# EUROPEAN PATENT SPECIFICATION

(11) **EP 3 818 067 B1**
(45) Date of publication and mention of the grant of the patent: **18.06.2025**
(21) Application number: 19830956.9
(22) Date of filing: 03.07.2019
(51) Int. Cl.: C07H 21/02, C12Q 1/68

(54) **SYSTEM, APPARATUS AND METHOD FOR COMPUTERIZED AUTOMATIC DIAGNOSIS**
SYSTEM, VORRICHTUNG UND VERFAHREN ZUR RECHNERGESTÜTZTEN AUTOMATISCHEN DIAGNOSE
SYSTÈME, APPAREIL ET PROCÉDÉ DE DIAGNOSTIC AUTOMATIQUE INFORMATISÉ

(30) Priority: 04.07.2018 WO PCT/IL2018/050726; 27.12.2018 WO PCT/IL2018/051400
(43) Date of publication of application: 12.05.2021
(73) Proprietor: Ador Diagnostics Ltd., Limassol, 3105 (CY)
(72) Inventor: SCHNELL, Amit, 3606811 Kiryat Tivon (IL); HODKO, Dalibor, San Diego, California 92064 (US); SWANSON, Paul D., Santee, California 92071 (US); HURGIN, Vladimir, 7085147 Gan Yavne (IL); SCHMITT, Mario, 66482 Zweibruecken (DE); WEBER, Lutz, 66981 Muenchweiler (DE); LIPSHITZ, Asaf, 7177108 Modiin (IL); ZAHAVI, Eran, 7630512 Rehovot (IL); NEUMEIER, Michel, 66399 Mandelbachtal (DE); ROZIN, Gideon, Kiryat Ono (IL); YAO, Zuxu, San Diego, California 92129 (US); HODKO, Nives, San Diego, California 92064 (US); SIRIS, Genrikh, 82024 Taufkirchen (DE); RZEHAK, Otto, 80798 Munich (DE); SECHSER, Benjamin, 82386 Oberhausen (DE); KAISER, Stefan, 80639 Munich (DE); DEUTER, Klaus, 85716 Unterschleissheim (DE); TADMOR, Ari, 4641213 Herzlia (IL)
(74) Representative: Evens, Paul Jonathan
(86) International application number: PCT/IL2019/050741
(87) International publication number: WO 2020/008463

(56) References cited:
- WO-A1-2018/065116
- WO-A1-2018/122852
- WO-A1-2018/122856
- WO-A2-2008/076395
- WO-A2-2011/071772
- GB-A- 2 531 615
- US-A1- 2010 062 454
- US-A1- 2013 331 298
- US-A1- 2018 099 276
- US-B1- 6 235 471
- US-B2- 11 609 472
- ALI MD MONSUR ET AL: "Enzymatic manipulations of DNA oligonucleotides on microgel: towards development of DNA-microgel bioassays", CHEMICAL COMMUNICATIONS, ROYAL SOCIETY OF CHEMISTRY, UK, no. 43, 21 November 2007 (2007-11-21), pages 4459 - 4461, XP009529190, ISSN: 1359-7345, [retrieved on 20070822], DOI: 10.1039/B709817K

## Description

### REFERENCE TO RELATED APPLICATIONS

Reference is hereby made to the following patent applications of applicant/assignee:
Israel Patent Application No. 249956, filed December 29, 2016 and entitled AN ELECTROPHERETIC CHIP FOR ELECTROPHORETIC APPLICATIONS;
Israel Patent Application No. 249957, filed December 29, 2016 and entitled AN ELECTROPHERETIC CHIP FOR ELECTROPHORETIC APPLICATIONS; and.
PCT Patent Application PCT/IL2017/051399, filed December 29, 2017 and entitled CARTRIDGE FOR USE IN IN-VITRO DIAGNOSTICS AND METHOD OF USE THEREOF.

The following patent applications, the priority from which is hereby claimed, are also related to the subject matter of the present application:
PCT Patent Application PCT/IL2018/050726, filed July 4, 2018 and entitled IMPROVED CARTRIDGE FOR USE IN IN-VITRO DIAGNOSTICS AND METHOD OF USE THEREOF; and
PCT Patent Application PCT/IL2018/051400, filed December 27, 2018 and entitled METHOD OF RAPIDLY DETECTING THE PRESENCE OF NUCLEIC ACID TARGET MOLECULES.

### FIELD OF THE INVENTION

The present invention relates generally to automated in-vitro diagnostic systems and methods generally, and more particularly to a computerized automated diagnostic system and a computerized automated diagnostic method.

### BACKGROUND OF THE INVENTION

Various types of automated in-vitro diagnostic systems and methods are known in the art.

US 2013/0331298 relates to an analyzer and disposable cartridge for molecular in vitro diagnostics, and discloses a computerized automated diagnostic system according to the pre-amble of appended independent claim 1.

WO 2011/071772 relates to assay cartridges and methods of using the same.

### SUMMARY OF THE INVENTION

In accordance with aspects of the present invention, there is provided a computerized automated diagnostic system as defined in appended independent claim 1, and a computerized automated diagnostic method as defined in appended independent claim 6. Embodiments of the present invention are defined in appended claims dependent on independent claim 1.

The present invention seeks to provide improved automated in-vitro diagnostic systems and methods.

There is thus provided in accordance with a preferred embodiment of the present invention a computerized automated diagnostic system including: a cartridge configured to contain reagents for carrying out a diagnostic biological process and to receive a biological sample to be tested using the reagents, said cartridge including a carbon array assembly mounted thereon; and an instrument operative to interact with the cartridge to carry out multiple steps in the diagnostic biological process, characterized in that:
the cartridge has multiple operative states including a non-functionalized state in which the cartridge does not include the reagents and does not include the biological sample, a partially functionalized state in which the cartridge does include the reagents and does include the biological sample and in which a carbon array assembly is mounted onto the cartridge and forms part of the cartridge and a fully functionalized state upon insertion of the cartridge, onto which the carbon array assembly is mounted, into clamped engagement with the instrument; and
the carbon array subassembly comprises: a double-sided adhesive layer formed with registration apertures and registration cut outs as well as liquid inlet apertures and liquid outlet apertures; and a cover assembly; with the carbon array subassembly also including a black background layer, a substrate layer formed over the black background layer and formed with registration apertures and registration cut outs as well as liquid inlet apertures and liquid outlet apertures, an array of carbon resistors formed onto the substrate layer, an electrode array formed over the array of carbon resistors onto the substrate layer and defining a plurality of electrode arrays, each of which includes a peripheral electrode and a counter electrode as well as two rows of working electrodes and a carbon array printed over the electrode array onto the substrate layer and defining a pair of carbon arrays, each of which includes a central carbon electrode as well as two rows of working carbon electrodes.

In accordance with a preferred arrangement of the present disclosure the cartridge includes reagents for carrying out amplification.

Preferably, the carbon array assembly includes a flow cell including multiple simultaneous detection regions. Additionally, the flow cell may include multiple simultaneous detection regions which are joined by a snake-shaped liquid pathway.

In accordance with a preferred arrangement of the present disclosure the cartridge includes a plurality of liquid flow pathways, at least one of which is configured such that passage therethrough by cells enhances lysis of the cells.

In accordance with a preferred arrangement of the present disclosure the cartridge includes a pump which is mechanically driven by the instrument for generating gas pressure for driving liquids through passageways formed in the cartridge.

Preferably, the cartridge includes multiple frangible seals along fluid flow pathways, which frangible seals are broken simultaneously upon clamped engagement of the cartridge by the instrument.

In accordance with a preferred embodiment of the present invention the cartridge includes a Polymerase Chain Reaction (PCR) amplification assembly, the PCR amplification assembly including a plurality of aliquot chambers receiving purified sample nucleic acid material, a plurality of different dry reagent plugs, each in liquid communication with one of the plurality of aliquot chambers, a plurality of PCR chambers, each in liquid communication with one of the plurality of different dry reagent plugs and a plurality of gas springs, each in liquid communication with one of the plurality of PCR chambers, the plurality of PCR chambers being located adjacent an edge of the cartridge.

In accordance with a preferred embodiment of the present invention the cartridge also includes: a fluid sealing layer having first and second pairs of apertures providing fluid communication with an interior of the carbon array assembly; a main cartridge element, having a three-dimensionally patterned fluid conduit defining surface which cooperates with the sealing layer to define a multiplicity of fluid conduits, the main cartridge element having a fluid conduit defining and liquid enclosure engagement surface opposite to the three-dimensionally patterned fluid conduit defining surface; and a plurality of elastomeric sealing layers which are sealingly joined to the fluid conduit defining and liquid enclosure engagement surface of the main cartridge element and define therewith a plurality of valves, the main cartridge element also including a plurality of through holes which cooperate with the sealing layer and the plurality of elastomeric sealing layers to define a plurality of frangible seals being normally closed and being simultaneously opened when the cartridge is in clamped engagement with the instrument. Additionally, the sealing layer may be formed with apertures for precise positioning of the cartridge in the instrument.

Preferably, the cartridge also includes a plurality of enclosure defining elements, which are sealingly joined to the fluid conduit defining and liquid enclosure engagement surface and define therewith a plurality of liquid enclosures.

In accordance with a preferred arrangement of the present disclosure the cartridge also includes a mechanically actuable gas pump mounted onto the main cartridge element.

Preferably, the plurality of liquid enclosures includes a sample receiving enclosure. Additionally, the plurality of liquid enclosures include a lysis bead containing chamber, a Protinease K containing chamber, a lysis mixing chamber, a dilution chamber and a Raffinose washing liquid containing chamber.

Preferably, the plurality of liquid enclosures include a plurality of wash buffer containing chambers containing different wash buffers, an elution buffer containing chamber, a mixing chamber, an amplicon dilution buffer dilution chamber and a sensor wash containing chamber. Additionally or alternatively, the plurality of liquid enclosures include a plurality of discriminator buffer containing chambers, a reporter buffer containing chamber and a waste container.

In accordance with a preferred arrangement of the present disclosure during initial functionalization of the cartridge, solid reagent plugs are loaded into the cartridge at reagent plug sockets defined therein.

In accordance with a preferred arrangement of the present disclosure the valves are defined by pairs of through holes formed in the main cartridge element cooperating with the sealing layer and the plurality of elastomeric sealing layers. Additionally, the valves are normally open and are closed when a respective one of the plurality of elastomeric sealing layers is pressed against the through holes.

Preferably, the cartridge includes a plurality of discriminator reservoirs and a reporter reservoir, each of which communicates via respective through holes in the main cartridge element with respective fill and venting ports.

In accordance with a preferred arrangement of the present disclosure the cartridge includes a plurality of optical liquid detection chambers.

In accordance with a preferred arrangement of the present disclosure the cartridge includes a Polymerase Chain Reaction (PCR) array including a plurality of eluent aliquot chambers and a plurality of reagent plug hydration chambers, which communicate with respective dry reagent plugs, which are located in respective reagent plug sockets. Additionally, the PCR array also includes a plurality of channels which communicate with the aliquot chambers, a plurality of PCR chambers, which communicate with respective ones of the hydration chambers and a plurality of gas springs, which communicate with respective ones of the PCR chambers.

Additionally or alternatively, the carbon array subassembly also includes a resistance heating layer.

Preferably, the carbon array subassembly also includes a dielectric layer, which is formed with a pair of elongate apertures which overlie and communicate with the carbon electrodes. Additionally, the dielectric layer defines a plurality of apertures, each of which overlies a working carbon electrode, which in turn overlies a working electrode.

Preferably, each of the plurality of apertures contains a droplet of a hydrophilic polymer used for binding.

In accordance with a preferred arrangement of the present disclosure the cover assembly includes a double-sided adhesive layer having formed therein two parallel snake-shaped cut outs which define liquid flow paths communicating with the working carbon electrodes of the carbon array subassembly, which in turn overlie the working electrodes of the carbon array subassembly and a transparent cover layer overlying the double-sided adhesive layer and sealing the snake-shaped cut outs.

In accordance with a preferred embodiment of the present invention the cartridge includes a room-temperature shelf-storable electrophoretic array including a multiplicity of immobilized, mutually spaced and mutually electrically separated microgel deposits, each of the multiplicity of immobilized, mutually spaced and mutually electrically separated microgel deposits containing materials suitable for performing rolling circle amplification and binding of at least one of the multiplicity of pre-selected nucleic acid target molecules, each of the microgel deposits containing at least the following elements pre-anchored therein: an RCA probe specific to of at least one of the multiplicity of pre-selected nucleic acid target molecules and at least one primer including at least one forward primer and at least one reverse primer.

In accordance with a preferred arrangement of the present disclosure the microgel deposits are dehydrated and are rehydratable when exposed to a solution containing at least one nucleic acid target molecule.

Preferably, the RCA probe is pre-hybridized to the at least one primer.

In accordance with a preferred arrangement of the present disclosure each of the microgel deposits when hydrated has a generally hemispherical shaped configuration. Additionally or alternatively, the multiplicity of immobilized, mutually spaced and mutually electrically separated microgel deposits define a corresponding multiplicity of immobilized, mutually spaced and mutually electrically separated microgel regions and the electrophoretic array is employed in carrying out a method including introducing the solution to each of the multiplicity of immobilized, mutually spaced and mutually electrically separated microgel regions, performing rolling circle amplification at least generally simultaneously at each of the multiplicity of immobilized, mutually spaced and mutually electrically separated microgel regions, while applying electric fields thereto during various stages of the rolling circle amplification and detecting the presence of at least one of the multiplicity of pre-selected nucleic acid target molecules at at least one corresponding one of the immobilized, mutually spaced and mutually electrically separated microgel regions, the detecting occurring within a short time period of the introducing, the short time period being less than 30 minutes.

Preferably, the applying electric fields thereto occurs during at least two different stages in the rolling circle amplification. Additionally or alternatively, the electric fields are at least generally the same at each of the immobilized, mutually spaced and mutually electrically separated microgel regions.

Preferably, the detecting occurs within a time duration of less than 20 minutes. More preferably, the detecting occurs within a time duration of less than 15 minutes.

In accordance with a preferred arrangement of the present disclosure the applying electric fields during the rolling circle amplification includes at least one of the following: applying an electric field to the immobilized, mutually spaced and mutually electrically separated microgel regions for driving nucleic acid target molecules in the solution to the microgel deposits, applying an electric field to the immobilized, mutually spaced and mutually electrically separated microgel regions for driving nucleic acid target molecule-RCA probe hybridization products in the solution to the microgel deposits, applying an electric field to the immobilized, mutually spaced and mutually electrically separated microgel regions for recapturing RCA amplicons that drift away from the microgel deposits, applying an electric field to the immobilized, mutually spaced and mutually electrically separated microgel regions for driving RCA probes into the microgel deposits for hybridization with at least one of capture probes and primers already bound to the microgel deposits, applying an electric field to the immobilized, mutually spaced and mutually electrically separated microgel regions for removing undesired molecules from the microgel regions, applying an electric field to the immobilized, mutually spaced and mutually electrically separated microgel regions for stretching RCA amplicons that are bound to the microgel deposits, applying an electric field to the immobilized, mutually spaced and mutually electrically separated microgel regions for compressing RCA amplicons that are bound to the microgel deposits, applying an electric field to the immobilized, mutually spaced and mutually electrically separated microgel regions for stirring RCA reagents in the vicinity of RCA amplicons that are bound to the microgel deposits, applying an electric field to the immobilized, mutually spaced and mutually electrically separated microgel regions for enhancing the speed of enzyme activity in RCA and applying electric field of sequentially reversing polarity to the immobilized, mutually spaced and mutually electrically separated microgel regions for enhancing stringency of binding of RCA amplicons to the microgel deposits.

### BRIEF DESCRIPTION OF THE DRAWINGS

The present invention will be understood and appreciated from the following detailed description, taken in conjunction with the drawings, in which:
Fig. 1 is a simplified pictorial illustration of an automated in-vitro diagnostic system constructed and operative in accordance with a preferred embodiment of the present invention;
Figs. 2A/1 and 2A/2 are simplified respective pictorial top-facing assembled view and pictorial bottom-facing assembled view illustrations of a non-functionalized cartridge forming part of the automated in-vitro diagnostic system of Fig. 1;
Figs. 2B and 2C are simplified respective pictorial top-facing exploded view and pictorial bottom-facing exploded view illustrations of the non-functionalized cartridge of Figs. 2A/1 and 2A/2;

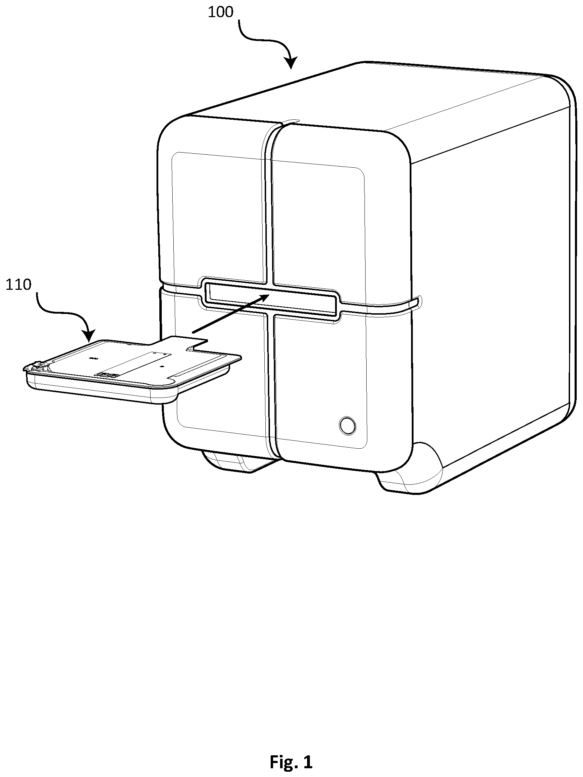

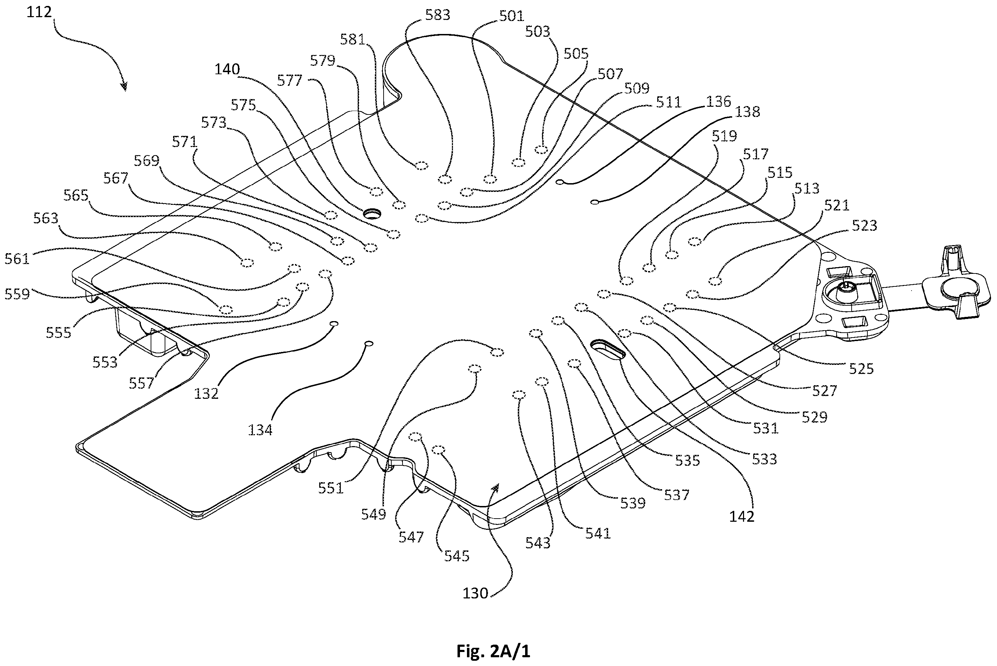

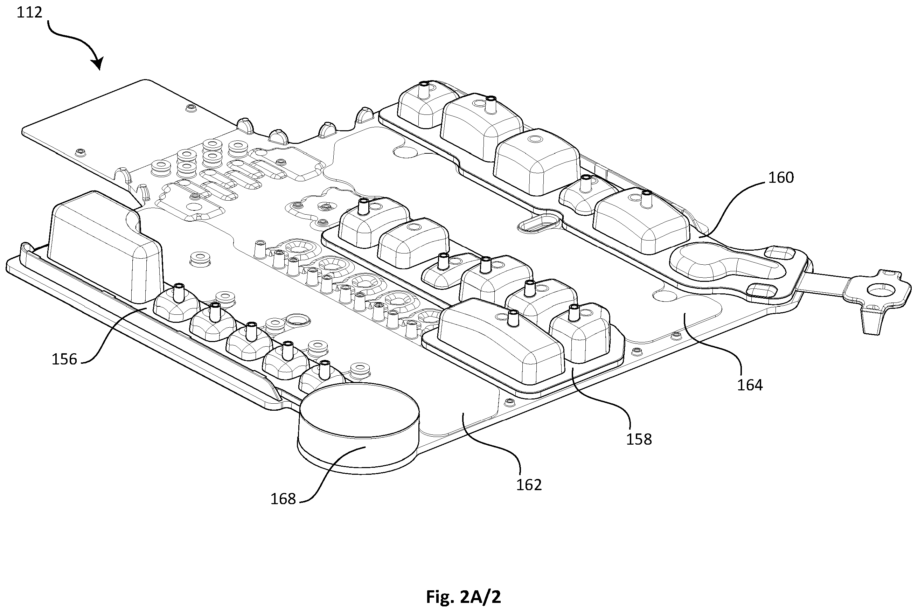

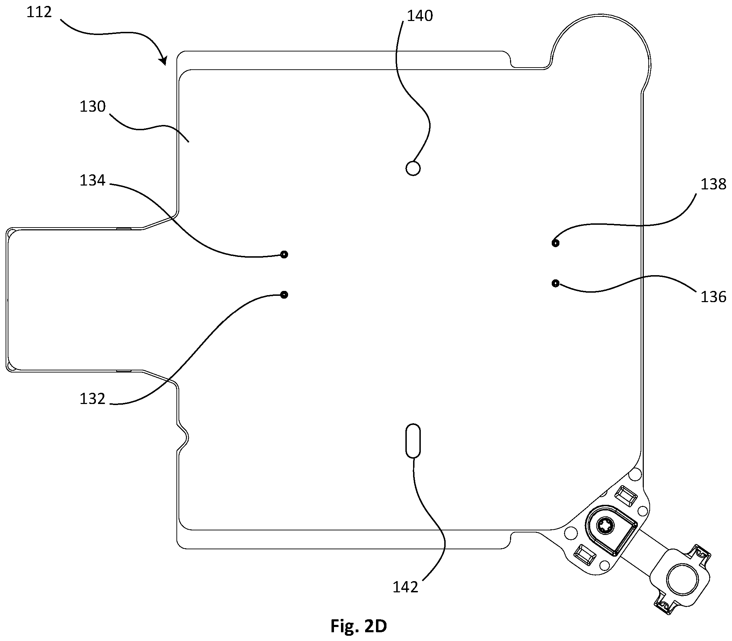

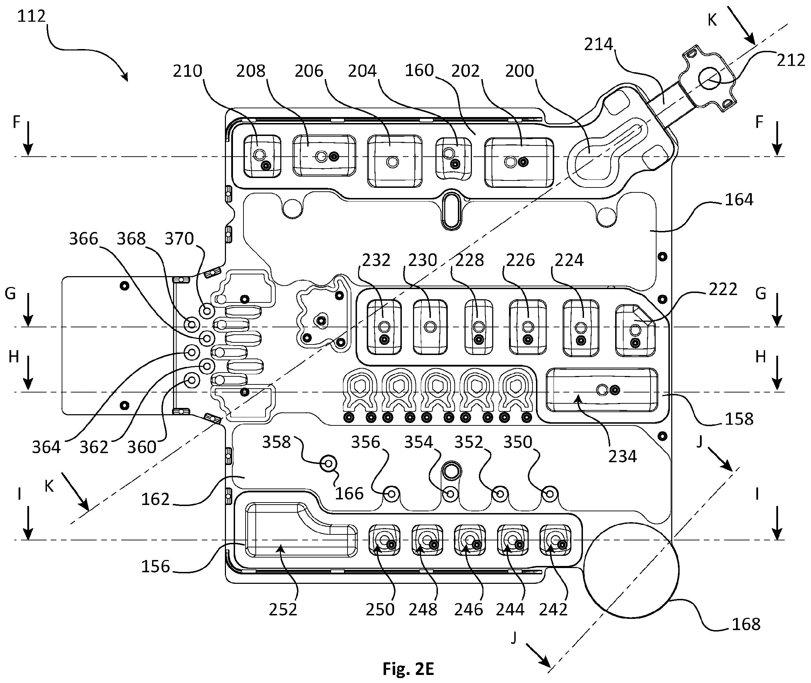

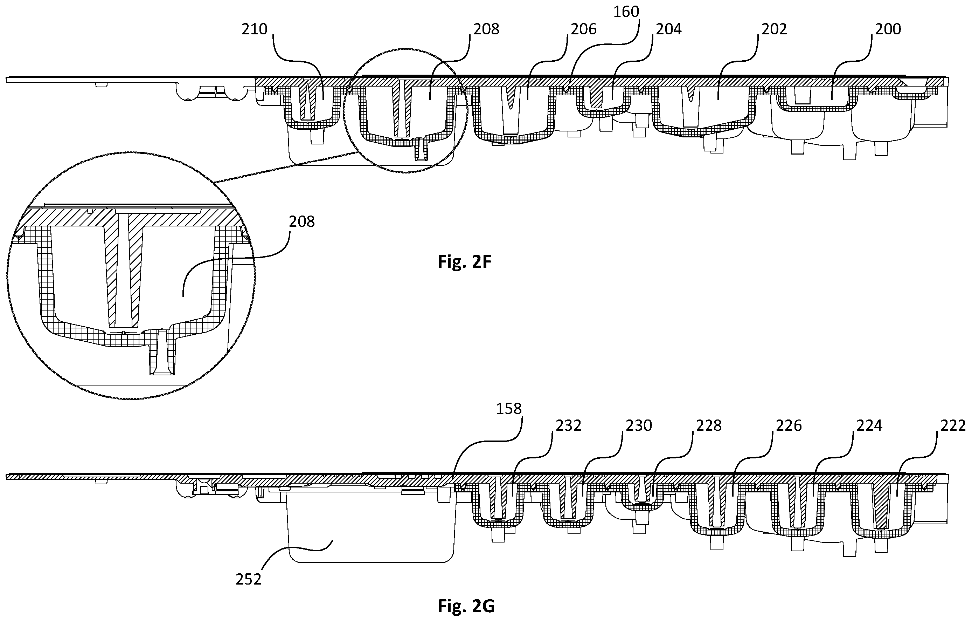

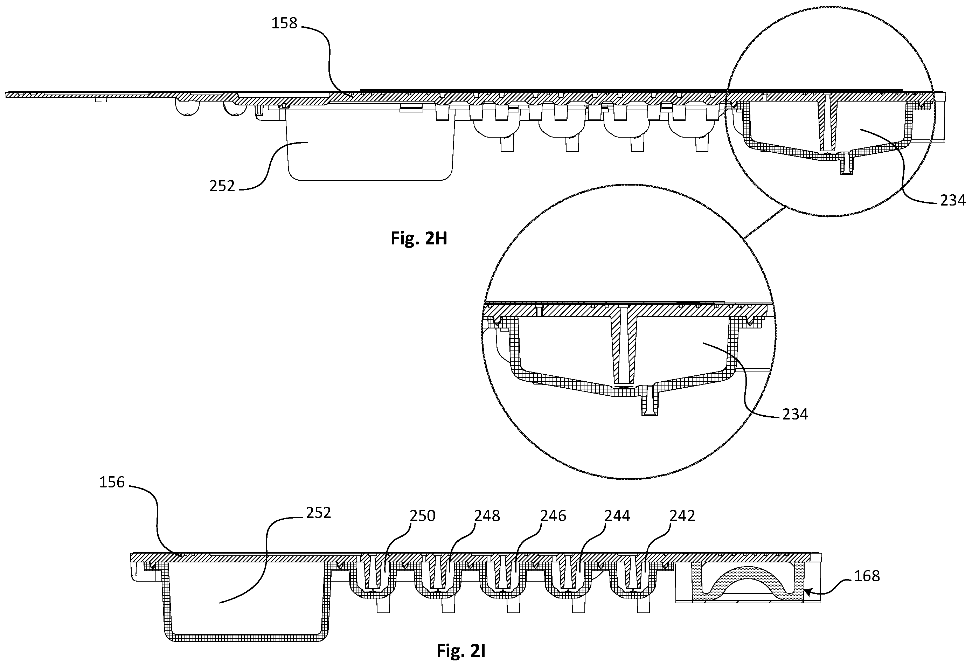

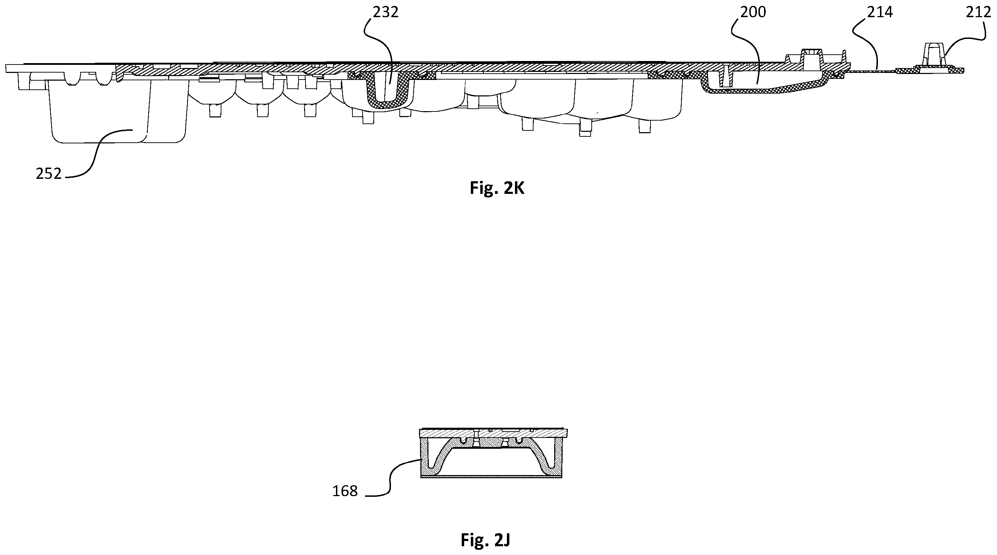

Figs. 2D, 2E, 2F, 2G, 2H, 2I, 2J and 2K are simplified respective planar top view, planar bottom view, first sectional view, second sectional view, third sectional view, fourth sectional view, fifth sectional view and sixth sectional view illustrations of the non-functionalized cartridge of Figs. 2A/1 - 2C, Fig. 2F being taken along lines F - F in Fig. 2E, Fig. 2G being taken along lines G - G in Fig. 2E, Fig. 2H being taken along lines H - H in Fig. 2E, Fig. 2I being taken along lines I - I in Fig. 2E, Fig. 2J being taken along lines J - J in Fig. 2E and Fig. 2K being taken along lines K - K in Fig. 2E, Fig. 2E being a bottom view of the non-functionalized cartridge and Figs. 2F - 2J being top views of the non-functionalized cartridge;
Figs. 3A and 3B are simplified respective pictorial top view and pictorial bottom view illustrations of a main element forming part of the non-functionalized cartridge of Figs. 2A/1 - 2K;

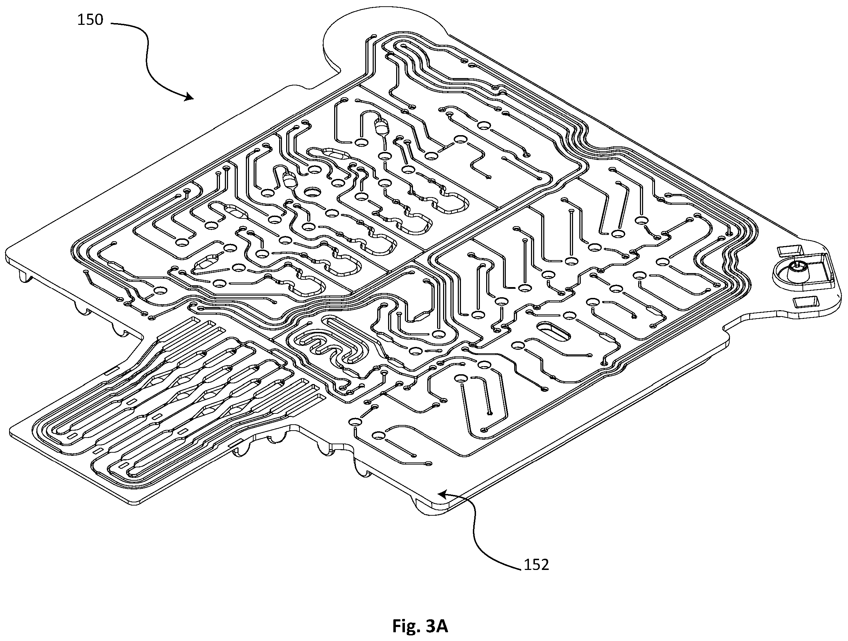

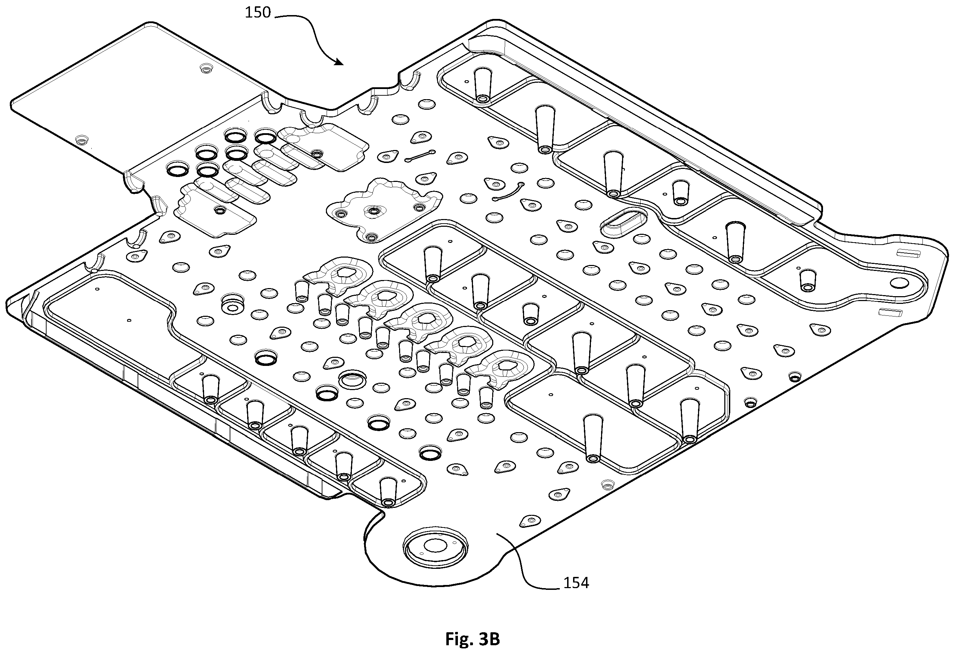

Figs. 3C/1, 3C/2, 3C/3, 3C/4, 3C/5, 3C/6, 3C/7, 3C/8, 3C/9 and 3C/10 are together a simplified composite planar top view illustration of the main element of Figs. 3A & 3B;
Figs. 3D/1, 3D/2, 3D/3 and 3D/4 are together a simplified composite planar bottom view illustration of the main element of Figs. 3A & 3B;

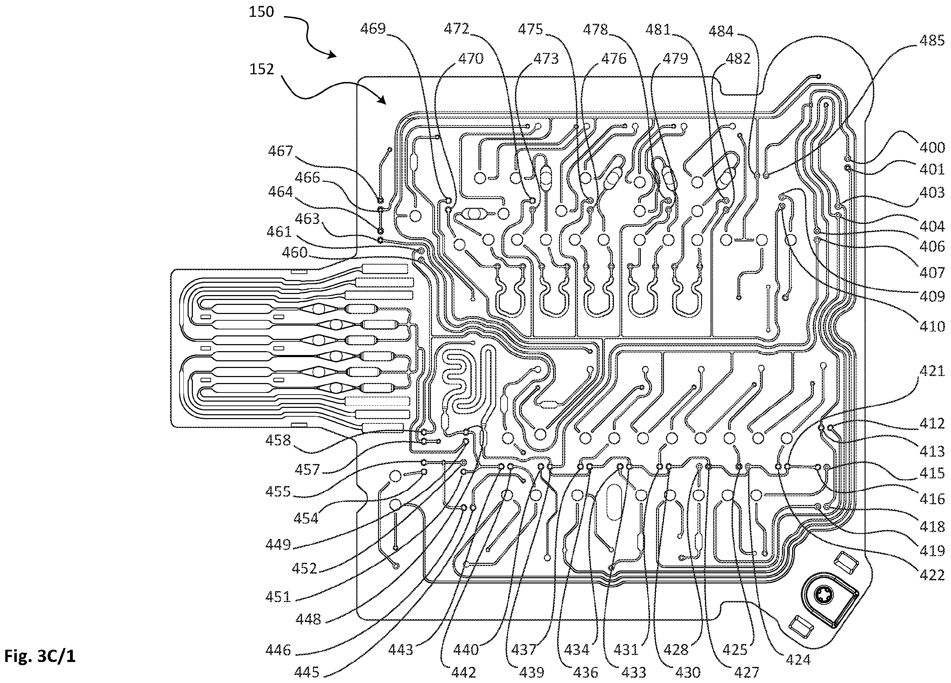

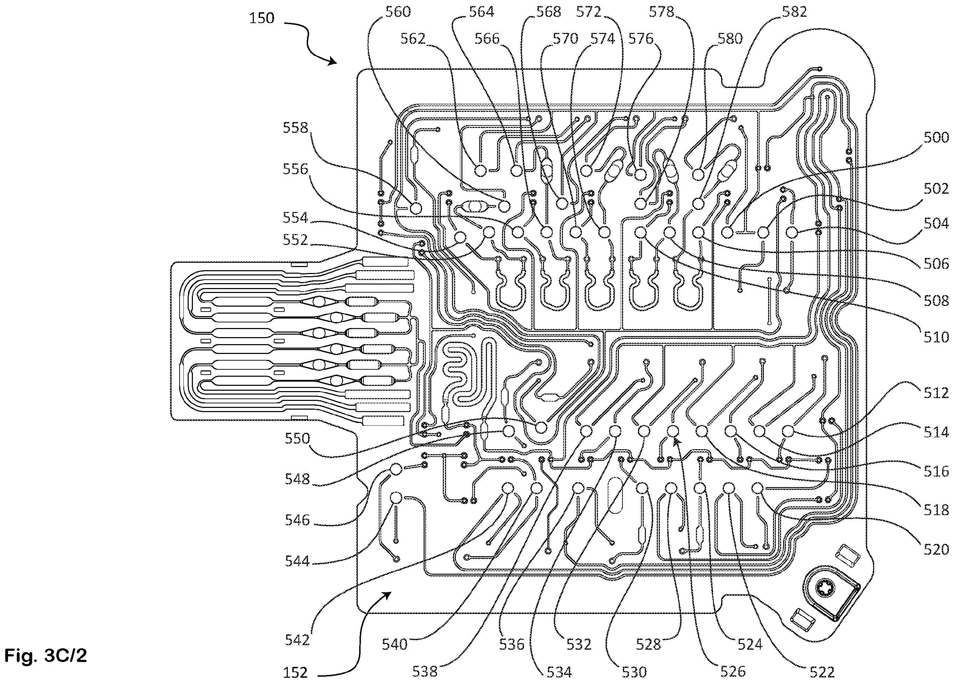

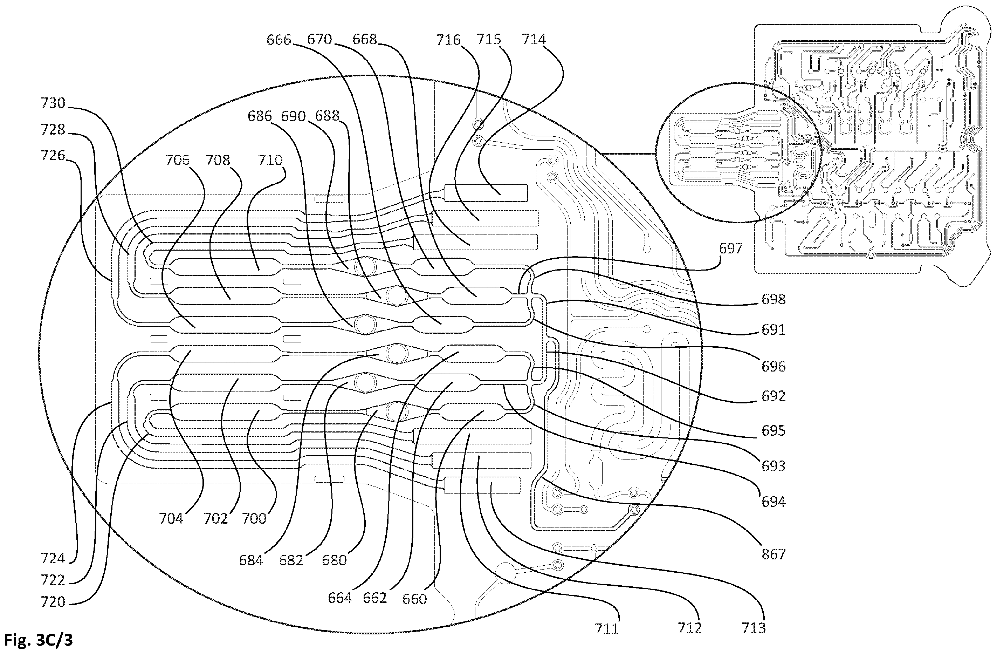

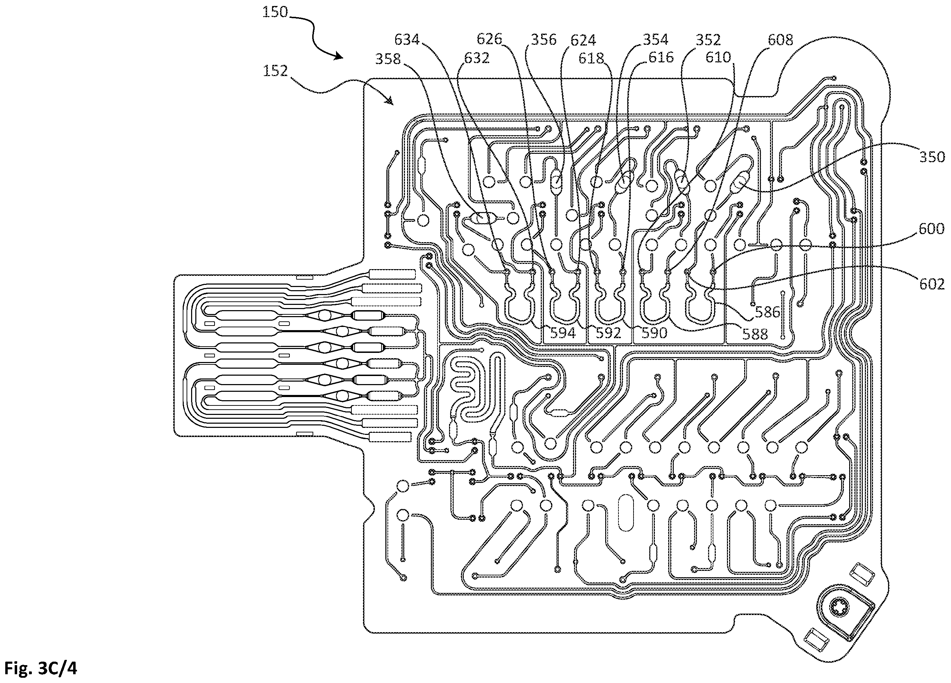

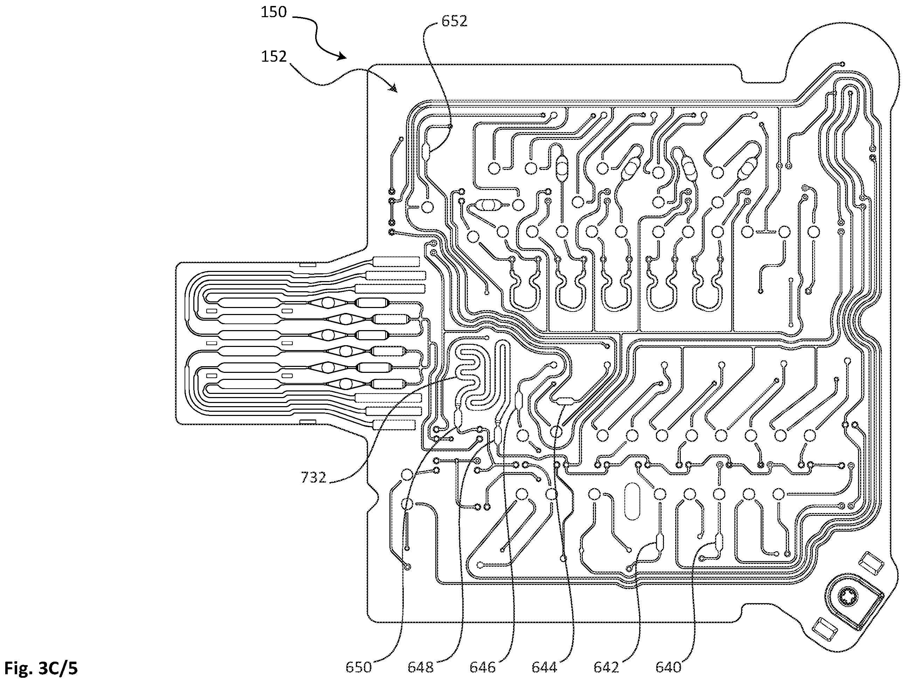

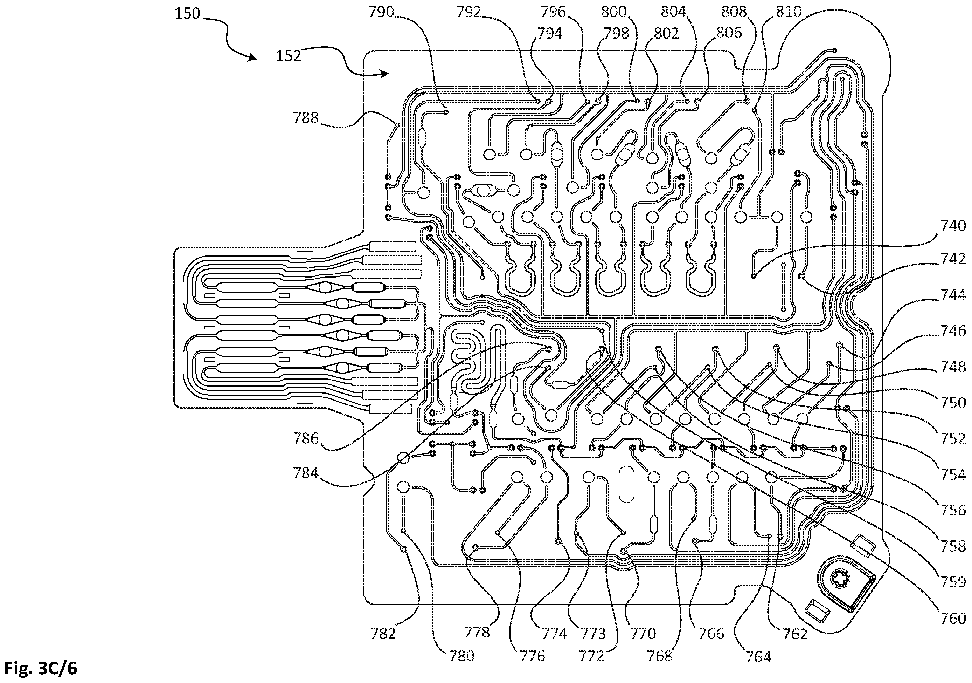

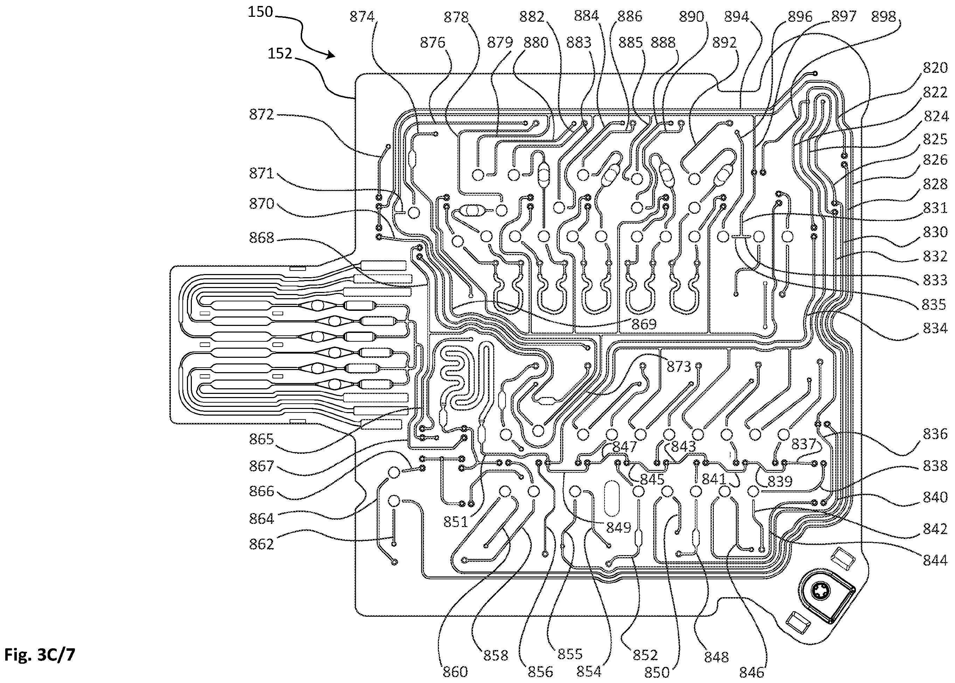

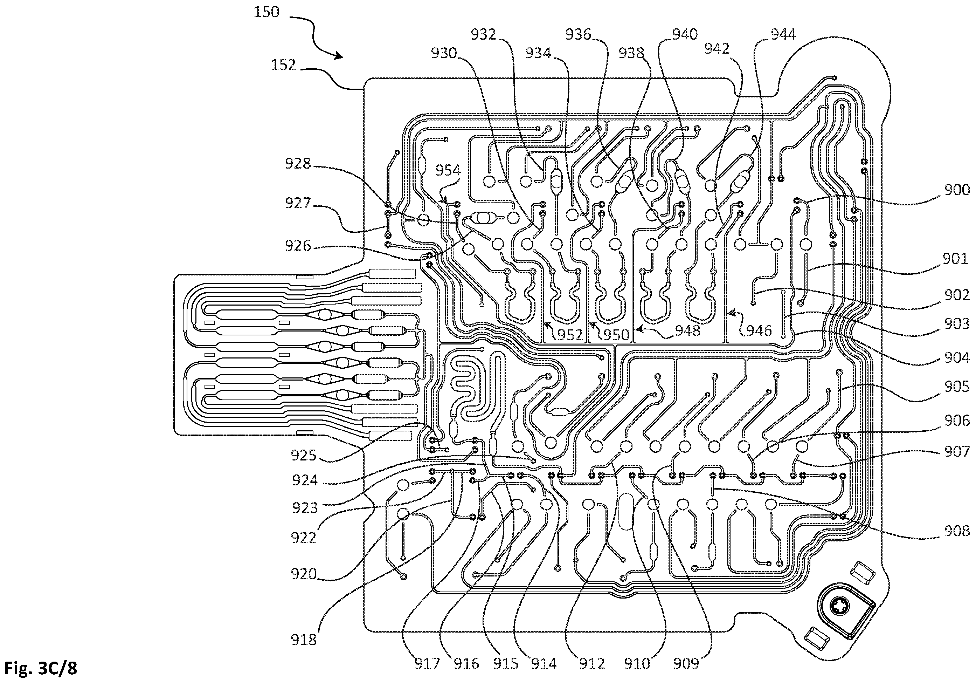

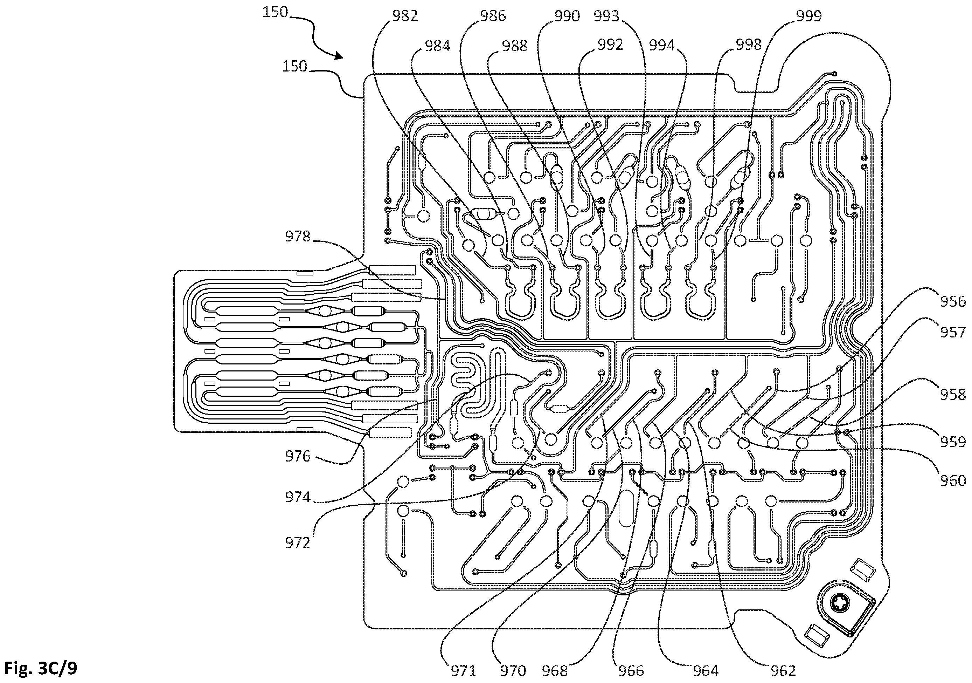

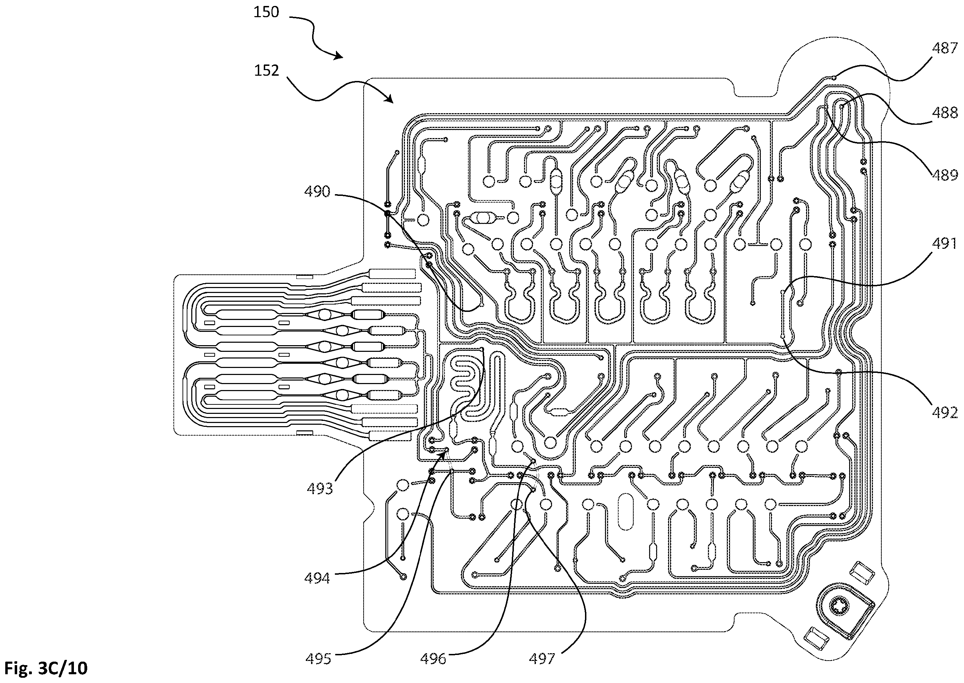

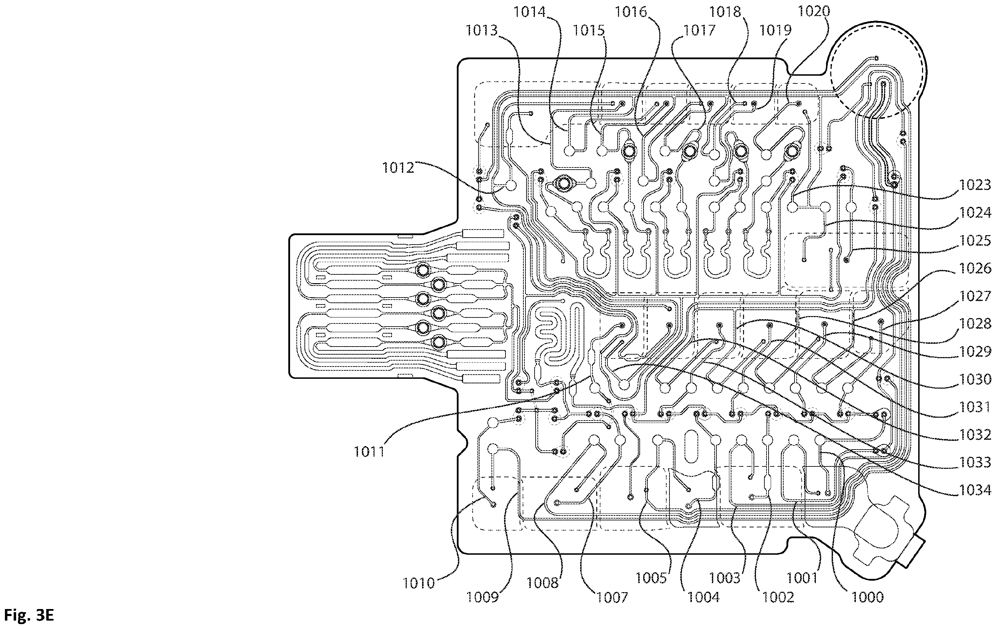

Fig. 3E is a simplified composite planar view illustration of the main element of Figs. 3A & 3B showing pathways defined by and between portions of the main element illustrated in Figs. 3C/1 - 3D3;

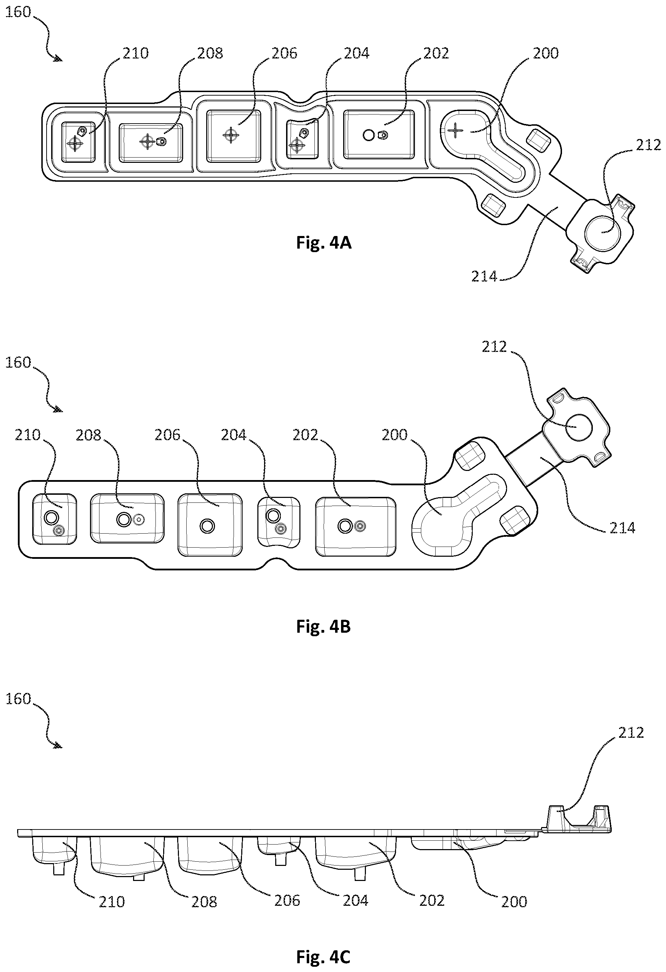

Figs. 4A, 4B and 4C are simplified respective planar top view, planar bottom view and side view illustrations of a first group of reagent containers forming part of the non-functionalized cartridge of Figs. 2A/1 - 2K;

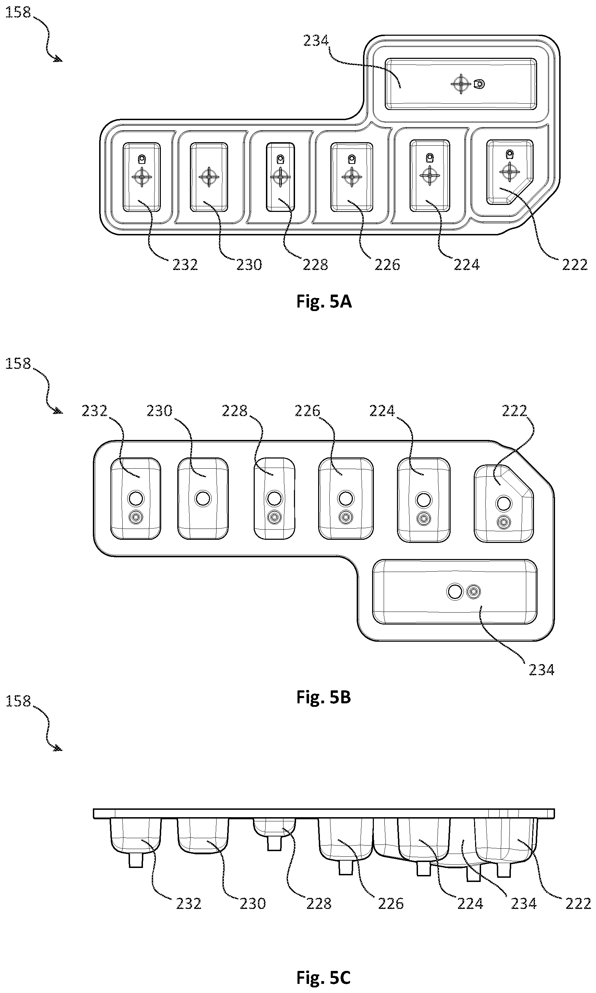

Figs. 5A, 5B and 5C are simplified respective planar top view, planar bottom view and side view illustrations of a second group of reagent containers forming part of the non-functionalized cartridge of Figs. 2A/1 - 2K;

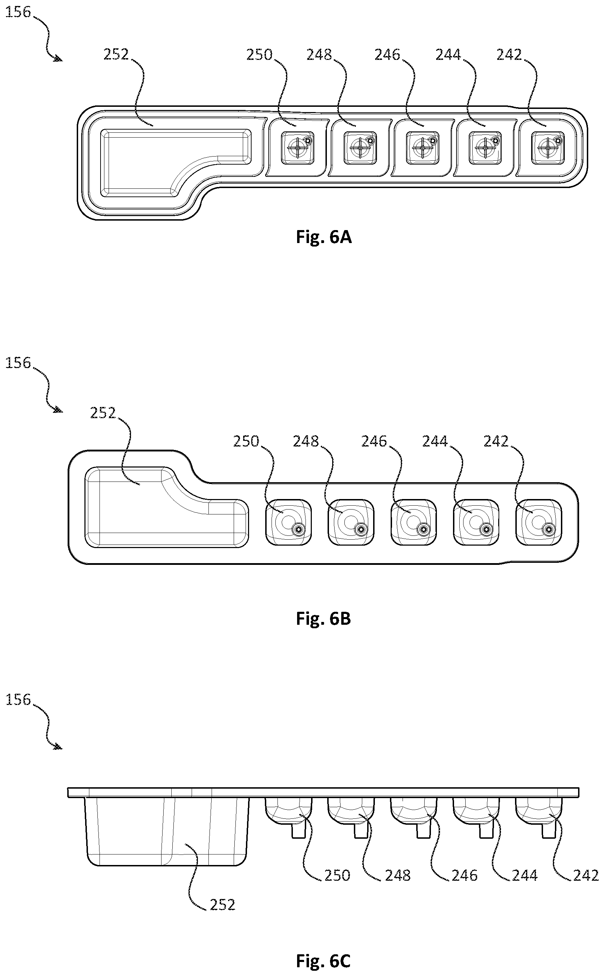

Figs. 6A, 6B and 6C are simplified respective planar top view, planar bottom view and side view illustrations of a third group of reagent containers forming part of the non-functionalized cartridge of Figs. 2A/1 - 2K;

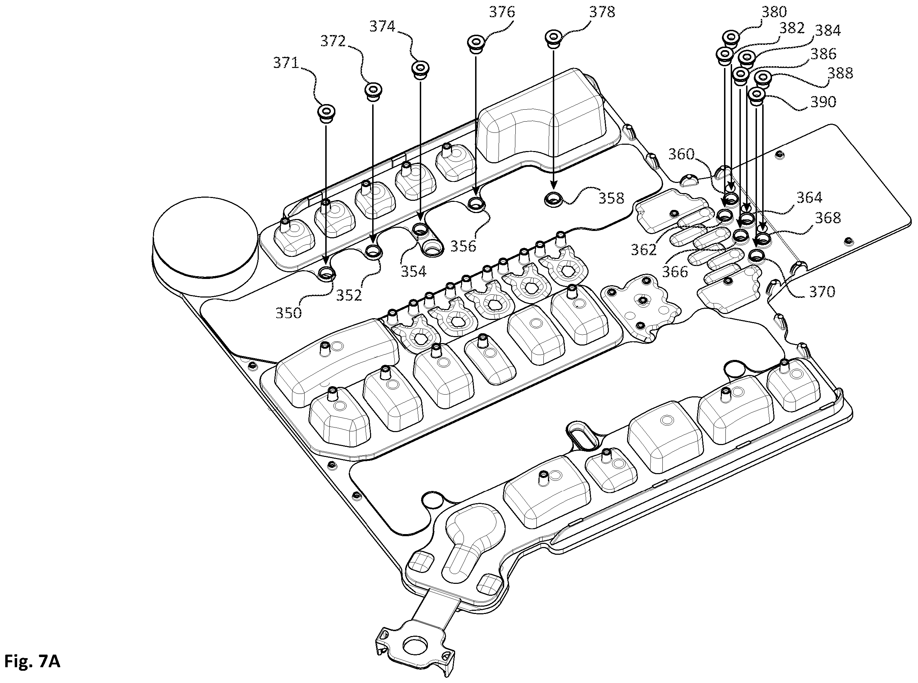

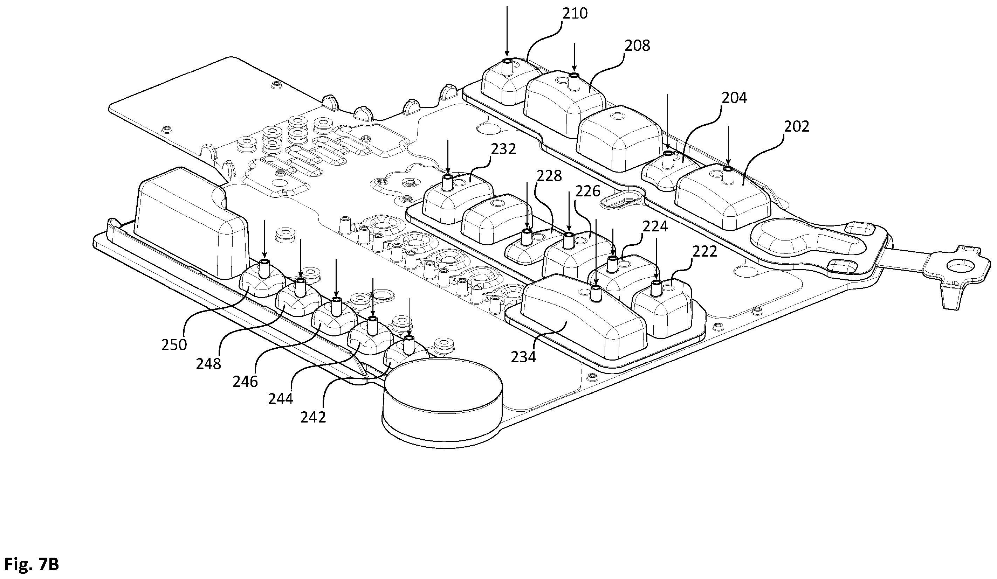

Figs. 7A and 7B are simplified illustrations of stages in the initial functionalization of the non-functionalized cartridge of Figs. 2A/1 to Fig. 6C;

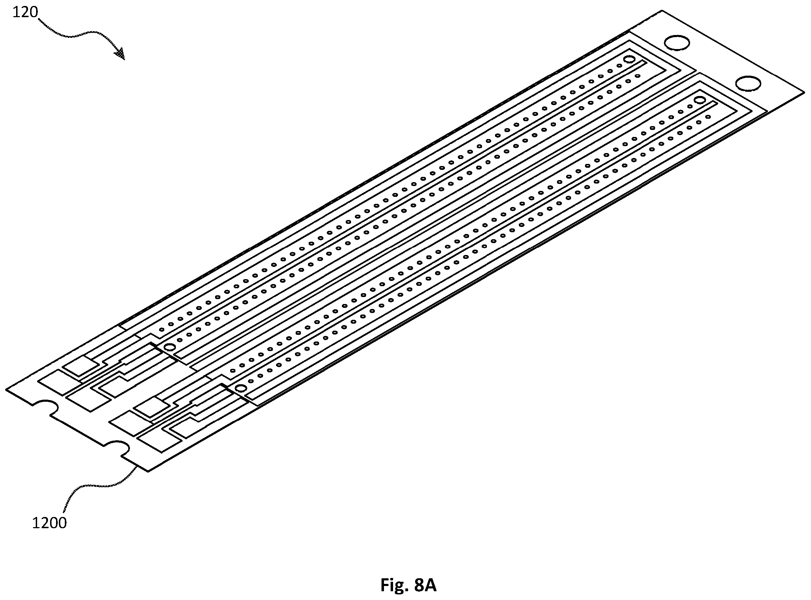

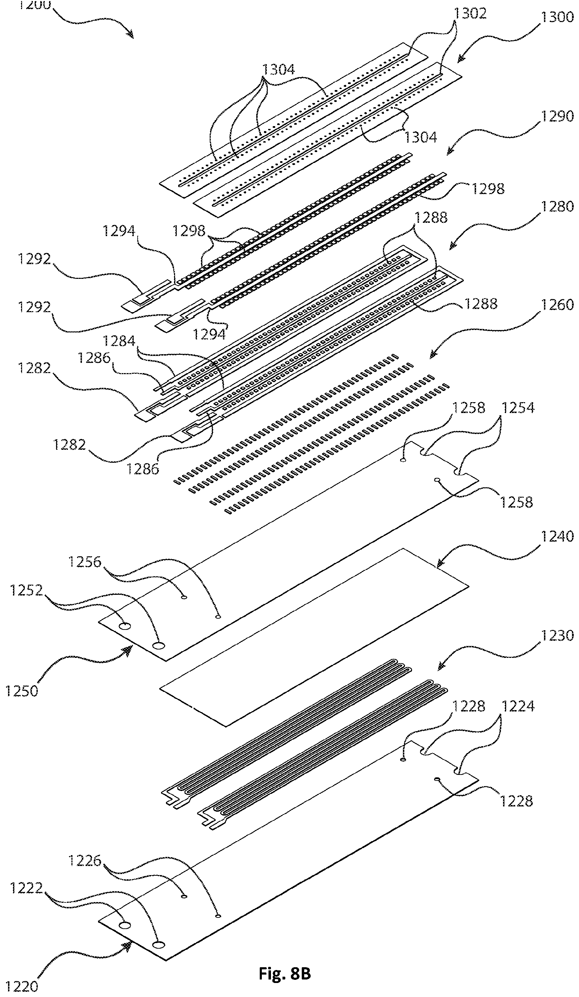

Figs. 8A and 8B are simplified respective pictorial assembled view and exploded view illustrations of a carbon array subassembly forming part of the carbon array assembly employed in the automated in-vitro diagnostic system of Fig. 1;

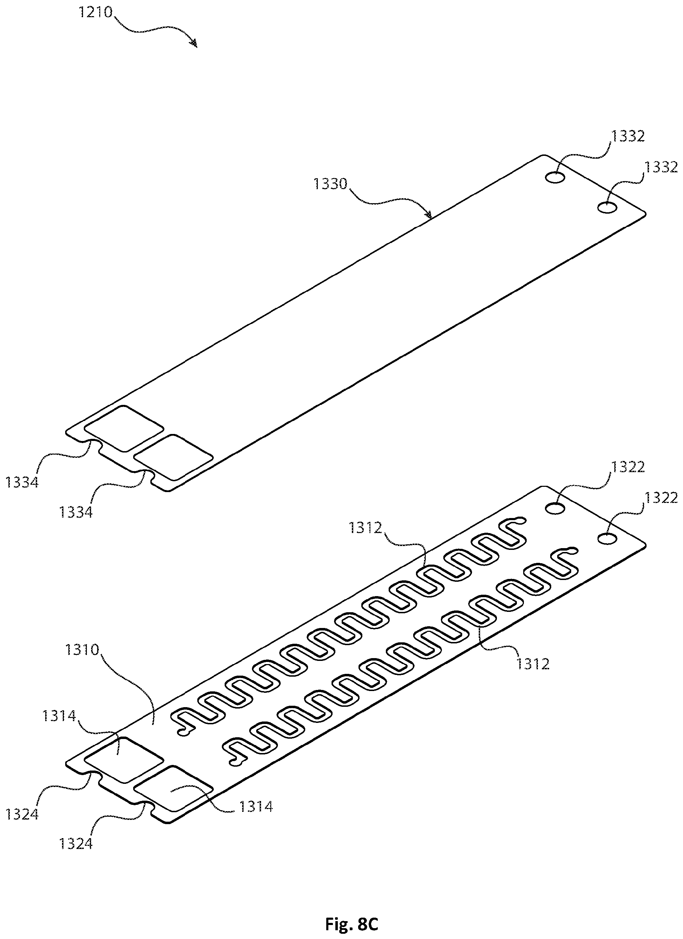

Fig. 8C is a simplified exploded view illustration of a cover assembly forming part of the carbon array assembly employed in the automated in-vitro diagnostic system of Fig. 1;

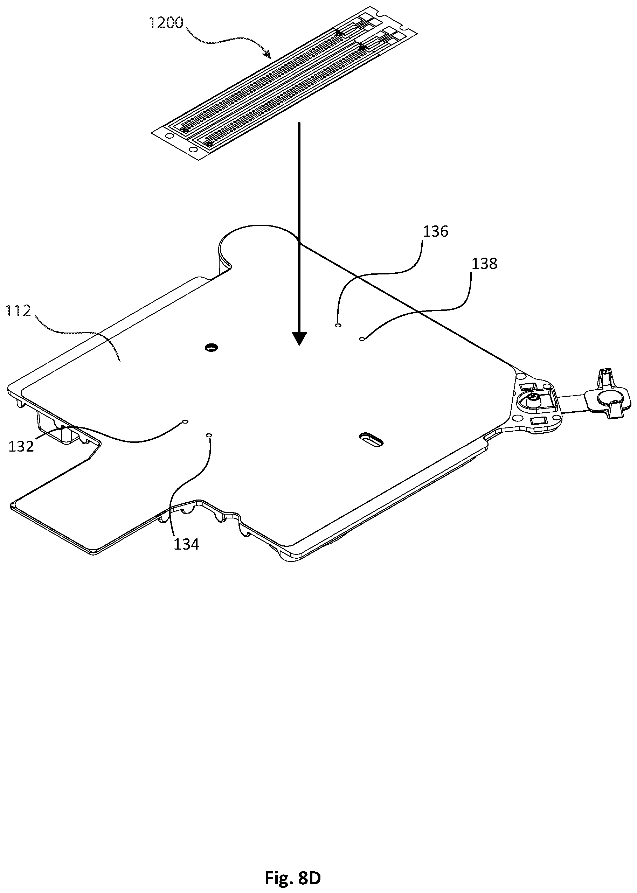

Fig. 8D is a simplified illustration of mounting of the carbon array subassembly of Figs. 8A - 8B onto the initially functionalized cartridge of Figs. 7A & 7B;

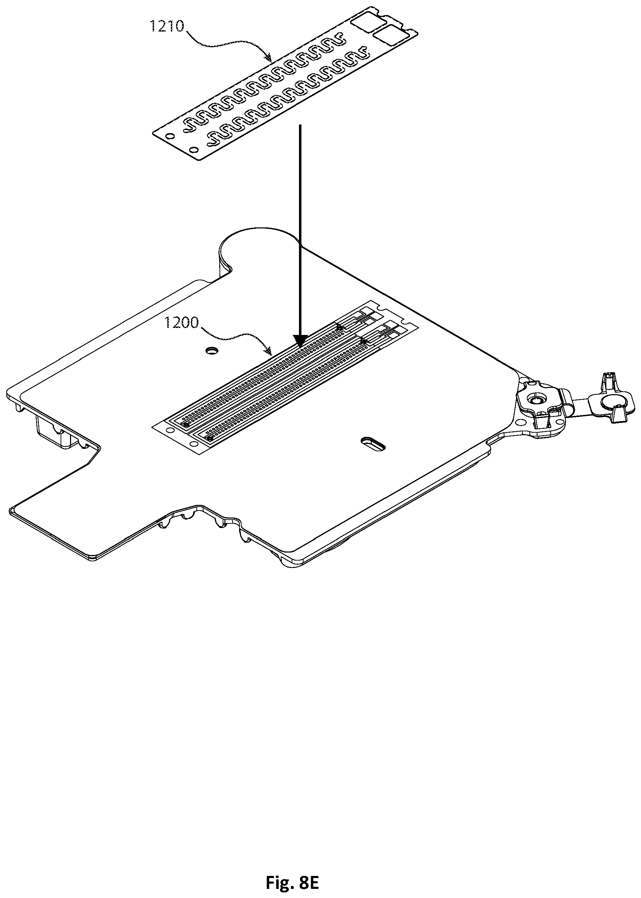

Fig. 8E is a simplified illustration of mounting of the cover assembly of Fig. 8C over the carbon array subassembly of Figs. 8A - 8B onto the initially functionalized cartridge of Figs. 7A & 7B;

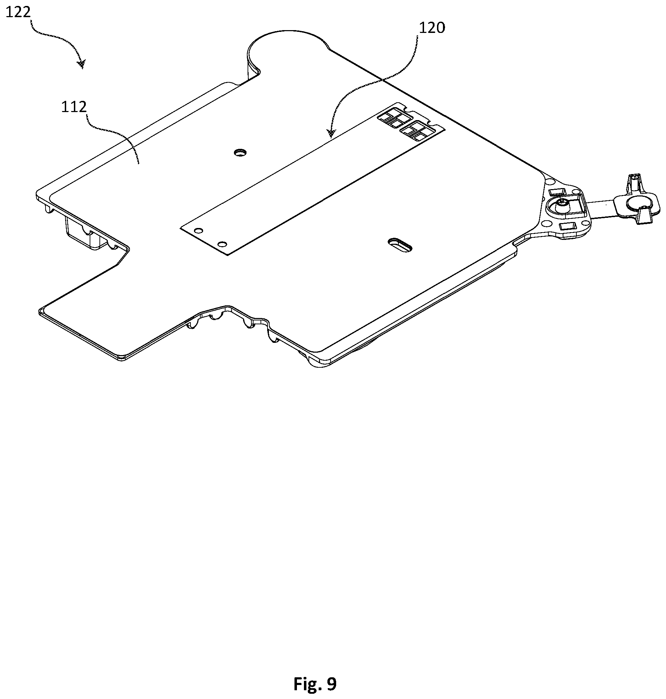

Fig. 9 is a simplified illustration of the fully assembled initially functionalized cartridge of Figs. 7A & 7B, including the carbon array subassembly of Figs. 8A - 8B and the cover assembly of Fig. 8C;

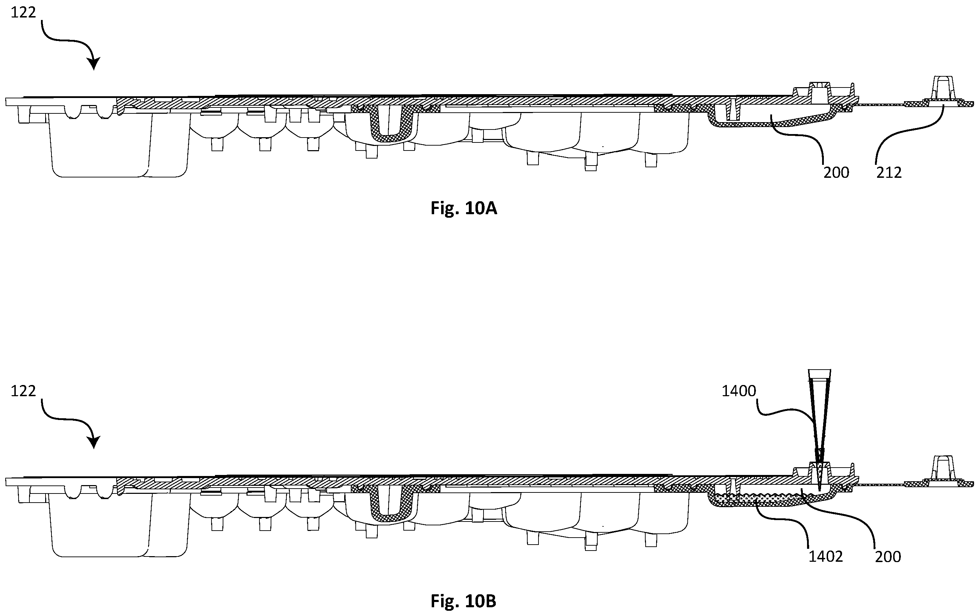

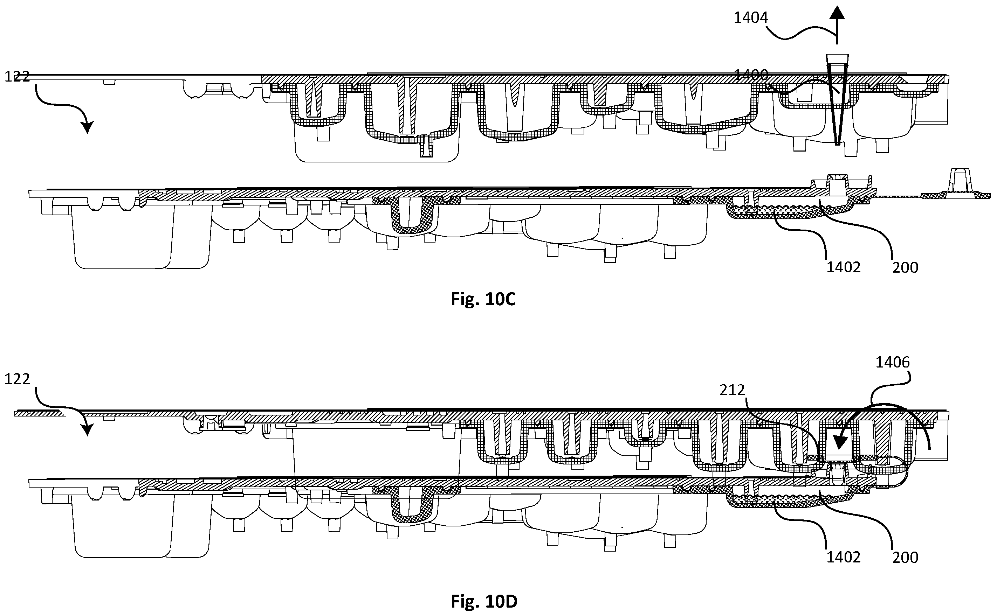

Figs. 10A, 10B, 10C and 10D are respective side view illustrations showing steps in the insertion of a sample into the cartridge of Figs. 2A/1 - 9;

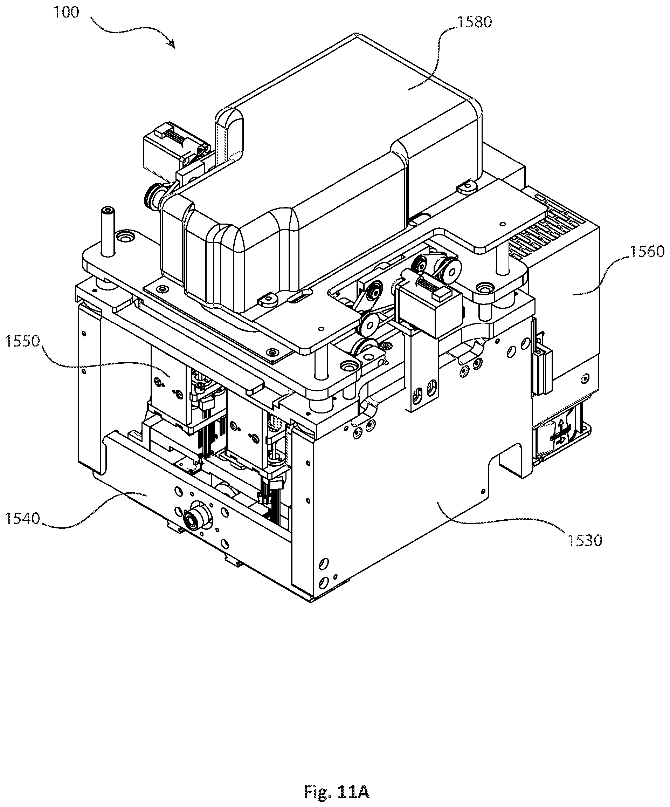

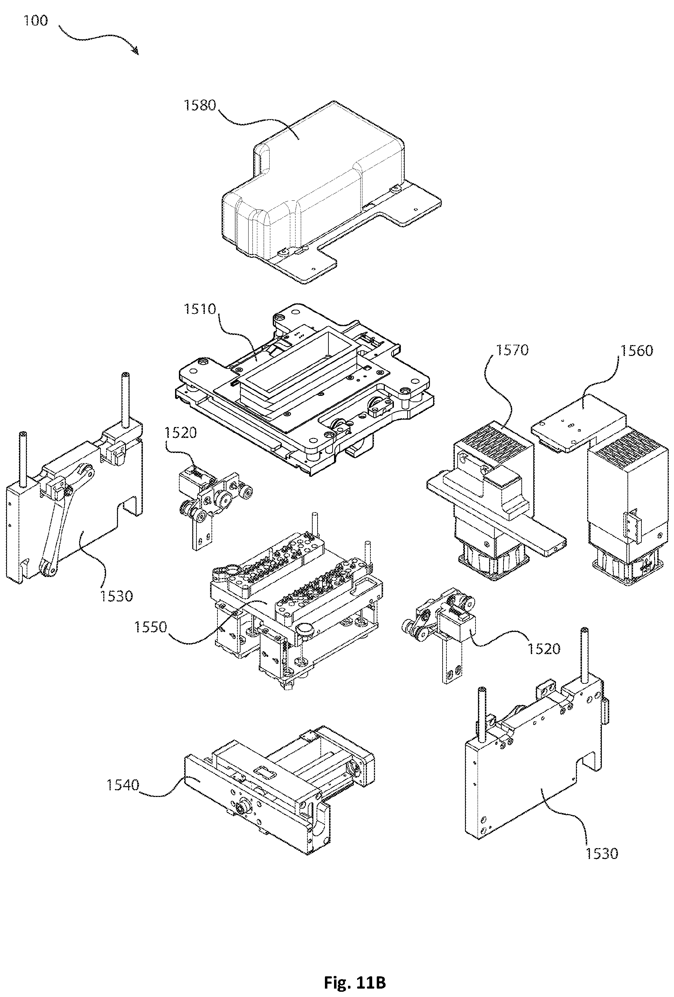

Figs. 11A and 11B are simplified respective pictorial assembled view and pictorial exploded view illustrations of an automated diagnostic instrument, which together with the cartridge of Figs. 2A/1 - 10D forms the automated in-vitro diagnostic system of Fig. 1;

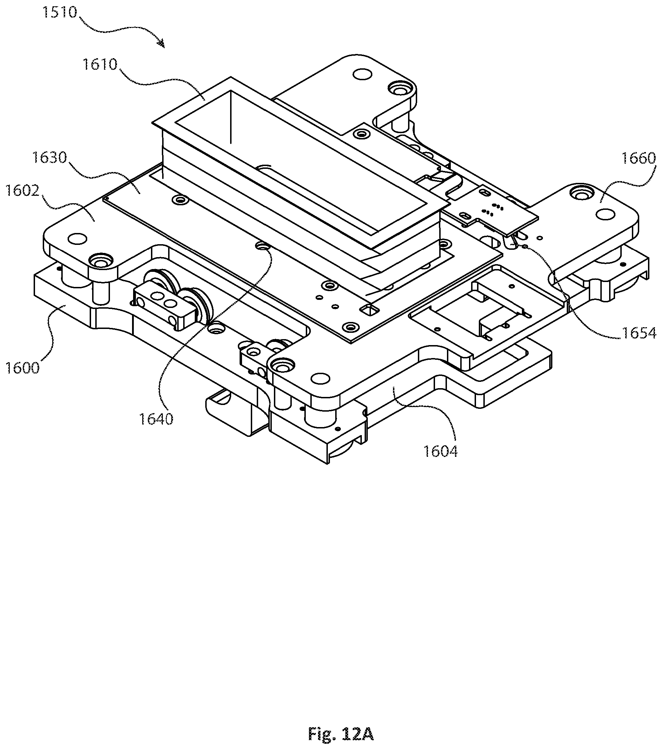

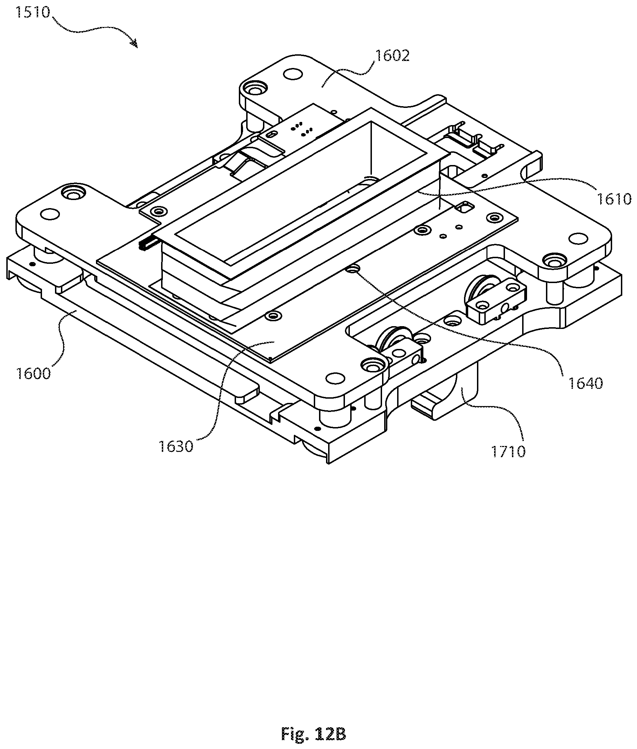

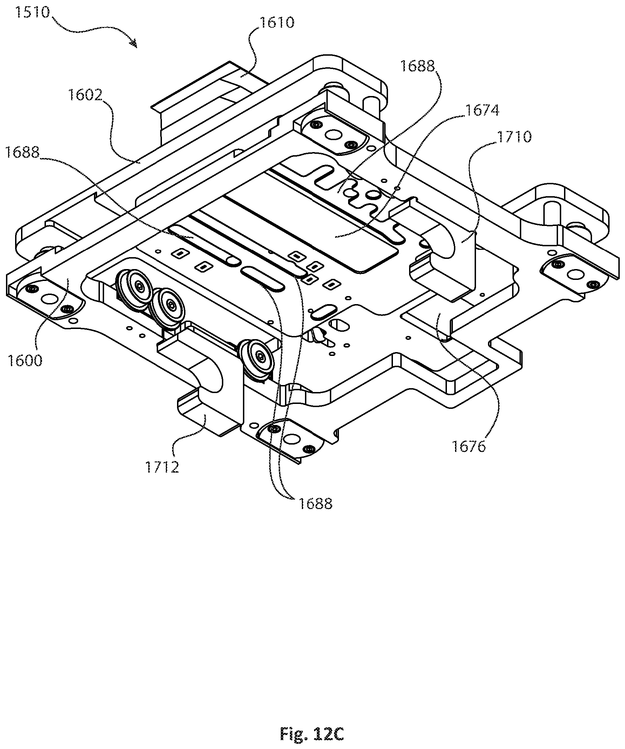

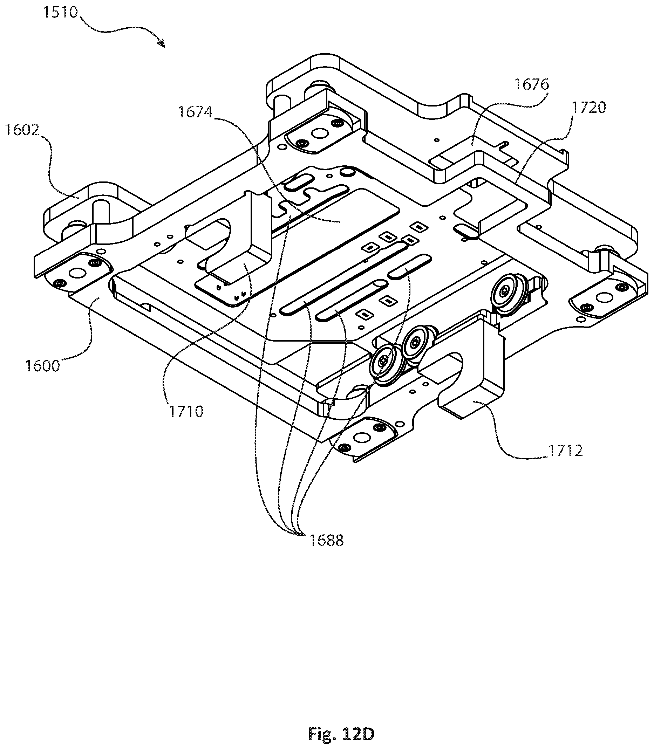

Figs. 12A, 12B, 12C and 12D are simplified assembled view illustrations, taken from various different directions, of a cartridge holder assembly forming part of the instrument of Figs. 11A and 11B;

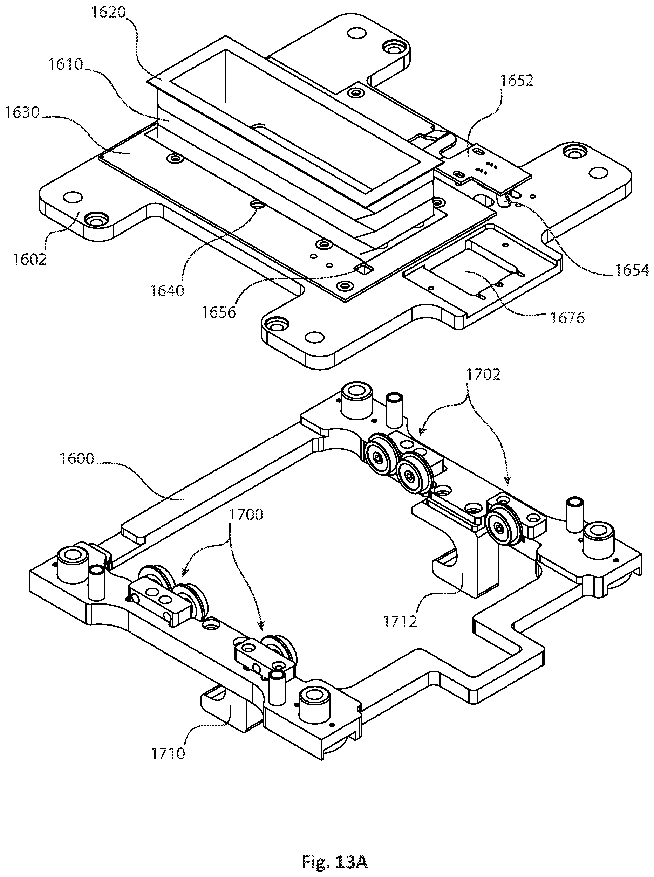

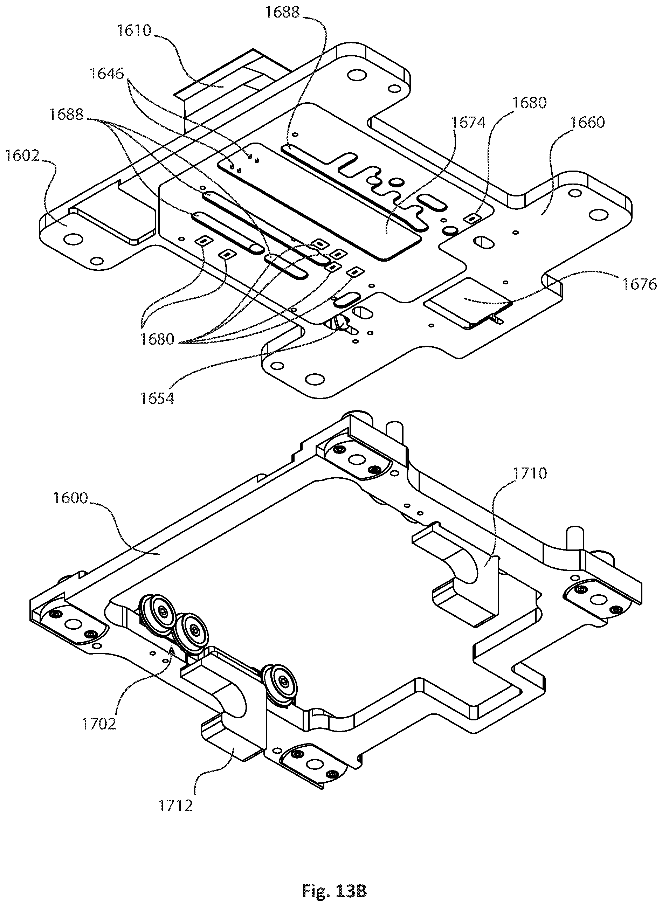

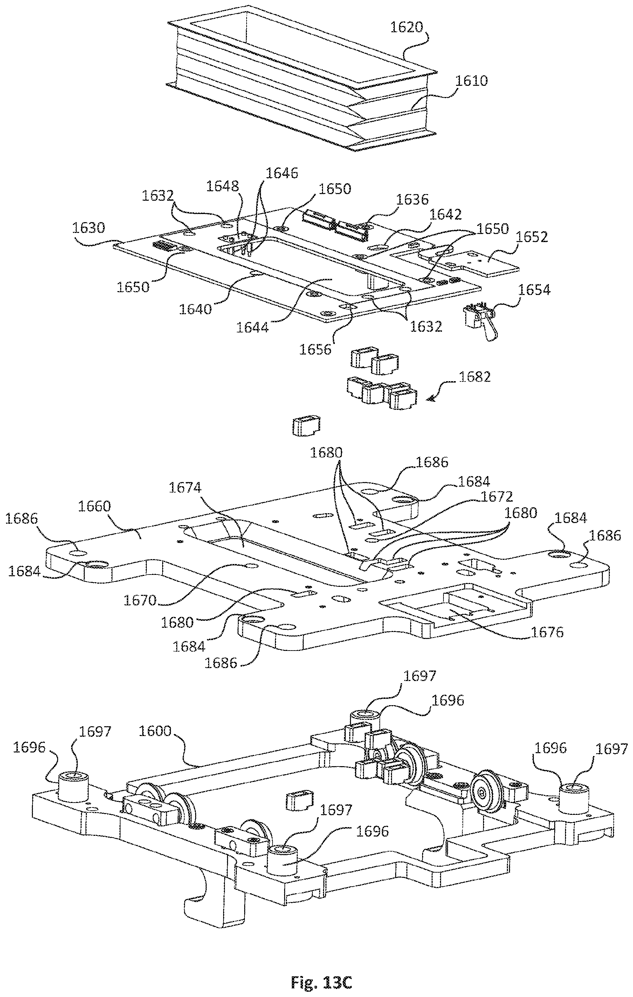

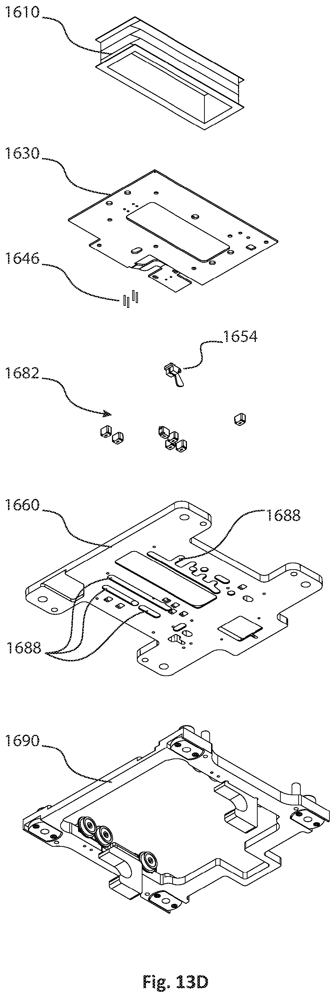

Figs. 13A, 13B, 13C and 13D are simplified exploded view illustrations taken from various mutually different directions of the cartridge holder assembly of Figs. 12A - 12C;

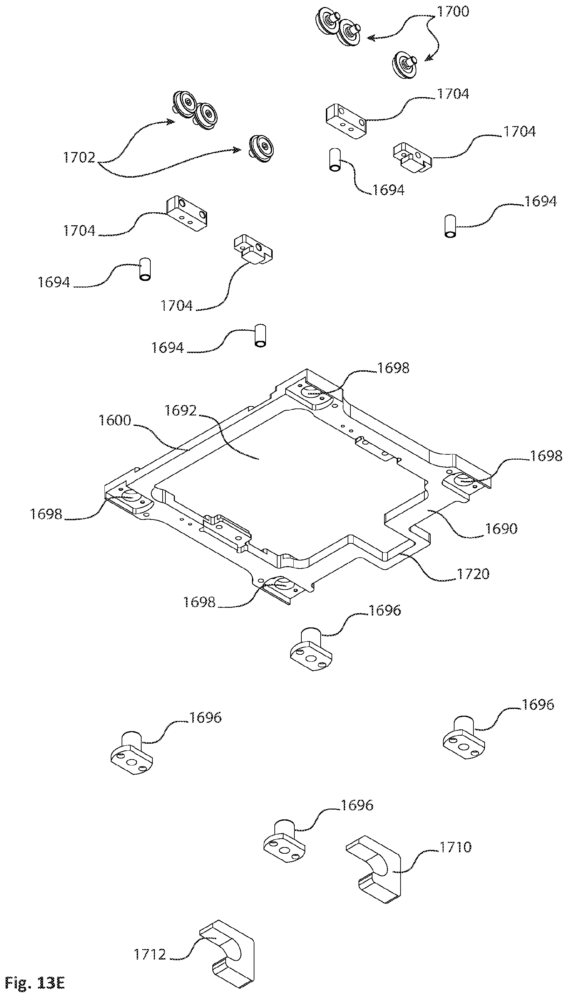

Fig. 13E is a simplified exploded view illustration of part of the cartridge holder assembly of Figs. 12A - 13D;

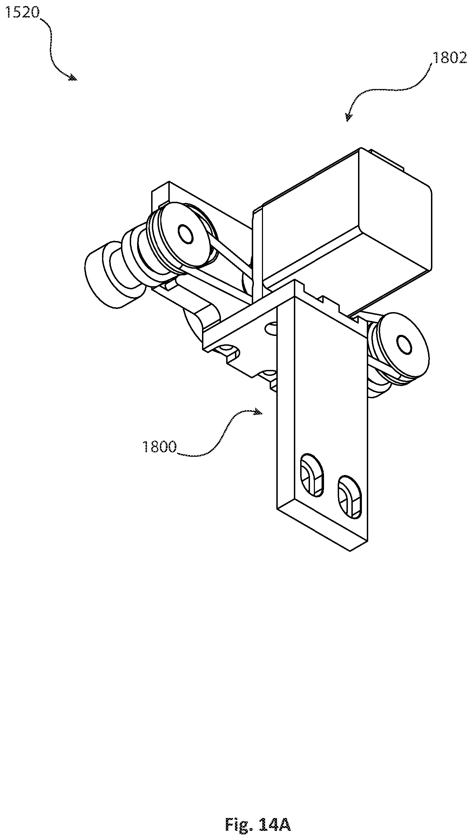

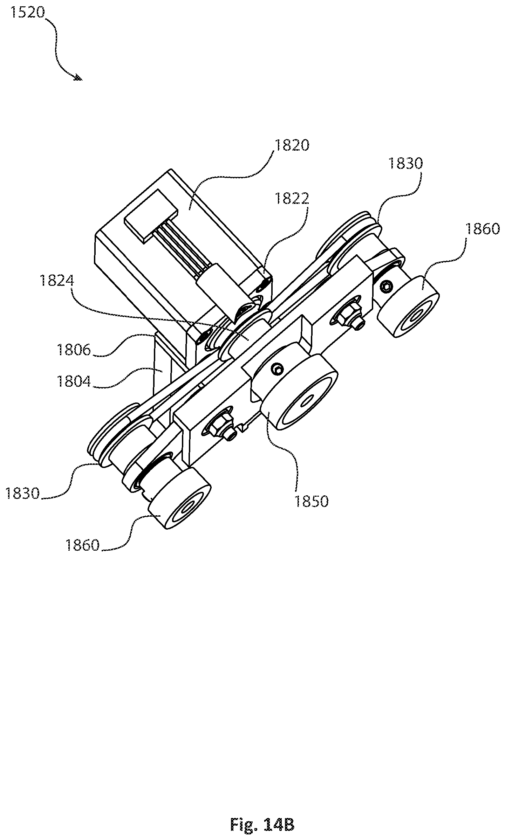

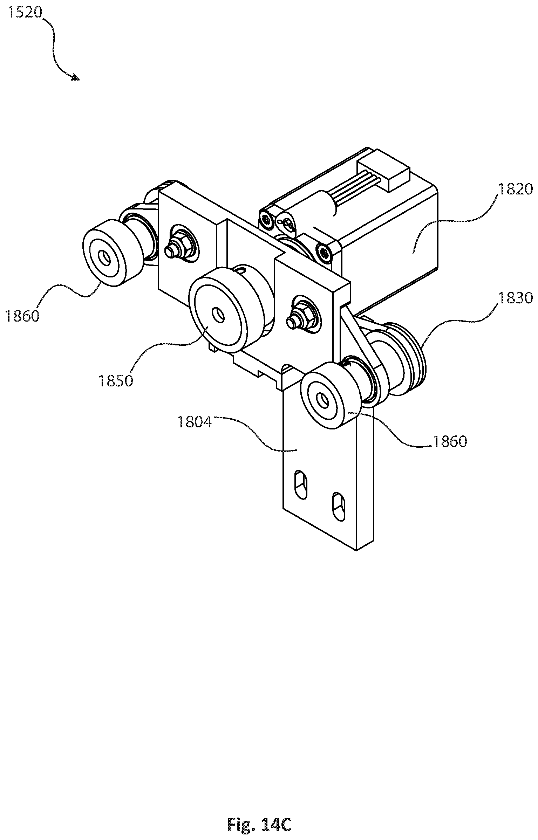

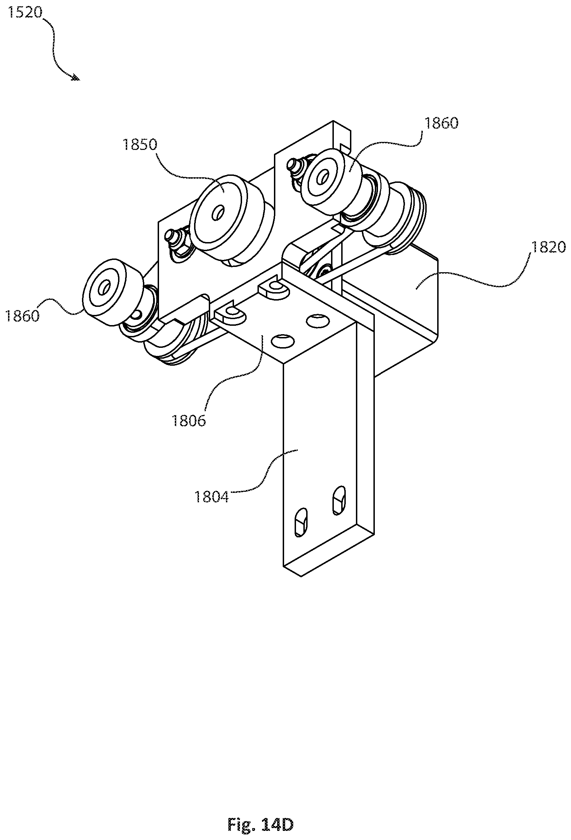

Figs. 14A, 14B, 14C and 14D are simplified assembled view illustrations, taken from various different directions, of a cartridge insertion drive assembly forming part of the instrument of Figs. 11A and 11B;

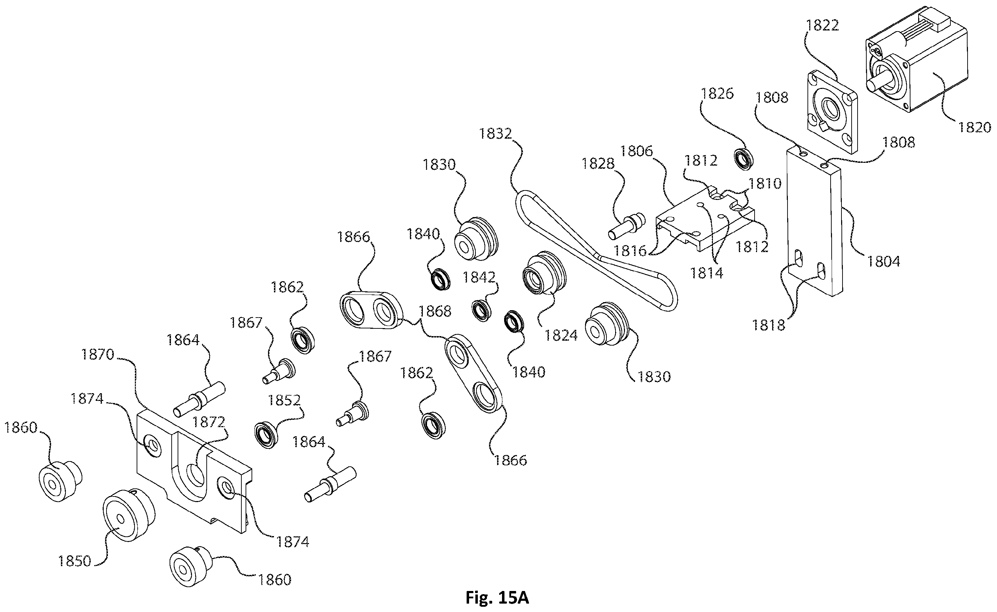

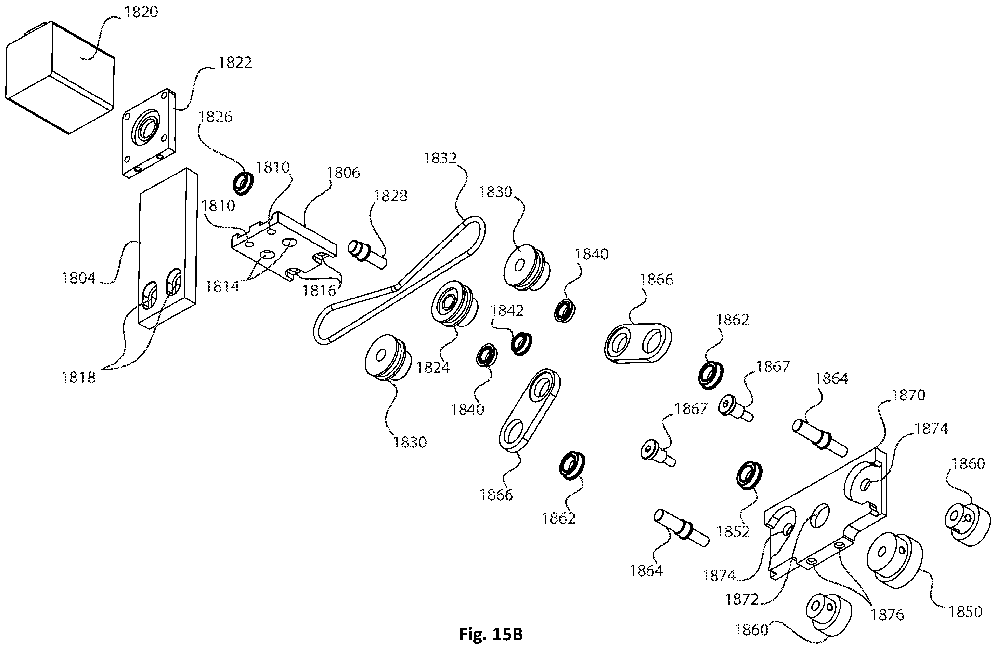

Figs. 15A and 15B are simplified exploded view illustrations, taken from different directions, of the cartridge insertion drive assembly of Figs. 14A - 14D;

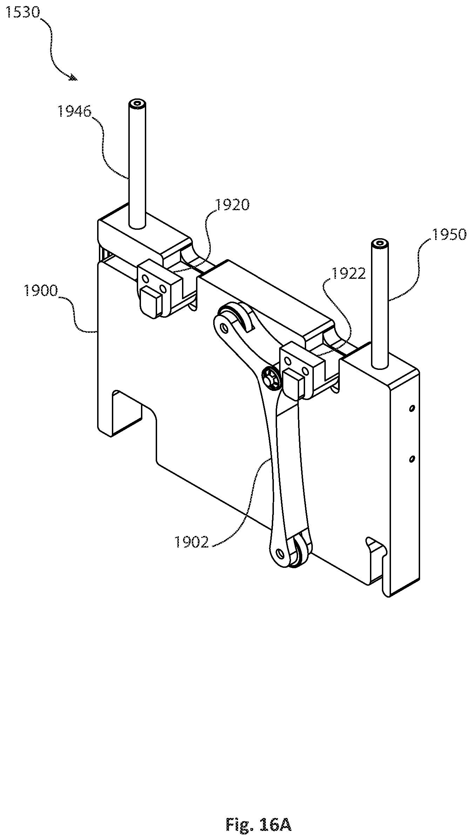

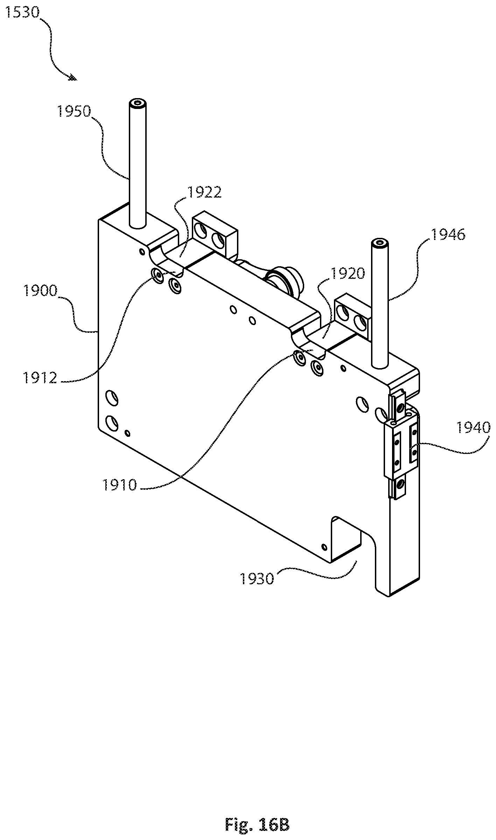

Figs. 16A and 16B are simplified assembled view illustrations, taken from different directions, of a cartridge clamping side sub-assembly forming part of the instrument of Figs. 11A and 11B;

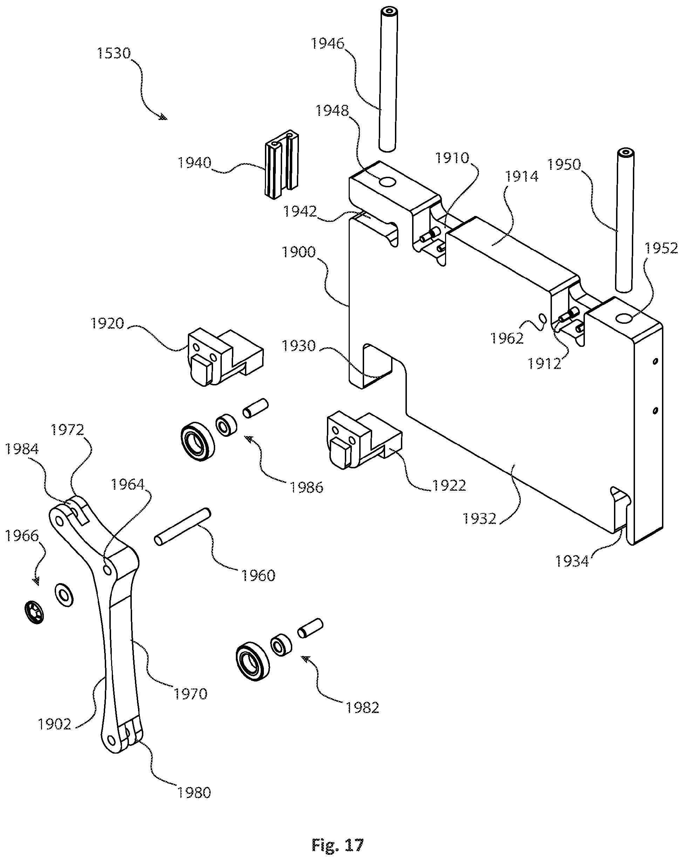

Fig. 17 is a simplified exploded view illustration of the cartridge clamping side sub-assembly of Figs. 16A & 16B;

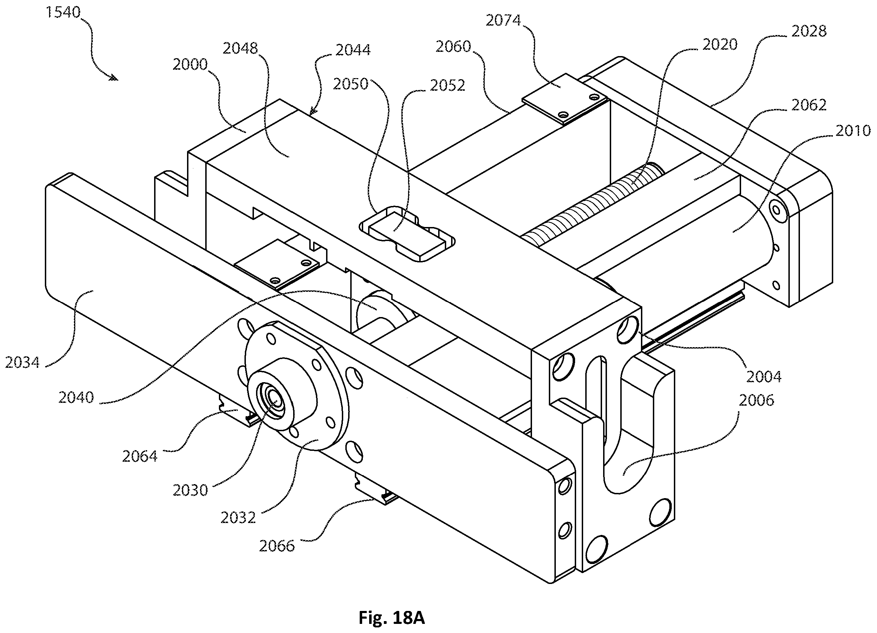

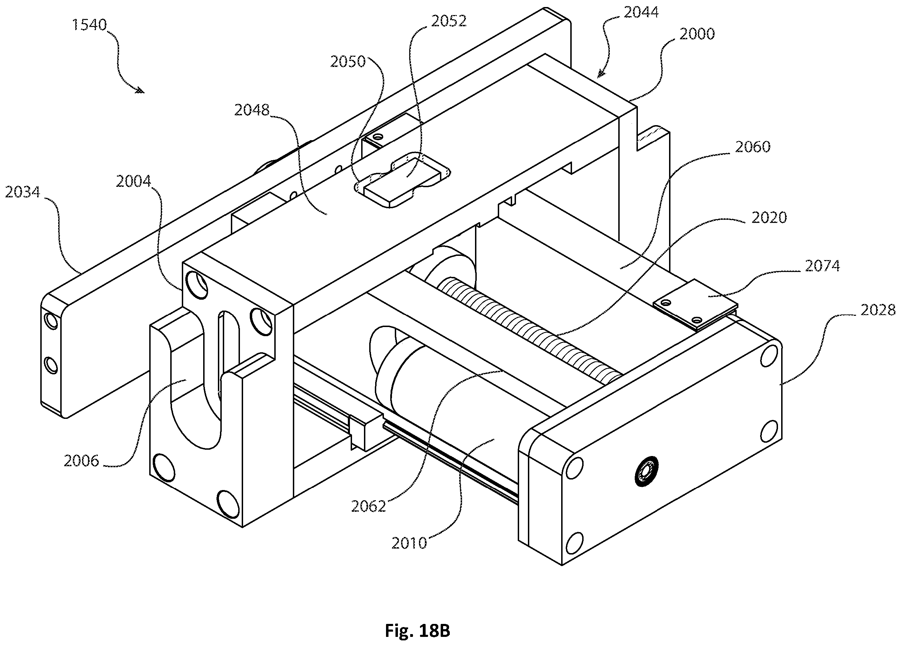

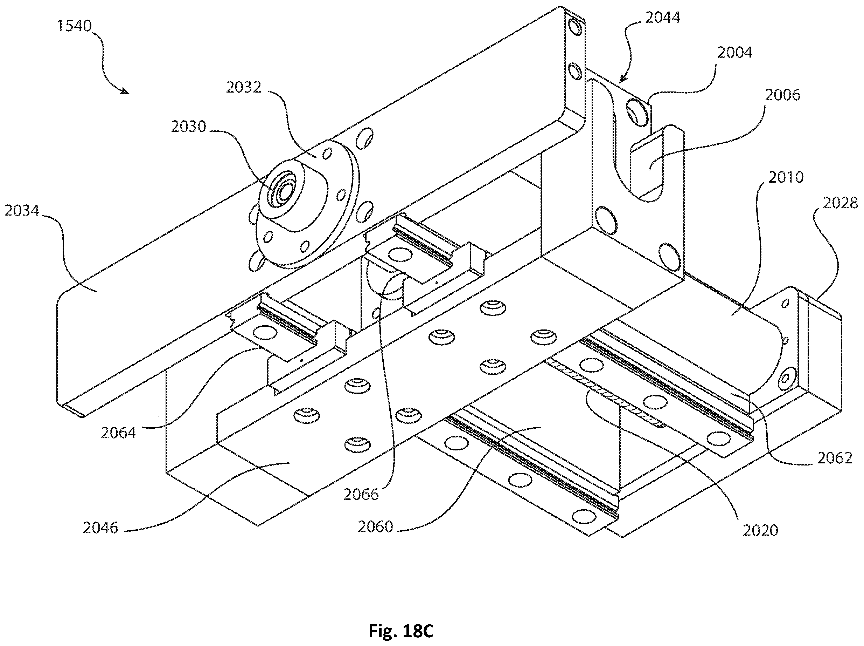

Figs. 18A, 18B and 18C are simplified assembled view illustrations, taken from various different directions, of a cartridge clamping drive assembly forming part of the instrument of Figs. 11A and 11B;

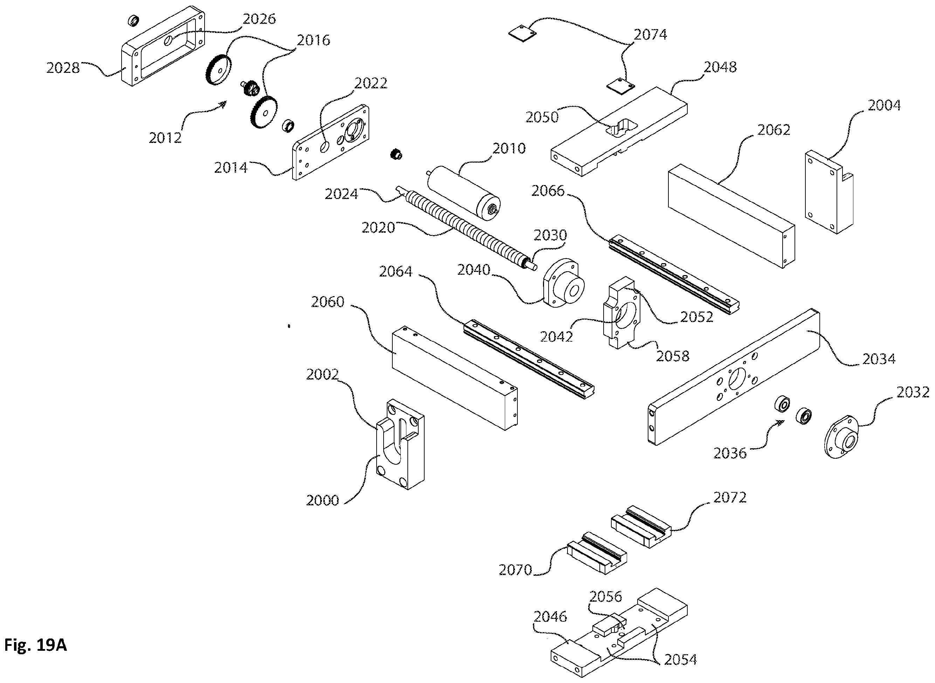

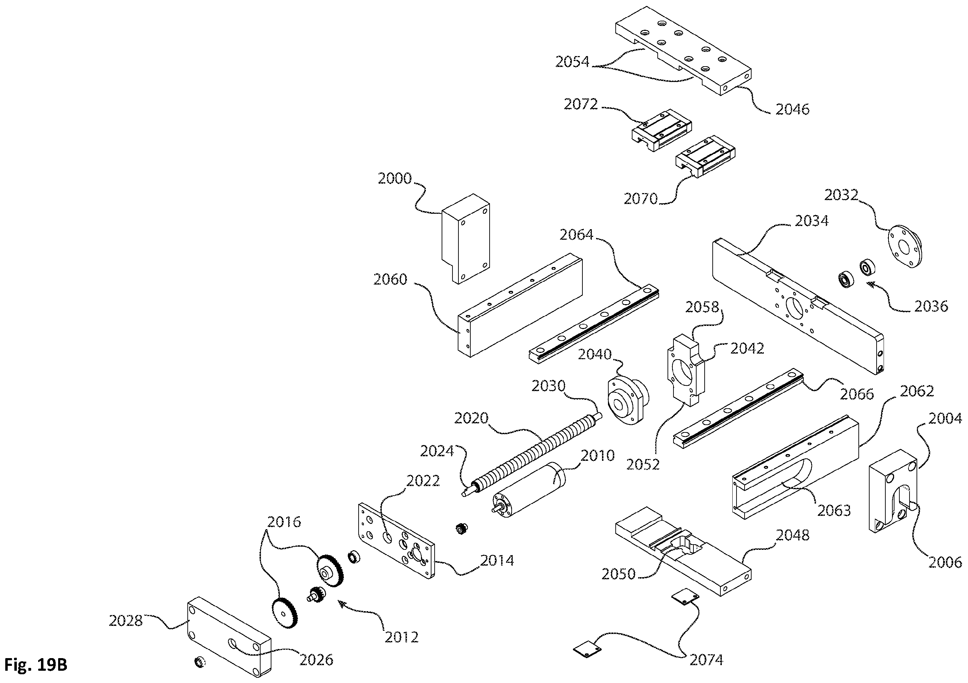

Figs. 19A and 19B are simplified exploded view illustrations, taken from different directions, of the cartridge clamping drive assembly of Figs. 18A - 18C;

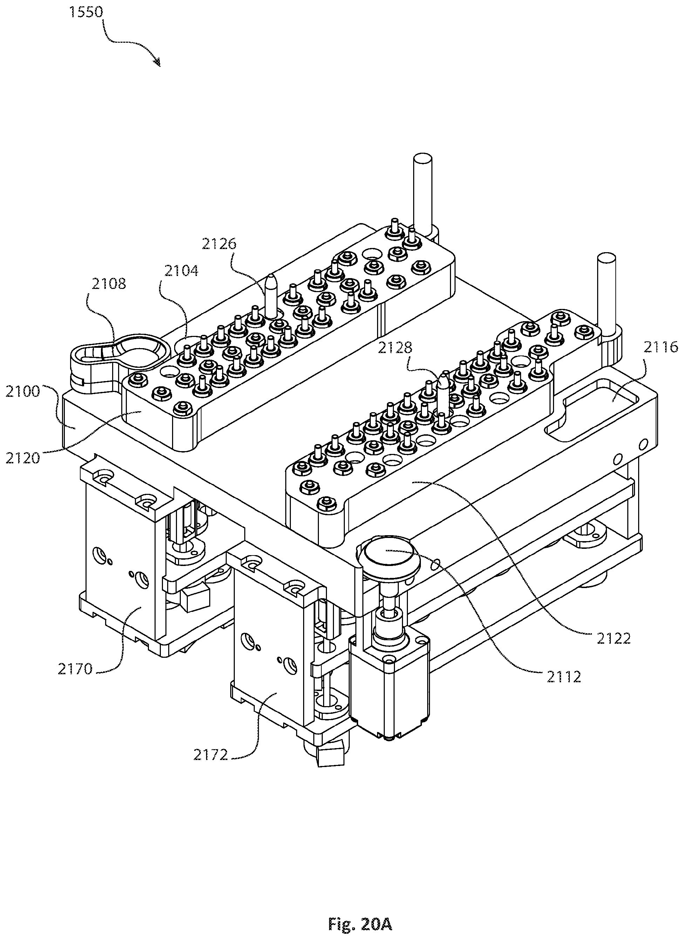

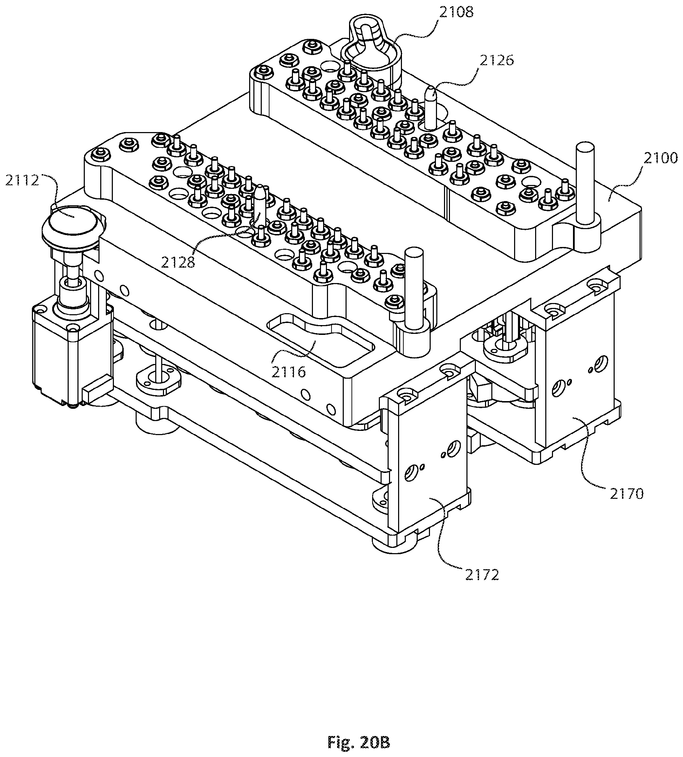

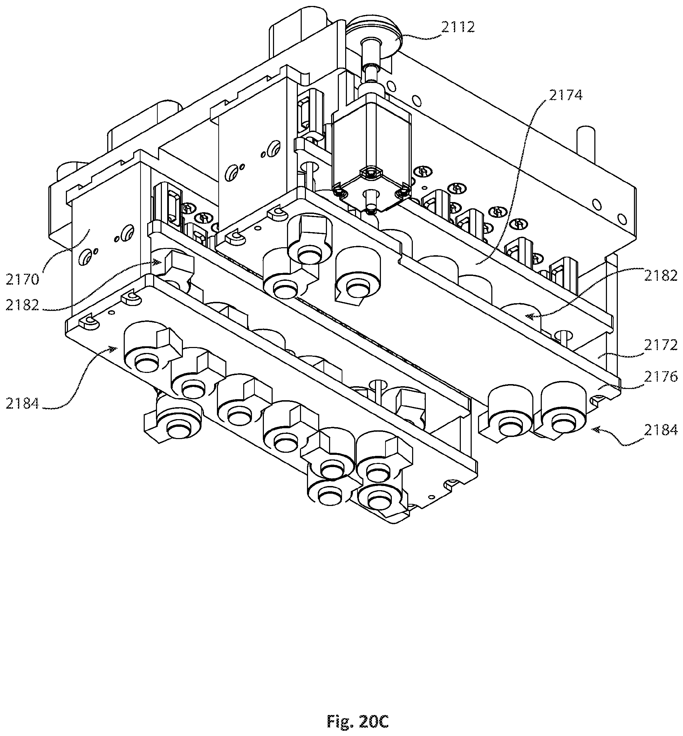

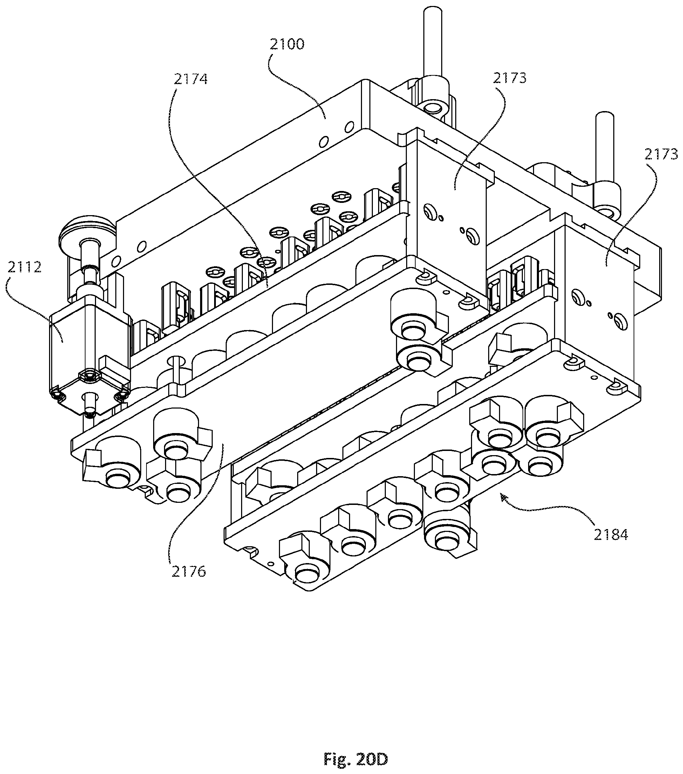

Figs. 20A, 20B, 20C and 20D are simplified assembled view illustrations, taken from various different directions, of a cartridge actuation assembly forming part of the instrument of Figs. 11A and 11B;

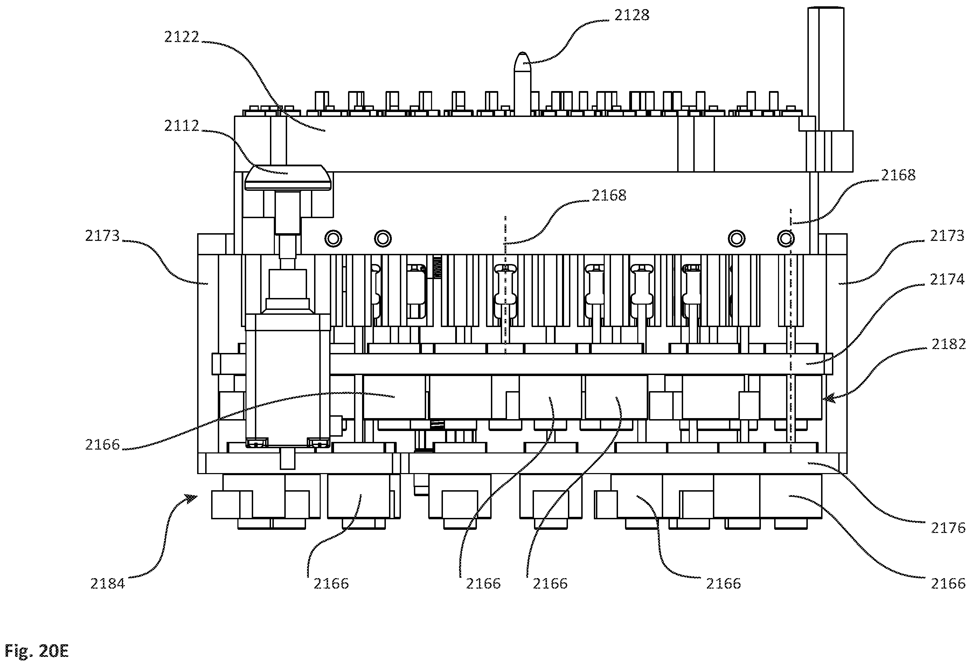

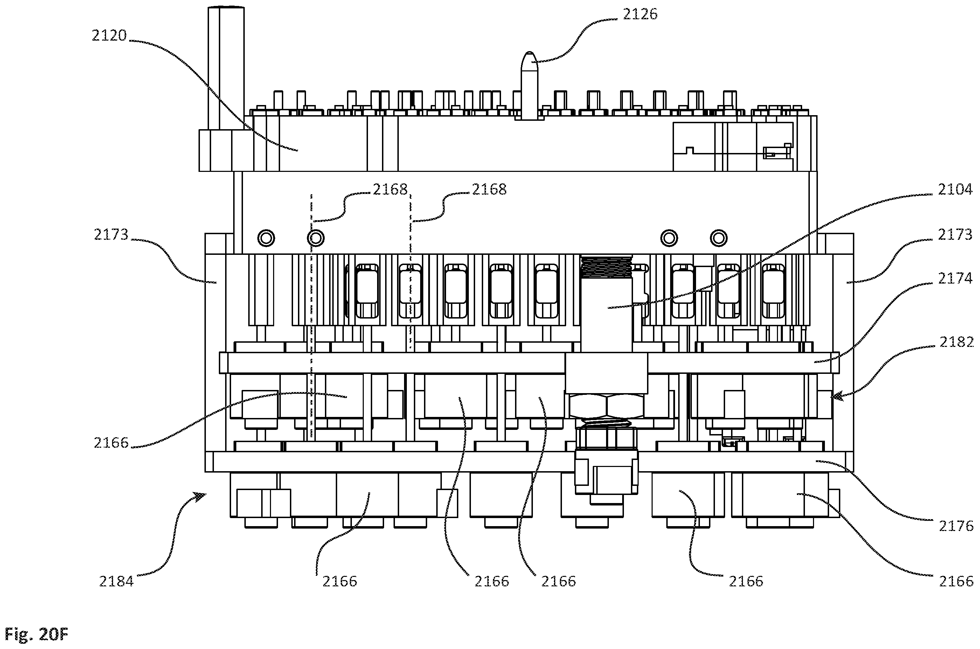

Figs. 20E and 20F are planar side views of the cartridge actuation assembly of Figs. 20A - 20D, taken from opposite directions thereof;

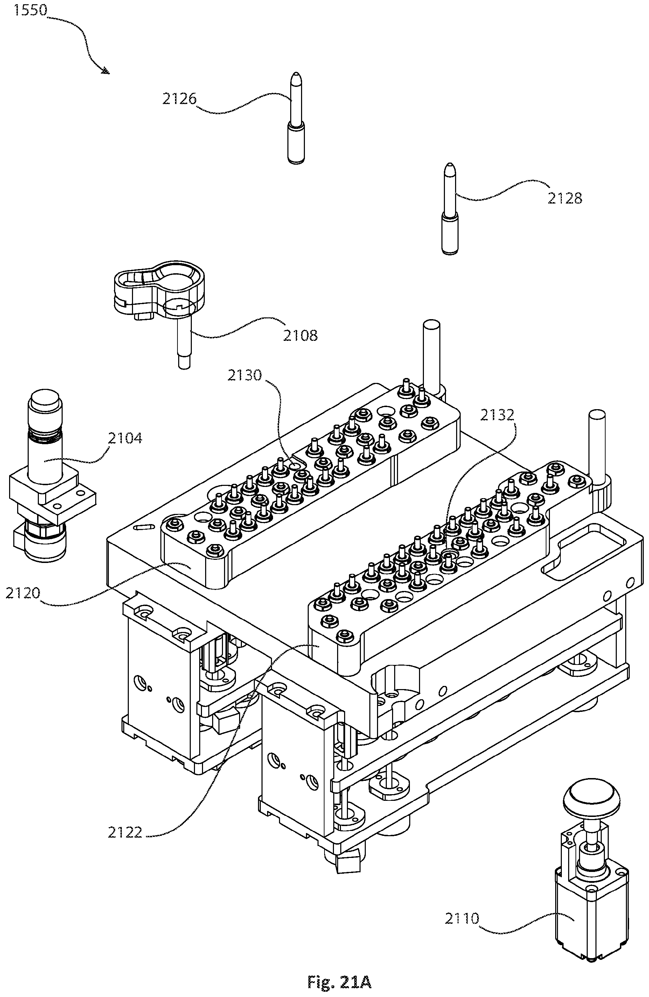

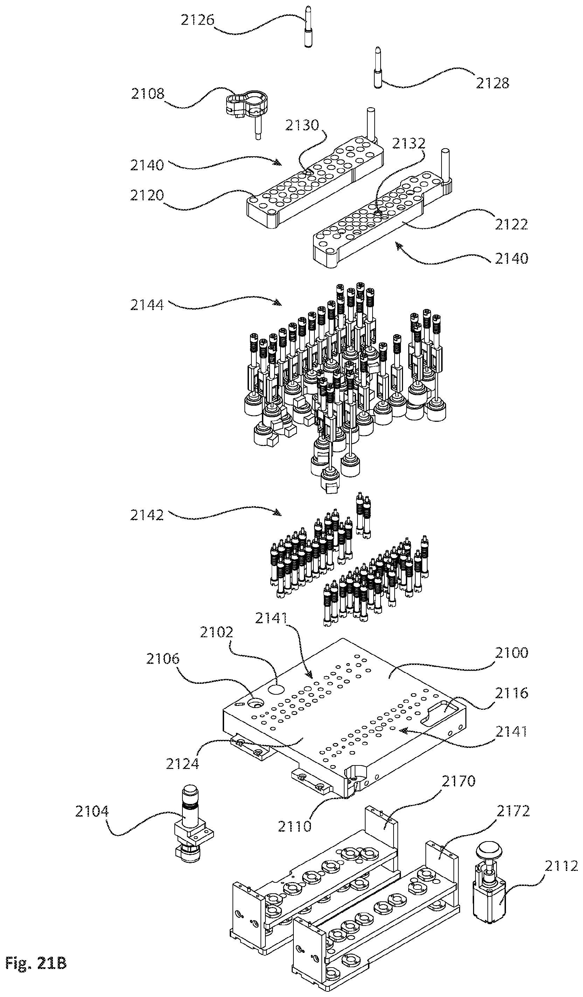

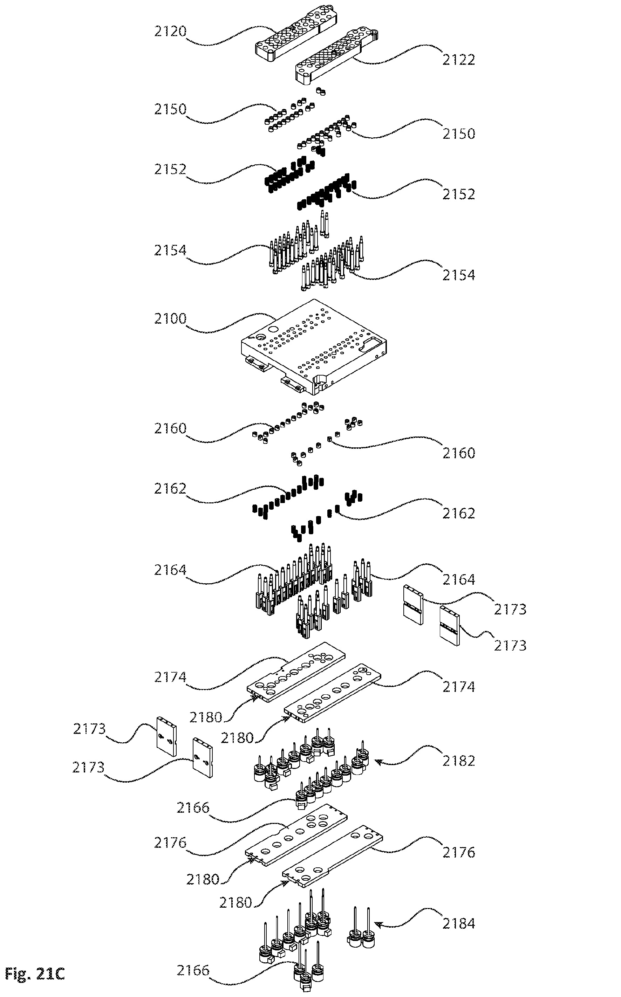

Figs. 21A, 21B and 21C are simplified exploded view illustrations taken from various mutually different directions of the cartridge actuation assembly of Figs. 20A - 20F;

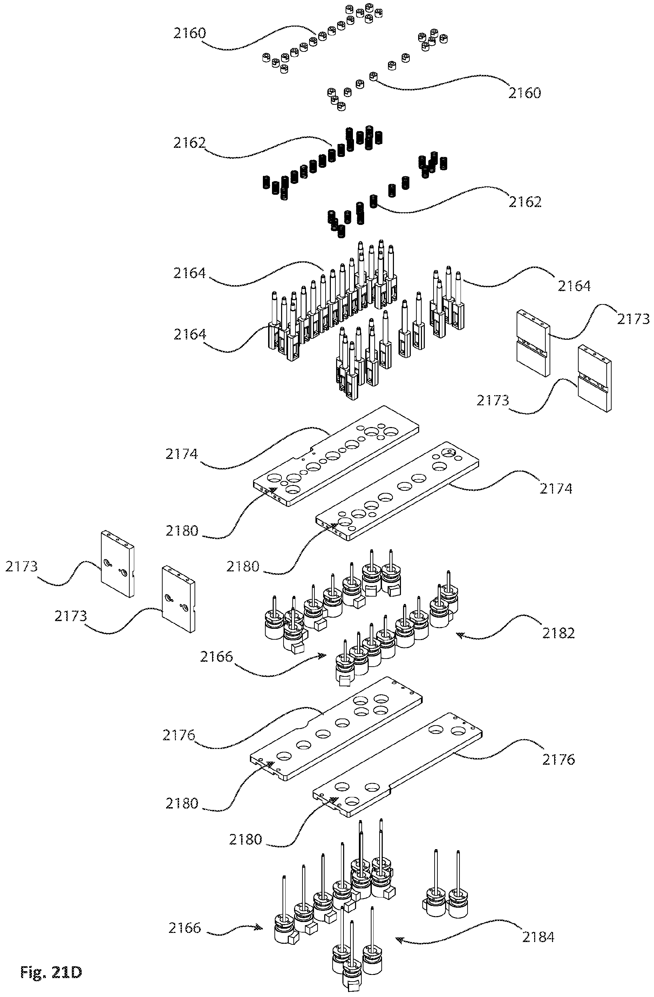

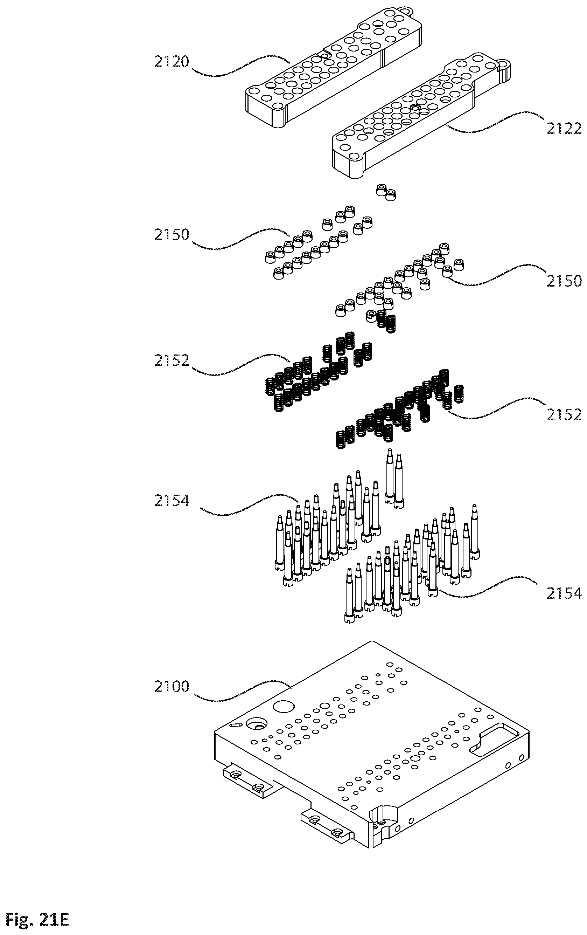

Figs. 21D and 21E are simplified enlarged exploded view illustrations of portions of the cartridge actuation assembly of Figs. 20A - 21C;

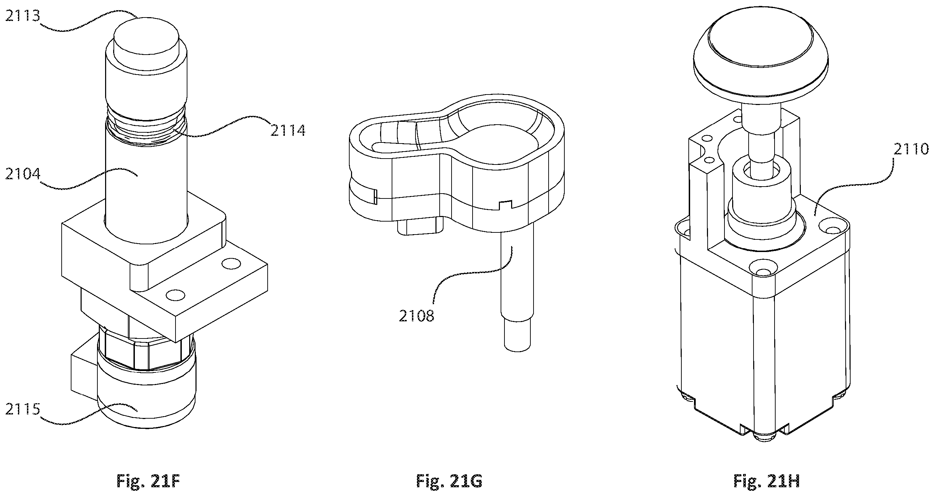

Figs. 21F, 21G and 21H are simplified illustrations of part of the cartridge actuation assembly of Figs. 20A - 21E;

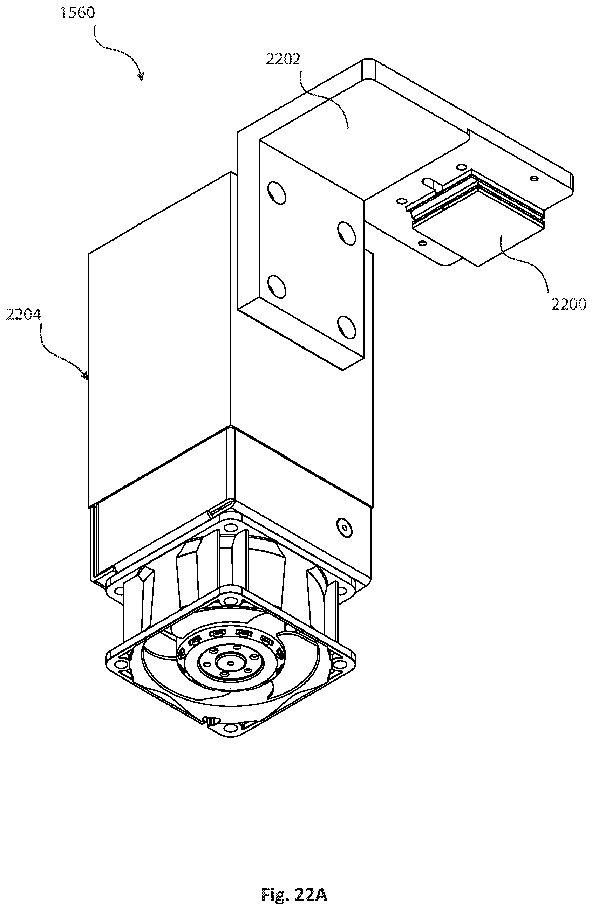

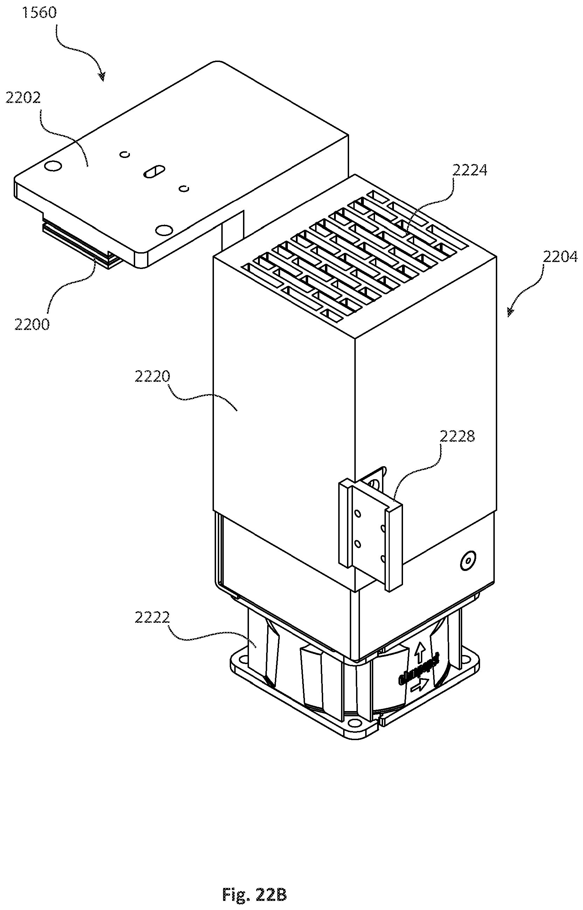

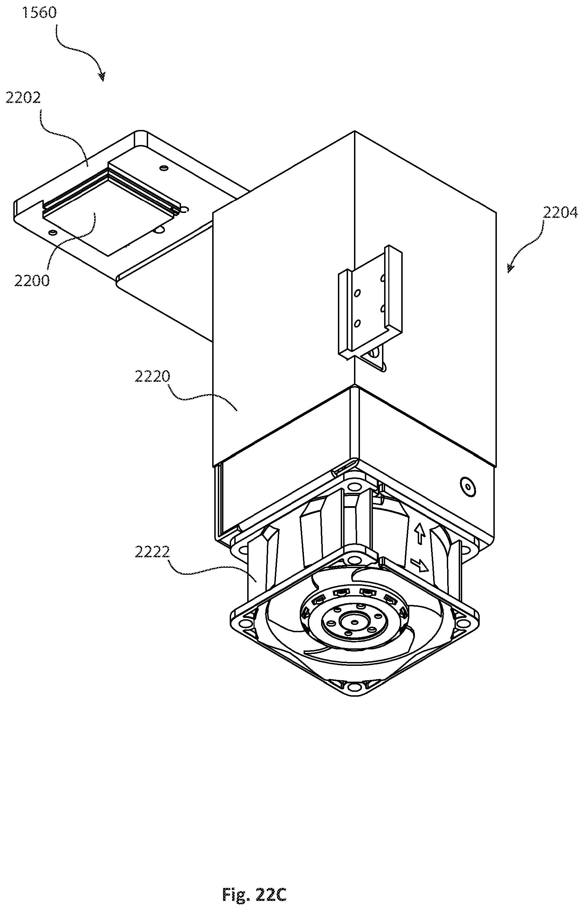

Figs. 22A, 22B and 22C are simplified assembled view illustrations, taken from various different directions, of an upper thermocycling assembly forming part of the instrument of Figs. 11A and 11B;

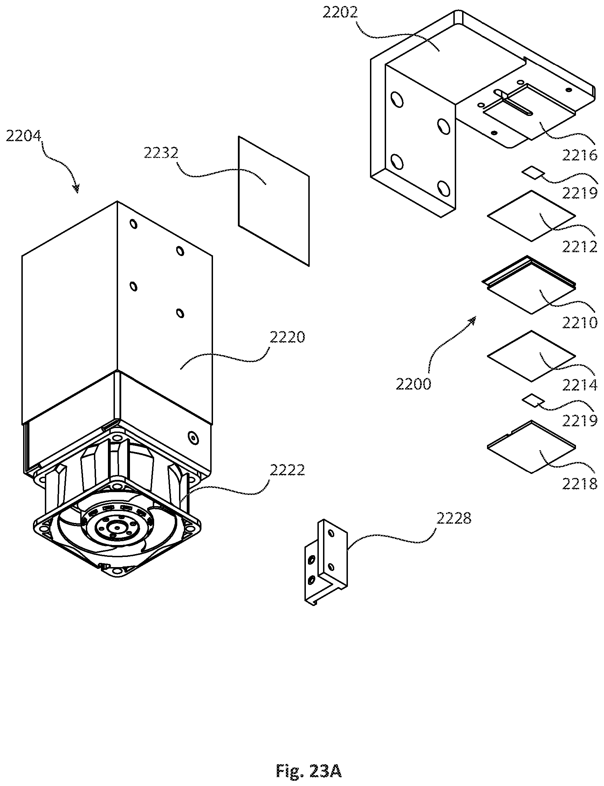

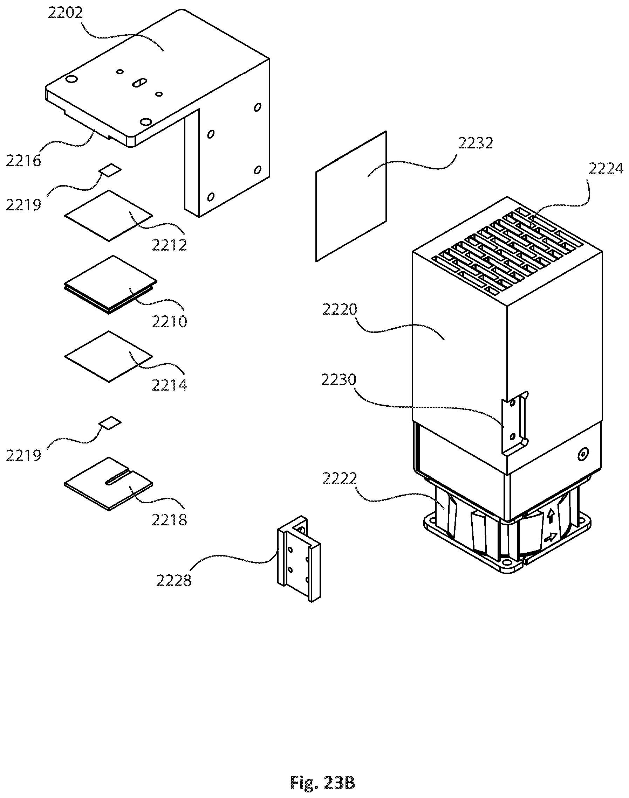

Figs. 23A and23B are simplified exploded view illustrations, taken from different directions, of the upper thermocycling assembly of Figs. 22A, 22B and 22C;

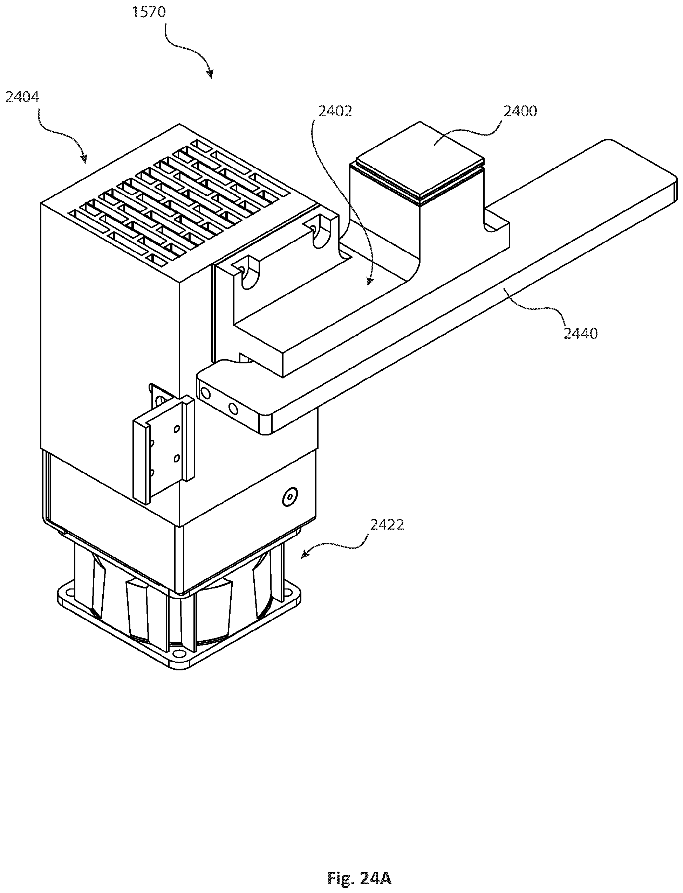

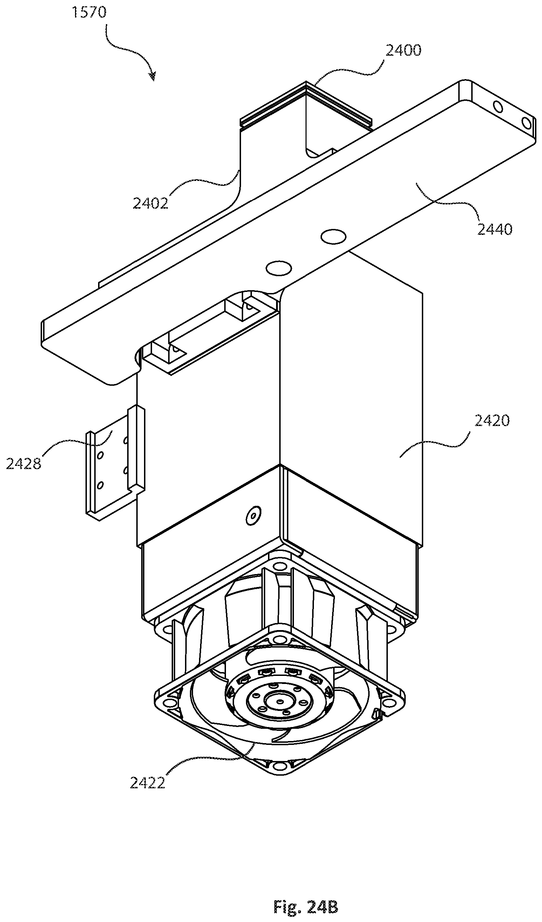

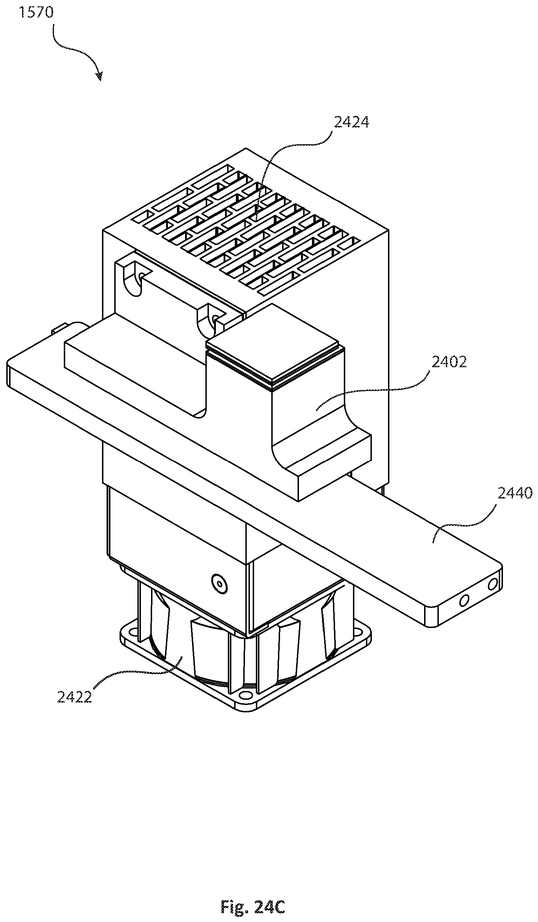

Figs. 24A, 24B and 24C are simplified assembled view illustrations, taken from various different directions, of a lower thermocycling assembly forming part of the instrument of Figs. 11A and 11B;

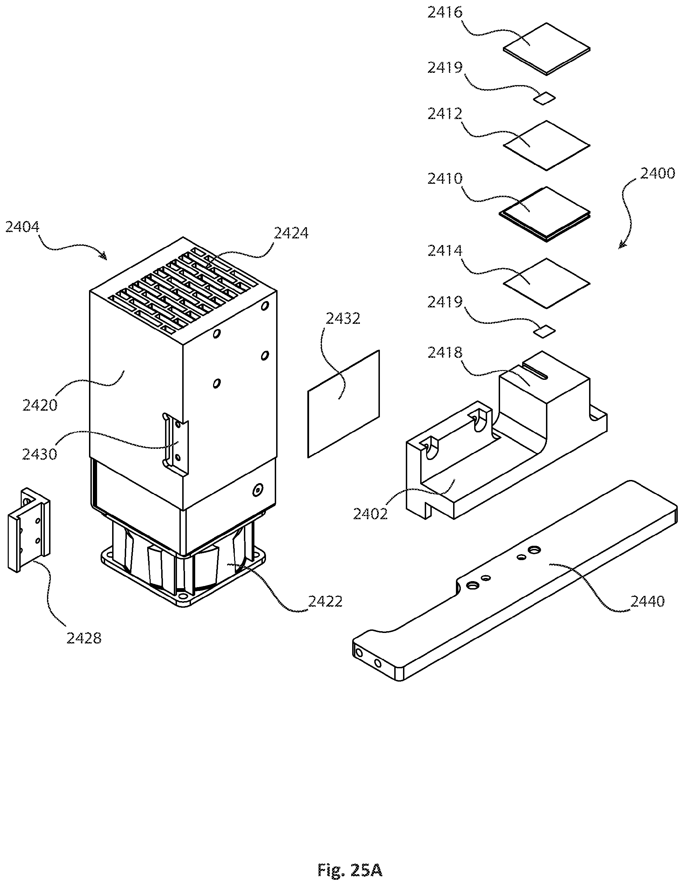

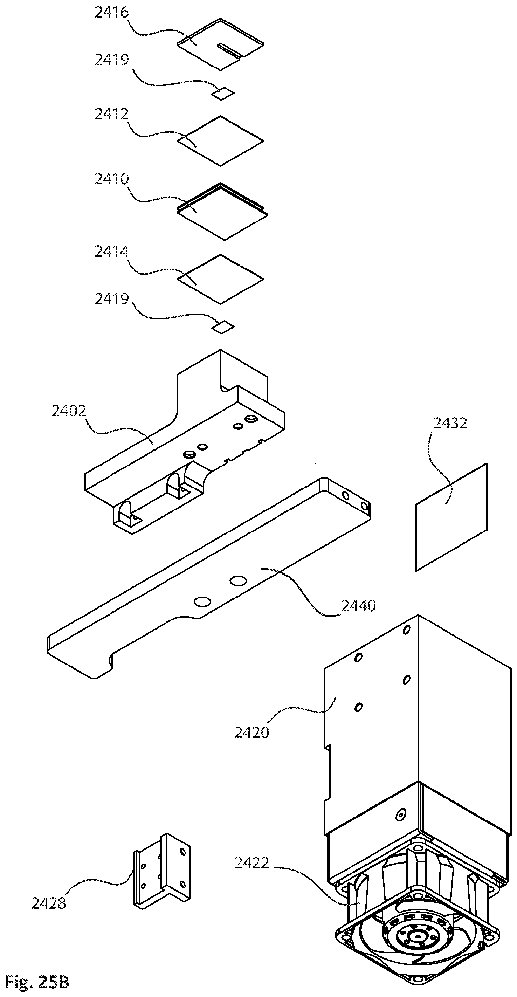

Figs. 25A and 25B are simplified exploded view illustrations, taken from different directions, of the lower thermocycling assembly of Figs. 24A - 24C;

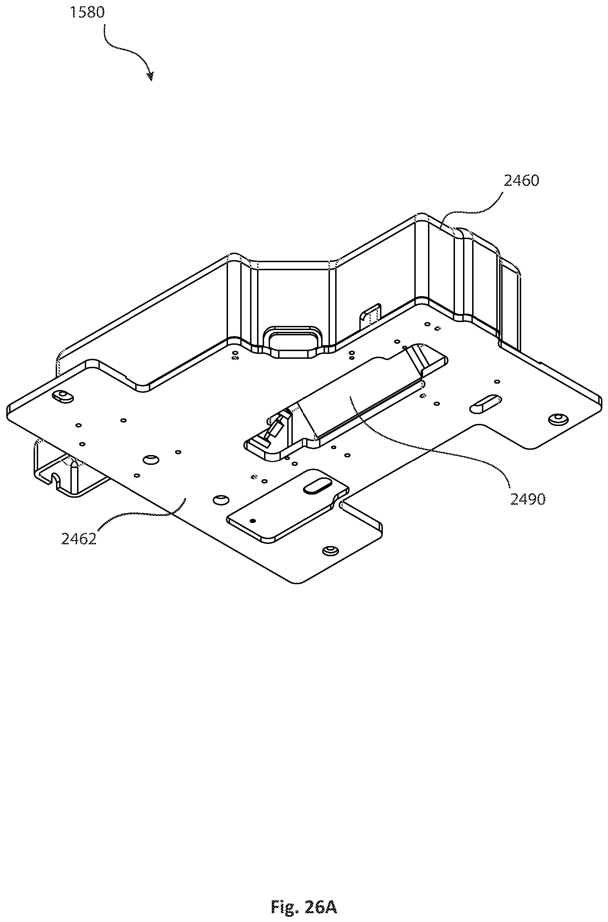

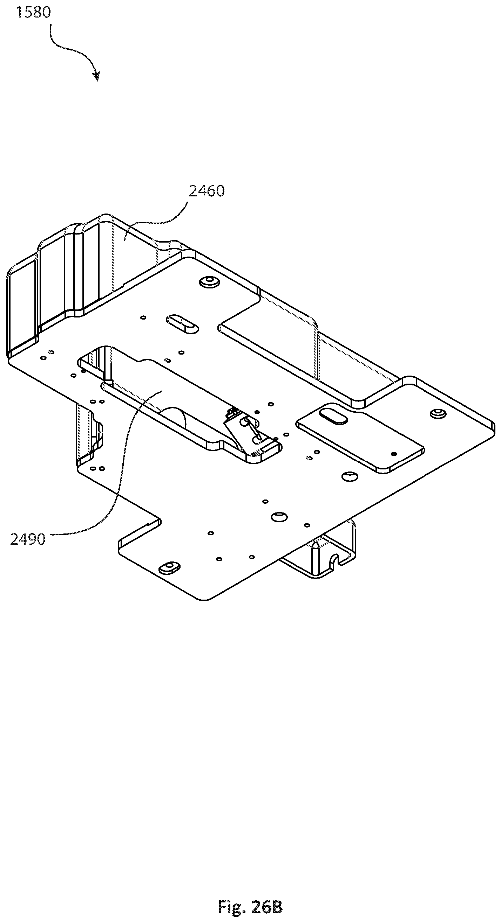

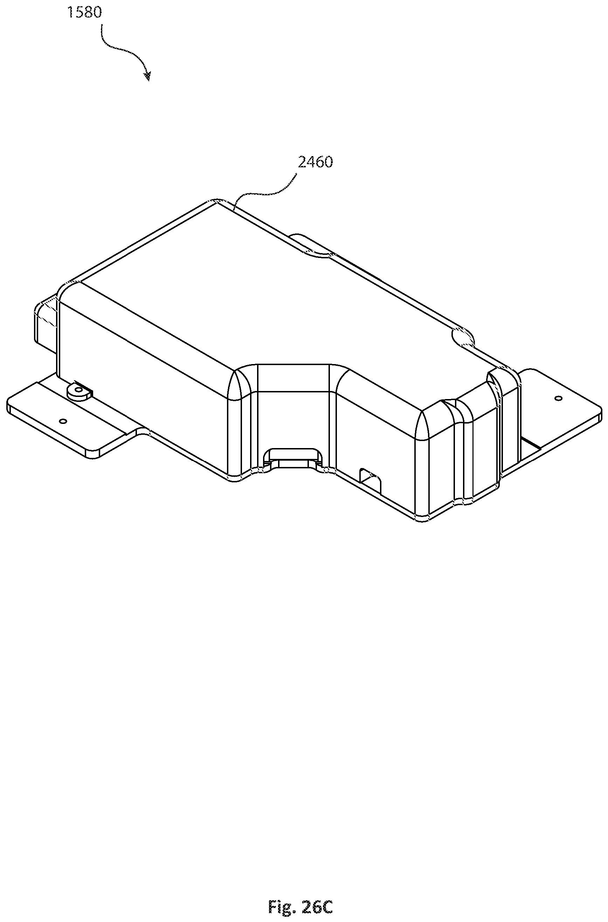

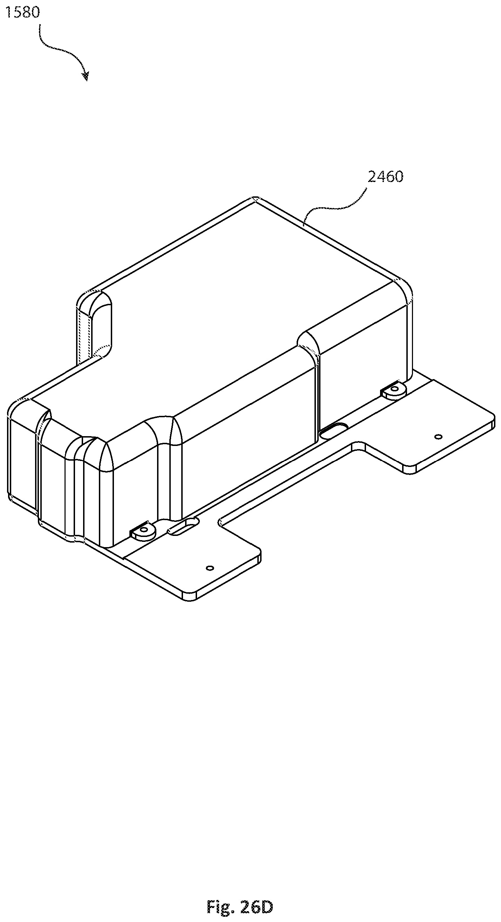

Figs. 26A, 26B, 26C and 26D are simplified assembled view illustrations, taken from various different directions, of an optical assembly forming part of the instrument of Figs. 11A and 11B;

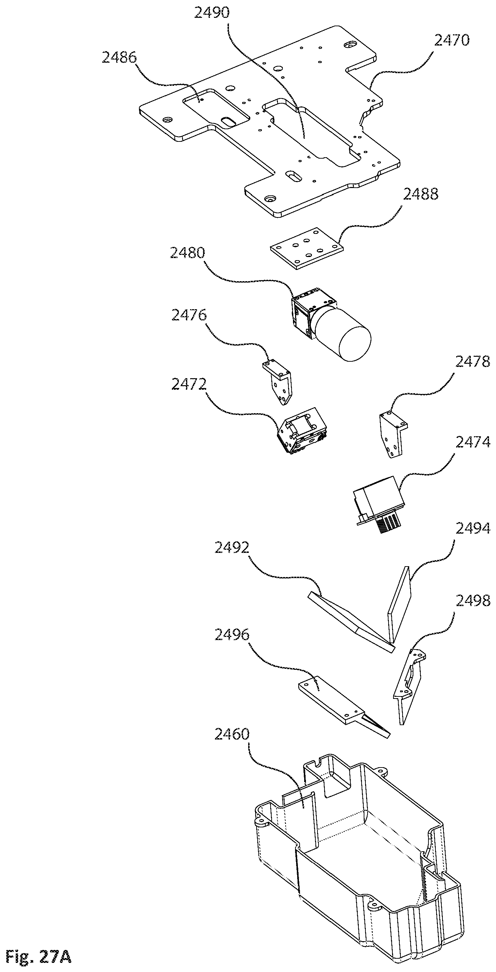

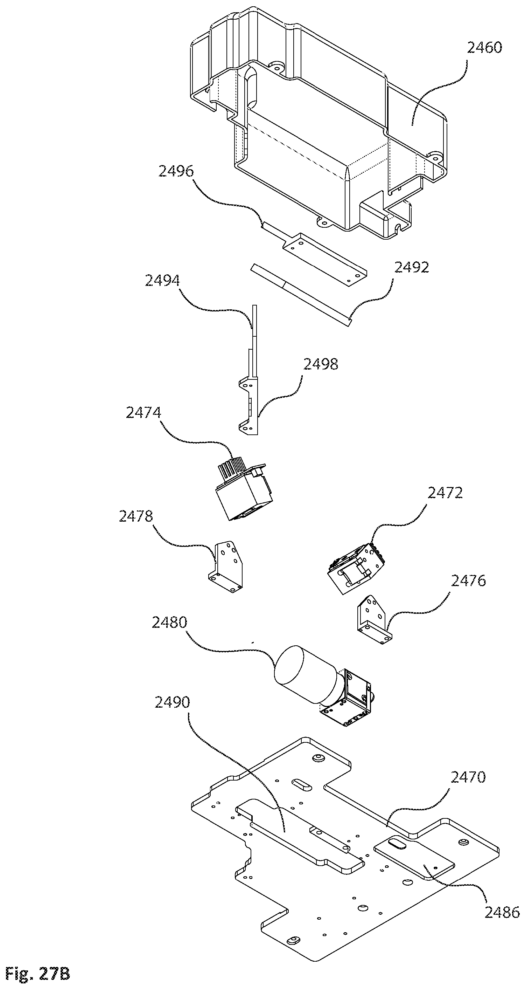

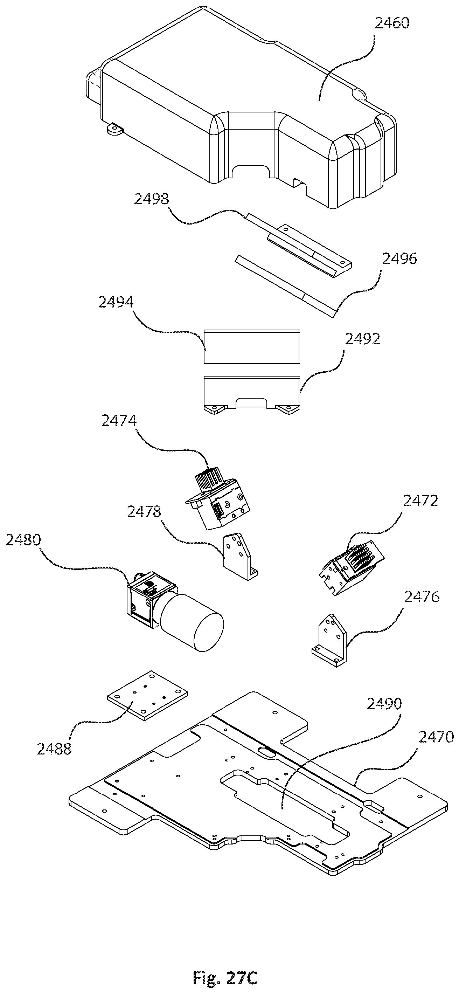

Figs. 27A, 27B and 27C are simplified exploded view illustrations, taken from various mutually different directions, of the optical assembly of Figs. 26A - 26C;

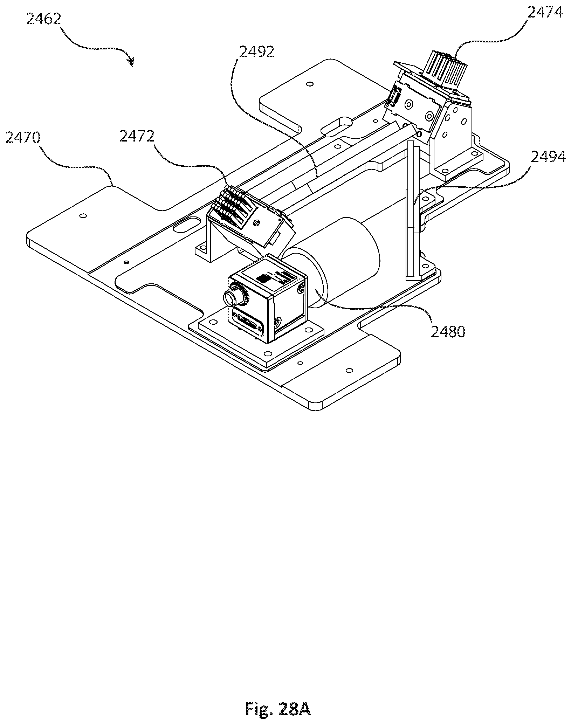

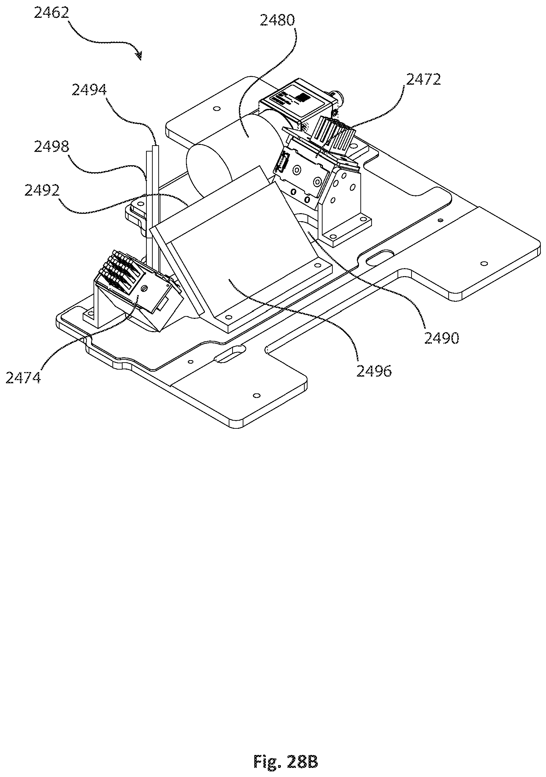

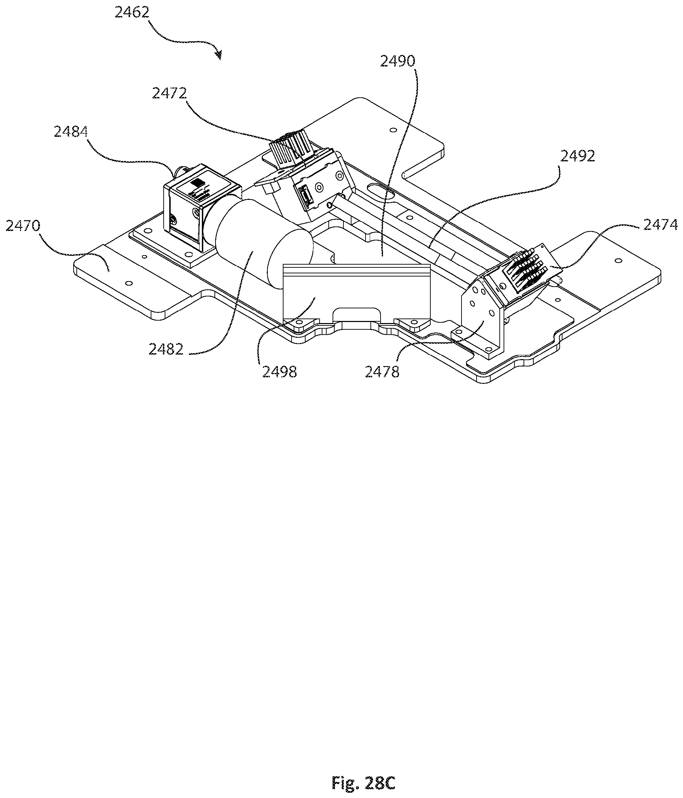

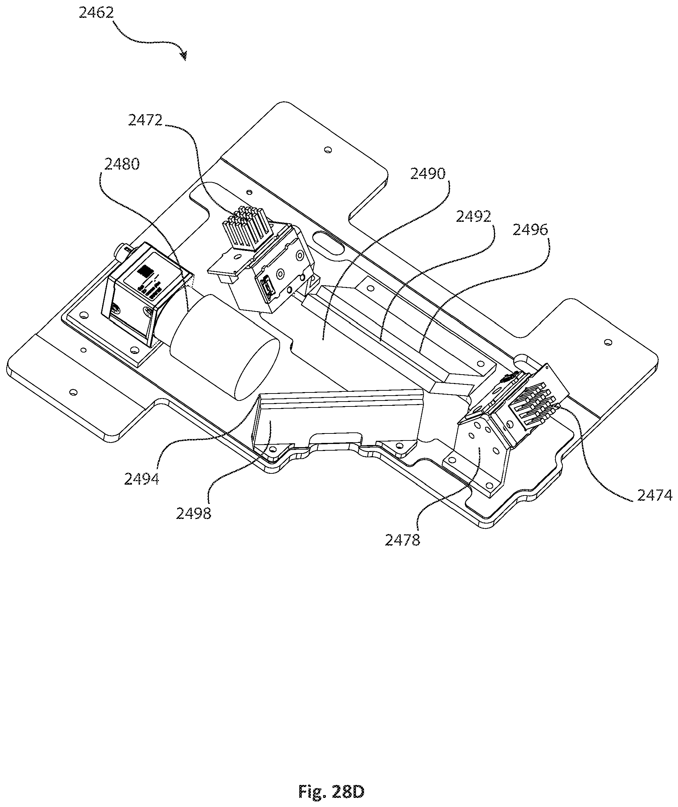

Figs. 28A, 28B, 28C and 28D are simplified illustrations taken from various mutually different directions of the optical assembly of Figs. 26A - 27C;

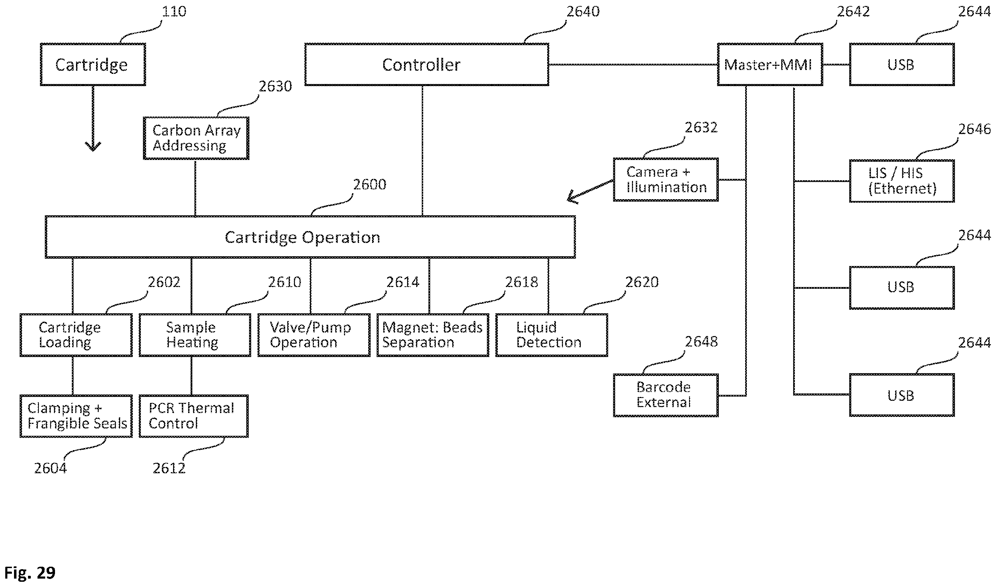

Fig. 29 is a simplified functional schematic illustration of the automated diagnostic instrument of Figs. 11A & 11B;

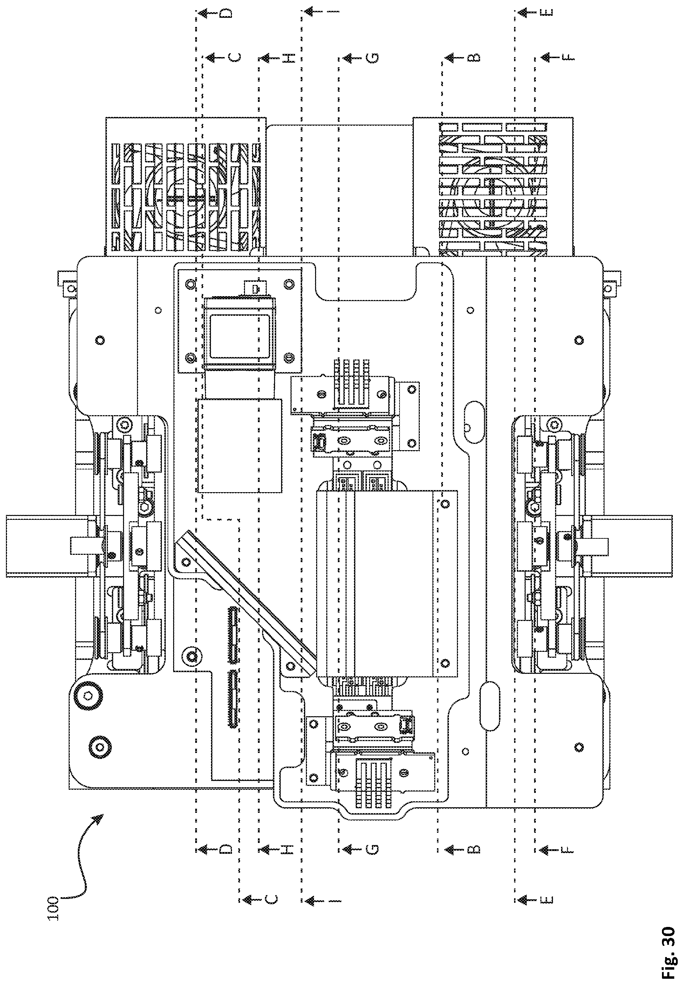

Fig. 30 is a simplified top view illustration of the instrument of Figs. 11A - 29, showing various section lines which are referenced hereinbelow;

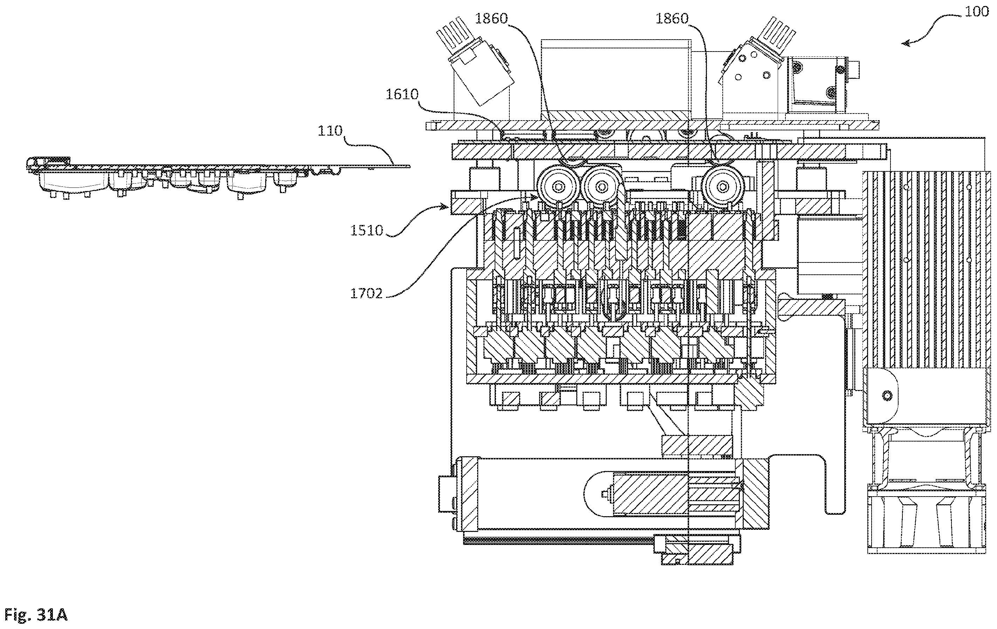

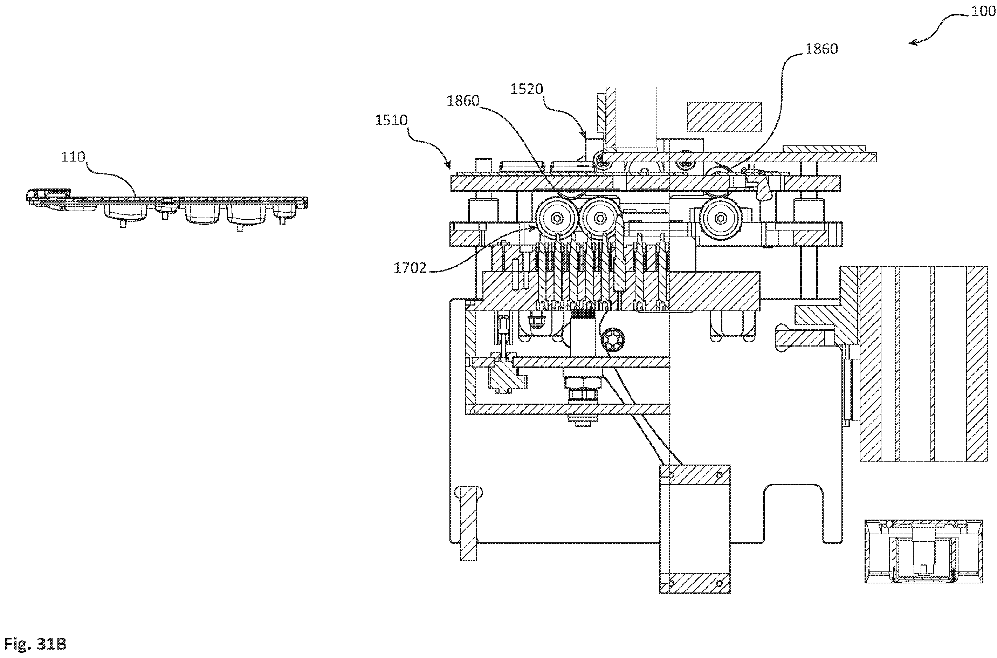

Figs. 31A and 31B are simplified respective first and second sectional illustrations of an initial stage just prior to insertion of the cartridge of Figs. 2A/1 - 10D into the instrument of Figs. 11A - 29, Figs. 31A and 31B being taken along respective section lines B - B and C - C in Fig. 30;

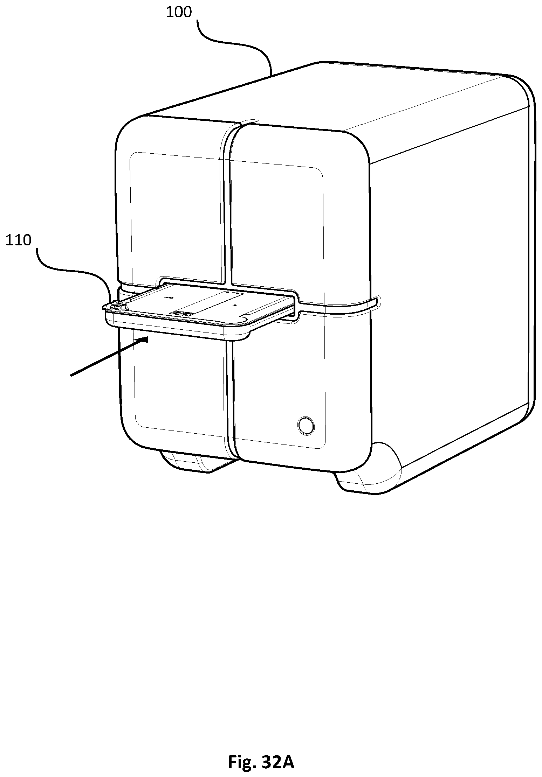

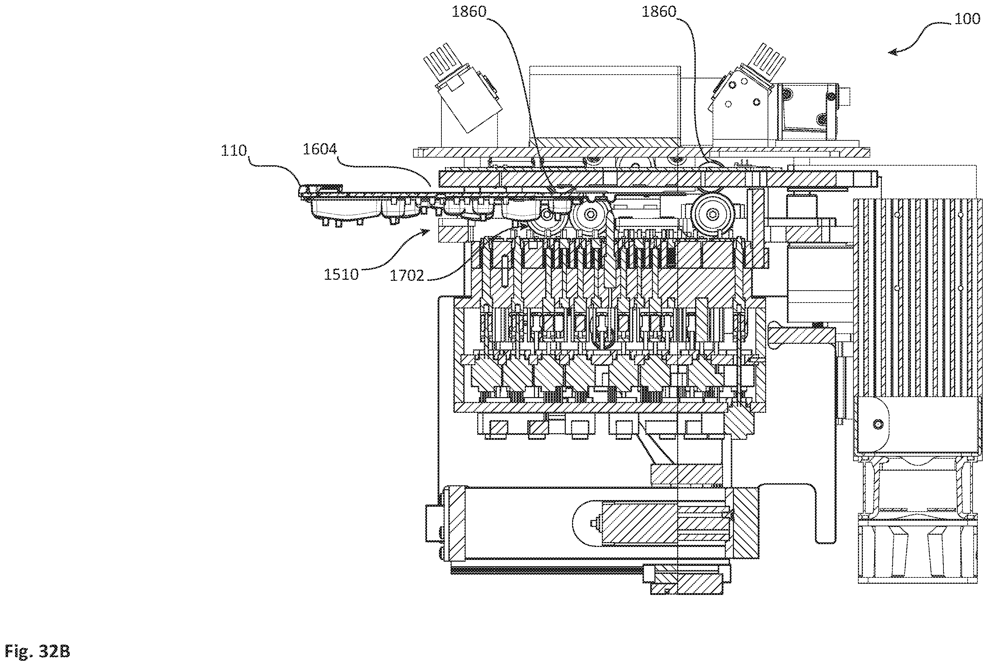

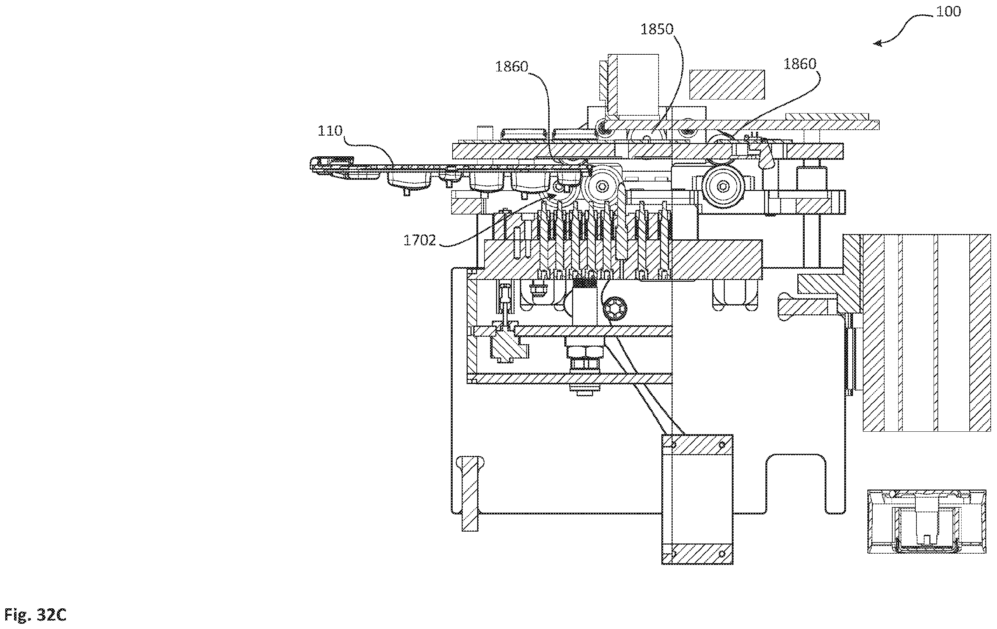

Figs. 32A, 32B and 32C are simplified respective pictorial and first and second sectional illustrations of a first stage of insertion of the cartridge of Figs. 2A/1 -10D into the instrument of Figs. 11A - 29, Figs. 32B and 32C being taken along section lines B - B and C - C in Fig. 30;

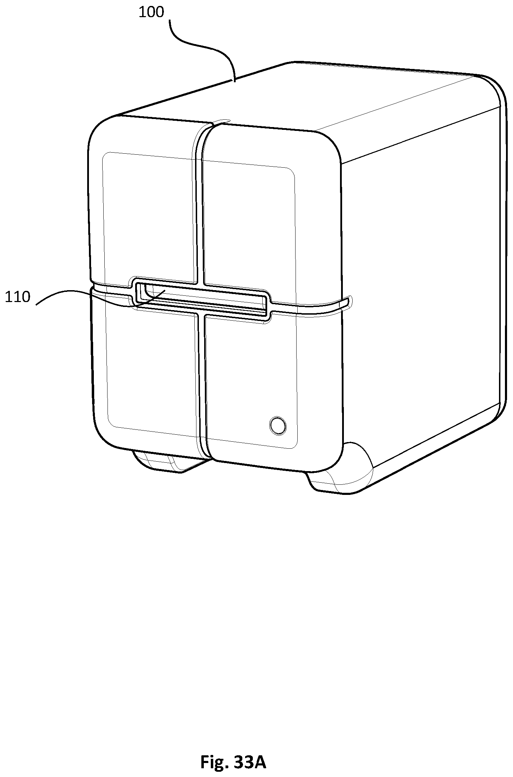

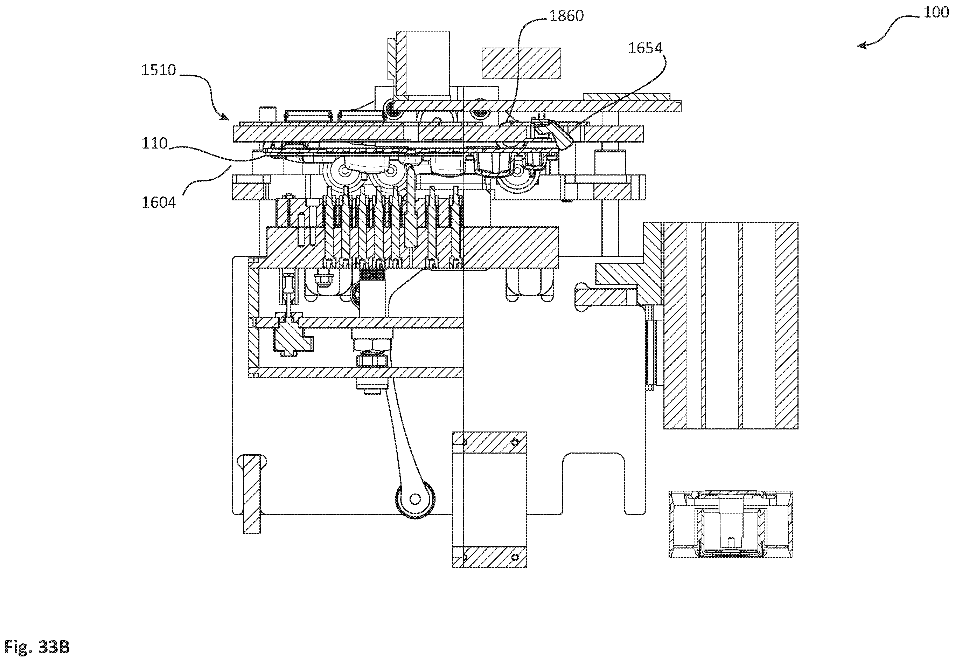

Figs. 33A and 33B are simplified respective pictorial and sectional illustrations of a second stage of insertion of the cartridge of Figs. 2A/1 - 10D into the instrument of Figs. 11A - 29, Fig. 33B being taken along section lines C - C in Fig. 30;

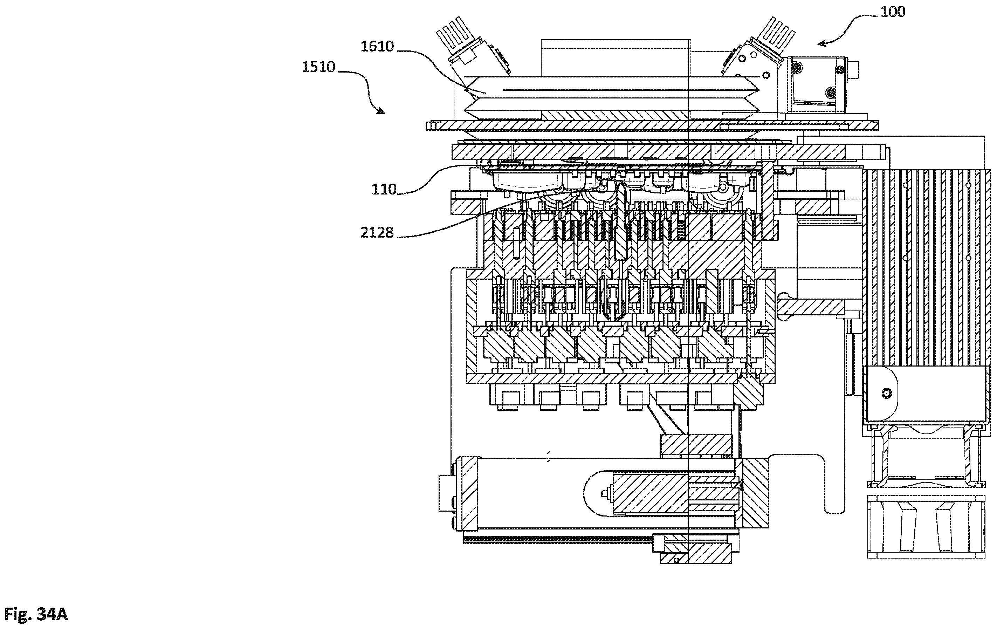

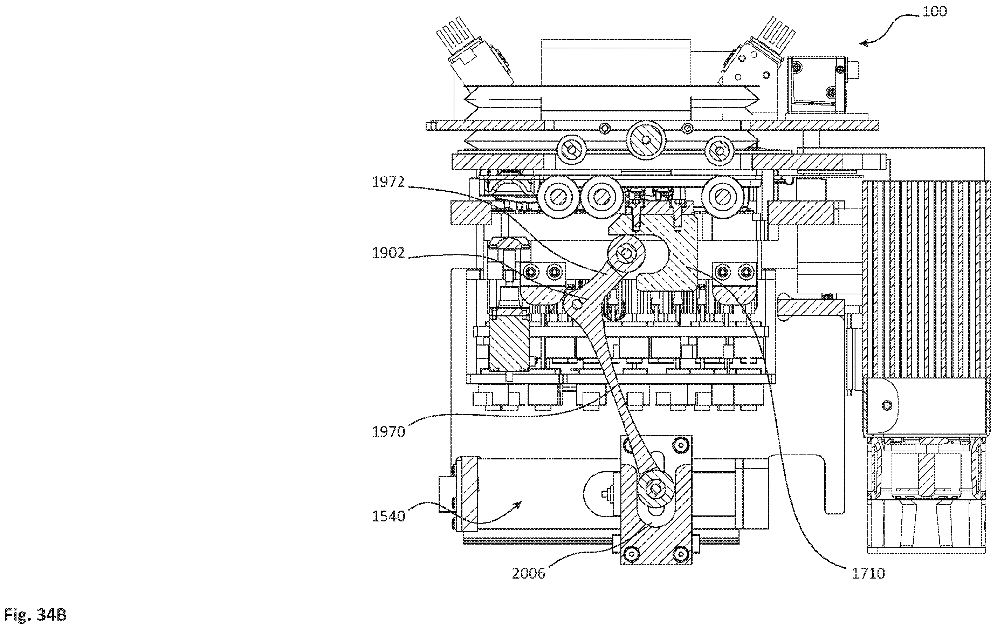

Figs. 34A & 34B are simplified respective sectional illustrations of an aspect of an initial stage of clamping of the cartridge of Figs. 2A/1 - 10D in the instrument of Figs. 11A - 29, taken along respective section lines B - B and F - F in Fig. 30;

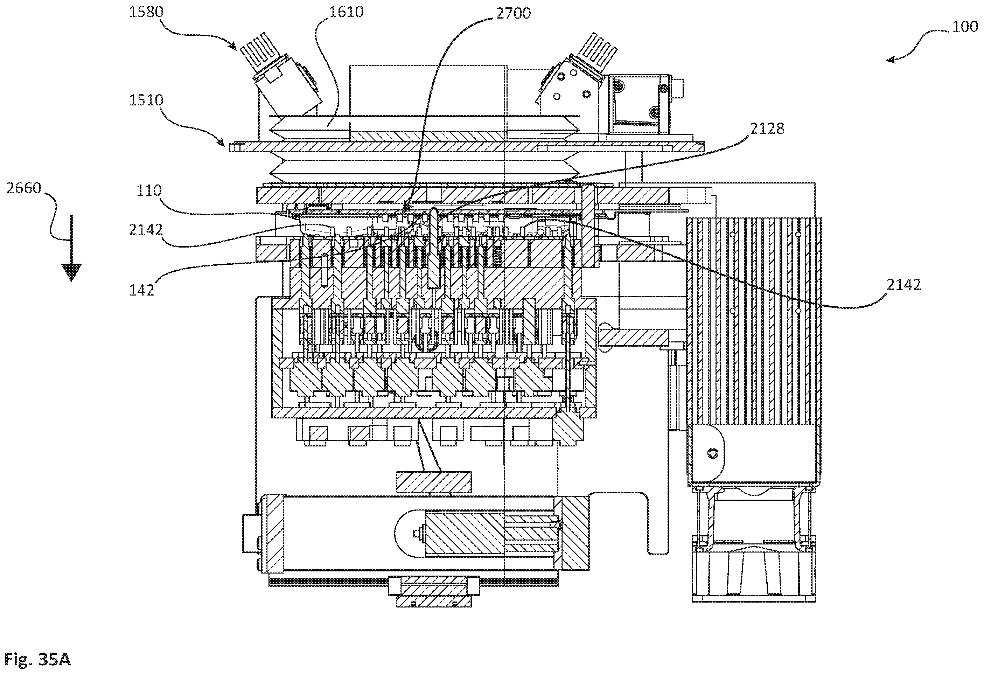

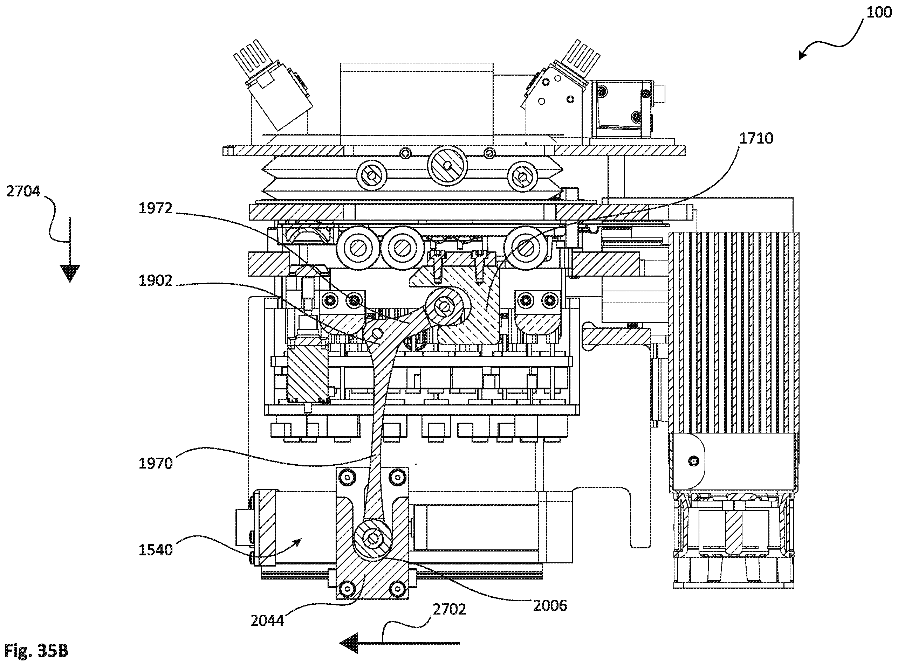

Figs. 35A & 35B are simplified sectional illustrations of an intermediate stage of clamping of the cartridge of Figs. 2A/1 - 10D in the instrument of Figs. 11A -29, taken along respective section lines B - B and F - F in Fig. 30;

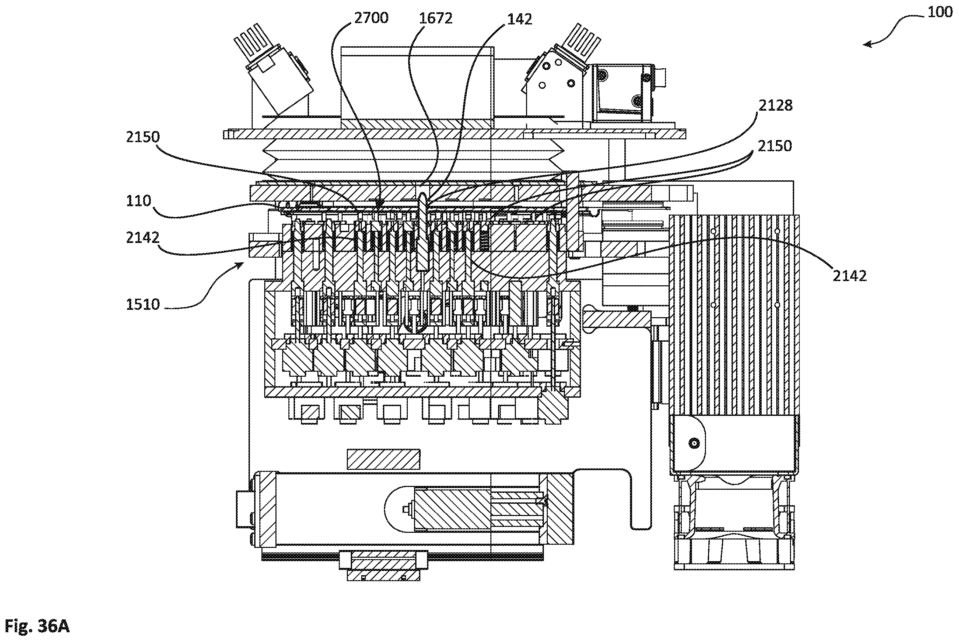

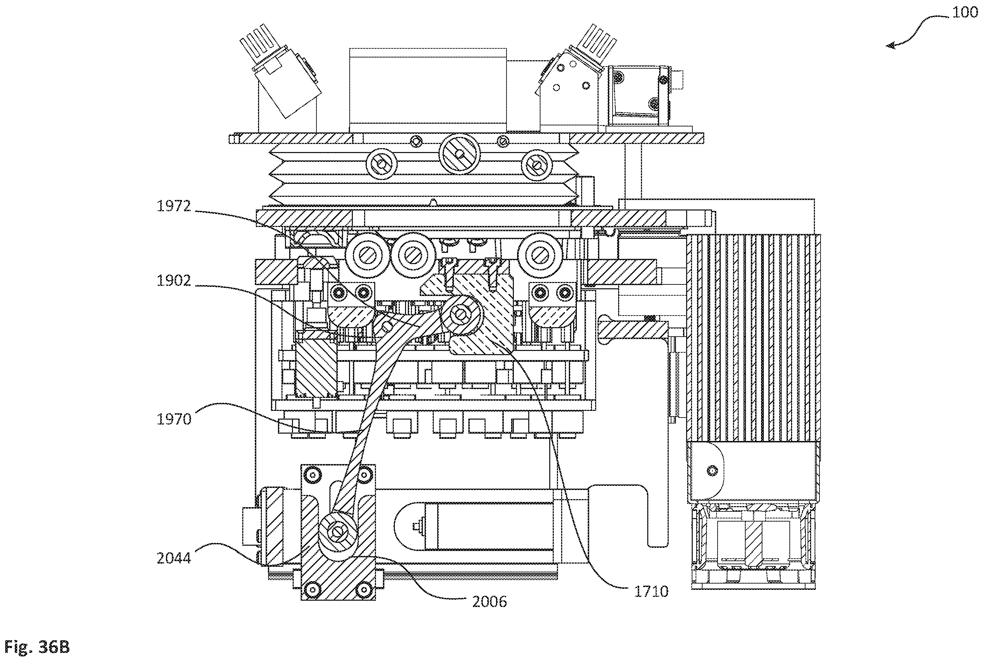

Figs. 36A & 36B are a simplified sectional illustrations of a final stage of clamping of the cartridge of Figs. 2A/1 - 10D in the instrument of Figs. 11A - 29, taken along respective section lines B - B and F - F in Fig. 30;

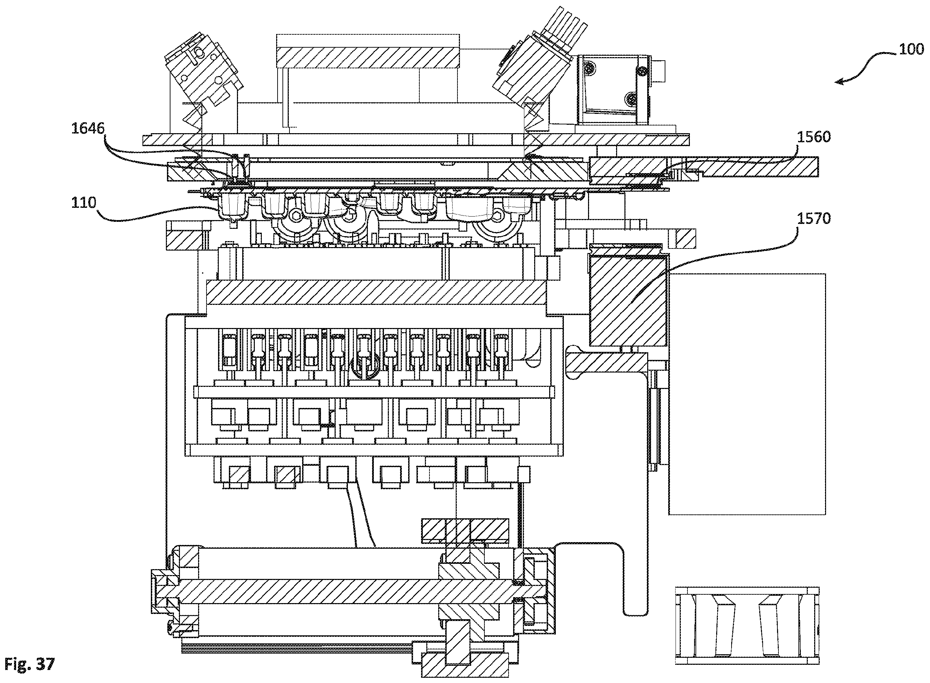

Fig. 37 is a simplified sectional illustration of another aspect of the initial stage of clamping of the cartridge of Figs. 2A/1 - 10D in the instrument of Figs. 11A -29, taken along section lines G - G in Fig. 30;

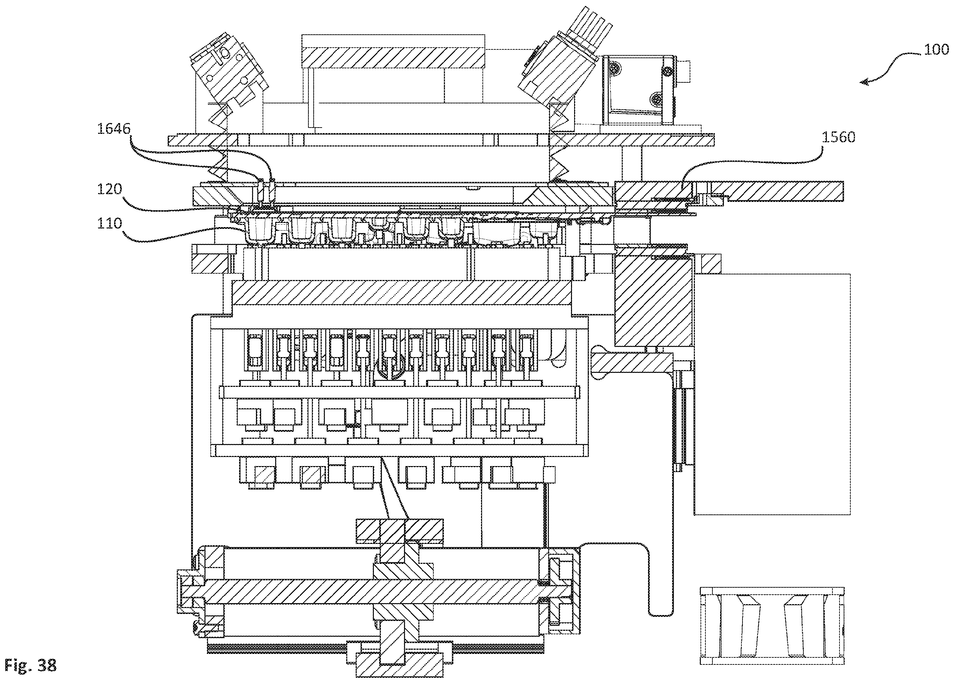

Fig. 38 is a simplified sectional illustration of another aspect of the intermediate stage of clamping of the cartridge of Figs. 2A/1 - 10D in the instrument of Figs. 11A - 29, taken along section lines G - G in Fig. 30;

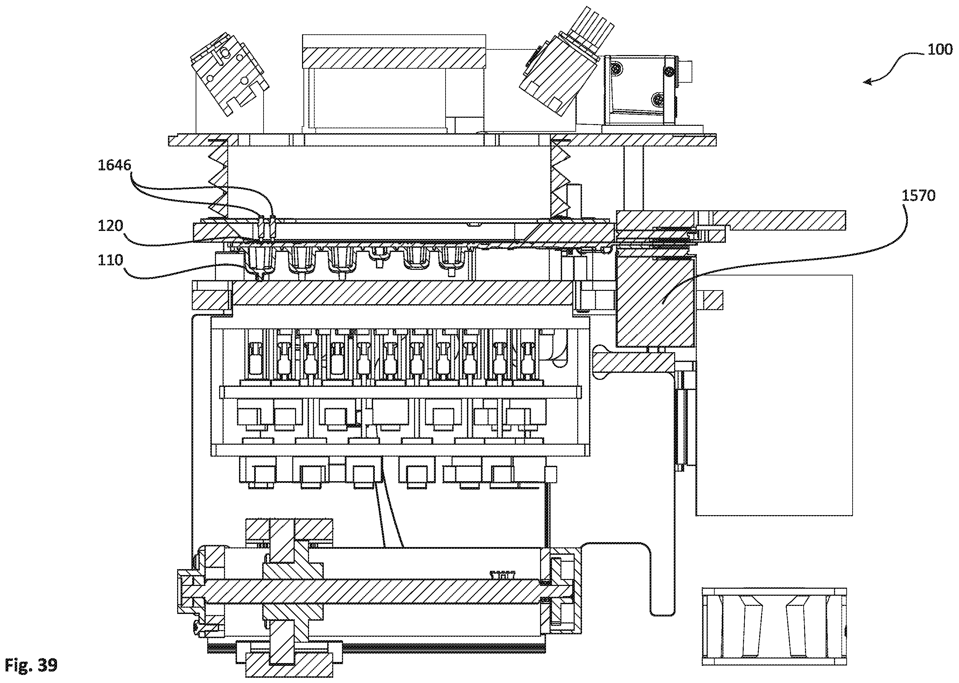

Figs. 39, 40, 41 and 42 are simplified sectional illustrations of other aspects of the final stage of clamping of the cartridge of Figs. 2A/1 - 10D in the instrument of Figs. 11A - 29, Figs. 39, 40, 41 and 42 being taken along respective section lines G - G, E - E, H - H and D - D in Fig. 30;

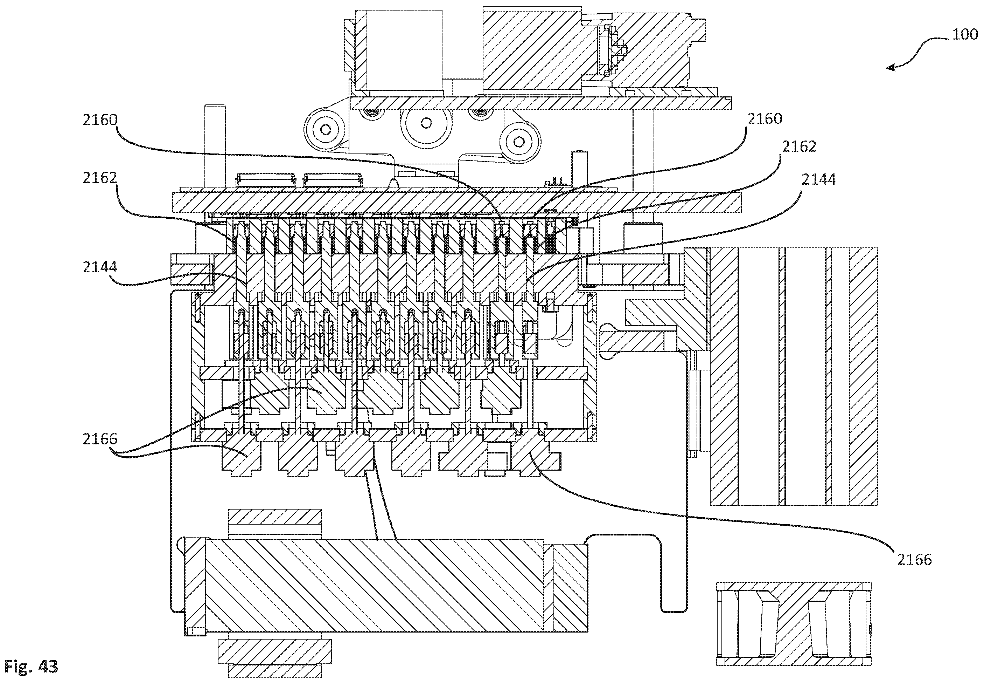

Fig. 43 is a simplified sectional illustration of valve actuators in a valve open operative orientation relative to the cartridge of Figs. 2A/1 - 10D in the instrument of Figs. 11A - 29, taken along section lines H - H in Fig. 30A;

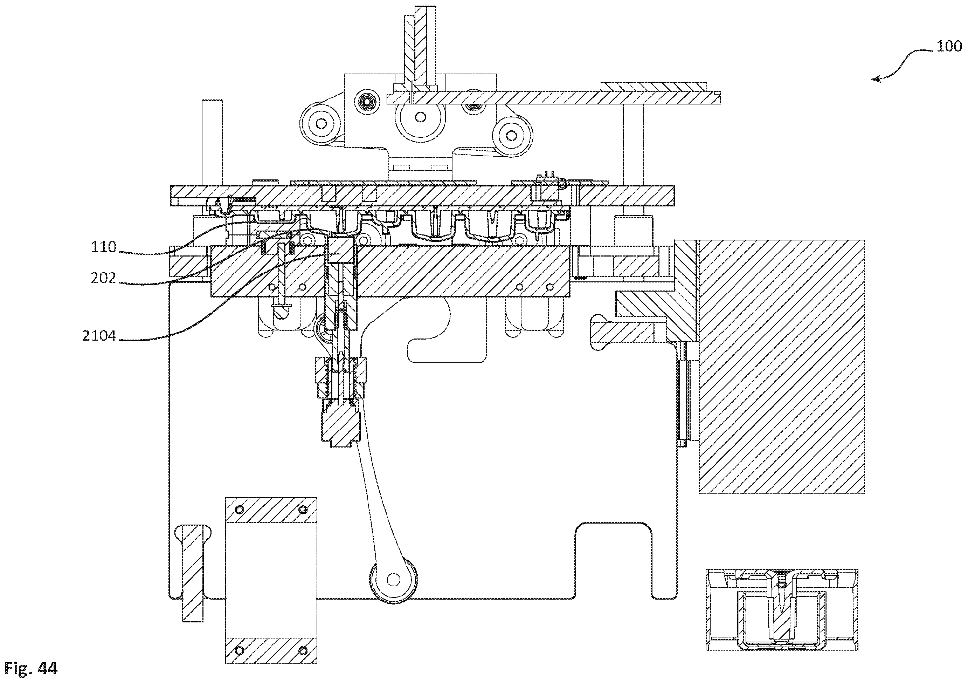

Fig. 44 is a simplified sectional illustration of an operative orientation of a magnet relative to the cartridge of Figs. 2A/1 - 10D in the instrument of Figs. 11A - 29, taken along section lines D - D in Fig. 30;

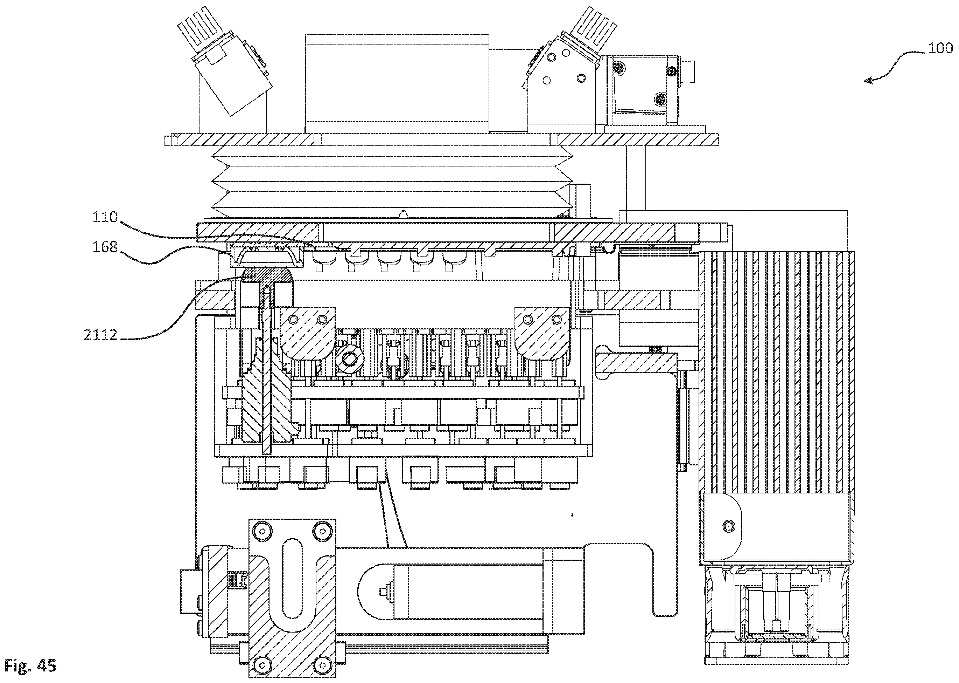

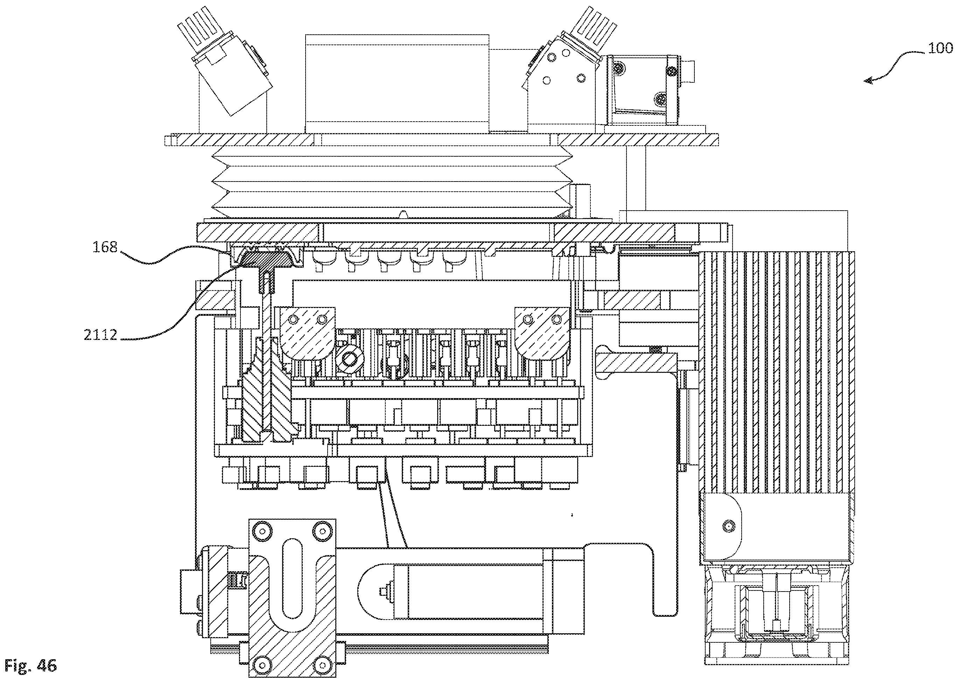

Fig. 45 and 46 are simplified sectional illustrations of respective first and second operative orientations of a pump actuator relative to the cartridge of Figs. 2A/1 -10D in the instrument of Figs. 11A - 29, both being taken along section lines E - E in Fig. 30;

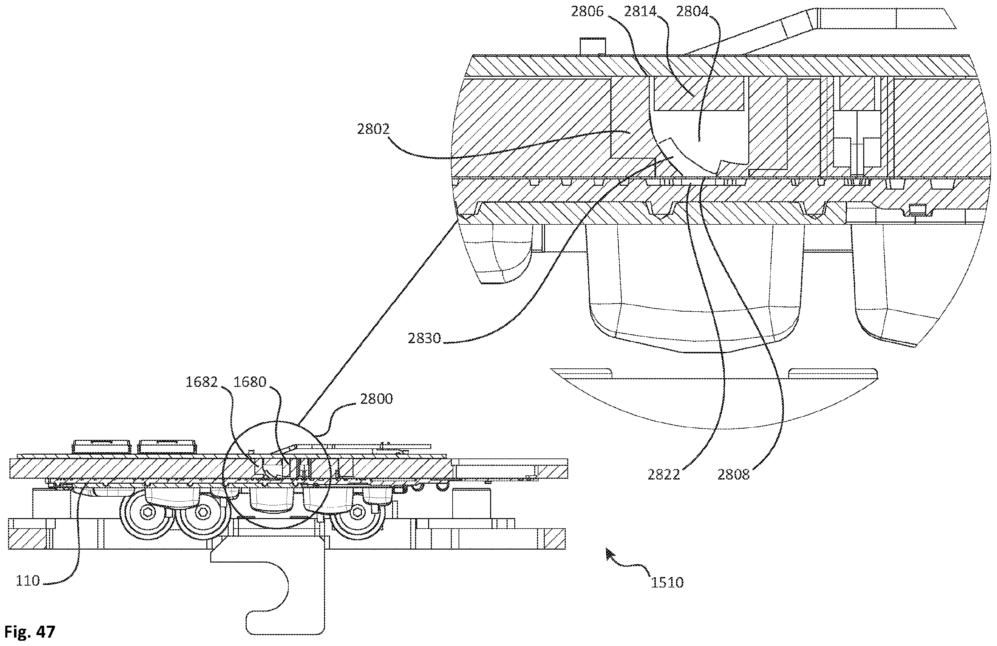

Figs. 47, 48 and 49 are simplified illustrations of the structure and operation of liquid sensors employed in the operation of the cartridge Figs. 2A/1 - 10D by the instrument of Figs. 11A - 29;
Figs. 50A/1, 50B/1, 50C/1, 50D/1, 50E/1, 50F/1, 50G/1, 50H/1, 50I/1, 50J/1, 50K/1, 50L/1, 50M/1, 50N/1, 50O/1, 50P/1, 50Q/1, 50R/1, 50S/1, 50T/1, 50U/1, 50V/1, 50W/1, 50X/1, 50Y/1, 50Z/1, 50AA/1, 50AB/1, 50AC/1, 50AD/1, 50AE/1, 50AF/1, 50AG/1, 50AH/1, 50AI/1, 50AJ/1, 50AK/1 and 50A/2, 50B/2, 50C/2, 50D/2, 50E/2, 50F/2, 50G/2, 50H/2, 50I/2, 50J/2, 50K/2, 50L/2, 50M/2, 50N/2, 50O/2, 50P/2, 50Q/2, 50R/2, 50S/2, 50T/2, 50U/2, 50V/2, 50W/2, 50X/2, 50Y/2, 50Z/2, 50AA/2, 50AB/2, 50AC/2, 50AD/2, 50AE/2, 50AF/2, 50AG/2, 50AH/2, 50AI/2, 50AJ/2, 50AK/2 are simplified respective pairs of schematic and flow illustrations of stages in the operation of the cartridge of Figs. 2A/1 - 10D by the instrument of Figs. 11A - 49;

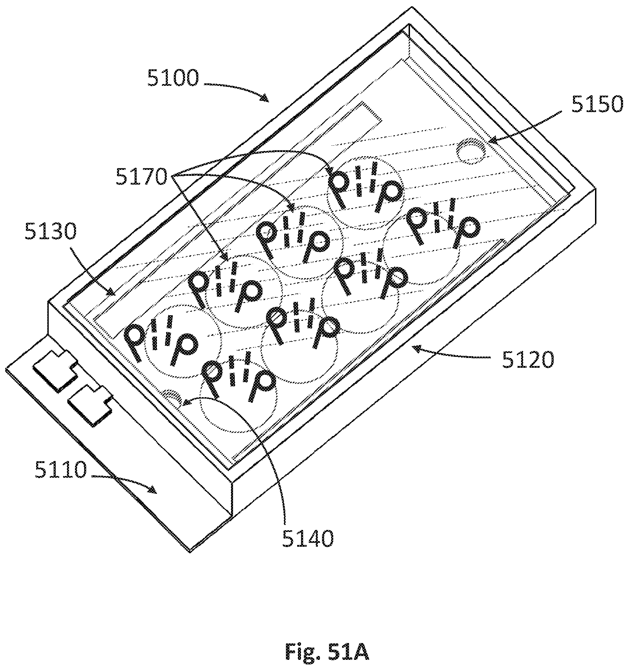

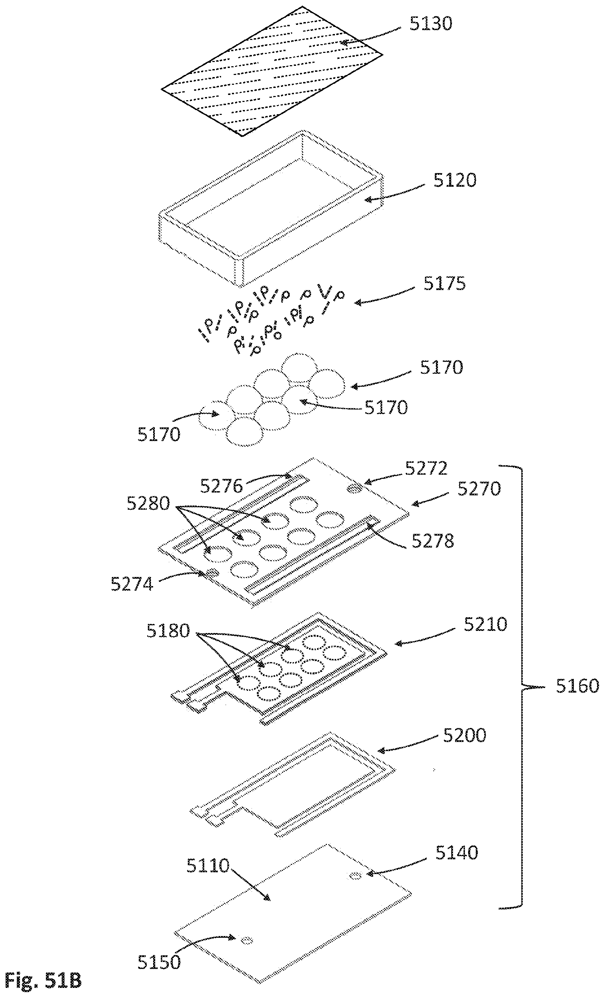

Figs. 51A and 51B are simplified pictorial assembled and exploded view illustrations of an electrophoretic array assembly constructed and operative in accordance with a preferred arrangement of the disclosure including a multiplicity of immobilized, mutually spaced and mutually electrically separated microgel regions during rollingcircle amplification (RCA) operation;

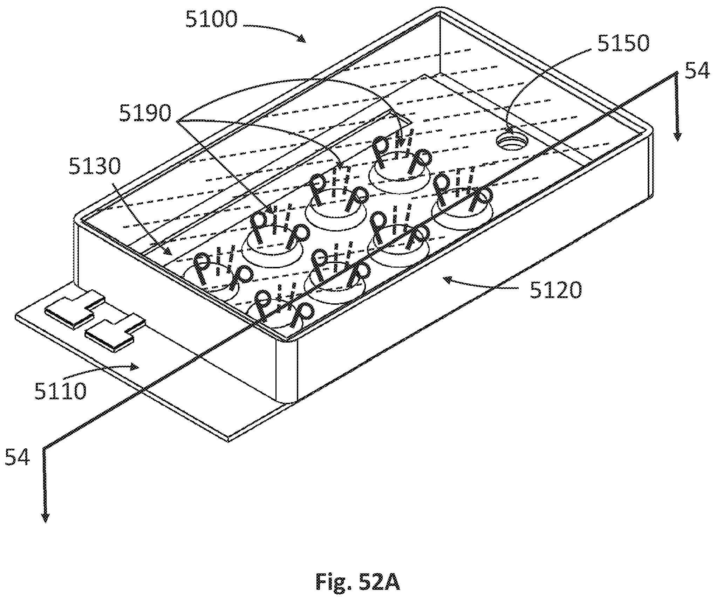

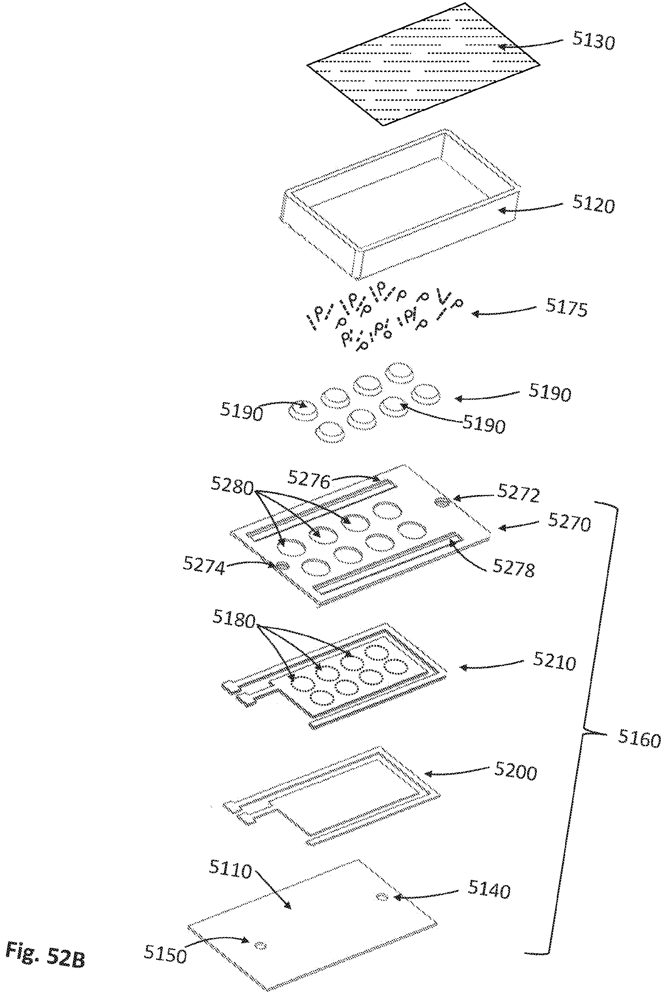

Figs. 52A and 52B are simplified pictorial assembled and exploded view illustrations of the electrophoretic array assembly of Figs. 51A and 51B including a multiplicity of immobilized, mutually spaced and mutually electrically separated microgel regions in a dehydrated storage operative orientation;

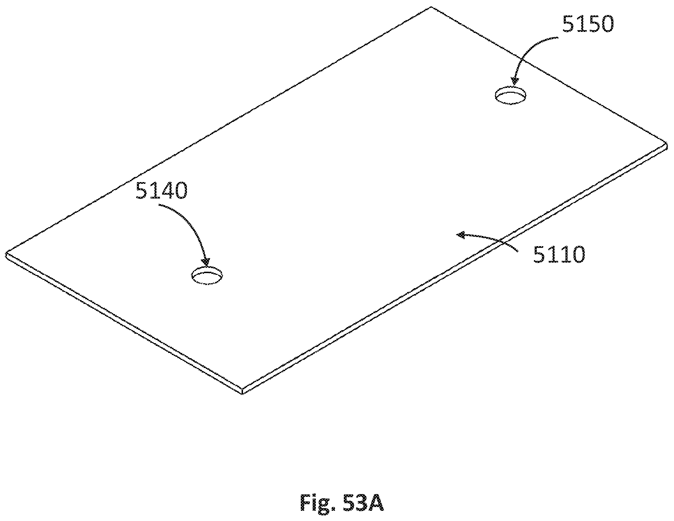

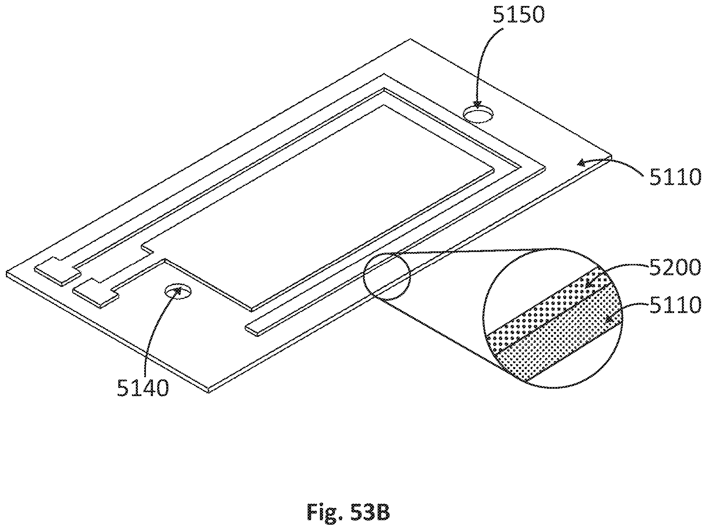

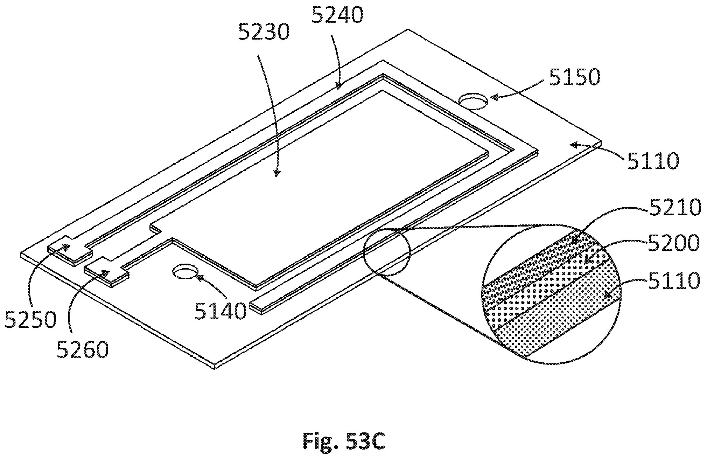

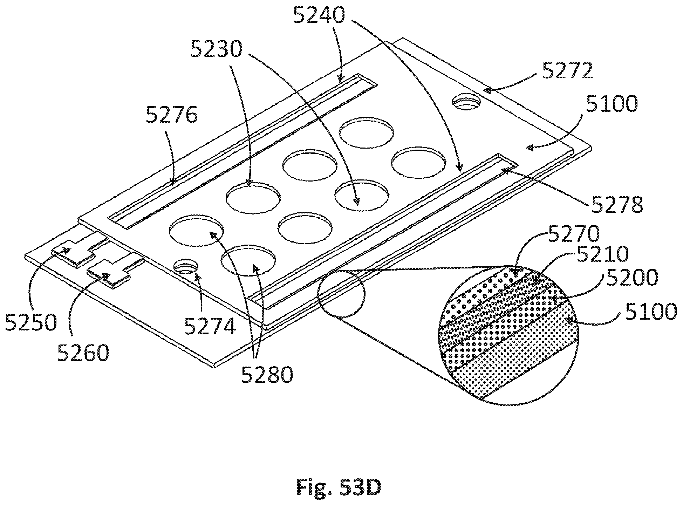

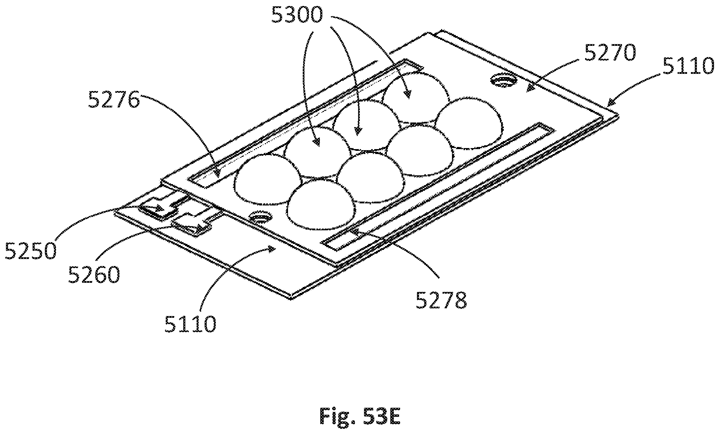

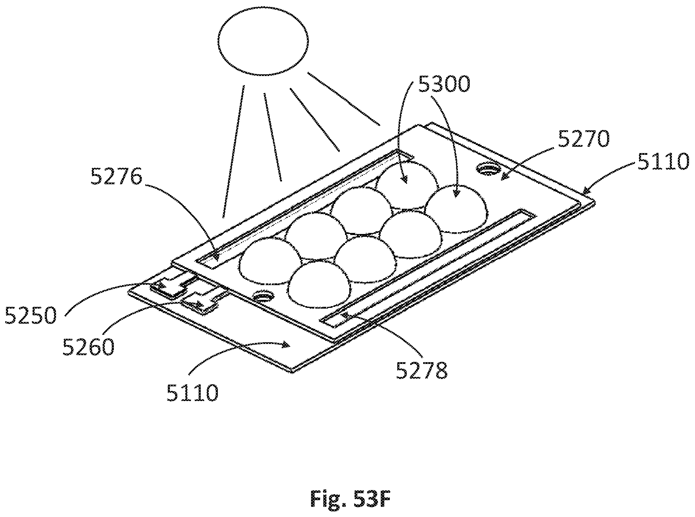

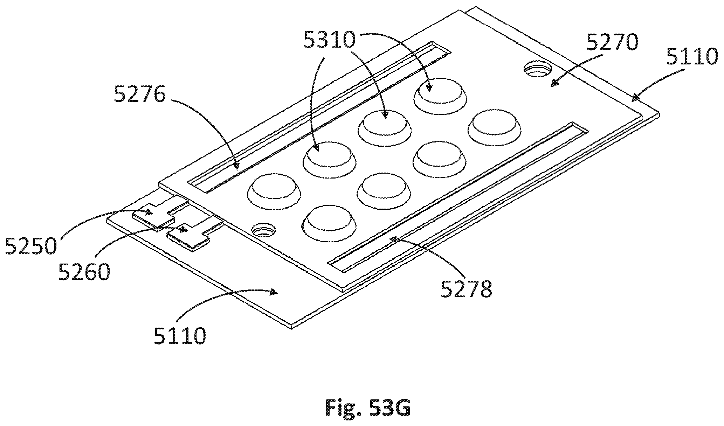

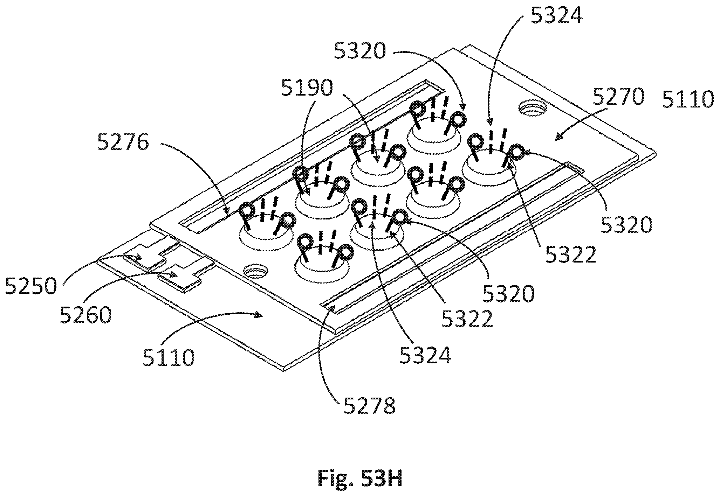

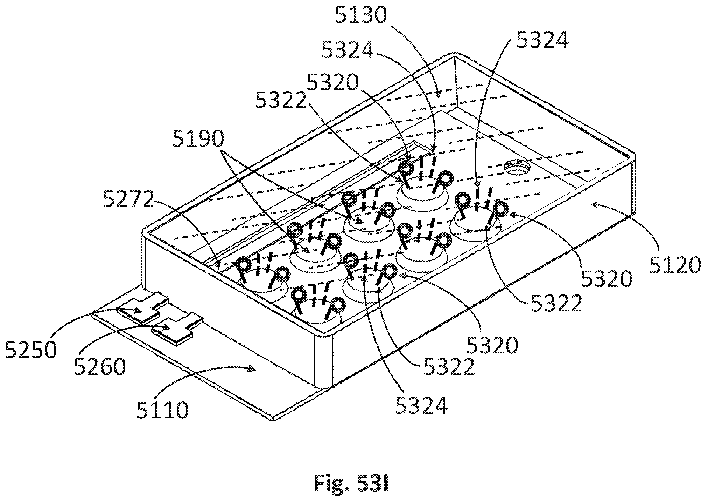

Figs. 53A, 53B, 53C, 53D, 53E, 53F, 53G, 53H and 53I are together a simplified illustration of a preferred method of producing the electrophoretic array employed in the arrangement of Figs. 51A - 52B;

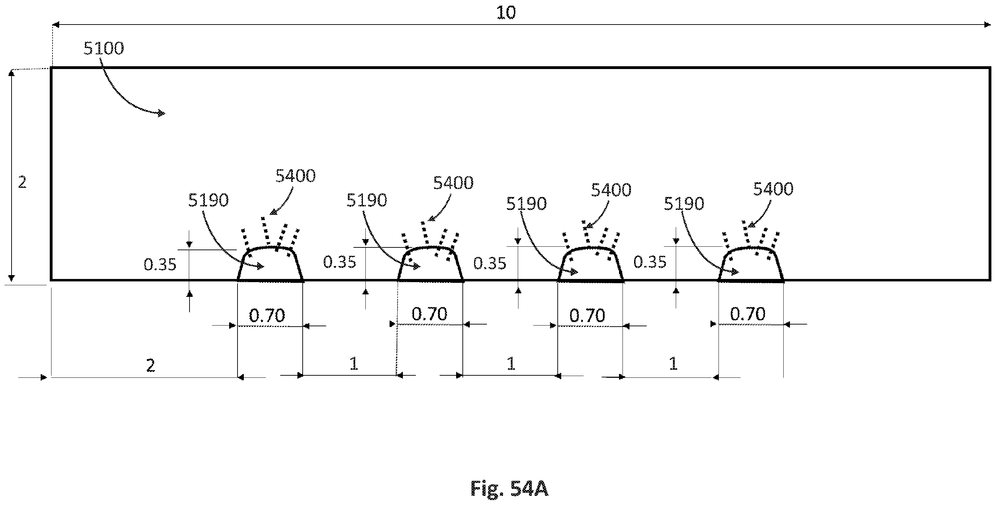

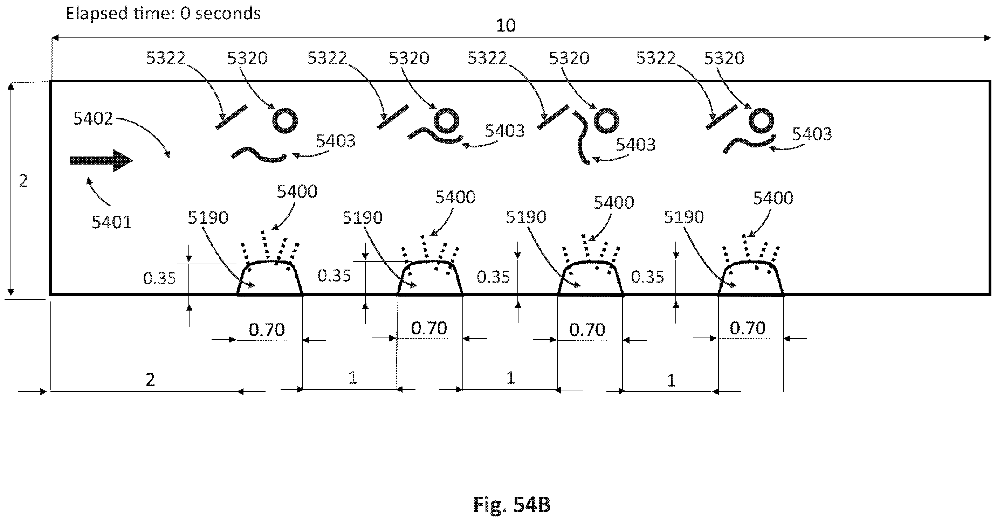

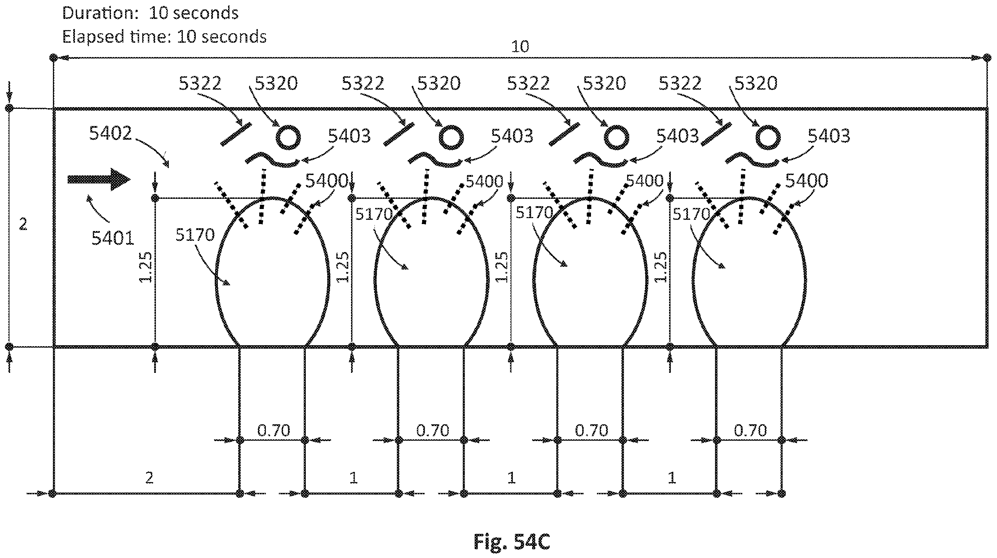

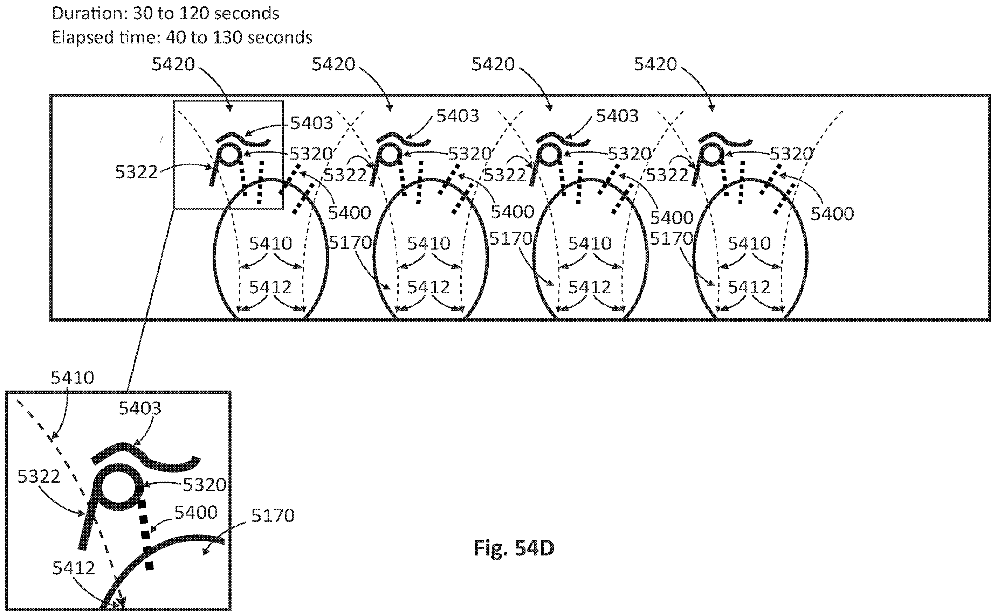

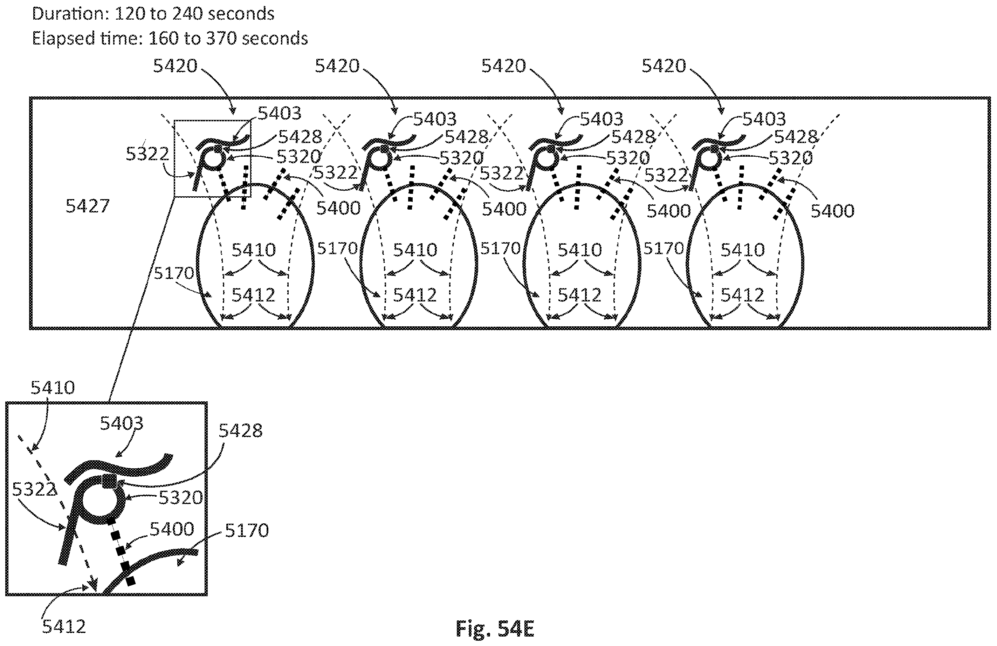

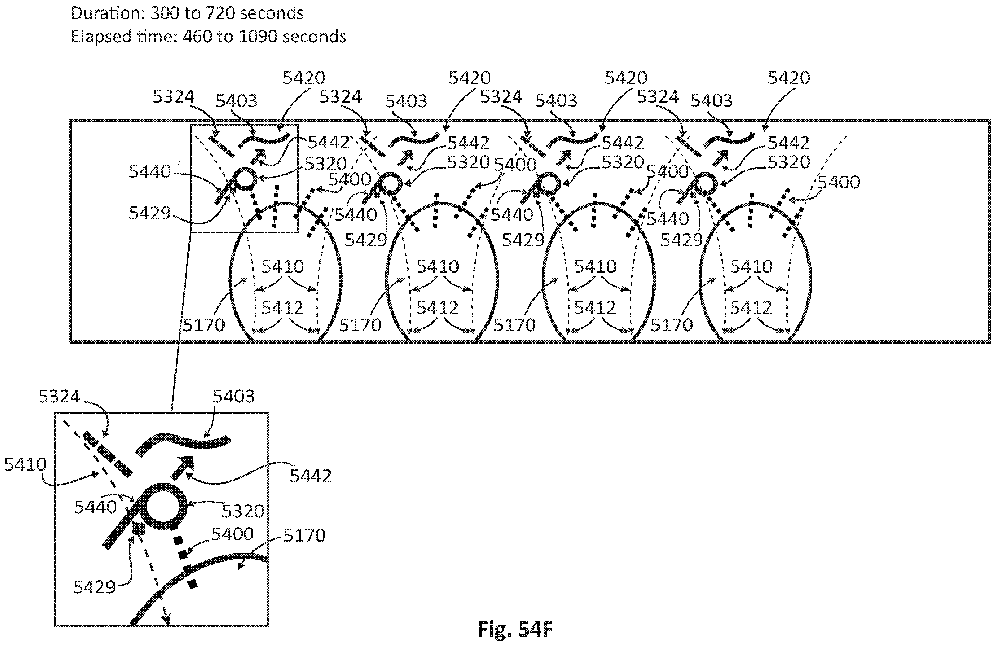

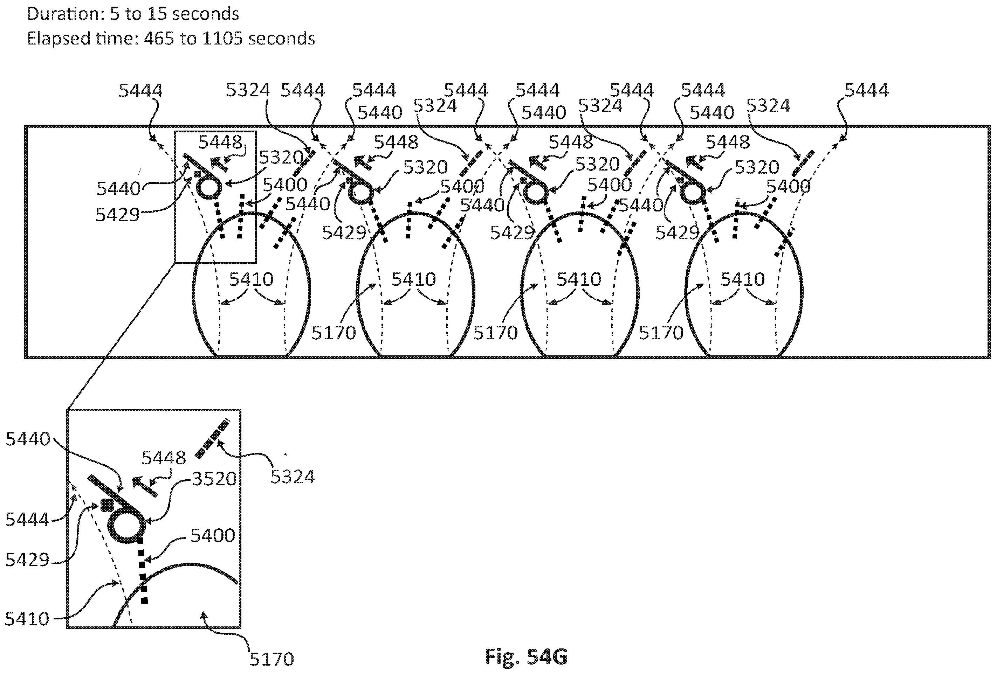

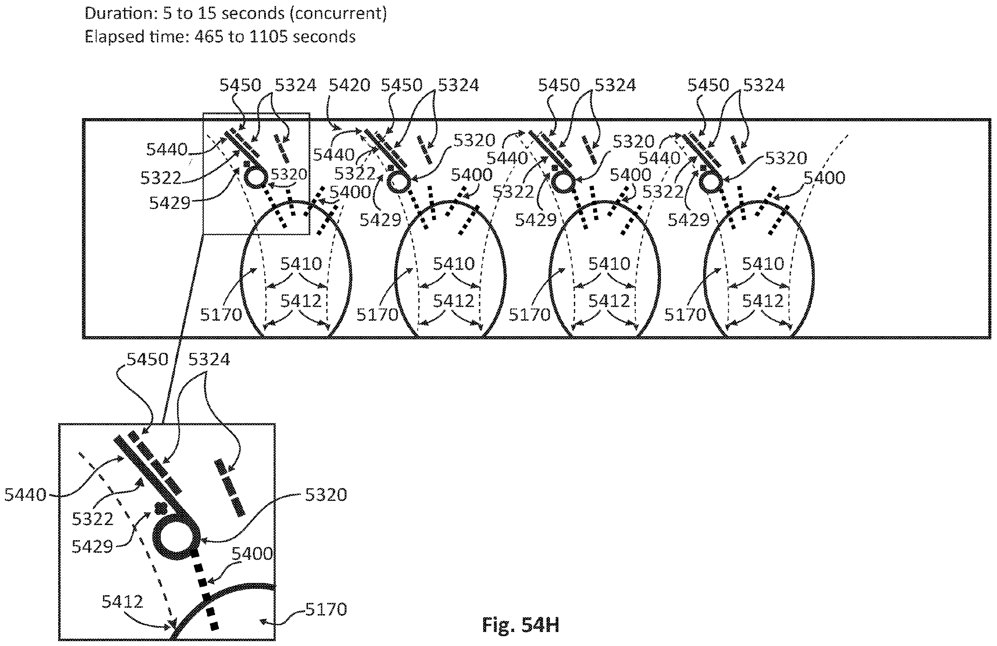

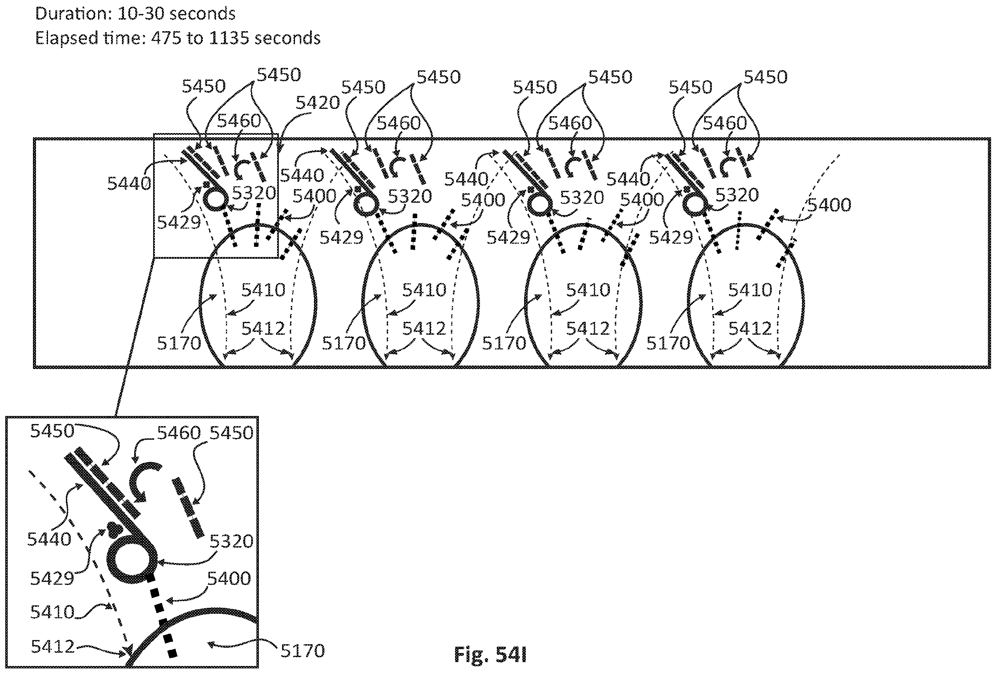

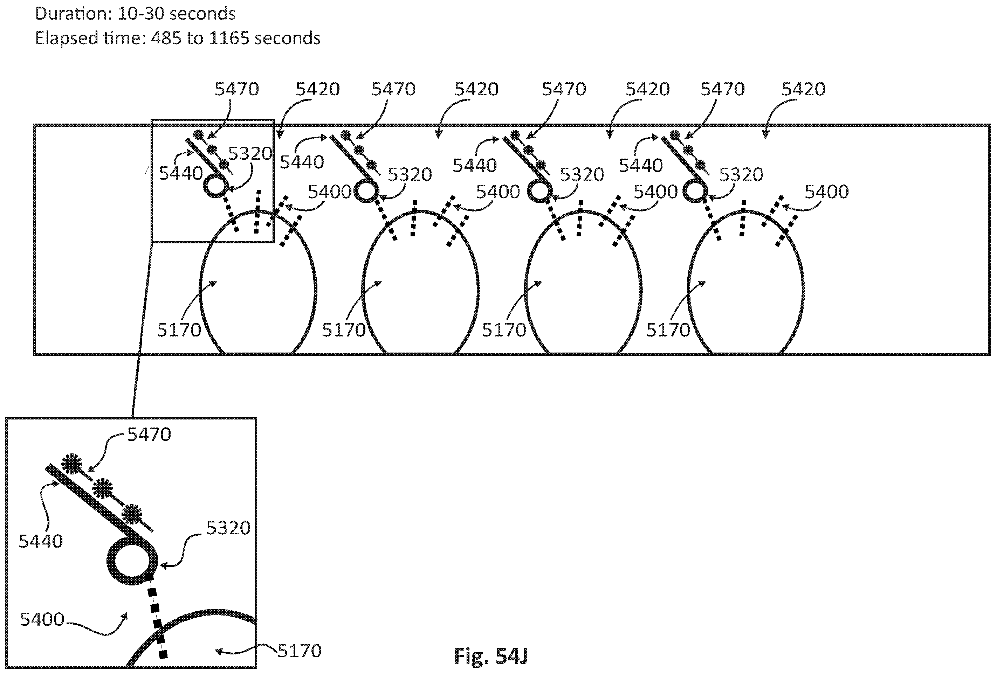

Figs. 54A, 54B, 54C, 54D, 54E, 54F, 54G, 54H, 54I and 54J are simplified illustrations of typical steps in rapid detection of the presence of at least one nucleic acid target molecule in accordance with one arrangement of the present disclosure;

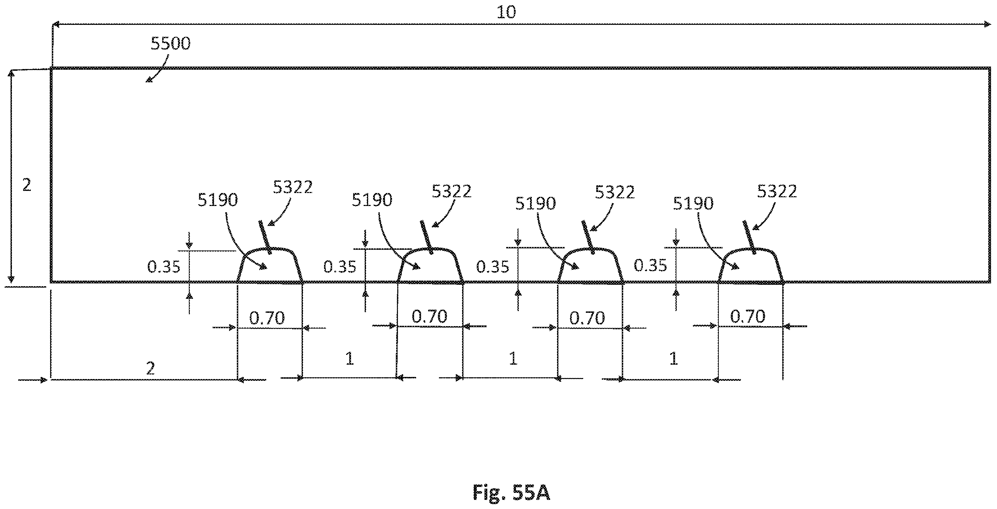

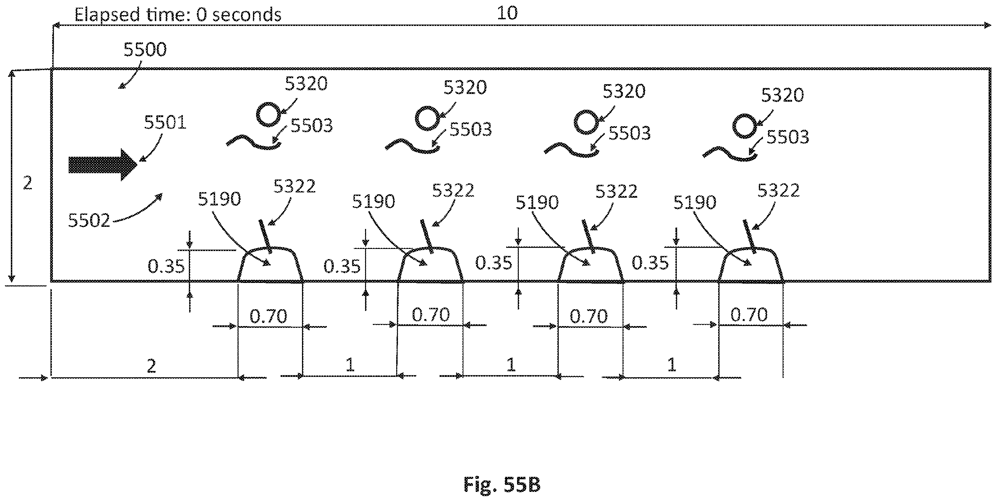

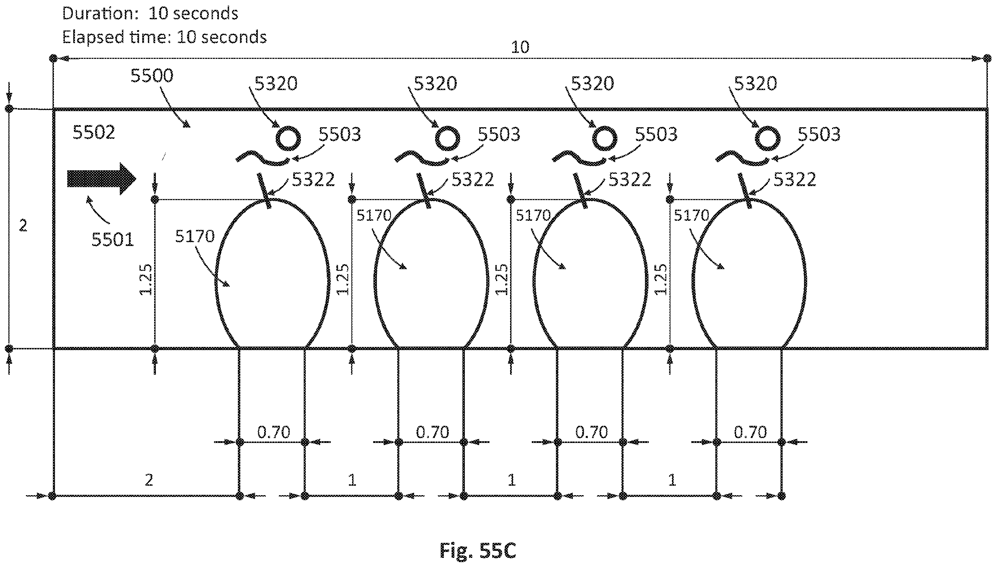

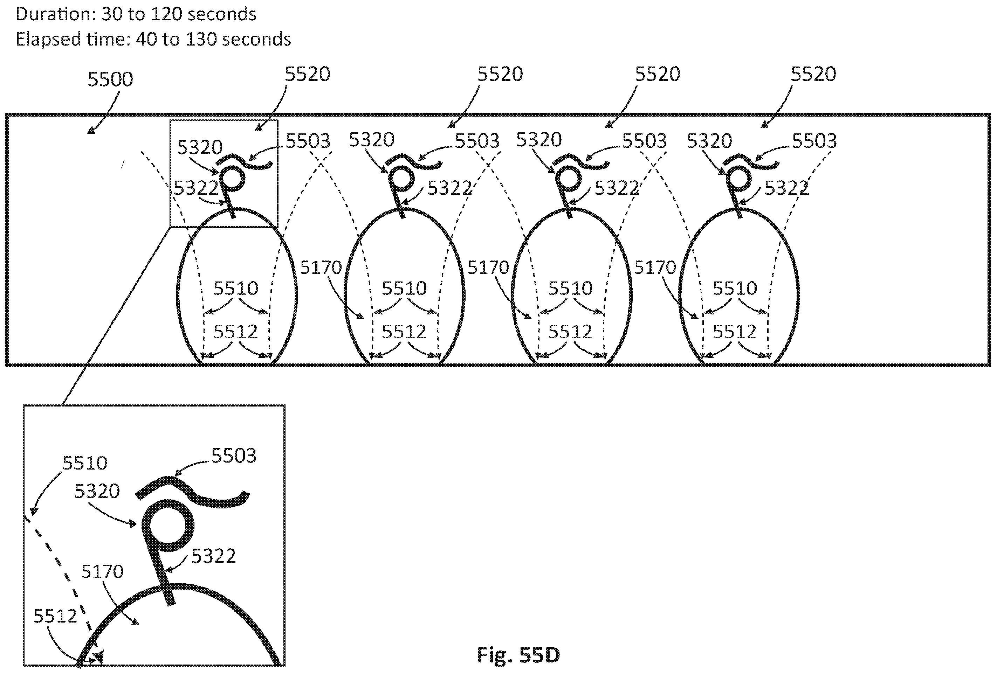

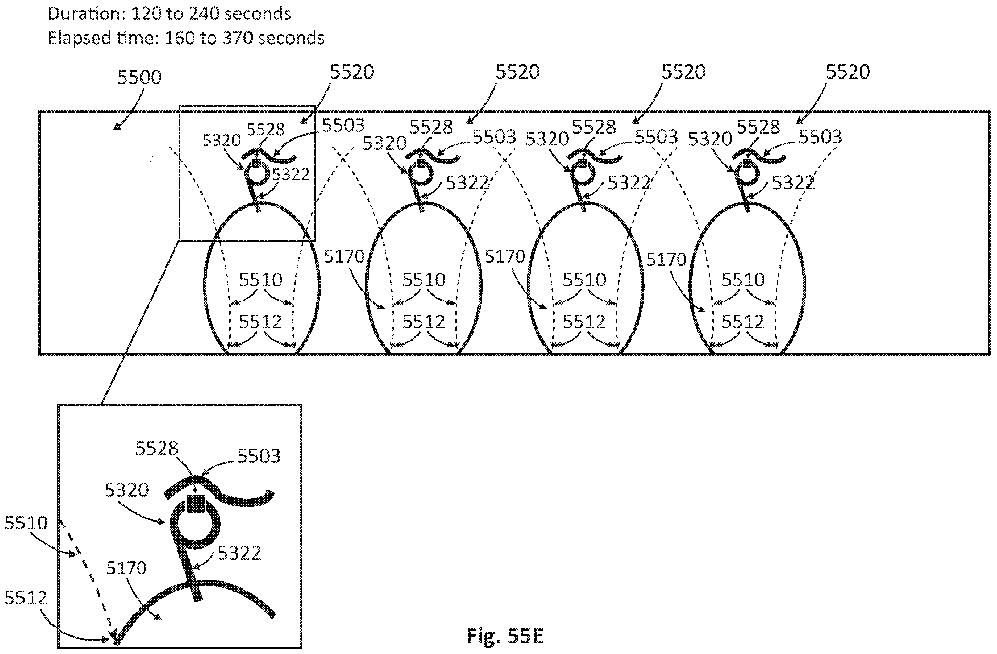

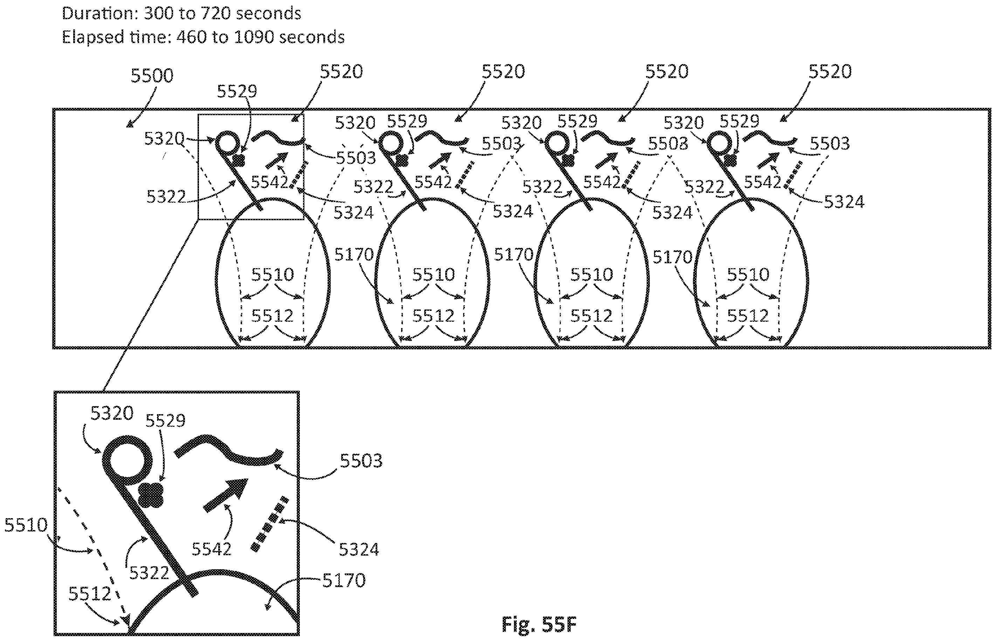

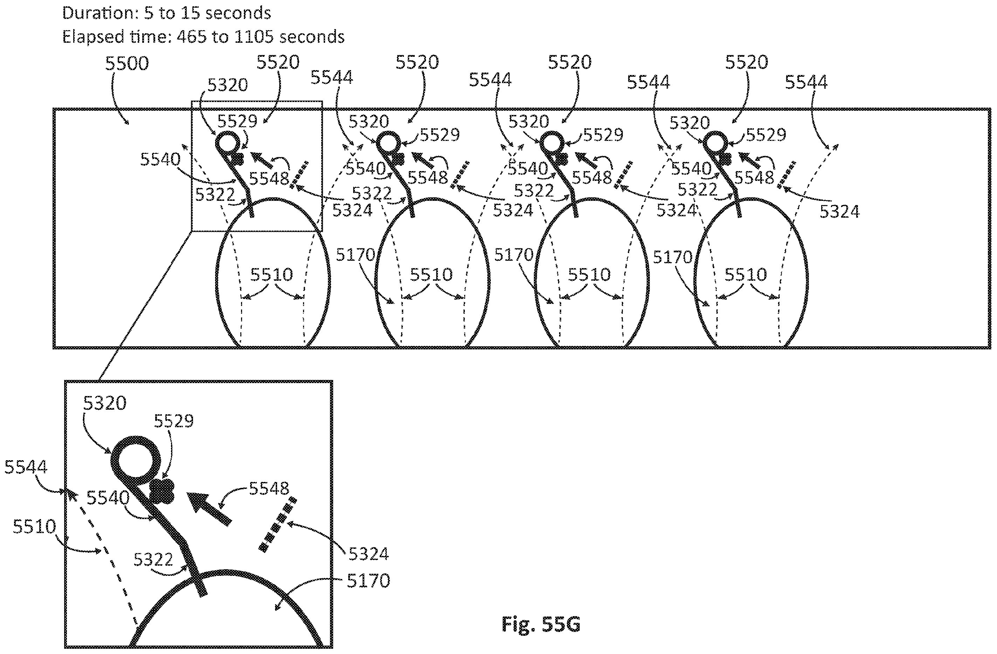

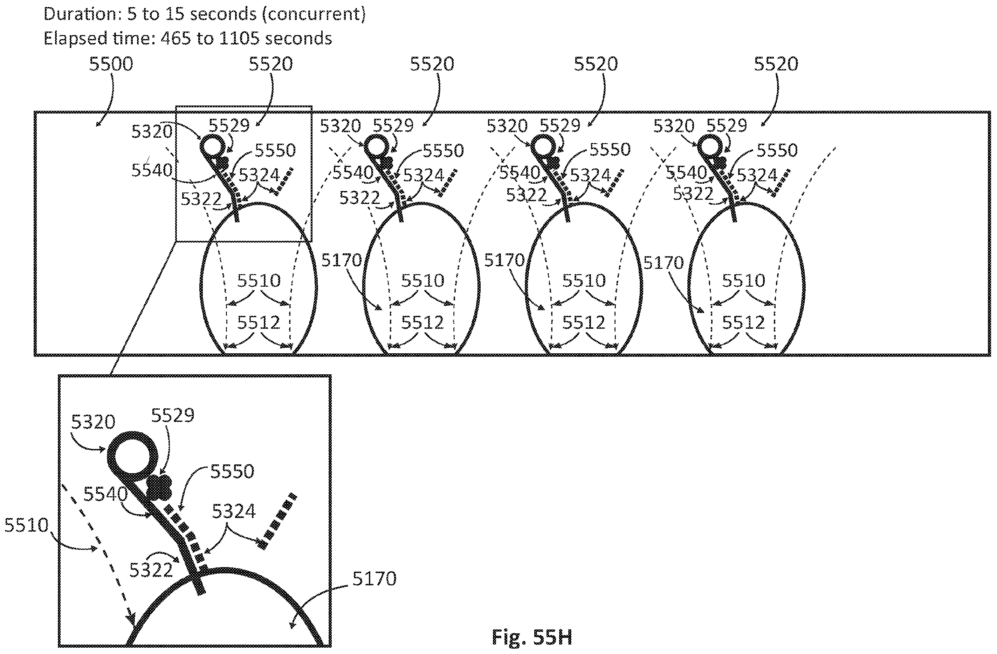

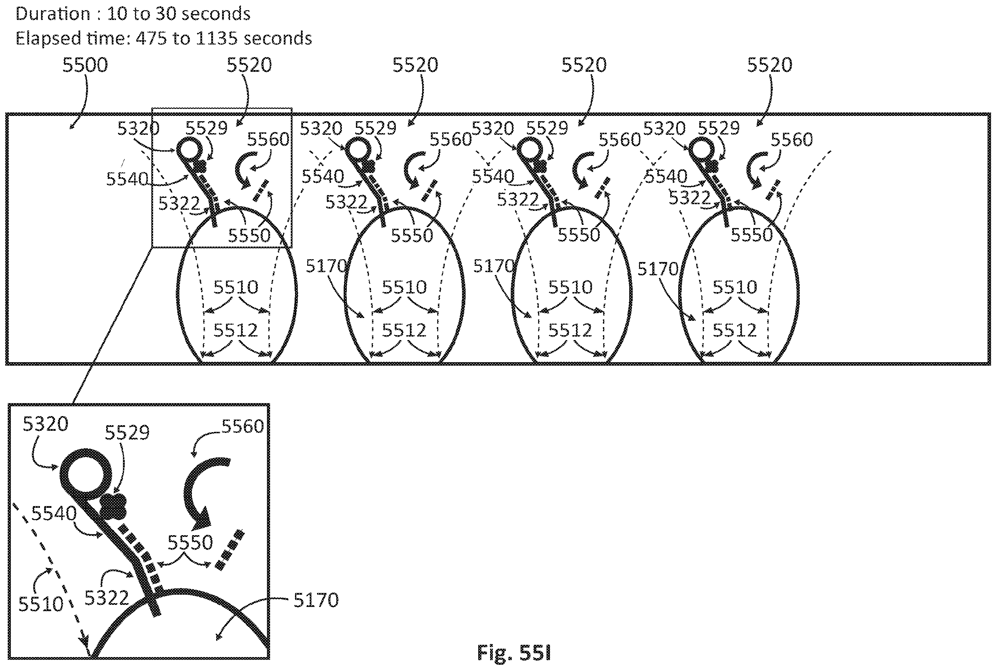

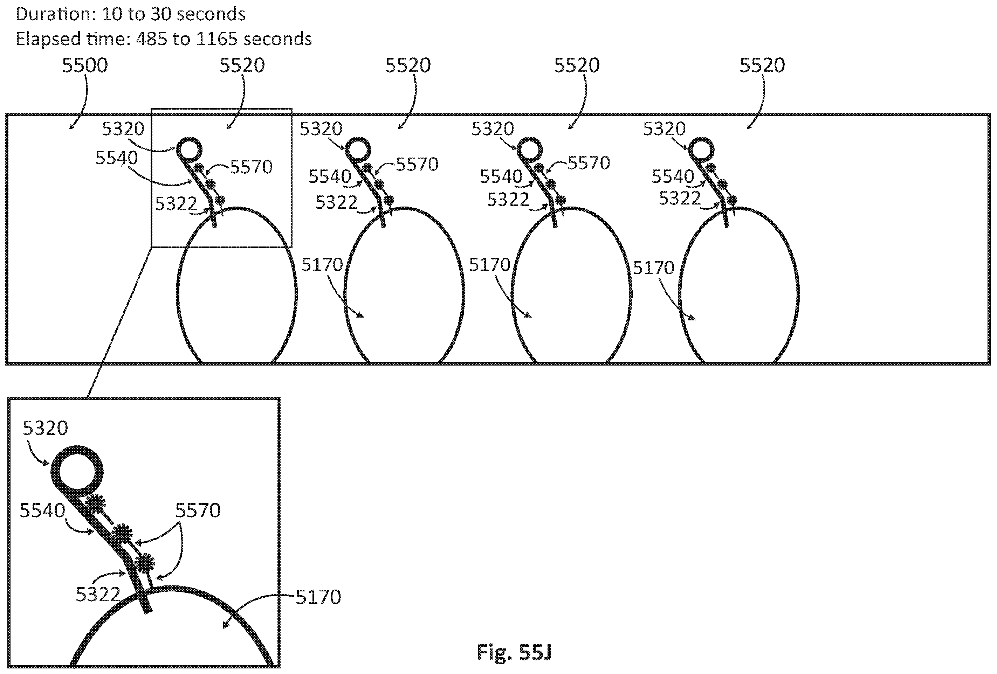

Figs. 55A, 55B, 55C, 55D, 55E, 55F, 55G, 55H, 55I and 55J are simplified illustrations of typical steps in rapid detection of the presence of at least one nucleic acid target molecule in accordance with one arrangement of the present disclosure;

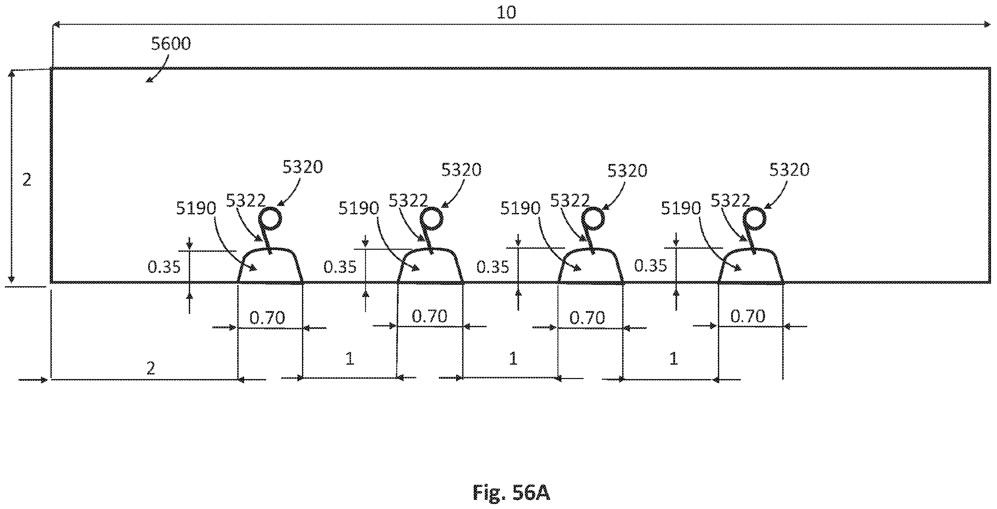

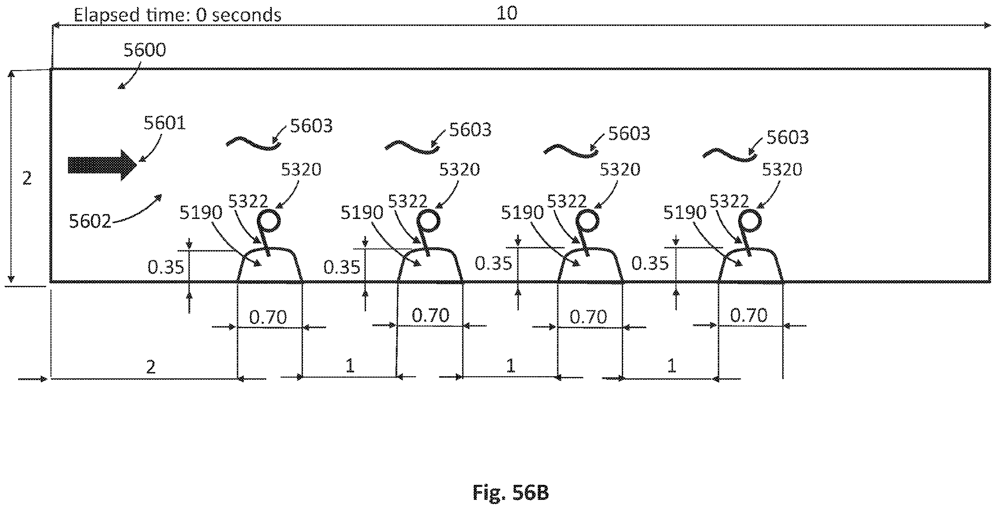

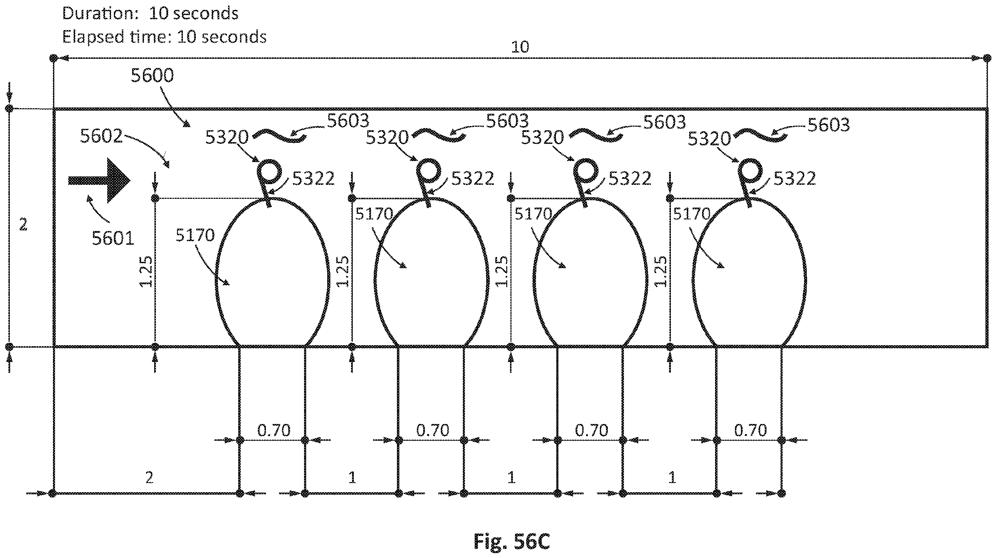

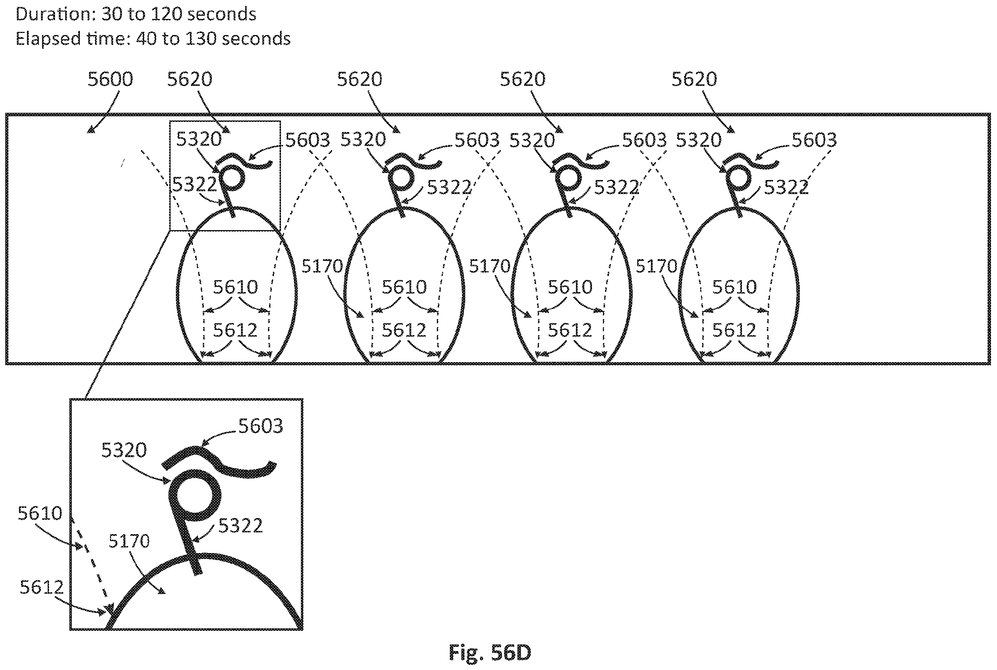

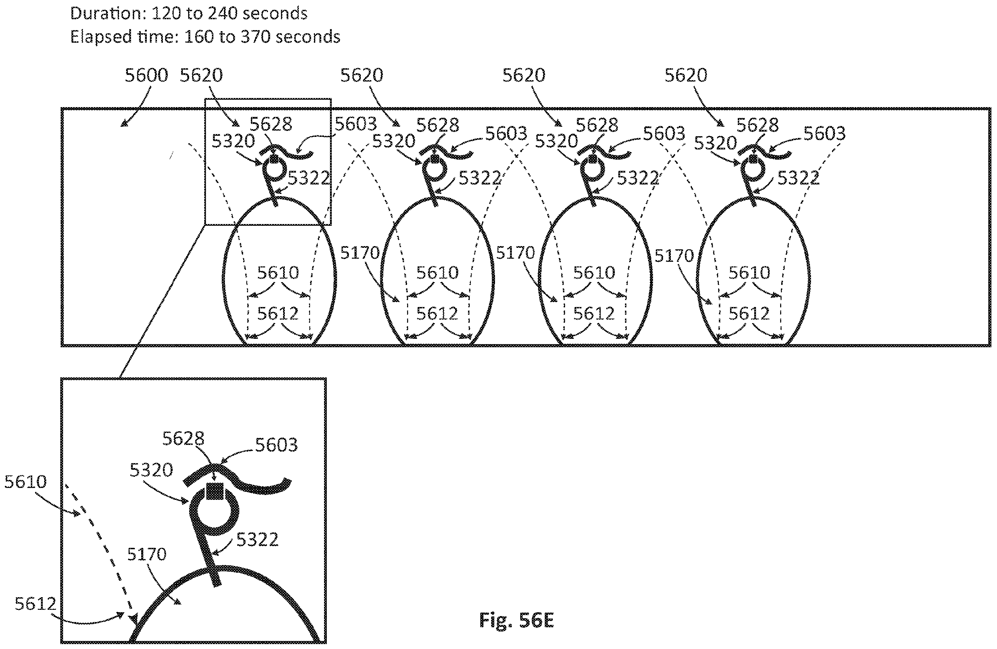

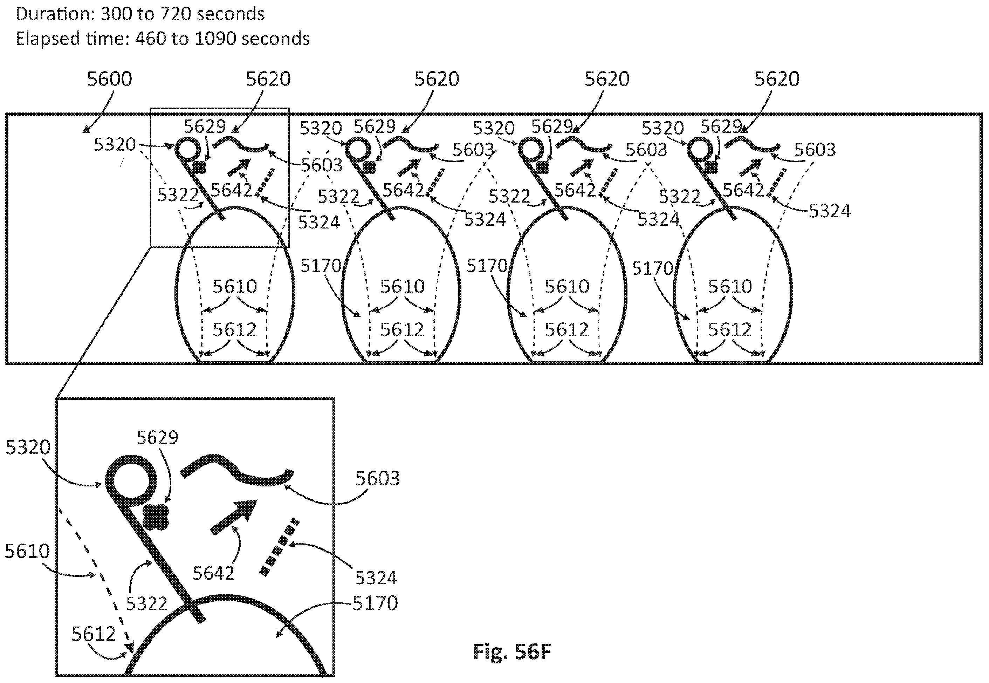

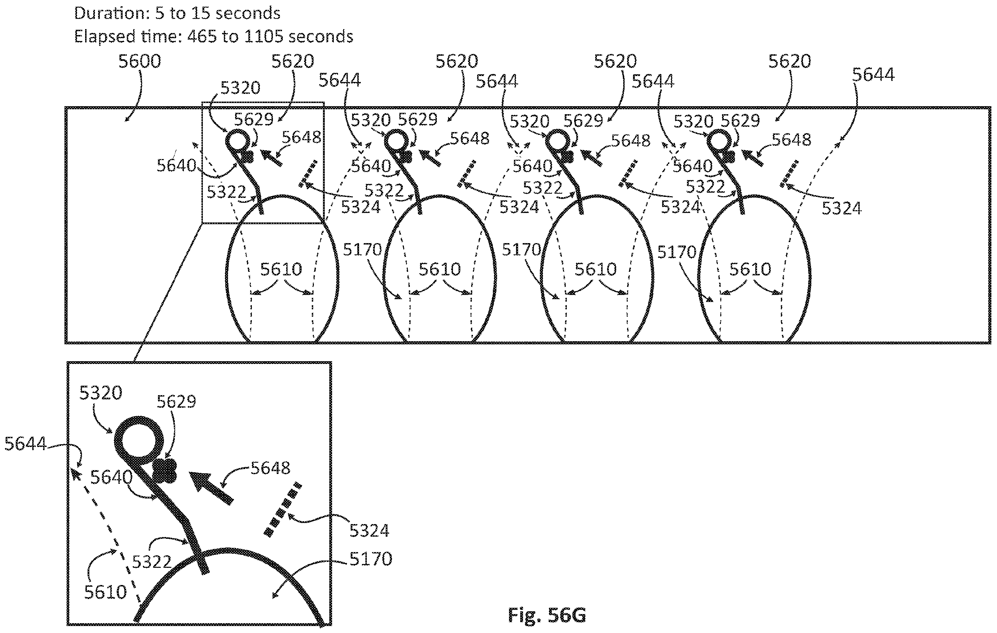

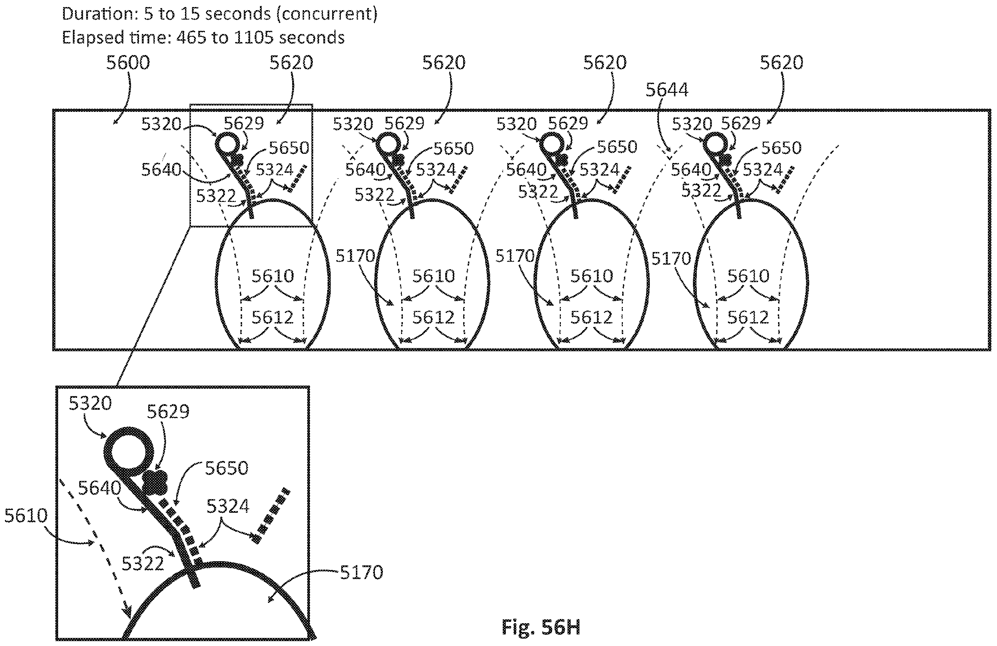

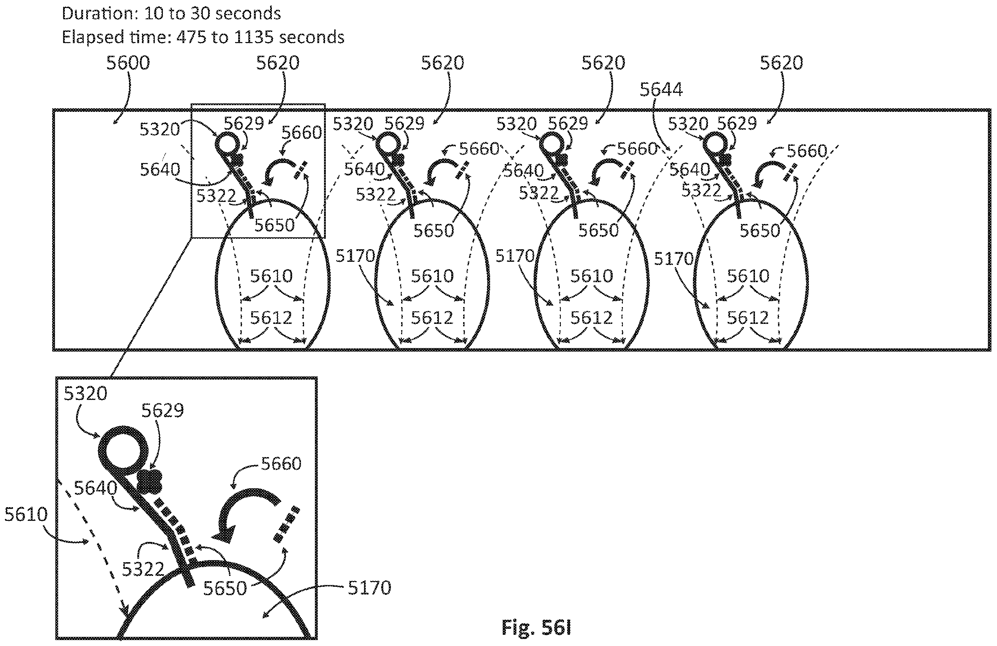

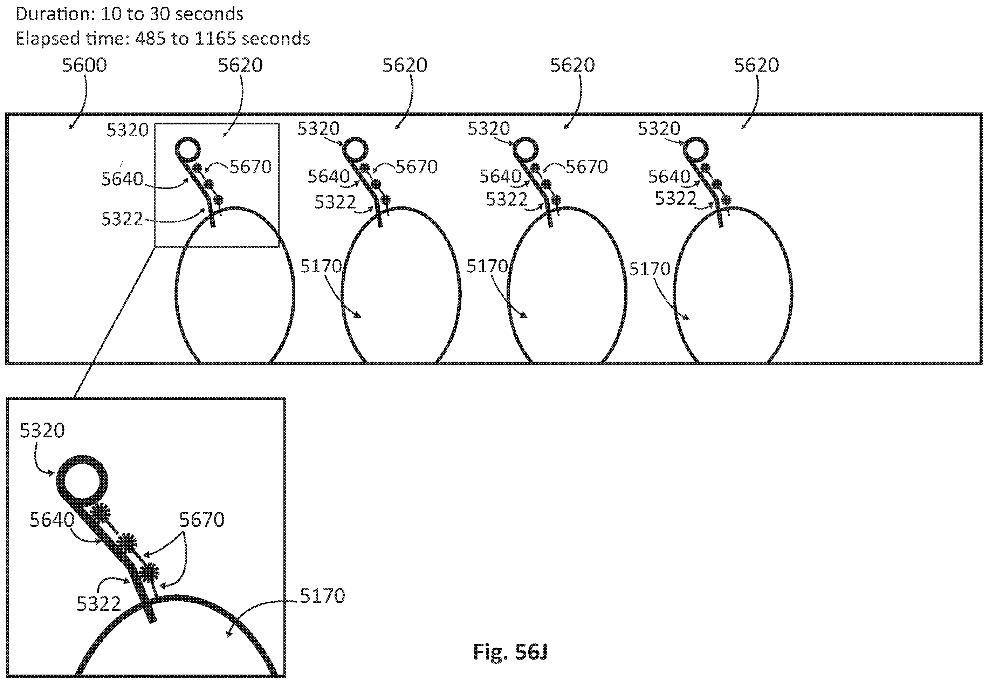

Figs. 56A, 56B, 56C, 56D, 56E, 56F, 56G, 56H, 56I and 56J are simplified illustrations of typical steps in rapid detection of the presence of at least one nucleic acid target molecule in accordance with one arrangement of the present disclosure;

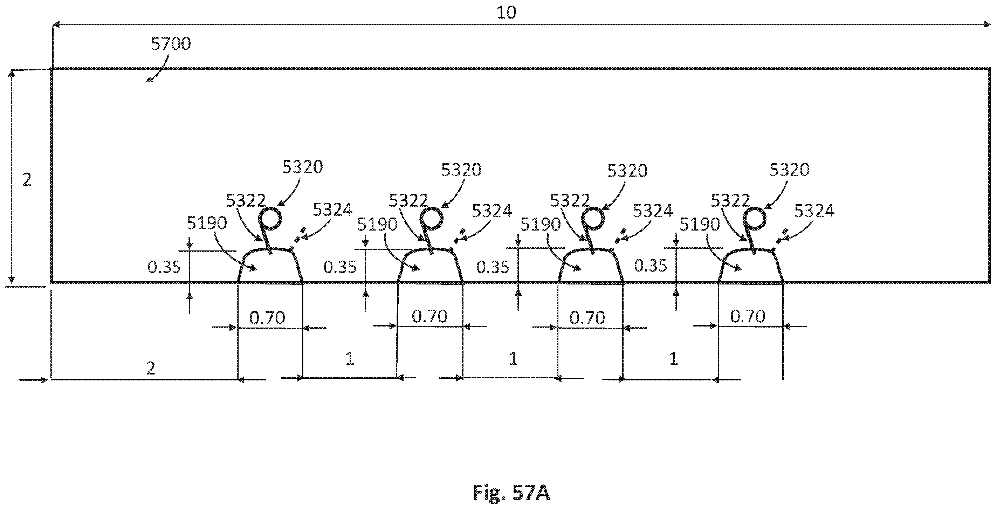

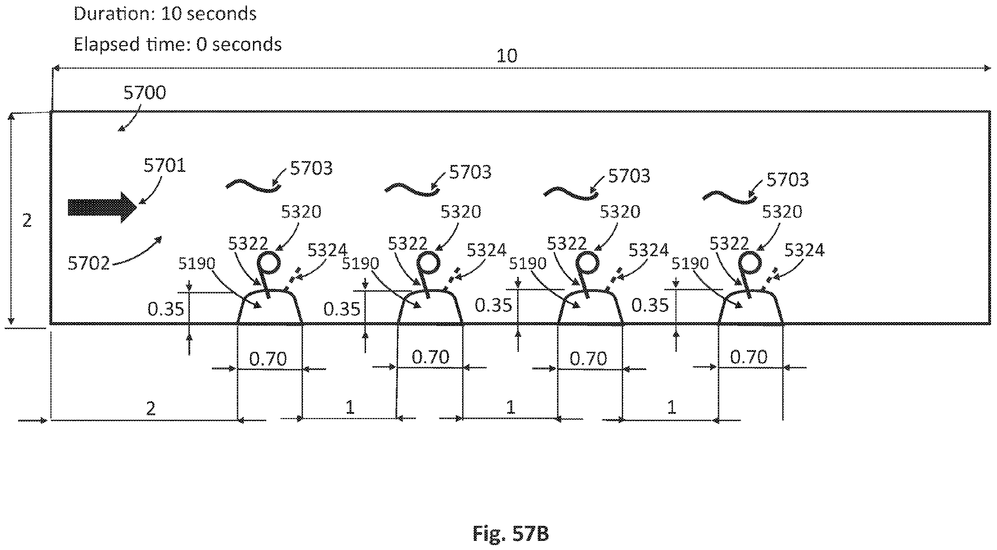

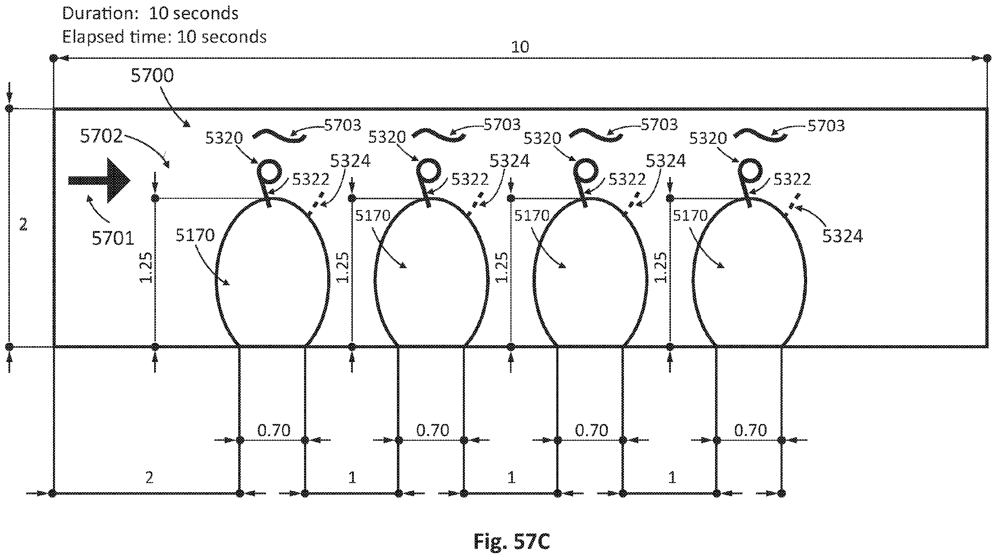

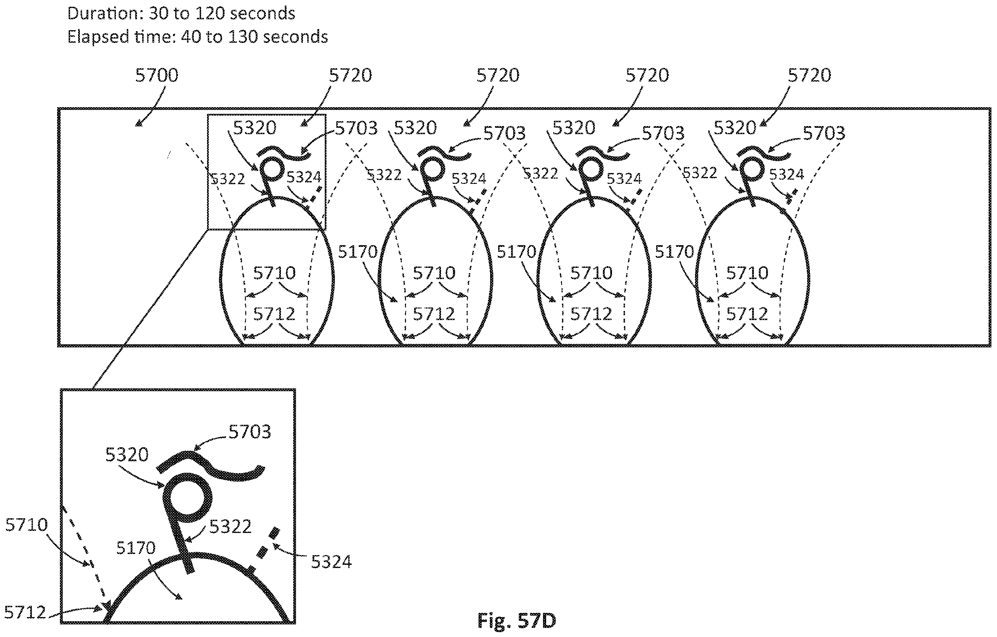

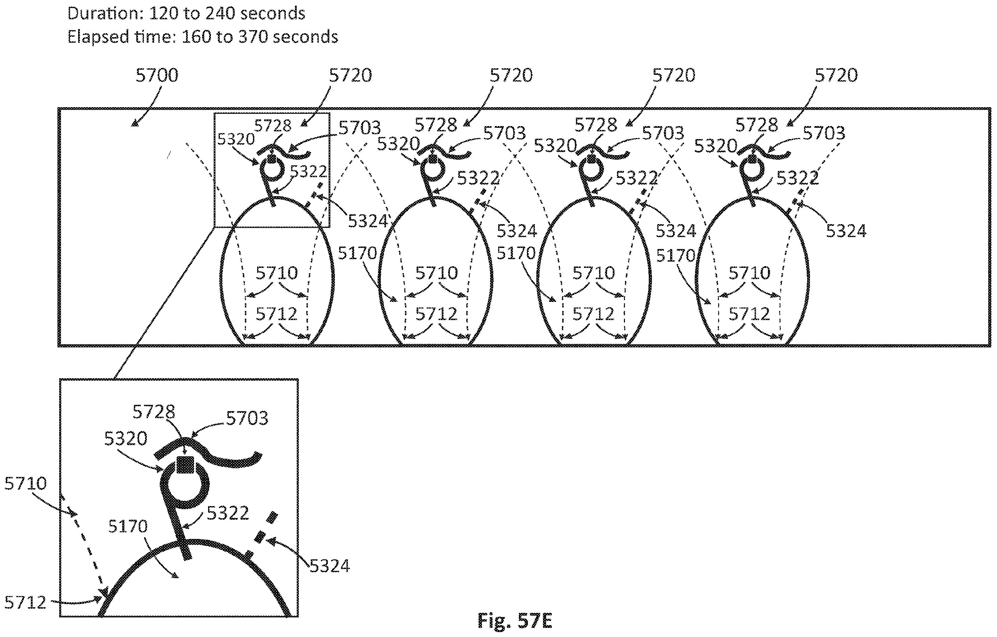

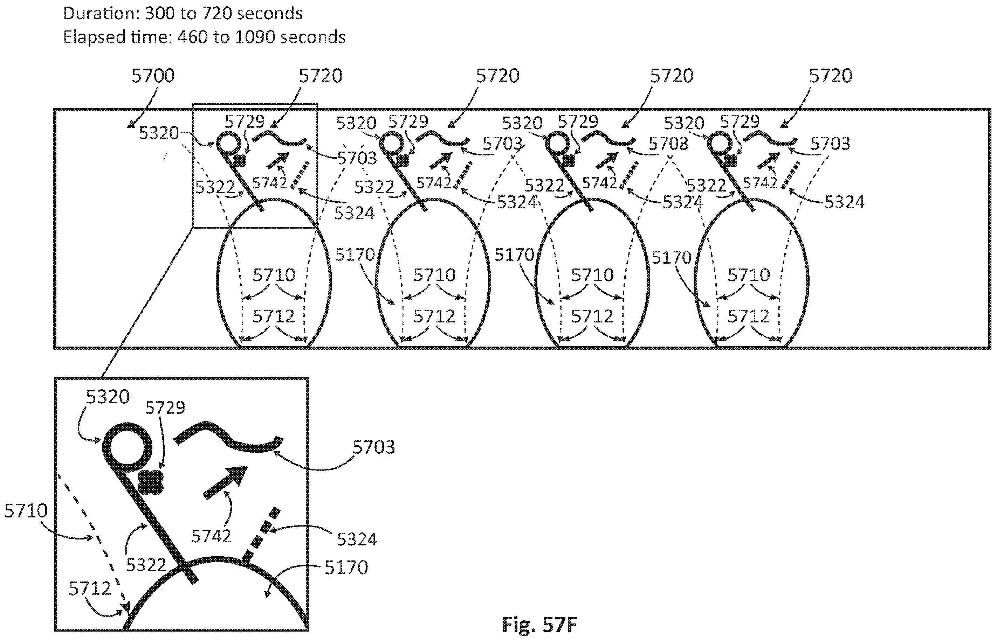

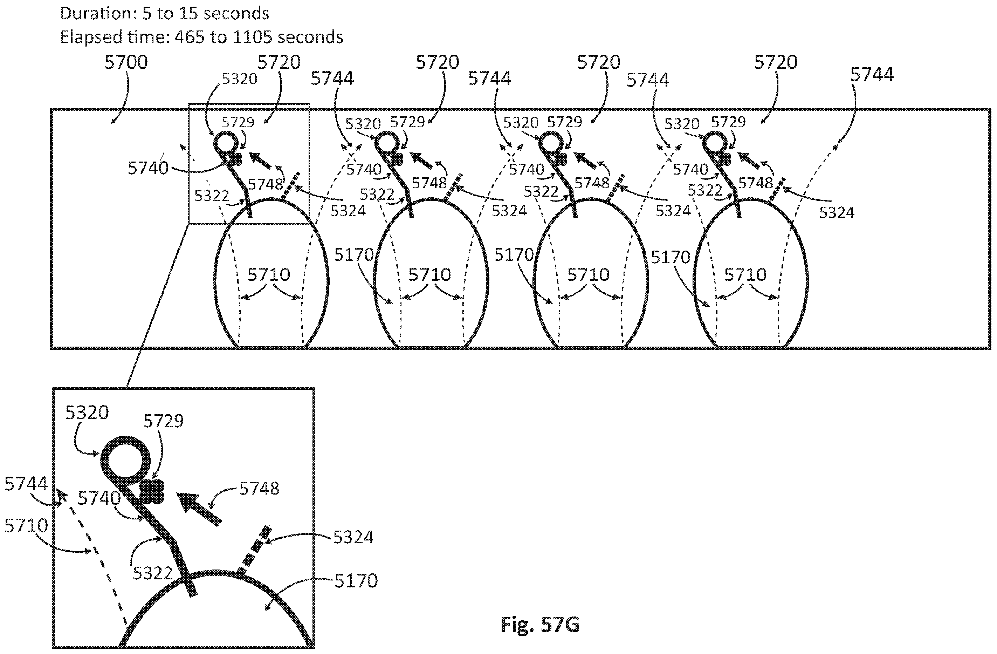

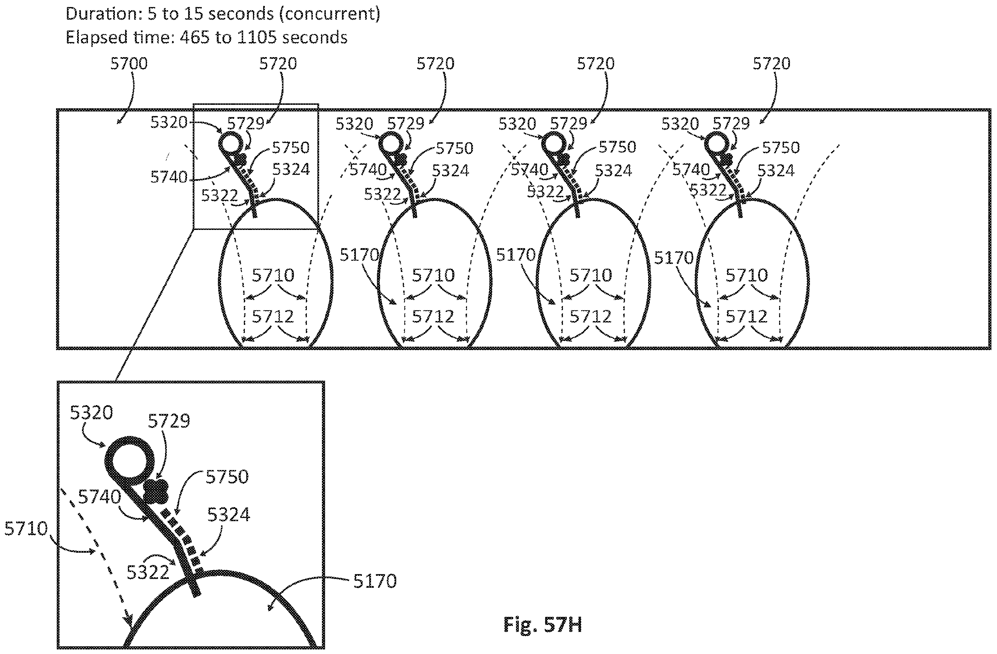

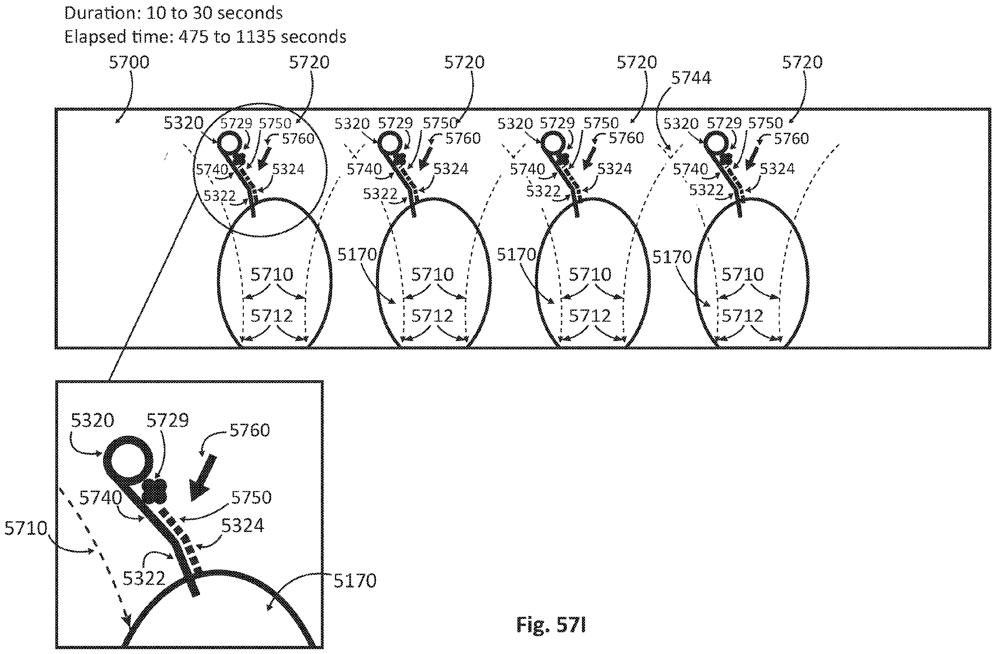

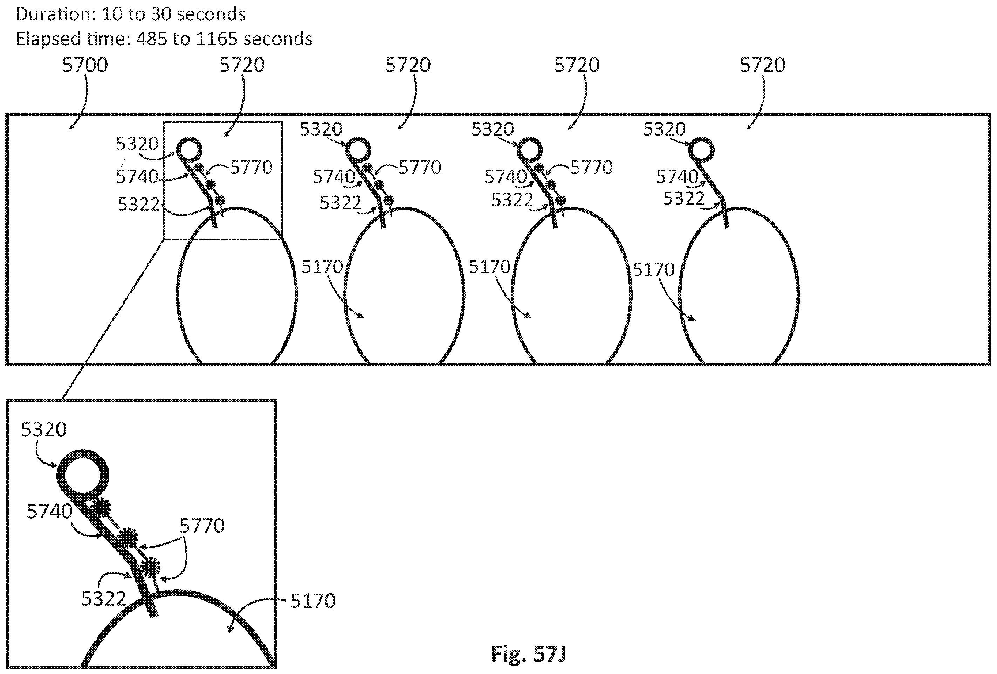

Figs. 57A, 57B, 57C, 57D, 57E, 57F, 57G, 57H, 57I and 57J are simplified illustrations of typical steps in rapid detection of the presence of at least one nucleic acid target molecule in accordance with one arrangement of the presentdisclosure;

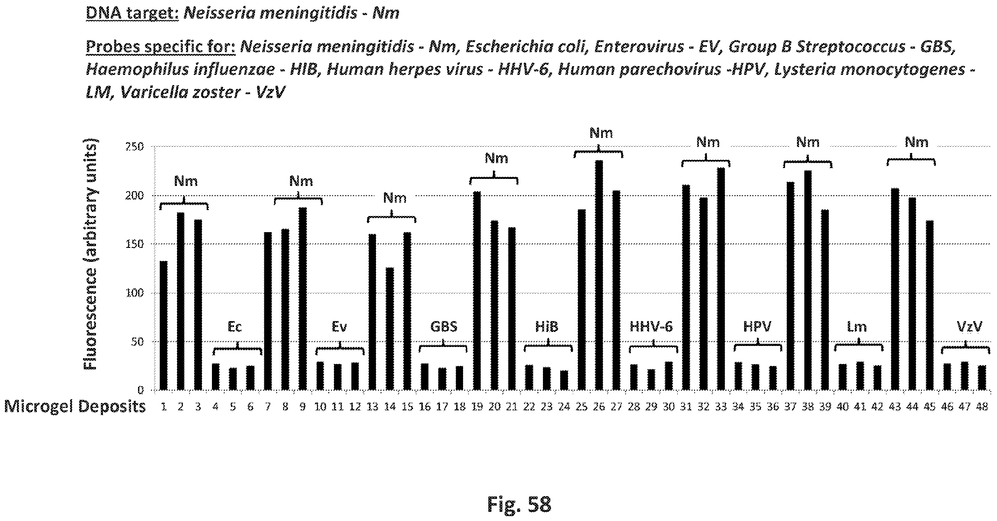

Fig. 58 is a diagram summarizing the results of Example I; and

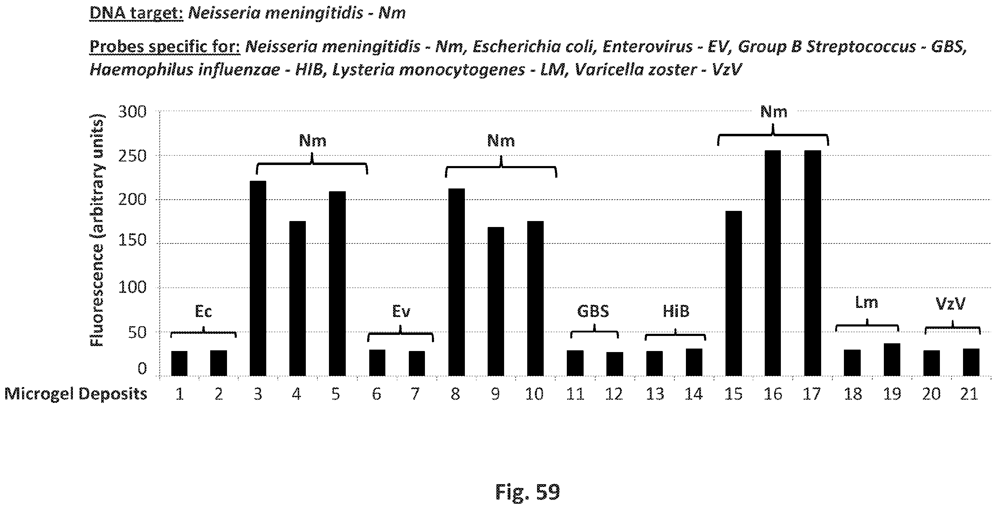

Fig. 59 is a diagram summarizing the results of Example II.

### DETAILED DESCRIPTION OF A PREFERRED EMBODIMENT

Reference is now made to Fig. 1, which is a simplified pictorial illustration of an automated in-vitro diagnostic system constructed and operative in accordance with a preferred embodiment of the present invention. The automated in-vitro diagnostic system of Fig. 1 comprises an automated diagnostic instrument 100 which automatically processes a cartridge 110 in order to provide automated in-vitro diagnosis of a sample inserted into the cartridge 110.

Cartridge 110 will now be described in detail hereinbelow with reference to Figs. 2A/1 - 9. It is appreciated that cartridge 110 is prepared in stages. Initially a non-functionalized cartridge is constructed. Cartridge 110, in the initial non-functionalized state thereof, is designated by reference numeral 112. The structure of non-functionalized cartridge 112 is described hereinbelow with reference to Figs. 2A/1 - 6C. The non-functionalized cartridge 112 is preferably subsequently initially functionalized by insertion thereto of reagents in solid form, as described hereinbelow with reference to Fig. 7A, and of reagents in liquid form, as described hereinbelow with reference to Fig. 7B, and by sealed mounting thereon of a carbon array assembly 120, one arrangement of which is described hereinbelow with reference to Figs. 8A - 8F, as described hereinbelow with reference to Fig. 9. Cartridge 110 in the initially functionalized state thereof is designated by reference numeral 122.

A sample may then be inserted in initially functionalized cartridge 122. Cartridge 110 is fully functionalized, following the addition of the sample, when fully inserted into and clamped by the automated diagnostic instrument 100 as described in detail hereinbelow.

Turning now to Figs. 2A/1 - 2J, there is seen a non-functionalized cartridge 112 including a fluid sealing layer 130, preferably having first and second pairs 132 & 134 and 136 & 138 of apertures providing fluid communication between the interior of the carbon array 120 (Figs. 8A - 8D) and fluid conduits within the remainder of the cartridge. Sealing layer 130 is also formed with apertures 140 and 142, which are employed for precise positioning of cartridge 110 in the automated diagnostic instrument 100 (Fig. 1).

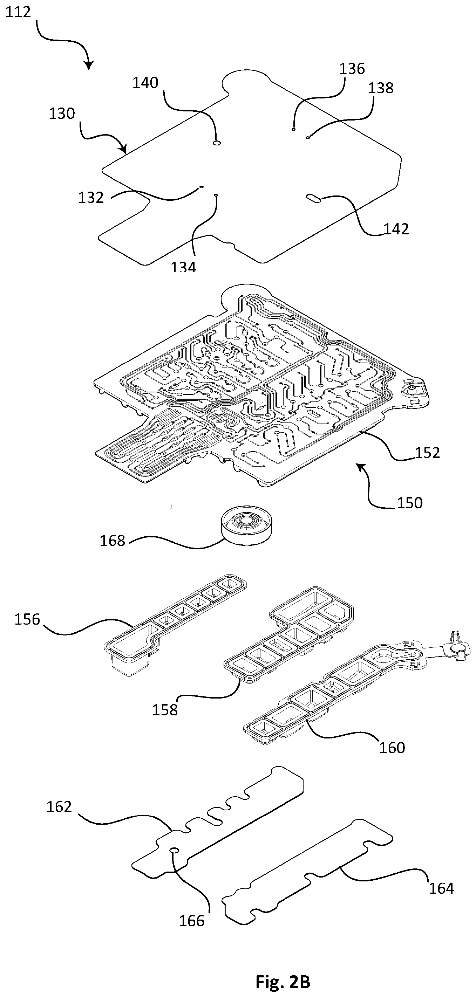

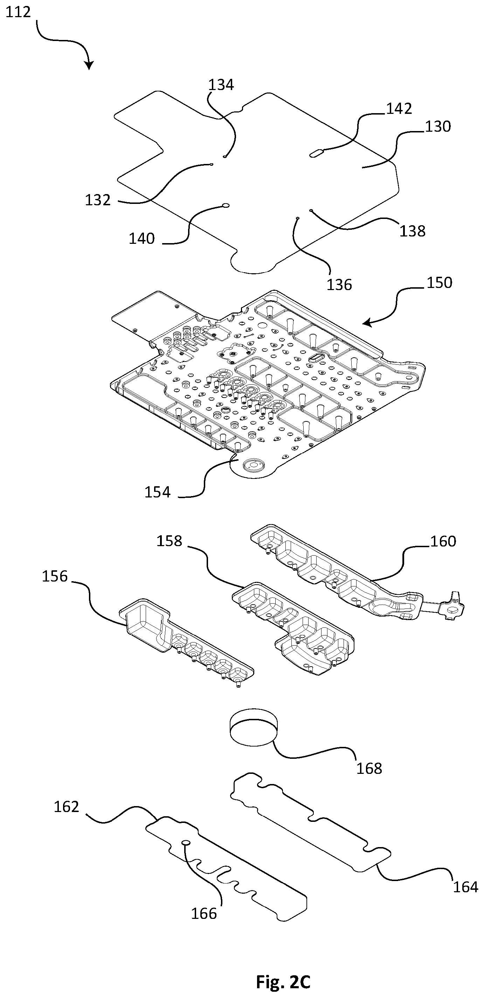

Disposed underlying sealing layer 130 is a main cartridge element 150, having a three-dimensionally patterned fluid conduit defining surface 152, which is seen in Figs. 2B and Figs. 3C/1 -3C/10 and which cooperates with sealing layer 130 to define a multiplicity of fluid conduits. On an opposite side of main cartridge element 150, as seen in Fig. 2C and Figs. 3D/1 - 3D/4, there is provided a fluid conduit defining and liquid enclosure engagement surface 154, which together with three enclosure defining elements 156, 158 and 160, which are sealingly joined to surface 154, define a plurality of liquid enclosures, which are described hereinbelow in detail.

A pair of elastomeric sealing layers 162 and 164 are sealingly joined to fluid enclosure engagement surface 154 of main cartridge element 150 alongside enclosure defining elements 156, 158 and 160. Elastomeric sealing layers 162 and 164 cooperate with main element 150 to define a plurality of valves, which are described hereinbelow in detail. Elastomeric sealing layer 162 is formed with an aperture 166. A mechanically actuable fluid pump 168, such as a gas pump, is mounted onto fluid enclosure engagement surface 154 of main cartridge element 150.

Turning now specifically to Figs. 2E - 2K and 4A - 4C, 5A -5C and 6A -6C, it is seen that enclosure defining element 160 preferably defines a sample receiving enclosure 200 and liquid enclosures 202, 204, 206, 208 and 210, as shown particularly in Fig. 2F, as well as a sample enclosure sealing plug 212, which is connected to the remainder of enclosure defining element 160 by a flexible connector 214. Liquid enclosure 202 is a lysis bead containing chamber. Liquid enclosure 204 is a Protinease K containing chamber. Liquid enclosure 206 is a lysis mixing chamber. Liquid enclosure 208 is a dilution chamber. Liquid enclosure 210 is a Raffinose washing liquid containing chamber.

Enclosure defining element 158 preferably defines liquid enclosures 222, 224, 226, 228, 230, 232 and 234, as shown particularly in Figs. 2G and 2H. Liquid enclosure 222 is a Wash Buffer I containing chamber. Liquid enclosure 224 is a Wash Buffer II containing chamber. Liquid enclosure 226 is a Wash Buffer III containing chamber. Liquid enclosure 228 is an elution buffer containing chamber. Liquid enclosure 230 is a Backend mixing chamber. Liquid enclosure 232 is an amplicon dilution buffer dilution chamber. Liquid enclosure 234 is a sensor wash containing chamber.

Enclosure defining element 156 preferably defines liquid enclosures 242, 244, 246, 248, 250 and 252, as shown particularly in Fig. 2I. Liquid enclosure 242 is a Discriminator Buffer I containing chamber. Liquid enclosure 244 is a Discriminator Buffer II containing chamber. Liquid enclosure 246 is a Discriminator Buffer III containing chamber. Liquid enclosure 248 is a Discriminator Buffer IV containing chamber. Liquid enclosure 250 is a reporter buffer containing chamber. Liquid enclosure 252 is a waste container.

Reference is now made to Figs. 3A, 3B, 3C/1 - 3C/10 and 3D/1 - 3D/4, which are simplified respective pictorial top view, pictorial bottom view, planar top view and planar bottom view illustrations of main element 150 of cartridge 110. It is appreciated that Figs. 3C/1 - 3C/10 together constitute a composite planar top view illustration of main element 150 and designate different elements therein. Similarly, it is appreciated that Figs. 3D/1 - 3D/4 together constitute a composite planar bottom view illustration of main element 150 and designate different elements therein. Multiple drawings are provided for clarity due to the large density of features appearing in these drawings.

Turning initially to Figs. 3B and 3D/1 - 3D/4, which illustrate fluid enclosure engagement surface 154, it is seen that the portions of fluid conduit defining and liquid enclosure engagement surface 154, which overlie and partially define each of the liquid enclosures are designated by the same reference numerals that designate the corresponding liquid enclosures in Fig. 2E. It is further seen that reagent supply ports 300, 302, 304, 306, 308 and 310 communicate with respective liquid enclosures 200, 202, 204, 206, 208 and 210. It is additionally seen that reagent supply ports 322, 324, 326, 328, 330, 332 and 334 communicate with respective fluid enclosures 222, 224, 226, 228, 230, 232 and 234. It is also seen that reagent supply ports 342, 344, 346, 348 and, 350 communicate with respective fluid enclosures 242, 244, 246, 248 and 250.

In accordance with a preferred arrangement of the disclosure, during initial functionalization of the non-functionalized cartridge 112, solid reagent plugs are loaded into the cartridge 112 at reagent plug sockets 350, 352, 354, 356, 358, 360, 362, 364, 366, 368 and 370. The reagent plugs are designated respectively by reference numerals 371, 372, 374, 376, 378, 380, 382, 384, 386, 388 and 390 in Fig. 7A.

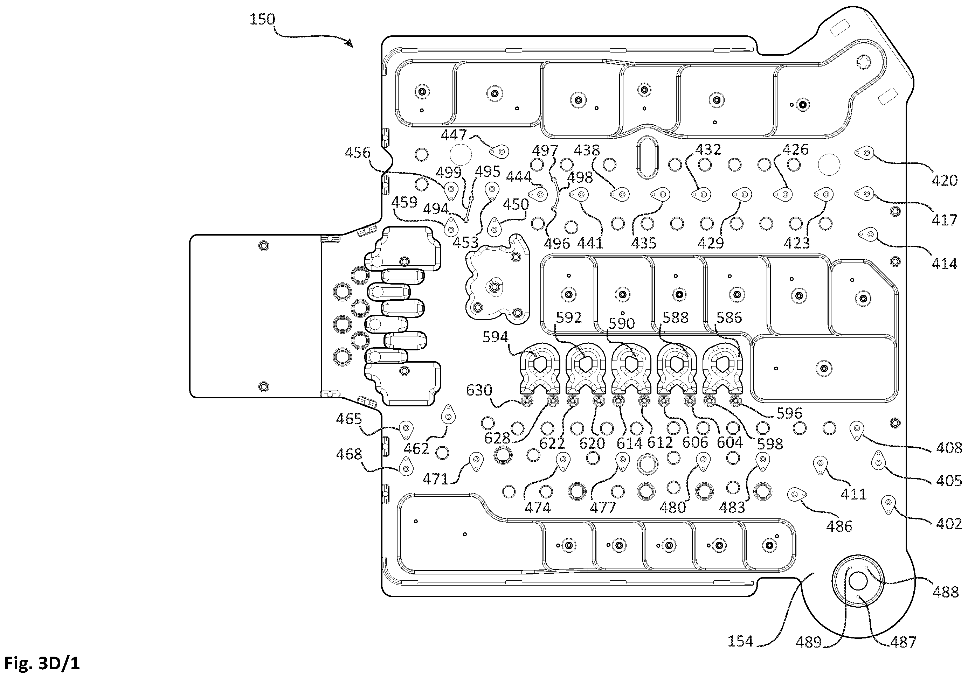

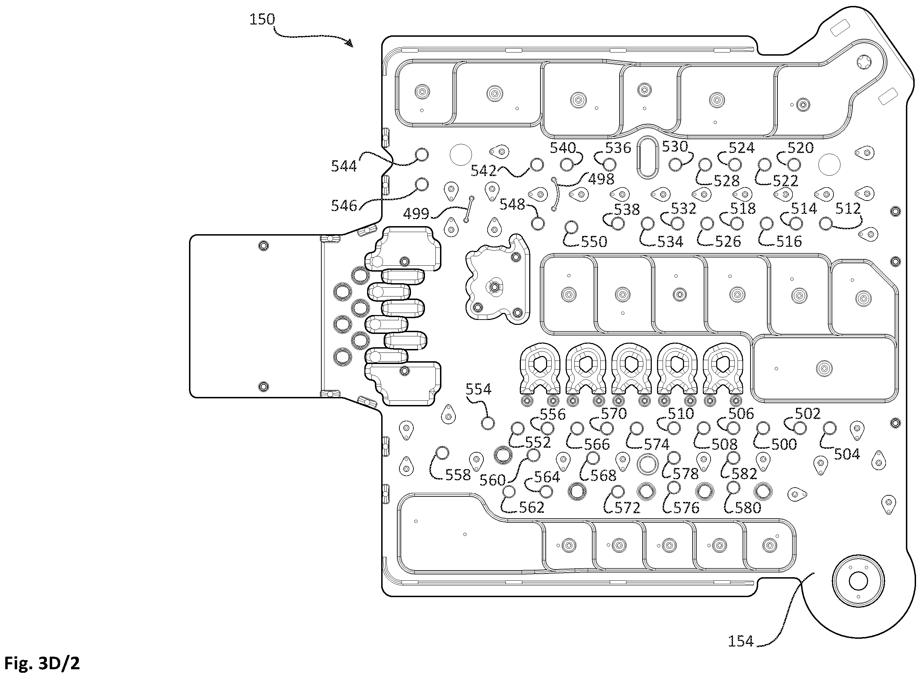

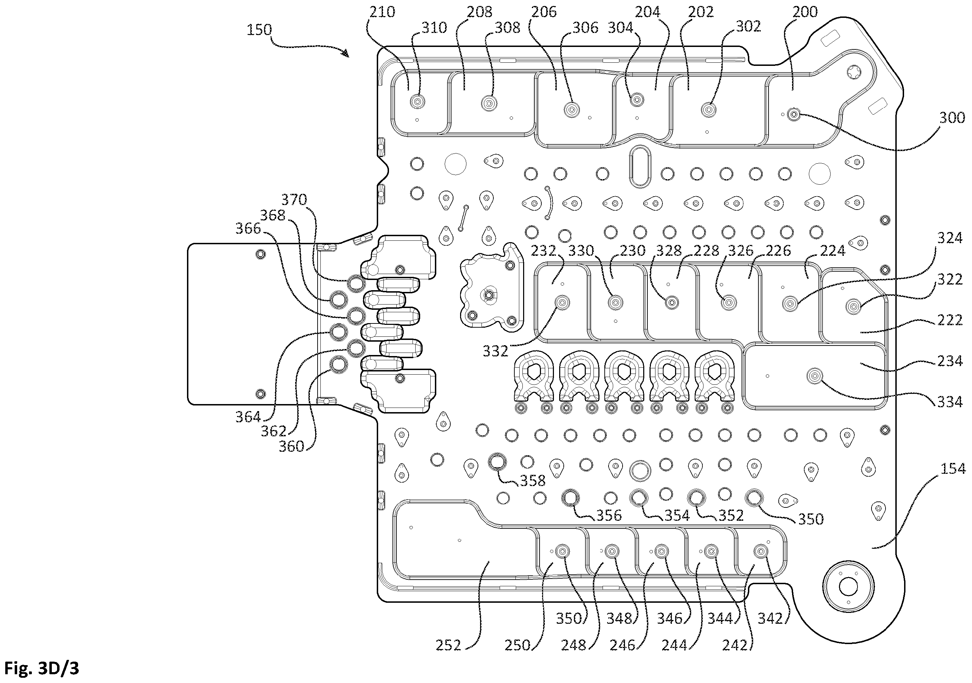

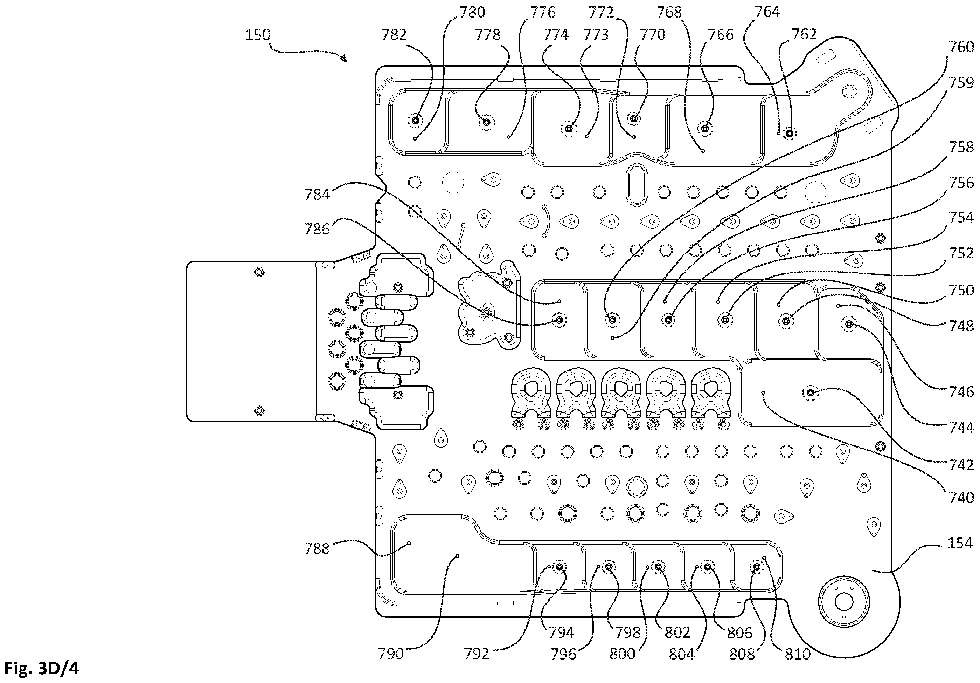

Turning now to Figs. 3A, 3C/1 and 3D1, a total of 29 pairs of through holes are seen. These pairs of through holes cooperate with sealing layer 130 sealed to a top surface of main cartridge element 150 and with one of elastomeric sealing layers 162 and 164 on a bottom surface of main cartridge element 150 to define valves, designated in Table 1 below, which are normally open when the respective elastomeric sealing layer 162 and 164 underlying them is not pressed against main cartridge element 150 and are closed by pressing the respective elastomeric sealing layer 162 and 164 thereagainst.

The correspondence between the pairs of through holes seen in Figs. 3C/1 and the valves seen in 3D/1 is set forth in Table 1:

**TABLE 1**

| Through Hole Pairs (Fig. 3C/1) | Valves (Fig. 3D/1) |
|---|---|
| 400, 401 | 402 |
| 403, 404 | 405 |
| 406, 407 | 408 |
| 409, 410 | 411 |
| 412, 413 | 414 |
| 415, 416 | 417 |
| 418, 419 | 420 |
| 421, 422 | 423 |
| 424, 425 | 426 |
| 427, 428 | 429 |
| 430, 431 | 432 |
| 433, 434 | 435 |
| 436, 437 | 438 |
| 439, 440 | 441 |
| 442, 443 | 444 |
| 445, 446 | 447 |
| 448, 449 | 450 |
| 451, 452 | 453 |
| 454,455 | 456 |
| 457, 458 | 459 |
| 460,461 | 462 |
| 463,464 | 465 |
| 466,467 | 468 |
| 469,470 | 471 |
| 472,473 | 474 |
| 475,476 | 477 |
| 478,479 | 480 |
| 481,482 | 483 |
| 484,485 | 486 |

Turning now to Fig. 3C/10 and again to Fig. 3D/1, there are seen additional through holes 487 - 489, which connect to a pump formed in the main cartridge element 150. Apertures 490 - 493, which are in fluid communication with carbon array assembly 120, are also formed in surface 152 of main cartridge element 150. Additionally, through holes 494 - 497 are formed in the main cartridge element 150. Also seen in Fig. 3D/1 are two channels 498 and 499 formed in liquid enclosure engagement surface 154.

The correspondence between the through holes seen in Figs. 3C/10 and 3D/1 and apertures seen in Fig. 3C/10 and the portions of the main cartridge element to which they are connected are set forth in Table 2:

**TABLE 2**

| **Through Holes/Apertures in** **Fig. 3C/10** | **Portion of Main Cartridge Element or other** |
|---|---|
| 487 | Through-hole to Pump |
| 488 | Through-hole to Pump |
| 489 | Through-hole to Pump |
| 490 | Aperture to aperture 132 in sealing layer 130 to carbon array assembly 120 |
| 491 | Aperture to aperture 136 in sealing layer 130 to carbon array assembly 120 |
| 492 | Aperture to aperture 138 in sealing layer 130 to carbon array assembly 120 |
| 493 | Aperture to aperture 134 in sealing layer 130 to carbon array assembly 120 |
| 494 | Through-hole to channel 499 |
| 495 | Through-hole to channel 499 |
| 496 | Through-hole to channel 498 |
| 497 | Through-hole to channel 498 |

Turning now to Fig. 3C/2 and 3D/2, a total of 42 through holes are seen. These through holes cooperate with sealing layer 130 sealed to a top surface of main cartridge element 150 and with one of elastomeric sealing layers 162 and 164 on a bottom surface of main cartridge element 150 to define frangible seals, which are in a sealed state prior to clamping operation of the instrument 100, when they are preferably all unsealed simultaneously. The structure of the frangible seals is described and claimed in U.S. Patent Nos. 8,950,424 and 10,173,215.

The correspondence between the through holes seen in Figs. 3C/2 and 3D/2 and the frangible seals seen in Fig. 2A/1 is set forth in Table 3:

**TABLE 3**

| Through Hole (Figs. 3C/2 & 3D/2) | Frangible Seal (Fig. 2A/1) |
|---|---|
| 500 | 501 |
| 502 | 503 |
| 504 | 505 |
| 506 | 507 |
| 508 | 509 |
| 510 | 511 |
| 512 | 513 |
| 514 | 515 |
| 516 | 517 |
| 518 | 519 |
| 520 | 521 |
| 522 | 523 |
| 524 | 525 |
| 526 | 527 |
| 528 | 529 |
| 530 | 531 |
| 532 | 533 |
| 534 | 535 |
| 536 | 537 |
| 538 | 539 |
| 540 | 541 |
| 542 | 543 |
| 544 | 545 |
| 546 | 547 |
| 548 | 549 |
| 550 | 551 |
| 552 | 553 |
| 554 | 555 |
| 556 | 557 |
| 558 | 559 |
| 560 | 561 |
| 562 | 563 |
| 564 | 565 |
| 566 | 567 |
| 568 | 569 |
| 570 | 571 |
| 572 | 573 |
| 574 | 575 |
| 576 | 577 |
| 578 | 579 |
| 580 | 581 |
| 582 | 583 |

Turning now to Fig. 3C/4 and again to Fig. 3D/1, reservoirs for Discriminator I, Discriminator II, Discriminator III and Discriminator IV are seen and designated by respective reference numerals 586, 588, 590 and 592. A reporter reservoir is designated by reference numeral 594.

Each of reservoirs 586, 588, 590, 592 and 594 communicates via respective through holes with respective fill and venting ports.

The correspondence between the reservoirs and their respective fill and venting ports and through holes appears in Table 4:

**TABLE 4**

| Reservoir | Fill Port | Venting Port | Fill Port Through Hole | Venting Port Through Hole |
|---|---|---|---|---|
| 586 | 596 | 598 | 600 | 602 |
| 588 | 604 | 606 | 608 | 610 |
| 590 | 612 | 614 | 616 | 618 |
| 592 | 620 | 622 | 624 | 626 |
| 594 | 628 | 630 | 632 | 634 |

Turning now to Fig. 3C/5, optical liquid detection chambers 640, 642, 644, 646, 648, 650 and 652 are seen.

Returning now to Fig. 3C/3, a Polymerase Chain Reaction (PCR) array is shown including six eluent aliquot chambers, designated by reference numerals 660, 662, 664, 666, 668 and 670 are shown. A corresponding six reagent plug hydration chambers, designated by reference numbers 680, 682, 684, 686, 688 and 690 are also shown and communicate with respective dry reagent plugs 390, 388, 386, 384, 382 and 380 (Fig. 7A), which are located in respective reagent plug sockets 370, 368, 366, 364, 362 and 360, seen in Fig 3D/3.

The PCR array also includes channels 691, 692, 693, 694, 695, 696, 697 and 698 and PCR chambers 700, 702, 704, 706, 708 and 710, which communicate with respective hydration chambers 680, 682, 684, 686, 688 and 690. Channels 693, 694, 695, 696, 697 and 698 respectively communicate with aliquot chambers 660, 662, 664, 666, 668 and 670. Communicating with each of PCR chambers 700, 702, 704, 706, 708 and 710 is a corresponding gas spring. The gas springs are designated by respective reference numerals 711, 712, 713, 714, 715 and 716. Gas springs 711, 712, 713, 714, 715 and 716 are respectively connected to PCR chambers 700, 702, 704, 706, 708 and 710 by channels 720, 722, 724, 726, 728 and 730.

Additionally seen in Fig. 3C/5 is a metering chamber 732.

Turning now to Figs. 3C/6 and 3D/4 there are designated a plurality of through holes designated by reference numerals 742, 744, 746, 748, 750, 752, 754, 756, 758 ,759, 760, 762, 764, 766, 768, 770, 772, 773, 774, 776, 778, 780, 782, 784, 786, 788, 790, 792, 794, 796, 798, 800, 802, 804, 806, 808 and 810, which communicate with various liquid enclosures seen in Fig. 2E.

The correspondence between the through holes seen in Figs. 3C/6 and 3D/2 and the liquid enclosures seen in Fig. 2E is set forth in Table 5:

**TABLE 5**

| Liquid Enclosures (Fig. 2E) | Through Holes (Figs. 3C/6 & 3D/4) |
|---|---|
| 200 | 762, 764 |
| 202 | 766, 768 |
| 204 | 770, 772 |
| 206 | 773, 774 |
| 208 | 776, 778 |
| 210 | 780, 782 |
| 222 | 744, 746 |
| 224 | 748, 750 |
| 226 | 752, 754 |
| 228 | 756, 758 |
| 230 | 759, 760 |
| 232 | 784, 786 |
| 234 | 740, 742 |
| 242 | 808, 810 |
| 244 | 804, 806 |
| 246 | 800, 802 |
| 248 | 796, 798 |
| 250 | 792, 794 |
| 252 | 788, 790 |

Turning now to Figs. 3C/7, 3C/8 and 3C/9 in which there are seen a plurality of channels formed in fluid conduit defining surface 152 designated by reference numerals 820, 822, 823, 824, 825, 826, 828, 830, 831, 832, 833, 834, 835, 836, 837, 838, 839, 840, 841, 842, 843, 844, 845, 846, 847, 848, 849, 850, 851, 852, 854, 855, 856, 858, 860, 862, 864, 865, 866, 867, 868, 869, 870, 871, 872, 873, 874, 876, 878, 879, 880, 882, 883, 884, 885, 886, 888, 890, 892, 894, 896, 897, 898, 900, 901, 902, 903, 904, 905, 906, 907, 908, 909, 910, 912, 914, 915, 916, 917, 918, 920, 922, 923, 924, 925, 926, 927, 928, 930, 932, 934, 936, 938, 940, 942, 944, 946, 948, 950, 952, 954, 956, 957, 958, 959, 960, 962, 964, 966, 968, 970, 971, 972, 974, 976, 978, 982, 984, 986, 988, 990, 992, 993, 994, 998 and 999, which provide fluid interconnections between portions of the cartridge.

The correspondence between the channels seen in Figs 3C/7, 3C/8 and 3C/9 and the end points thereof is set forth in Table 6, in which V is a Valve through hole, P is a Pump through hole, CA is a Carbon Array aperture, TC is a Through hole to a Channel on liquid enclosure engagement surface 154, LE is a Liquid Enclosure, PFS is in Proximity to a Frangible Seal through hole, I is an Intersection, C is a Concentrate, CC is a Connector to Concentrate, CH is a Channel, LS is a Liquid Sensor, RP is a Reagent Plug and MC is a Metering Chamber.

**TABLE 6**

| **Channel #** | **End 1 #** | **End 1 Type** | **End 2 #** | **End 2 Type** | **On Channel** |
|---|---|---|---|---|---|
| **820** | 400 | V | 489 | P | |
| **822** | 489 | P | 406 | V | |
| **824** | 488 | P | 404 | V | |
| **825** | 403 | V | CHs: 957, 959, 966, 971 | I | |
| **826** | 544 | PFS | CHs: 897, 885, 883, 879, 871, 869 | I | |
| **828** | 401 | V | 542 | PFS | |
| **830** | 403 | V | 773 | LE | |
| **831** | 484 | V | CHs: 833, 835 | I | |
| **832** | 404 | V | 413 | V | |
| **833** | CH 831 | I | 502 | PFS | |
| **834** | 407 | V | 439 | V | |
| **835** | CH 831 | I | 500 | PFS | |
| **836** | 413 | V | 418 | V | |
| **837** | 416 | V | 421 | V | |
| **838** | 415 | V | 520 | PFS | |
| **839** | 421 | V | 424 | V | |
| **840** | 412 | V | 528 | PFS | |
| **841** | 424 | V | 427 | V | |
| **842** | 762 | LE | 520 | PFS | |
| **843** | 427 | V | 430 | V | |
| **844** | 419 | V | 522 | PFS | |
| **845** | 430 | V | 433 | V | |
| **846** | 764 | LE | 522 | PFS | |
| **847** | 433 | V | 436 | V | |
| **848** | 766 | LE | 524 | FPS | LS 640 |
| **849** | 436 | V | 439 | V | |
| **850** | 768 | LE | 528 | PFS | |
| **851** | 439 | V | 448 | V | LS 648, LS 650, MC 732 |
| **852** | 770 | LE | 530 | PFS | LS 642 |
| **854** | 772 | LE | 536 | PFS | |
| **855** | 773 | LE | 536 | PFS | |
| **856** | 774 | LE | 440 | V | |
| **858** | 778 | LE | 540 | PFS | |
| **860** | 776 | LE | 542 | PFS | |
| **862** | 780 | LE | 544 | PFS | |
| **864** | 782 | LE | 546 | PFS | |
| **865** | CH 873 | I | 457 | V | |
| **866** | 454 | V | 546 | PFS | |
| **867** | 449 | V | CHS: 691, 692 | I | |
| **868** | 461 | V | CHs:873 | I | |
| **869** | CH 826 | I | 550 | PFS | |
| **870** | 463 | V | 759 | LE | |
| **871** | CH 826 | I | 558 | PFS | |
| **872** | 467 | V | 788 | LE | |
| **873** | CH 904 | I | CHs 865, 868 | I | |
| **874** | 490 | CA | 790 | LE | LS 652 |
| **876** | 792 | LE | 558 | PFS | LS 652 |
| **878** | 794 | LE | 560 | PFS | |
| **879** | CH 826 | I | 562 | PFS | |
| **880** | 796 | LE | 562 | PFS | |
| **882** | 798 | LE | 564 | PFS | |
| **883** | CH 826 | I | 568 | PFS | |
| **884** | 800 | LE | 568 | PFS | |
| **885** | CH 826 | I | 576 | PFS | |
| **886** | 802 | LE | 572 | PFS | |
| **888** | 804 | LE | 576 | PFS | |
| **890** | 806 | LE | 578 | PFS | |
| **892** | 808 | LE | 580 | PFS | |
| **894** | 487 | P | 466 | V | |
| **896** | 810 | LE | 500 | PFS | |
| **897** | CH 826 | I | 484 | V | |
| **898** | 489 | P | 485 | V | |
| **900** | 409 | V | 504 | PFS | |
| **901** | 742 | LE | 504 | PFS | |
| **902** | 740 | LE | 502 | PFS | |
| **903** | 491 | CA | 492 | CA | |
| **904** | 410 | V | CHs: 946, 948, 950, 952, 954 | I | |
| **905** | 744 | LE | 512 | PFS | |
| **906** | 425 | V | 516 | PFS | |
| **907** | 422 | V | 512 | PFS | |
| **908** | 428 | V | 524 | PFS | |
| **909** | 431 | V | 526 | PFS | |
| **910** | 434 | V | 530 | PFS | |
| **912** | 437 | V | 534 | PFS | |
| **914** | 442 | V | 540 | PFS | |
| **915** | 443 | V | CH 923 | I | |
| **916** | 445 | V | 497 | TC | |
| **917** | 451 | V | CH 923 | I | |
| **918** | 452 | V | 495 | TC | |
| **920** | 446 | V | 495 | TC | |
| **922** | 455 | V | 495 | TC | |
| **923** | 448 | V | CH 915, 917 | I | |
| **924** | 496 | B | TC | PFS | |
| **925** | 457 | V | 494 | TC | |
| **926** | 560 | PFS | 552 | PFS | RP 358 |
| **927** | 464 | V | 466 | V | |
| **928** | 470 | V | 554 | PFS | |
| **930** | 473 | V | 556 | PFS | |
| **932** | 564 | PFS | 566 | PFS | RP 356 |
| **934** | 476 | V | 570 | PFS | |
| **936** | 572 | PFS | 574 | PFS | RP 354 |
| **938** | 479 | V | 510 | PFS | |
| **940** | 578 | PFS | 508 | PFD | RP 352 |
| **942** | 481 | V | 506 | PFS | |
| **944** | 580 | PFS | 582 | PFS | RP 350 |
| **946** | CH 904 | I | 482 | V | |
| **948** | CH 904 | I | 478 | V | |
| **950** | CH 904 | I | 475 | V | |
| **952** | CH 904 | I | 472 | V | |
| **954** | CH 904 | I | 469 | V | |
| **956** | 748 | LE | 516 | PFS | |
| **957** | CH 825 | I | 514 | PFS | |
| **958** | 746 | LE | 514 | PFS | |
| **959** | CH 825 | I | 518 | PFS | |
| **960** | 750 | LE | 518 | PFS | |
| **962** | 752 | LE | 526 | PFS | |
| **964** | 754 | LE | 532 | PFS | |
| **966** | CH 825 | I | 532 | PFS | |
| **968** | 756 | LE | 534 | PFS | |
| **970** | 758 | LE | 538 | PFS | |
| **971** | CH 825 | I | 538 | PFS | |
| **972** | 784 | LE | 550 | PFS | |
| **974** | 786 | LE | 548 | PFS | LS 646 |
| **976** | 458 | V | 493 | A | |
| **978** | 460 | V | 760 | LE | LS644 |
| **982** | 634 | CC | 554 | PFS | C 594 |
| **984** | 632 | CC | 552 | PFS | C 594 |
| **986** | 626 | CC | 556 | PFS | C 592 |
| **988** | 624 | CC | 566 | PFS | C 592 |
| **990** | 618 | CC | 570 | PFS | C 590 |
| **992** | 616 | CC | 574 | PFS | C 590 |
| **993** | 610 | CC | 510 | PFS | C 588 |
| **994** | 608 | CC | 508 | PFS | C 588 |
| **998** | 602 | CC | 582 | PFS | C 586 |
| **999** | 600 | CC | 506 | PFS | C 586 |

As noted above, initially functionalized cartridge 122 is fully functionalized when fully inserted into and clamped by the automated diagnostic instrument 100 as described in detail hereinbelow. This clamping preferably simultaneously unseals all of the frangible seals.

Reference is now made to Fig. 3E, which illustrates various fluid pathways defined in the cartridge 110 when all of the frangible seals are broken. These pathways will be referred to hereinbelow in connection with a detailed description of operation of the automated diagnostic instrument 100 and the cartridge 110 in accordance with one arrangement of the present disclosure.

The correspondence between the pathways in Fig. 3E and various channels and portions of the cartridge is set forth in Table 7, in which TH is a through hole to a chamber, V is a Valve through hole and LS is a Liquid Sensor.

**TABLE 7**

| **Pathway** | **Frangible seal(s)** | **From-To** | **Channels connected** |
|---|---|---|---|
| **1000** | 520 | 762 (Sample Chamber TH), 415 (V) | 842, 838 |
| **1001** | 522 | 764 (Sample Chamber TH), 419 (V) | 846, 844 |
| **1002** | 524 | 766 (Lysis/Beads TH), 428 (V) | 848, 908 (LS 640) |
| **1003** | 528 | 768 (Lysis/Beads TH), 412(V) | 850, 840 |
| **1004** | 530 | 770 (PK TH), 434 (V) | 852, 910 (LS 642) |
| **1005** | 536 | 772 (PK TH), 403 (V) | 854, 855, 830 |
| **1007** | 540 | 778 (Dilution TH), 442 (V) | 858, 914 |
| **1008** | 542 | 776 (Dilution TH), 401 (V) | 860, 828 |
| **1009** | 544 | 780 (Raffinose Wash TH), 484 (V) | 862, 826, 897 |
| **1010** | 546 | 782 (Dilution TH), 454 (V) | 864, 866 |
| **1011** | 548 | 786 (Amplicon Dilution TH), 496 (TH to channel 498 on engagement surface 154) | 924, 974, (LS 646) |
| **1012** | 558 | 792 (Reporter TH), 484 (V) *Part of shared channel. Pathway formed during flow | 876, 871, 826, 897 |
| **1013** | 560, 552, 554 | 794 (Reporter TH), 470 (V) | 878, 926, 984, 982, 928 |
| **1014** | 562 | 796 (Discr. 4 TH), 484 (V) *Part of shared channel. Pathway formed during flow | 880, 826, 897, 879 |
| **1015** | 564, 566, 556 | 798 (Discr. 4 TH), 473 (V) | 882, 932, 988, 986, 930 |
| **1016** | 568 | 800 (Discr. 3 TH), 484 (V) *Part of shared channel. Pathway formed during flow | 884, 826, 897, 883 |
| **1017** | 572, 574, 570 | 802 (Discr. 3 TH), 476 (V) | 886, 936, 934, 922, 990 |
| **1018** | 576 | 804 (Discr. 2 TH), 484 (V) *Part of shared channel. Pathway formed during flow | 888, 826, 897, 885 |
| **1019** | 578, 508, 510 | 806 (Discr. 2 TH), 479 (V) | 890, 940, 994, 993, 938 |
| **1020** | 580, 582, 506 | 808 (Discr. 1 TH), 481 (V) | 892, 944, 998, 999, 942 |
| **1023** | 500 | 810 (Discr. 1 TH), 484 (V) *Part of shared channel. Pathway formed during flow | 896, 835, 831 |
| **1024** | 502 | 740 (Sensor Wash TH), 484 (V) | 902, 833, 831 |
| | | *Part of shared channel. Pathway formed during flow | |
| **1025** | 504 | 742 (Sensor Wash TH), 409 (V), | 901, 900 |
| **1026** | 514 | 746 (Wash 1 TH), 403 (V) | 958, 957 |
| **1027** | 512 | 744 (Wash 1 TH), 422 (V) | 905, 907 |
| **1028** | 518 | 750 (Wash 2 TH), 403 (V) | 960, 825, 959 |
| **1029** | 516 | 748 (Wash 2 TH), 425 (V) | 956, 906 |
| **1030** | 532 | 754 (Wash 3 TH), 403 (V) | 964, 966 |
| **1031** | 526 | 752 (Wash 3 TH), 431 (V) | 962, 909 |
| **1032** | 538 | 758 (Elution TH), 403 (V) | 970, 971 |
| **1033** | 534 | 756 (Elution TH), 437 (V) | 968, 912 |
| **1034** | 550 | 784 (Amplicon Dilution TH), 484 (V) | 972, 873 |
| | | *Part of shared channel. Pathway formed during flow | |

Reference is now made to Figs. 7A and 7B, which are simplified illustrations of stages in the initial functionalization of the non-functionalized cartridge of Figs. 2A/1 to Fig. 6C.

As seen in Fig. 7A, dry reagents, in the form of reagent plugs 371, 372, 374, 376, 378, 380, 382, 384, 386, 388 and 390 are inserted, as indicated by respective arrows, into respective reagent plug sockets 350, 352, 354, 356, 358, 360, 362, 364, 366, 368 and 370, which are then preferably heat sealed. The descriptions of the reagent plugs are detailed below in Table 8.

**TABLE 8**

| Reagent Plug | Description |
|---|---|
| 371 | Discriminator 1 |
| 372 | Discriminator 2 |
| 374 | Discriminator 3 |
| 376 | Discriminator 4 |
| 378 | Reporter Buffer |
| 380 | PCR 1 |
| 382 | PCR 2 |
| 384 | PCR 3 |
| 386 | PCR 4 |
| 388 | PCR 5 |
| 390 | PCR 6 |

As seen in Fig. 7B, liquid reagents are injected into liquid enclosures 202, 204, 208, 210, 222, 224, 226, 228, 232, 234, 242, 244, 246, 248 and 250 via respective nozzles, as indicated by respective arrows, which are then preferably heat sealed. The descriptions of the reagent plugs are detailed below in Table 9.

**TABLE 9**

| Liquid Enclosures (Figs. 2E/7B) | Description | Quantity (µl) |
|---|---|---|
| 202 | Lysis/Beads | 600 |
| 204 | PK | 200 |
| 208 | Dilution | 1800 |
| 210 | Raffinose Wash | 300 |
| 222 | Sample Wash 1 | 600 |
| 224 | Sample Wash 2 | 600 |
| 226 | Sample Wash 3 | 600 |
| 228 | Sample Elution | 180 |
| 232 | Amplicon Dilution | 300 |
| 234 | Sensor Wash | 2000 |
| 242 | Discriminator 1 Buffer | 100 |
| 244 | Discriminator 2 Buffer | 100 |
| 246 | Discriminator 3 Buffer | 100 |
| 248 | Discriminator 4 Buffer | 100 |
| 250 | Reporter Buffer | 100 |

Reference is now made to Figs. 8A - 9, which illustrate carbon array assembly 120 and mounting thereof onto the cartridge of Figs. 7A and 7B, to define initially functionalized cartridge 122.

Turning initially to Figs. 8A - 8C, it is seen that the carbon array assembly 120 comprises a carbon array subassembly 1200, seen in Figs. 8A and 8B, and a cover assembly 1210, seen in 8C. The construction of carbon array subassembly 1200 is shown particularly in Fig. 8B. It is seen that the carbon array subassembly 1200 preferably includes a double-sided adhesive layer 1220. Double-sided adhesive layer 1220 is preferably formed with registration apertures 1222 at a first end thereof and with registration cut outs 1224 at an opposite end thereof. Double-sided adhesive layer 1220 is also formed with liquid inlet apertures 1226 and liquid outlet apertures 1228.

Adhered to double-sided adhesive layer 1220 is an optional resistance heating layer 1230.

Formed over a portion of double-sided adhesive layer 1220 and over optional resistance heating layer 1230 is a black background layer 1240 typically formed by painting a PET layer with black ink.

Disposed above black background layer 1240 is a substrate layer 1250. Substrate layer 1250 is preferably formed with registration apertures 1252 at a first end thereof and with registration cut outs 1254 at an opposite end thereof. Substrate layer 1250 is also formed with liquid inlet apertures 1256 and liquid outlet apertures 1258.

An array of carbon resistors 1260, typically including 4 rows of 50 resistors each, is formed, preferably by screen printing onto substrate layer 1250.

An electrode array 1280, preferably formed of silver, is preferably screen printed over the array of carbon resistors 1260 onto substrate layer 1250 and defines a pair of electrode arrays 1282, each of which includes a peripheral electrode 1284 and a counter electrode 1286 as well as two rows of working electrodes 1288.

A carbon array 1290 is preferably screen printed over the electrode array 1280 onto substrate layer 1250 and defines a pair of carbon arrays 1292, each of which includes a central carbon electrode 1294 as well as two rows of working carbon electrodes 1298.

Formed over substrate layer 1250 and overlying arrays 1260, 1280 and 1290 is a dielectric layer 1300 which is formed with a pair of elongate apertures 1302 which overlie and communicate with carbon electrodes 1294, which in turn overlie counter electrodes 1286. Dielectric layer 1300 also defines a plurality of apertures 1304 each of which overlies one of working carbon electrodes 1298, which in turn overlies one of working electrodes 1288. Each of apertures 1304 preferably contains a droplet of a hydrophilic polymer used for binding.

Reference is now made to Fig 8C which illustrates cover assembly 1210. Cover assembly 1210 includes a double-sided adhesive layer 1310 having formed therein two parallel snake-shaped cut outs 1312 and two corresponding square cut outs 1314. Double-sided adhesive layer 1310 is preferably formed with registration apertures 1322 at a first end thereof and with registration cut outs 1324 at an opposite end thereof. Snake-shaped cut outs 1312 define liquid flow paths communicating with the working carbon electrodes 1298 of carbon array subassembly 1200, which in turn overlie working electrodes 1288 of carbon array subassembly 1200.

Overlying double-sided adhesive layer 1310 is a transparent cover layer 1330, preferably formed of polypropylene, which seals the snake-shaped cut outs 1312. Transparent cover layer 1330 is preferably formed with registration apertures 1332 at a first end thereof and with registration cut outs 1334 at an opposite end thereof.

Fig. 8D shows the mounting of the carbon array subassembly 1200 onto the initially functionalized cartridge 122 of Figs. 7A & 7B and Fig. 8F shows the mounting of the cover assembly 1210 over the carbon array subassembly 1200 in sealing engagement with the initially functionalized cartridge of Figs. 7A & 7B.

It is appreciated that alternatively the cover assembly 1210 may be sealed to the carbon array subassembly 1200 prior to mounting of the carbon array subassembly 1200 onto the initially functionalized cartridge of Figs. 7A & 7B.

It is appreciated that carbon array assembly 120 may be constructed and operative in accordance with the teachings of one or more of the following Patent Applications of applicant/assignee:
Israel Patent Application No. 249956, filed December 29, 2016 and entitled AN ELECTROPHERETIC CHIP FOR ELECTROPHORETIC APPLICATIONS;
Israel Patent Application No. 249957, filed December 29, 2016 and entitled AN ELECTROPHERETIC CHIP FOR ELECTROPHORETIC APPLICATIONS;
PCT Patent Application PCT/IL2017/051399, filed December 29, 2017 and entitled CARTRIDGE FOR USE IN IN-VITRO DIAGNOSTICS AND METHOD OF USE THEREOF; and
PCT Patent Application PCT/IL2019/ 050726 filed July 4, 2019 and entitled IMPROVED CARTRIDGE FOR USE IN IN-VITRO DIAGNOSTICS AND METHOD OF USE THEREOF.

Fig. 9 shows the initially functionalized cartridge 122 ready to receive a sample and to be inserted into the instrument 100.

Teachings of U.S. Patent Nos. 9,149,802; 10,232,367 and 10,315,197 may also be relevant to the cartridge 110 described hereinabove.

Reference is now made to Figs 10A, 10B, 10C and 10D, which are respective side view illustrations showing steps in the insertion of a sample into the initially functionalized cartridge 122 of Figs. 2A/1 - 9. It is appreciated that the sample preferably is a liquid biological sample including nucleic acids.

As seen in Fig. 10A, sample enclosure sealing plug 212 of cartridge 122 is in the open position and sample receiving enclosure 200 is accessible for the addition of a sample. In Fig. 10B, a pipette 1400 containing a sample 1402 is placed within the opening of sample receiving enclosure 200 and sample 1402 is transferred into sample receiving enclosure 200.

As seen in Fig. 10C, following the transfer of sample 1402 from pipette 1400 to sample receiving enclosure 200, pipette 1400 is withdrawn from sample receiving enclosure 200, as indicated by an arrow 1404. In Fig. 10D, sample enclosure sealing plug 212 of cartridge 122 is then closed, sealing sample 1402 in sample receiving enclosure 200.

Reference is now made to Figs. 11A and 11B, which are simplified respective pictorial assembled view and pictorial exploded view illustrations of automated diagnostic instrument 100 which, together with the cartridge 110 of Figs. 2A/1 - 10D, forms the automated in-vitro diagnostic system of Fig. 1.

As seen in Figs. 11A and 11B, the automated diagnostic instrument 100 comprises a cartridge holder assembly 1510, which is described hereinbelow in detail with reference to Figs. 12A - 13E, and a pair of cartridge insertion drive assemblies 1520, which are described hereinbelow in detail with reference to Figs. 14A - 15B. The automated diagnostic instrument 100 also comprises a pair of cartridge clamping side sub-assemblies 1530, which are described hereinbelow in detail with reference to Figs. 16A - 17 and a cartridge clamping drive assembly 1540, which is described hereinbelow in detail with reference to Figs. 18A - 19B.

The automated diagnostic instrument 100 additionally comprises a cartridge actuation assembly 1550, which is described hereinbelow in detail with reference to Figs. 20A - 21H, and an upper thermocycling assembly 1560, which is described hereinbelow in detail with reference to Figs. 22A - 23B. The automated diagnostic instrument 100 further comprises a lower thermocycling assembly 1570, which is described hereinbelow in detail with reference to Figs. 24A - 25B. The automated diagnostic instrument 100 still further comprises an optical assembly 1580, which is described hereinbelow in detail with reference to Figs. 26A - 28D.

Reference is now made to Figs. 12A, 12B, 12C and 12D, which are simplified assembled view illustrations, taken from various different directions, of cartridge holder assembly 1510 forming part of the instrument of Figs. 11A and 11B, to Figs. 13A, 13B, 13C and 13D, which are simplified exploded view illustrations taken from various mutually different directions of the cartridge holder assembly of Figs. 12A - 12D, and to Fig. 13E, which is a simplified exploded view illustration of part of the cartridge holder assembly of Figs. 12A - 13D.

As seen in Figs. 12A - 13E, cartridge holder assembly 1510 preferably comprises a base portion 1600 and a cover portion 1602 mounted thereon. An opening 1604 is defined between base portion 1600 and cover portion 1602, for insertion of cartridge 110. Cartridge holder assembly 1510 is preferably operative to hold cartridge 110 within instrument 100 during registration, clamping, functionalization and subsequent operation thereof, as is further detailed below with reference to Figs. 31A - 36B.

As seen particularly in Figs. 13C and 13D, cover portion 1602 preferably comprises a flexible concertinaed element 1610, preferably coupled to optical assembly 1580 (Fig. 11B) at an end 1620 thereof. Concertinaed element 1610 is preferably formed of a light absorbing material and provides a light seal between cartridge holder assembly 1510 and optical assembly 1580.

Cover portion 1602 further preferably comprises a cartridge holder assembly plate 1630, preferably embodied as a printed circuit board (PCB). Concertinaed element 1610 is preferably mounted on cartridge holder assembly plate 1630 via four mounting holes 1632. Cartridge holder assembly plate 1630 is preferably formed with a first circular registration aperture 1640 and a second elliptical registration aperture 1642, for enabling precise positioning of cartridge holder assembly plate 1630 and hence of cartridge 110 within instrument 100. First and second registration apertures 1640 and 1642 are respectively located on opposite sides of an illumination and imaging aperture 1644 formed in cartridge holder assembly plate 1630, through which illumination and imaging aperture 1644 a portion of cartridge 110 may be both illuminated and imaged by optical assembly 1580 (Fig. 11B) during operation of cartridge 110.

Mounted on cartridge holder assembly plate 1630 along a rim of illumination and imaging aperture 1644 is a pair of pogo pins 1646. Pogo pins 1646 are preferably mounted on a pogo pin mounting plate 1648 adhered to cartridge holder assembly plate 1630. Pogo pins 1646 are preferably operative to electrically connect to electrodes of carbon array assembly 120 (Fig. 8A), as is further detailed hereinbelow with reference to Figs. 37 - 39.

Cartridge holder assembly plate 1630 is preferably additionally formed with a plurality of holes 1650 for the mechanical mounting of cartridge holder assembly plate 1630. Cartridge holder assembly plate 1630 is preferably further formed with a serpentine portion 1652 adapted for mounting thereon of a cartridge location switch 1654. Cartridge location switch 1654 serves to limit the motion of cartridge holder assembly plate 1630 within machine 100, by changing position upon cartridge 110 being fully inserted within instrument 100, as is further detailed hereinbelow with reference to Fig. 33B.

Cartridge holder assembly plate 1630 is preferably further formed with a generally square aperture 1656 useful for initial alignment of cartridge 110 within instrument 100, during insertion of cartridge 110.

Cover portion 1602 additionally preferably comprises a base plate 1660 adapted for the mounting of cartridge holder assembly plate 1630 thereon. Base plate 1660 is preferably formed with a pair of first and second symmetrical registration apertures 1670 and 1672 respectively located on opposite sides of a base plate illumination and imaging aperture 1674 and respectively underlying first and second registration apertures 1640 and 1642. Base plate illumination aperture 1674 preferably underlies illumination aperture 1644. An additional aperture 1676 is preferably also formed in base plate 1660 to accommodate upper and lower thermocycling assemblies 1560 and 1570 (Fig. 11B), as is further detailed hereinbelow with reference to Fig. 39.

Base plate 1660 is additionally formed with seven liquid sensor sockets 1680 housing seven liquid sensors 1682 correspondingly therein. Liquid sensors 1682 serve to sense the flow of liquid in cartridge 110 during the operation thereof. Further details concerning the structure and operation of liquid sensors 1682 are provided hereinbelow with reference to Figs. 47 - 49.

Base plate 1660 preferably further includes four holes 1684 and an additional four mechanical mounting holes 1686. As seen particularly in Fig. 13D, a plurality of grooves 1688 is preferably formed on a lower surface of base plate 1660. Grooves 1688 are preferably shaped so as to accommodate frangible seal opening elements when cartridge 110 is clamped in instrument 100, as is further detailed hereinbelow with reference to Fig. 36A.

As seen particularly in Figs. 13C and 13E, base portion 1600 of cartridge holder assembly 1510 preferably comprises a frame 1690 defining a central recess 1692. Four posts 1694 are preferably mounted at respective corners of frame 1690, for connection to corresponding holes 1684. An additional four tubular elements 1696 are preferably inserted through four corresponding apertures 1698 in frame 1690. Tubular elements 1696, having throughgoing bores 1697, are preferably aligned with corresponding mechanical mounting holes 1686, in order to facilitate the connection of cartridge holder assembly 1510 to cartridge clamping side sub-assemblies 1530 (Fig. 11B), as is further detailed hereinbelow with reference to Figs. 16A - 17.

A first set of three wheels 1700 is preferably located on frame 1690 on a first side of recess 1692 and a second set of three wheels 1702 is preferably located across therefrom. Wheels 1700 and 1702 are preferably mounted on frame 1690 by way of wheel supports 1704 and are preferably operative as passive wheels, facilitating motion of cartridge insertion drive assembly 1520 (Fig. 11B) therealong. Wheels 1700 and 1702 are preferably formed of a high friction material, such as rubber.

A first hooked element 1710 is preferably appended to frame 1690 in the region of first set of wheels 1700 and a second hooked element 1712 is preferably appended to frame 1690 in the region of second set of wheels 1702. First and second hooked elements 1710 and 1712 are adapted for engagement with cartridge clamping side sub-assembly 1530 (Fig. 11B) in order to clamp cartridge 110 in instrument 100, as is detailed hereinbelow with reference to Figs. 16A - 17B.

Frame 1690 is preferably formed with a generally square protruding portion 1720, adapted for the entry of a portion of lower thermocycling assembly 1570 (Fig. 11B) therein, as is described in further detail hereinbelow with reference to Figs. 24A - 25B.

Reference is now made to Figs. 14A, 14B, 14C and 14D, which are simplified assembled view illustrations, taken from various different directions, of a first cartridge insertion drive assembly 1520, forming part of the instrument of Figs. 11A and 11B, and to Figs. 15A and 15B, which are simplified exploded view illustrations, taken from various different directions, of the cartridge insertion drive assembly 1520 of Figs. 14A - 14D. It is appreciated that the second cartridge insertion drive assembly 1520 is a mirror image of the first cartridge insertion drive assembly.

As seen in Figs. 14A - 15B, first cartridge insertion drive assembly 1520 preferably comprises a support portion 1800 for the mounting thereof on a corresponding cartridge clamping side sub-assembly 1530 (Fig. 11B) and a motorized head portion 1802. Cartridge insertion drive assembly 1520 is preferably operative to drive cartridge holder assembly 1510 and thereby move cartridge 110 within instrument 100, as is detailed hereinbelow with reference to Figs. 35B and 36B.

Support portion 1800 preferably comprises an erect support member 1804 and an upper shoulder member 1806 attached thereto. As seen particularly in Figs. 15A and 15B, erect support member 1804 is preferably formed with a pair of mounting holes 1808 mateable with a corresponding pair of holes 1810 formed in scalloped recesses 1812 on upper shoulder member 1806. Upper shoulder member 1806 is additionally formed with mounting holes 1814 and 1816 for facilitating the mounting of components of head portion 1802 thereon. Support member 1804 is preferably additionally formed with a pair of mounting holes 1818 on a lower portion thereof.

Head portion 1802 preferably comprises a motor 1820 coupled to a motor holder 1822. A motor wheel 1824 is preferably cooperatively connected to motor 1820 by a bearing 1826 and an axle 1828. A pair of pulley wheels 1830 is preferably provided straddling motor wheel 1824. A drive belt 1832 is preferably looped over pulley wheels 1830. Two bearings 1840 are preferably respectively coupled to pulley wheels 1830 and an additional bearing 1842 is preferably coupled to motor wheel 1824.

Head portion 1802 preferably additionally comprises a central wheel 1850 aligned with motor wheel 1824 by way of an additional bearing 1852. Head portion 1802 preferably further comprises two peripheral wheels 1860 respectively aligned with pulley wheels 1830 by way of yet additional bearings 1862 and respective axles 1864. In operation of cartridge insertion drive assembly 1520, central wheel 1850 is preferably driven by motor wheel 1824, which in turn produces motion of peripheral wheels 1860.

Peripheral wheels 1860 are preferably respectively strung on two arms 1866. Arms 1866 are preferably pivotable, about pivot pins 1867, at respective ends 1868 thereof, such that peripheral wheels 1860 may be raised in order to allow the passage of cartridge 110 therebeneath, as is further detailed hereinbelow with reference to Figs. 32B and 32C.

Central wheel 1850 and peripheral wheels 1860 are preferably rotatably mounted on an anterior mounting plate 1870. Mounting plate 1870 is formed with a central aperture 1872 therein for the mounting of central wheel 1850 thereon by way of bearing 1852. Mounting plate 1870 is additionally formed with two peripheral apertures 1874 respectively located on either side of central aperture 1872 for the respective seating of pivot pins 1867 therein. Mounting plate 1870 preferably includes a pair of mounting apertures 1876 formed in a bottom surface thereof which are preferably coupled to mounting holes 1816 via screws (not shown).

Central and peripheral wheels 1850 and 1860 are preferably formed of a high friction material, such as rubber, and are preferably operative to cooperate with respective wheels 1700 and 1702 of cartridge holder assembly 1510 (Fig. 11B).

Reference is now made to Figs. 16A and 16B, which are simplified assembled view illustrations, taken from different directions, a first cartridge clamping side sub-assembly 1530, forming part of the instrument of Figs. 11A and 11B, and to Fig. 17, which is a simplified exploded view illustration of the cartridge clamping side subassembly 1530 of Figs. 16A & 16B. It is appreciated that the second cartridge clamping side sub-assembly 1530 is a mirror image of the first cartridge clamping side subassembly.

As seen in Figs. 16A - 17, cartridge clamping side sub-assembly 1530 comprises a body portion 1900 having a pivotable arm 1902 mounted thereon. Body portions 1900 of cartridge clamping side sub-assemblies 1530 preferably form the sides of the chassis of instrument 100 and lend support to the entire structure thereof.

As seen particularly in Fig. 17, body portion 1900 is preferably formed with a first notch 1910 and a second notch 1912 along an upper edge 1914 thereof. A first support member 1920 is preferably engaged with first notch 1910 and a second support member 1922 is preferably engaged with second notch 1912. Body portion 1900 is preferably additionally formed with a throughgoing recess 1930 along a lower edge 1932 thereof for allowing wiring to pass therethrough, and an indent 1934, also along lower edge 1932, for facilitating attachment of cartridge clamping drive assembly 1540 (Fig. 11B) to body portion 1900 of cartridge clamping side sub-assembly 1530.

A rail 1940 is preferably attached to body portion 1900 in the region of an indent 1942. Rail 1940 serves to mount upper thermocycling assembly 1560 (Fig. 11B) on body portion 1900. A first pillar 1946 preferably extends from a first pillar mounting hole 1948 and a second pillar 1950 preferably extends from a second pillar mounting hole 1952, which first and second pillar mounting holes 1948 and 1952 are preferably located at respective ends of upper edge 1914. First and second pillars 1946 and 1950 are preferably insertable through the respective bores 1697 of tubular elements 1696 of cartridge holder assembly 1510 (Fig. 12A) in order to facilitate the mounting of cartridge holder assembly 1510 on cartridge clamping side sub-assembly 1530.

Pivotable arm 1902 is preferably pivotably attached to body portion 1900 by way of a pivot pin 1960 insertable in a pivot pin aperture 1962 in body 1900. Pivotable arm 1902 is preferably formed with an additional pivot pin aperture 1964 adapted to allow pivot pin 1960 to pass therethrough and be attached thereto by a pivot pin cover 1966.

Pivotable arm 1902 preferably comprises a driving handle portion 1970 and a driven head portion 1972. Back and forth motion of handle portion 1970, as driven by cartridge clamping drive assembly 1540 (Fig. 11B), serves to create up and down motion of head portion 1972, such that head portions 1972 of cartridge clamping side sub-assemblies 1530 may engage with respective hooked element 1710 and 1712 of base portion 1600 (Fig. 13E), in a manner described in detail hereinbelow with reference to Figs. 34A-36B. Handle portion 1970 is preferably formed with an apertured notch 1980 at an extremity thereof to allow entry of a wheel 1982 therein. Head portion 1972 is preferably also formed with an apertured notch 1984 therein at an extremity thereof, in order to allow entry of another wheel 1986 therein. Wheels 1982 and 1986 serve to assist the motion of pivotable arm 1902.

Reference is now made Figs. 18A, 18B and 18C, which are simplified assembled view illustrations, taken from various different directions, of cartridge clamping drive assembly 1540, forming part of the automated diagnostic instrument 100 of Figs. 11A and 11B, and to Figs. 19A and 19B, which are simplified exploded view illustrations taken from different directions of cartridge clamping drive assembly 1540 of Figs. 18A - 18C.

As seen in Figs. 18A - 19B, cartridge clamping drive assembly 1540 preferably comprises a first side wall 2000 having a first U-shaped notch 2002 therein and a second side wall 2004 having a second U-shaped notch 2006 therein. Notches 2002 and 2006 are preferably adapted to respectively receive handle portions 1970 (Fig. 17) of cartridge clamping side sub-assemblies 1530 (Fig. 11B) of automated diagnostic instrument 100, thereby allowing pivotable arms 1902 of cartridge clamping side sub-assemblies 1530 to be driven by cartridge clamping drive assembly 1540 so as to clamp cartridge 110 within instrument 100, as is further detailed hereinbelow with reference to Figs. 34A-36B.

As seen particularly in Figs. 19A and 19B, cartridge clamping drive assembly 1540 is preferably driven by a motor 2010 coupled to a gear box 2012 by way of a motor mounting plate 2014. Gear box 2012 preferably includes a variety of gears 2016 for controlling the drive force of motor 2010. A screw 2020 is preferably linearly driven by motor 2010 via the interaction of gears 2016 therewith. Motor mounting plate 2014 is preferably formed with a screw hole 2022 therein, for insertion therethrough of screw 2020.

A first end 2024 of screw 2020 is preferably adapted to rest in a screw hole 2026 of a motor base plate 2028, anterior to which motor base plate 2028 gears 2016 are located. A second end 2030 of screw 2020 is preferably adapted to rest on an outer bolt 2032. Outer bolt 2032 is preferably bolted to a front plate 2034 via connecting components 2036. A bolt 2040 and a corresponding bolt holder 2042 are preferably provided along the body of screw 2020, between motor base plate 2028 and front plate 2034.

As seen particularly in Figs. 18B and 18C, a movable frame 2044 coupled to screw 2020 is preferably formed by first and second side walls 2000 and 2004, fastened to a base element 2046 and an upper bridge element 2048 via screws (not shown). Upper bridge element 2048 is preferably formed with an aperture 2050 therein, adapted to accommodate an upper rim 2052 of bolt holder 2042 therein. Base element 2046 is preferably formed with a pair of rectangular recesses 2054 connected by a longitudinal recess 2056. Longitudinal recess 2056 accommodates a lower rim 2058 of bolt holder 2042. It is appreciated that motion of screw 2020 as driven by motor 2010 thus serves to move the entirety of moveable frame 2044, including first and second side walls 2000 and 2004, base element 2046 and upper bridge element 2048.

Cartridge clamping drive assembly 1540 additionally preferably comprises a first static inner wall 2060, adjacent to first side wall 2000, and a second static inner wall 2062, adjacent to second side wall 2004. Static wall 2062 is formed with an elongate U-shaped notch 2063 for mounting motor 2010 therein. A first guide rail 2064 is preferably abutted to a base of first static inner wall 2060 and a second guide rail 2066 is preferably abutted to a base of second static inner wall 2062. Movable frame 2044 is preferably slideable along first and second guide rails 2064 and 2066, respectively, by way of first and second slidable members 2070 and 2072, which are seated in rectangular recesses 2054 and fastened to base element 2046 via screws (not shown). A pair of limiters 2074 may be mounted on an upper edge 2076 of first static inner wall 2060.

Reference is now made to Figs. 20A, 20B, 20C and 20D, which are simplified assembled view illustrations, taken from various different directions, of cartridge actuation assembly 1550, forming part of the instrument of Figs. 11A and 11B, to Figs. 20E and 20F, which are planar side views of the cartridge actuation assembly 1550 of Figs. 20A - 20D, to Figs. 21A, 21B and 21C, which are simplified exploded view illustrations taken from various mutually different directions of the cartridge actuation assembly 1550 of Figs. 20A - 20F, to Figs. 21D and 21E, which are simplified enlarged exploded view illustrations of portions of cartridge actuation assembly 1550 of Figs. 20A - 21C, and to Figs. 21F, 21G and 21H, which are simplified illustrations of part of cartridge actuation assembly 1550 of Figs. 20A - 21E.

Cartridge actuation assembly 1550 is preferably the principal active component of instrument 100 and serves to register, clamp, functionalize and operate cartridge 110 therein, as is further detailed hereinbelow with reference to Figs. 34A - 46.

Turning now to Figs. 20A - 21H, cartridge actuation assembly 1550 is seen to comprise a base element 2100 having formed therein a magnet recess 2102 for the mounting of a magnet 2104 therein, a heater recess 2106 for the mounting of a heater 2108 therein and a pump actuator indent 2110 for the mounting of a pump actuator 2112 therein. As seen in Fig. 21F, magnet 2104 preferably comprises a magnetic portion 2113, a spring 2114 and magnet motor 2215.

Base element 2100 is additionally formed with a waste enclosure recess 2116, for accommodating liquid waste enclosure 252 (Fig. 2I) of cartridge 110.

A first template piece 2120 and a second template piece 2122 are preferably disposed on an upper surface 2124 of base element 2100. A first registration pin 2126 and a second registration pin 2128 are preferably respectively mounted in a first and a second registration pin aperture 2130 and 2132, which first and a second registration pin apertures 2130 & 2132 are preferably respectively formed in first and second template pieces 2120 & 2122. First and second registration pins 2126 and 2128 serve to align and register cartridge 110 within instrument 100, prior to the functionalization thereof, as is described in further detail hereinbelow with reference to Figs. 35A - 36B.

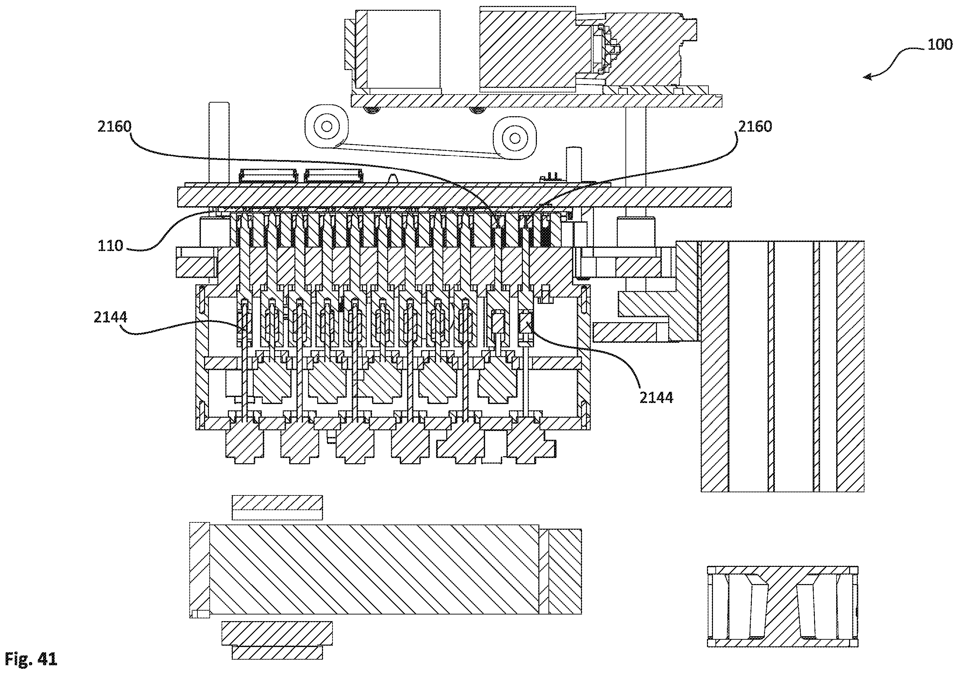

First and second template pieces 2120 and 2122 are each formed with an array of apertures 2140 therein, corresponding to an array of apertures 2141 formed in base element 2100. As best appreciated from consideration of Fig. 21B, two types of pins, namely frangible seal opening pins 2142 and linearly displaceable valve pins 2144, are provided extending through apertures 2140 and 2141. Frangible seal opening pins 2142 are preferably operative to unseal frangible seals in cartridge 110, in order to fully functionalize cartridge 110, as described in greater detail hereinbelow with reference to Fig. 36A. Valve pins 2144 are preferably operative during the operation of cartridge 110, following the full functionalization thereof, in order to control fluid flow therein, as described in greater detail hereinbelow with reference to Figs. 41 and 43.

Preferably, 20 frangible seal opening pins 2142 are provided extending through base element 2100 and first template piece 2120 and 22 frangible seal opening pins 2142 are preferably provided extending through base element 2100 and second template piece 2122. As seen in Figs. 21C and 21E, each frangible seal opening pin 2142 preferably comprises a frangible seal opening pin tip 2150, a spring portion 2152 and a pin body portion 2154.

Preferably, 16 linearly displaceable valve pins 2144 are provided extending through base element 2100 and first template piece 2120 and 13 linearly displaceable valve pins 2144 are preferably provided extending through base element 2100 and second template piece 2122. As seen in Figs. 21C and 21D, each linearly displaceable valve pin 2144 preferably comprises a valve pin tip 2160, a spring portion 2162 and a pin body portion 2164. Each linearly displaceable valve pin 2144 additionally comprises a valve pin linear driving motor 2166, for controlling the motion of the respective linearly displaceable valve pin 2144, along a pin displacement axis 2168, with respect to a corresponding valve within cartridge 110, so as to open and close respective valves within cartridge 110 during the operation thereof.

It is appreciated that a relatively very high density of linearly displaceable valve pins 2144 is required. It is a particular feature of an arrangement of the present disclosure that in order to realize this relatively high density of linearly displaceable valve pins 2144, there is provided a plurality of valve pin linear driving motors 2166 comprising first and second arrays of valve pin linear driving motors 2166, the arrays being arranged in first and second planes extending generally perpendicularly to pin displacement axis 2168, the first plane lying above the second plane and the valve pin linear driving motors 2166 of the first and second arrays in the first and second planes being mutually offset from each other, in an interdigitated manner. It is appreciated that pin displacement axis 2168 is preferably vertical, in the sense of Fig. 20E, and first and second planes are generally horizontal in the sense of Fig. 20E.

A first motor housing 2170 and a second motor housing 2172 are preferably provided in respective alignment with first and second template pieces 2120 and 2122, for housing valve pin linear driving motors 2166 therein. As seen particularly in Figs. 21C and 21D, each of first and second motor housings 2170 and 2172 preferably includes a pair of side support pieces 2173, between which side support pieces 2173 are arranged an upper motor plate 2174 and a lower motor plate 2176. Motor plates 2174 and 2176 are preferably arranged in a tiered manner in order to facilitate a compact arrangement of valve pin linear driving motors 2166. Motor plates 2174 and 2176 are each preferably formed with motor apertures 2180 therein, through which motor apertures 2180 valve pin linear driving motors 2166 extend. As seen particularly in Fig. 21D, a first set 2182 of shorter valve pin linear driving motors 2166 is associated with each upper motor plate 2174 and a second set 2184 of longer valve pin linear driving motors 2166 is associated with each lower motor plate 2176.

Reference is now made to Figs. 22A, 22B and 22C, which are simplified assembled view illustrations, taken from various different directions, of upper thermocycling assembly 1560 forming part of the instrument of Figs. 11A and 11B, and to Figs. 23A and 23B, which are simplified exploded view illustrations taken from mutually different directions of upper thermocycling assembly 1560 of Figs. 22A, 22B and 22C.

As seen in Figs. 22A - 23B, upper thermocycling assembly 1560 preferably comprises a heating element 2200 mounted on a clamp portion 2202 and a heat dissipation assembly portion 2204 also mounted on clamp portion 2202. Heating element 2200 of upper thermocycling assembly 1560 is preferably operative to provide highly accurate heating of an upper surface of cartridge 110, when cartridge 110 is clamped by clamp portion 2202 during the operation thereof within instrument 100.

As seen particularly in Figs. 23A and 23B, heating element 2200 is preferably embodied as a Peltier heater 2210. Adhered to an upper surface of Peltier heater 2210 is a first double sided adhesive layer 2212. Adhered to a lower surface of Peltier heater 2210 is a second double sided adhesive layer 2214. A first flexible thermal layer 2216 is preferably adhered to first double-sided adhesive layer 2212, interfacing first double-sided adhesive layer 2212 and clamp portion 2202. A second flexible thermal layer 2218 is preferably adhered to second double-sided adhesive layer 2214, interfacing second double sided adhesive layer 2214 and a portion of cartridge 110, when cartridge 110 is held within instrument 100.

A pair of heat sensors 2219 is preferably disposed in proximity to respective upper and lower surfaces of Peltier heater 2210 in order to sense the temperatures thereof. One of heat sensors 2219 is preferably disposed between first double sided adhesive layer 2212 and first flexible thermal layer 2216 and the other one of heat sensors 2219 is preferably disposed between second double sided adhesive layer 2214 and second flexible thermal layer 2218. Wiring (not shown) associated with heat sensors 2219 may be accommodated by notches formed in first and second flexible thermal layers 2216 and 2218.

Heat dissipation assembly portion 2204 preferably comprises a heat sink 2220 and a fan 2222 coupled thereto. Heat sink 2220 preferably comprises a multiplicity of internal ribs 2224 to ensure efficient heat transfer therewithin, so as to maintain a required temperature gradient across Peltier heater 2210. Upper thermocycling assembly 1560 is preferably mountable, by a rail 2228 attached thereto, at a notch 2230 formed in heat sink 2220. A thermal film 2232 is preferably disposed between clamp portion 2202 and heat sink 2220, in order to maximize heat transfer therebetween.

Reference is now made to Figs. 24A, 24B and 24C, which are simplified assembled view illustrations, taken from various mutually different directions of lower thermocycling assembly 1570, forming part of the instrument of Figs. 11A and 11B and to Figs. 25A and 25B, which are simplified exploded view illustrations taken from different directions of lower thermocycling assembly 1570 of Figs. 24A - 24C.

As seen in Figs. 24A - 25B, lower thermocycling assembly 1570 preferably comprises a heating element 2400 mounted on a base 2402 and a heat dissipation assembly portion 2404, attached to base 2402. Heating element 2400 of lower thermocycling assembly 1570 is preferably operative to provide highly accurate heating of a lower surface of cartridge 110, when cartridge 110 is held within instrument 100.

As seen particularly in Figs. 25A and 25B, heating element 2400 is preferably embodied as a Peltier heater 2410. Adhered to an upper surface of Peltier heater 2410 is a first double sided adhesive layer 2412. Adhered to a lower surface of Peltier heater 2410 is a second double sided adhesive layer 2414. A first flexible thermal layer 2416 is preferably adhered to first double-sided adhesive layer 2412, for interfacing first double sided adhesive layer 2412 and a portion of cartridge 110, when cartridge 110 is held within instrument 100. It is appreciated that Peltier heater 2410 having first double sided adhesive layer 2412 and first flexible thermal layer 2416 adhered thereto is preferably adapted for entry into protrusion 1720 of frame 1690 of base 1600 (Fig. 13E). A second flexible thermal layer 2418 is preferably adhered to second double-sided adhesive layer 2414, between second double-sided adhesive layer 2414 and base 2402.

A pair of heat sensors 2419 is preferably disposed in proximity to respective upper and lower surfaces of Peltier heater 2210 in order to sense the temperatures thereof. One of heat sensors 2419 is preferably disposed between first double sided adhesive layer 2412 and first flexible thermal layer 2416 and the other one of heat sensors 2419 is preferably disposed between second double sided adhesive layer 2414 and second flexible thermal layer 2418. Wiring (not shown) associated with heat sensors 2419 may be accommodated by notches formed in first and second flexible thermal layers 2416 and 2418.

Heat dissipation assembly portion 2404 preferably comprises a heat sink 2420 and a fan 2422 coupled thereto. Heat sink 2420 preferably comprises a multiplicity of internal ribs 2424 to ensure efficient heat transfer therewithin, so as to maintain a required temperature gradient across Peltier heater 2410. Lower thermocycling assembly 1570 is preferably mountable by a rail 2428 inserted in a notch 2430 formed within heat sink 2420. A thermal film 2432 is preferably disposed interfacing base 2402 and heat sink 2420, in order to maximize heat transfer therebetween.

Lower thermocycling assembly 1570 preferably also includes an elongate generally planar member 2440 located abutting base 2402. Elongate member 2440 serves to support base 2402 and additionally to allow mounting of lower thermocycling assembly 1570 within instrument 100, by insertion of elongate member 2244 within indent 1934 of cartridge clamping side sub-assembly 1530 (Fig. 16A).

Reference is now made to Figs. 26A, 26B, 26C and 26D, which are simplified assembled view illustrations, taken from various different directions, of optical assembly 1580, forming part of the instrument of Figs. 11A and 11B, to Figs. 27A, 27B and 27C, which are simplified exploded view illustrations taken from various mutually different directions of optical assembly 1580 of Figs. 26A - 27C, to Figs. 28A, 28B, 28C and 28D, which are simplified optical illustrations taken from various mutually different directions of optical assembly 1580 of Figs. 26A - 27C, and to Fig. 28E, which is a simplified illustration of part of the optical assembly of Figs. 26A - 28D.

As seen in Figs. 26A - 28D, optical assembly 1580 preferably comprises a cover 2460 and an illumination and imaging portion 2462 enclosed thereby. Optical assembly 1580 is preferably operative to illuminate and image cartridge 110 during the operation thereof within instrument 100, particularly in order to detect fluorescence arising from carbon array assembly 120 therein.

As seen particularly in Figs. 27A - 28D, illumination and imaging portion 2462 preferably comprises a base plate 2470 having a first illuminator 2472 and a second illuminator 2474 mounted thereon, preferably embodied as LEDs. First and second illuminators 2472 and 2474 are preferably respectively attached to base plate 2470 by first and second illuminator mounts 2476 and 2478. A camera 2480 is preferably additionally mounted on base plate 2470. Camera 2480 preferably comprises a lens portion 2482 and a sensor 2484 coupled thereto, such as, by way of example, a CMOS sensor. Base plate 2470 is preferably formed with an indented region 2486 therein adapted to receive a camera mounting plate 2488 for the mounting of camera 2480 thereon.

Base plate 2470 is preferably formed with a central aperture 2490 therein. Aperture 2490 allows illumination provided by first and second illuminators 2472 and 2474 to reach cartridge 110 when cartridge 110 is held in instrument 100 and further allows fluorescence arising from cartridge 110 to arrive at camera 2480, for the imaging thereof.

A first mirror 2492 is preferably mounted adjacent to aperture 2490 on a first side thereof and a second mirror 2494 is preferably mounted adjacent to aperture 2490 across therefrom. First and second mirrors 2492 and 2494 are preferably respectively attached to base plate 2470 by first and second mirror supports 2496 and 2498.

In operation of illumination and imaging portion 2462, illuminators 2472 and 2474 are preferably operative to illuminate cartridge 110 with illumination in the 615 - 650 nm wavelength. Illuminators 2472 and 2474 preferably each include a filter in order to limit emission to the 615 - 650 nm wavelength range. Fluorescence arising from the carbon array assembly 120 of cartridge 110 preferably propagates through aperture 2490 towards first mirror 2492, is reflected therefrom towards second mirror 2494, and is in turn reflected from second mirror 2490 towards camera 2480. It is appreciated that the arrangement of first and second mirrors 2492 and 2494 allows illumination and imaging portion 2462 to be formed in a highly compact manner.

Camera 2480 preferably includes a filter so as to allow light entry only in the 670 - 710 nm wavelength range. It is understood that the non-overlapping of the bandwidths of illuminators 2472 and 2474 and camera 2480 serves to prevent illumination from illuminators 2472 and 2474 illuminating camera 2480 and thus interfering with the imaging of the fluorescence arising from cartridge 110.

Reference to now made to Fig. 29, which is a simplified functional block-diagram representation of operation of the automated diagnostic instrument of Figs. 11A & 11B with respect to the cartridge of Figs. 2A/1 - 10D.

As seen in Fig. 29, automated diagnostic instrument 100 preferably includes cartridge operation functionality 2600. Cartridge operation functionality 2600 preferably includes cartridge loading functionality 2602, for transporting cartridge 110 into instrument 100 in initial alignment therewith. Cartridge operation functionality 2600 further preferably includes cartridge clamping and frangible seal opening functionality 2604, for precisely aligning cartridge 110 within instrument 100, clamping cartridge 110 and unsealing frangible seals within cartridge 110 in order to fully functionalize cartridge 110.

Cartridge operation functionality 2600 additionally preferably includes sample heating functionality 2610, for heating sample 1402 in sample receiving enclosure 200, and PCR thermal control functionality 2612, for thermal control of the PCR array of cartridge 110. Cartridge operation functionality 2600 still additionally preferably includes valve and pump operation functionality 2614, for selective actuation of valves and activation of pump 168 during various stages in the operation of cartridge 110.

Cartridge operation functionality 2600 yet further preferably includes magnetic bead separation functionality 2618, for retaining magnetic beads in lysis bead containing chamber 202 at certain stages in the operation of cartridge 110. Cartridge operation functionality 2600 still further preferably includes liquid detection functionality 2620, for detecting the presence of liquid at various locations within cartridge 110 during the operation thereof.

Automated diagnostic instrument 100 additionally preferably includes carbon array addressing functionality 2630, for electrically interfacing with carbon array assembly 120 and for connecting a current source thereto. Automated diagnostic instrument 100 still additionally preferably includes camera and illumination functionality 2632, for illuminating and imaging fluorescence arising from carbon array assembly 120.

Automated diagnostic instrument 100 additionally preferably includes a controller 2640. Controller 2640 is preferably a real time processing module operative to control instrument 100 during the operation of cartridge 110 thereby. Controller 2640 is preferably in communication with a user interface module 2642, which user interface module 2642 may interface with a plurality of USB connections 2644 and an internet connection 2646.

Automated diagnostic instrument 100 may optionally include barcode reading functionality 2648, for reading an identifying barcode which may be present on cartridge 110.

Particularly preferred arrangement of components of instrument 100 providing cartridge operation functionality 2600, carbon array addressing functionality 2630 and camera and illumination functionality 2632 have been described hereinabove with reference to Figs. 11B - 28D. Operation of the components of Figs. 11B - 28D is now described hereinbelow with reference to Figs. 30 - 49.

Reference is now made to Fig. 30, which is a simplified top view illustration of the instrument 100 of Figs. 11A - 29, showing various section lines referenced hereinbelow, to Figs. 31A and 31B, which are simplified respective first and second sectional illustrations of an initial stage just prior to insertion of the cartridge 110 of Figs. 2A/1 - 10D into the instrument 100 of Figs. 11A - 29, Figs. 31A and 31B being taken along respective section lines B - B and C - C in Fig. 30, to Figs. 32A - 32C, which are simplified respective pictorial and first and second sectional illustrations of a first stage of insertion of the cartridge 110 of Figs. 2A/1 - 10D into the instrument 100 of Figs. 11A - 29, Figs. 32A and 32B being taken along respective section lines B - B and C - C in Fig. 30, and to Figs. 33A and 33B, which are simplified respective pictorial and sectional illustrations of a second stage of insertion of the cartridge 110 of Figs. 2A/1 -10D into the instrument 100 of Figs. 11A - 29, Figs. 33A and 33B being taken along respective section lines B - B and C - C in Fig. 30.

As seen in Figs. 31A and 31B, and also illustrated pictorially in Fig. 1, at an initial stage just prior to insertion of cartridge 110 in instrument 100, cartridge 110 is entirely external to instrument 100. At this stage, peripheral wheels 1860 of cartridge insertion drive assemblies 1520 are resting upon static wheels 1702 and 1700 (Fig. 13A) of cartridge holder assembly 1510. Cartridge holder assembly 1510 is in an uppermost position thereof, such that concertinaed element 1610 is fully compressed.

Turning now to Figs. 32A - 32C, which show a first stage of insertion of cartridge 110 into instrument 100, cartridge 110 is seen to be partially inserted into instrument 100. As seen particularly in Figs. 32B and 32C, cartridge 110 is inserted into opening 1604 of cartridge holder assembly 1510, interfacing with a first one of peripheral wheels 1860 and a first one of static wheels 1702 and 1700 (Fig. 13A). At this stage, motor 1820 (Figs. 15A and 15B) is activated by controller 2640 (Fig. 29) to rotate central wheel 1850, which in turn rotates peripheral wheels 1860, which peripheral wheels 1860 transport cartridge 110 into instrument 100. Operation of controller 2640 may be initiated via user interface module 2642 (Fig. 29).

Turning now to Figs. 33A and 33B, showing a second stage of insertion of cartridge 110 into instrument 100, cartridge 110 is seen to be fully inserted into instrument 100. As seen particularly in Fig. 33B, cartridge 110 continues to progress through opening 1604 of cartridge holder assembly 1510 until cartridge 110 reaches and activates cartridge location switch 1654. It is appreciated that cartridge holder assembly 1510 preferably remains stationary during the progression of cartridge 110 therethrough. Upon activation of cartridge location switch 1654, controller 2640 (Fig. 29) preferably turns off motor 1820 (Figs. 15A and 15B), thus stopping rotation of central wheel 1850 and hence peripheral wheels 1860, so that cartridge 110 does not advance further within instrument 100.

Reference is now made to Figs. 34A & 34B, which are simplified respective sectional illustrations of an aspect of an initial stage of clamping of the cartridge 110 of Figs. 2A/1 - 10D in the instrument of Figs. 11A - 29, taken along respective section lines B - B and F - F in Fig. 30.

As seen in Figs. 34A and 34B, following the full insertion of cartridge 110 within instrument 100, as shown in Figs. 33A & 33B, cartridge 110 is lowered by cartridge holder assembly 1510 in preparation for registration and clamping. As cartridge holder assembly 1510 is lowered, cartridge 110 approaches registration pin 2128, seen in Fig. 34A, and registration pin 2126 (Fig. 20A) and concertinaed element 1610 becomes extended, as seen particularly in Fig. 34A.

As seen in Fig. 34B, handle portions 1970 of pivotable arms 1902 rest in an upper portion of U-shaped notch 2006, seen in Fig. 34B, and notch 2002 (Fig. 18A) of cartridge clamping drive assembly 1540. Head portions 1972 of pivotable arms 1902 are correspondingly positioned at an entrance to hooked element 1710, seen in Fig. 34B, and hooked element 1712 (Fig. 13E). At this stage, operation of motor 2010 (Fig. 18D) of cartridge clamping drive assembly 1540 is initiated by controller 2640 (Fig. 29) in order to horizontally displace handle portions 1970 and hence vertically displace head portions 1972, thereby lowering cartridge clamping drive assembly 1540.

Reference is now made to Figs. 35A & 35B, which are simplified sectional illustrations of an intermediate stage of clamping of the cartridge 110 of Figs. 2A/1 - 10D in the instrument 100 of Figs. 11A - 29 taken along respective section lines B - B and F - F in Fig. 30.

As seen in Figs. 35A and 35B, at an intermediate stage of the clamping of cartridge 110, cartridge 110 is further lowered by cartridge holder assembly 1510.

As seen in Fig. 35A, as cartridge 110 is further lowered, registration pin 2128, seen in Fig. 35A, and registration pin 2126 (Fig. 20A) respectively enter aperture 142, seen in Fig. 35A, and aperture 140 of cartridge 110. It is appreciated that registration pins 2126 and 2128 are static and that cartridge 110 moves with respect to the registration pins 2126 and 2128. Concertinaed element 1610 is further extended as cartridge holder assembly 1510 is lowered, in a direction indicated by an arrow 2660, away from optical assembly 1580.

As further appreciated from consideration of Fig. 35A, when cartridge 110 is in the intermediate stage of clamping thereof, frangible seal opening pins 2142 are offset from cartridge 110, such that frangible seals of cartridge 110, here collectively designated by reference number 2700, remain sealed.

As seen particularly in Fig. 35B, movable frame 2044 of cartridge clamping drive assembly 1540 is horizontally displaced by motor 2010 (Fig. 18A) in a direction indicated by an additional arrow 2702. Handle portions 1970 of pivotable arms 1902 lying in U-shaped notch 2006, seen in Fig. 35B, and notch 2002 (Fig. 18A) are generally horizontally displaced by the movement of movable frame 2044 and head portions 1972 of pivotable arms 1902 are correspondingly generally vertically displaced, in a direction indicated by yet an additional arrow 2704. Head portions 1972 further engage with hooked element 1710, seen in Fig. 35B, and hooked element 1712 (Fig. 13E), thereby lowering hooked elements 1710 and 1712 and thus lowering the entirety of cartridge holder assembly 1510 along direction 2660.

Reference is now made to Figs. 36A & 36B, which are simplified sectional illustrations of a final stage of clamping of the cartridge 110 of Figs. 2A/1 - 10D in the instrument 100 of Figs. 11A - 29, taken along respective section lines B - B and F - F in Fig. 30.

As seen in Figs. 36A and 36B, at the final stage of the clamping of cartridge 110, cartridge 110 is still further lowered by cartridge holder assembly 1510 to reach a final, fully lowered, position.

In the fully lowered position of cartridge 110, shown in Figs. 36A and 36B, registration pin 2128, seen in Fig. 36A, and registration pin 2126 (Fig. 20A) respectively pass through aperture 142, seen in Fig. 36A, and aperture 140 and respectively enter registration aperture 1672, seen in Fig. 36A, and registration aperture 1670 (Fig. 13C) of cartridge holder assembly 1510. It is appreciated that as a result of the alignment of registration pins 2126 and 2128 with registration apertures both in cartridge 110 and in cartridge holder assembly 1510, cartridge 110 may be precisely positioned within instrument 100 prior to the operation thereof. It is understood that the use of two registration pins 126 and 128 allows precise alignment of cartridge 110 in two dimensions.

As further appreciated from consideration of Fig. 36A, when cartridge 110 is in the fully lowered position thereof, frangible seal opening pin tips 2150 of frangible seal opening pins 2142 enter frangible seals 2700 on cartridge 110, thereby simultaneously unsealing all of the frangible seals 2700 of cartridge 110 and defining the combined pathways between the various channels and through holes, as shown in Fig. 3E and delineated in Table 7. Cartridge 110 is thus rendered ready for operation. It is appreciated that frangible seal opening pin tips 2150 rest in grooves 1688 (Fig. 13D) of cartridge holder assembly 1510 when cartridge 110 is fully clamped in instrument 100.

As seen particularly in Fig. 36B, movable frame 2044 of cartridge clamping drive assembly 1540 is further horizontally displaced by motor 2010 (Fig. 18A) in a direction along additional arrow 2702 (Fig. 35B). Handle portions 1970 of pivotable arms 1902 lying in U-shaped notch 2006, seen in Fig. 36B, and notch 2002 (Fig. 18A) are further generally horizontally displaced by the movement of movable frame 2044 and head portions 1972 of pivotable arms 1902 are correspondingly further generally vertically displaced, along the direction indicated by arrow 2704 (Fig. 35B). Head portions 1972 still further engage with hooked element 1710, seen in Fig. 36B, and hooked element 1712 (Fig. 13E), thereby further lowering hooked elements 1712 and 1710 and thus lowering the entirety of cartridge holder assembly 1510 along direction 2660 (Fig. 35A).

Reference is now made to Fig. 37, which is a simplified sectional illustration of another aspect of the initial stage of clamping of the cartridge 110 of Figs. 2A/1 - 10D in the instrument 100 of Figs. 11A- 29, taken along section lines G - G in Fig. 30.

As seen in Fig. 37, at the initial stage of clamping of cartridge 110, pogo pins 1646 are disposed above cartridge 110, such that no electrical contact is formed between cartridge 110 and pogo pins 1646. Furthermore, upper thermocycling assembly 1560 is not in contact with from cartridge 110.

Reference is now made to Fig. 38, which is a simplified sectional illustration of another aspect of the intermediate stage of clamping of the cartridge 110 of Figs. 2A/1 - 10D in the instrument 100 of Figs. 11A - 29 taken along section lines G - G in Fig. 30.

As seen in Fig. 38, at the intermediate stage of clamping of cartridge 110, pogo pins 1646 contact carbon array assembly 120 of cartridge 110. Furthermore, upper thermocycling assembly 1560 rests on the upper surface of cartridge 110, thereby applying a force thereto.

Reference is now made to Figs. 39, 40, 41 and 42, which are simplified sectional illustrations of other aspects of the final stage of clamping of the cartridge 110 of Figs. 2A/1 - 10D in the instrument of Figs. 11A - 29, Figs. 39, 40, 41 and 42 being taken along respective section lines G - G, E - E, H - H and D - D in Fig. 30.

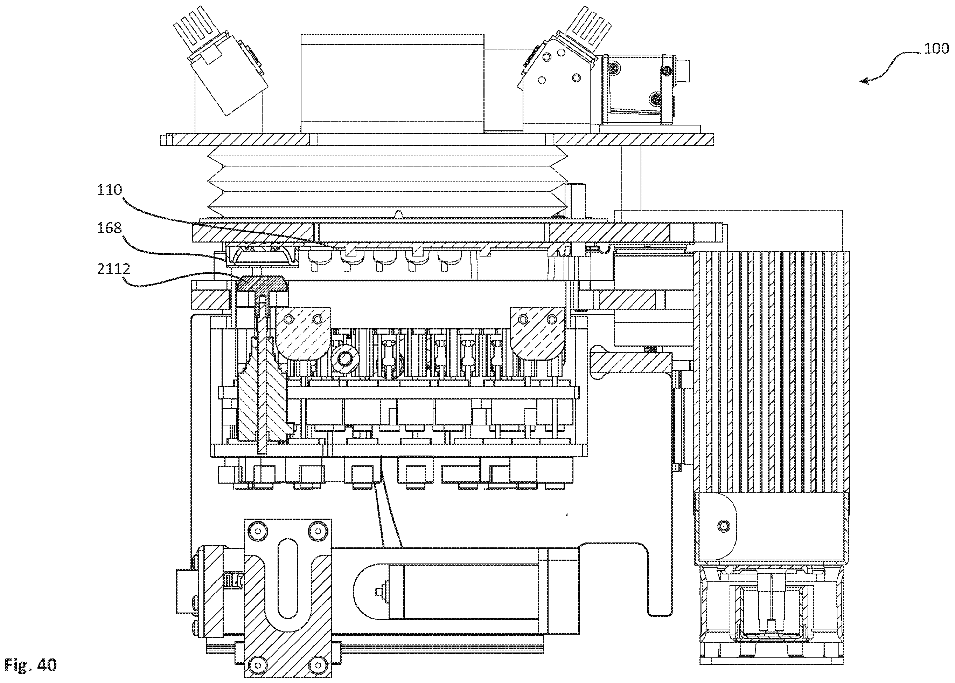

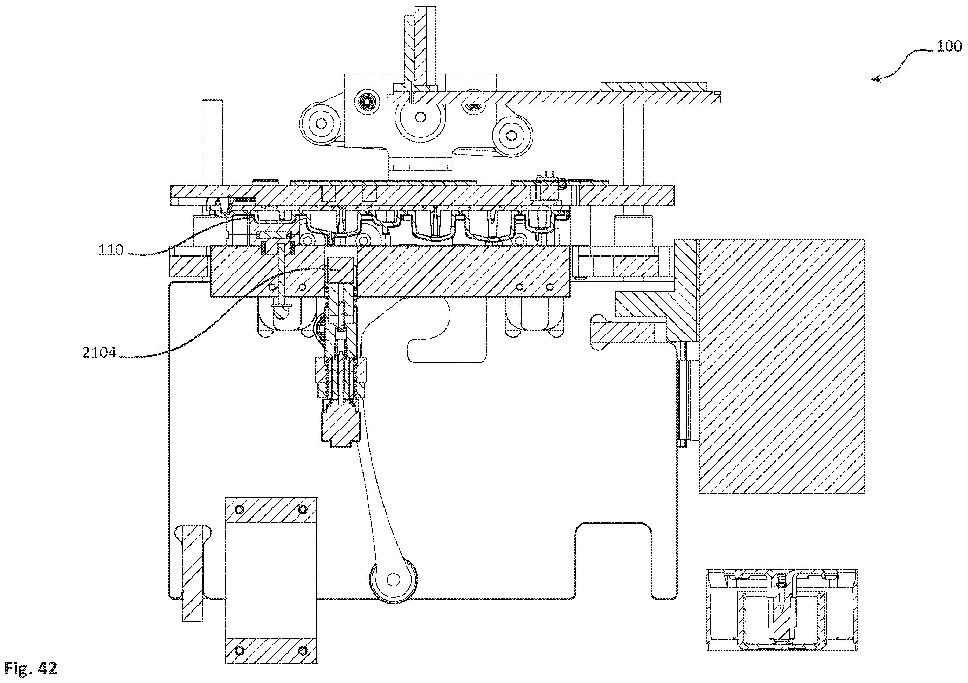

As seen in Fig. 39, at the final stage of clamping of cartridge 110 pogo pins 1646 are compressed against carbon array assembly 120 of cartridge 110. Lower thermocycling assembly 1570 is in contact with the lower surface of cartridge 110. As further seen in Fig. 40, pump actuator 2112 is in a lowered position, offset from cartridge 110. As additionally seen in Fig. 41, valve pin tips 2160 of valve pins 2144 are in contact with cartridge 110, such that all valves of cartridge 110 are closed. As yet further seen in Fig. 42, magnet 2104 is in a lowered non-operative position, offset from cartridge 110.

Reference is now made to Fig. 43, which is a simplified sectional illustration of valve actuators in a valve open operative orientation relative to the cartridge 110 of Figs. 2A/1 - 10D in the instrument 100 of Figs. 11A - 29, taken along section lines H - H in Fig. 30A.

As described hereinabove with reference to Fig. 41, at the final stage of clamping of cartridge 110 all valves of cartridge 110 are closed by valve pins 2144. It is understood, however, that during stages in the operation of cartridge 110 various ones of the valves thereof may be opened in order to allow the flow of fluid therethrough. As seen in Fig. 43, in order to open valves of cartridge 110, valve pin linear driving motors 2166 may be activated by controller 2640 (Fig. 29) so as to lower valve pins 2144, such that spring portions 2162 of valve pins 2144 are compressed and valve pin tips 2160 are withdrawn from cartridge 110.

Reference is now made to Fig. 44, which is a simplified sectional illustration of an operative orientation of a magnet relative to the cartridge 110 of Figs. 2A/1 - 10D in the instrument 100 of Figs. 11A - 29, Fig. 44 being taken along section lines D - D in Fig. 30.

As described hereinabove with reference to Fig. 42, at the final stage of clamping of cartridge 110, magnet 2104 is in a lowered position, offset from cartridge 110. It is understood, however, that during certain stages in the operation of cartridge 110, magnet 2104 is preferably brought in proximity thereto. As seen in Fig. 44, magnet 2104 may be brought into proximity with cartridge 110 by the raising thereof, such that magnet 2104 contacts liquid enclosure 202 of cartridge 110. Operation of magnet 2104 is preferably controlled by controller 2640 (Fig. 29).

Reference is now made to Figs. 45 and 46, which are simplified sectional illustrations of respective first and second operative orientations of a pump actuator relative to the cartridge 110 of Figs. 2A/1 - 10D in the instrument 100 of Figs. 11A - 29, both being taken along section lines E - E in Fig. 30.

As described hereinabove with reference to Fig. 41, at the final stage of clamping of cartridge 110 pump actuator 2112 is in a lowered position, offset from cartridge 110 and thus not contacting pump 168. It is understood, however, that, during certain stages in the operation of cartridge 110, pump actuator 2112 is preferably brought into contact with pump 168 of cartridge 110 in order to actuate pump 168 by changing the volume thereof. An intermediate position of pump actuator 2112 is seen in Fig. 45, at which intermediate position pump actuator 2112 is somewhat raised with respect to the fully lowered position thereof shown in Fig. 41, but pump actuator 2112 is not compressing pump 168. A fully extended position of pump actuator 2112 is seen in Fig. 46, at which fully extended position pump actuator 2112 is pressed against pump 168, thereby changing the volume thereof and actuating pump 168 to pump fluids through channels and passageways in cartridge 110.

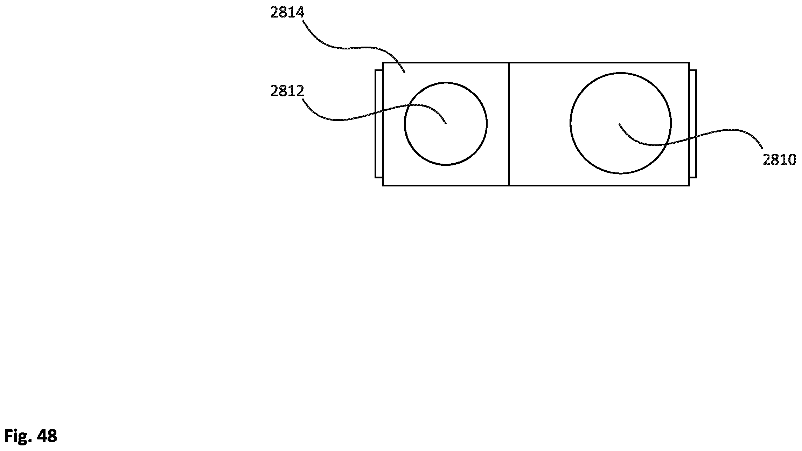

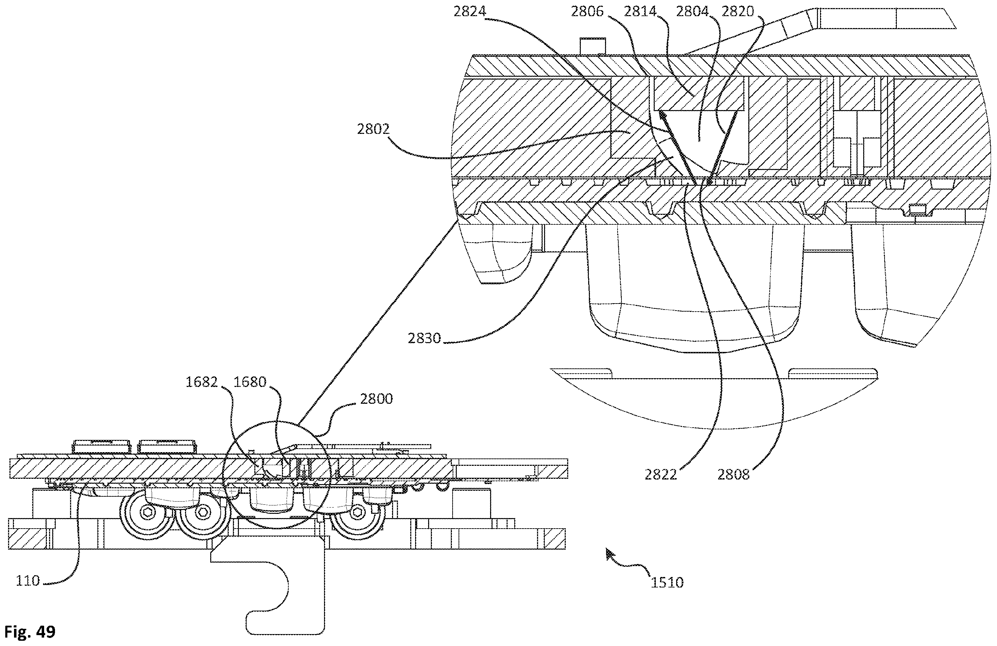

Reference is now made to Fig. 47, which is a simplified illustration of the structure of a liquid sensor 1682 employed in the operation of the cartridge 110 of Figs. 2A/1 - 10D by the instrument 100 of Figs. 11A - 43, Fig. 47 being taken along section lines I - I in Fig. 30; to Fig. 48, which is an top view of a portion of the liquid sensor shown in Fig. 47, and to Fig. 49, which is a functional illustration of the liquid sensor shown in Figs. 47 and 48.

As described hereinabove with reference to Figs. 12A - 13E, cartridge holder assembly 1510 includes a plurality of liquid sensors 1682. Liquid sensors 1682 are preferably operative to detect the presence of liquid in corresponding plurality of liquid detection chambers 640, 642, 644, 646, 648, 650 and 652 (Fig. 3C/5) during the operation of cartridge 110.

As seen in Fig. 47, liquid sensor 1682 is disposed in liquid sensor socket 1680 in cartridge holder assembly 1510, above an upper surface of cartridge 110. As seen particularly at an enlargement 2800, each liquid sensor 1682 preferably includes a housing 2802, defining an internal recess 2804 having a recess upper opening 2806 and a recess lower opening 2808. As seen particularly in Fig. 48, a light source 2810 and a light sensor 2812 are preferably mounted on a mounting element 2814 located within housing 2800 at recess upper opening 2804.

As seen in Fig. 49, light from light source 2810 preferably propagates through recess 2804 and recess lower opening 2808 in a direction indicated by a light propagation arrow 2820, towards a corresponding liquid detection chamber, here designated by reference number 2822. Light reflected from liquid detection chamber 2822 preferably propagates through recess lower opening 2808 and recess 2804 towards sensor 2812, in a direction indicated by a light reflection arrow 2824. The light signal detected by sensor 2812 is preferably indicative of the presence or absence of liquid in liquid detection chamber 2822.

Each liquid sensor 1682 preferably also includes a curved light absorbing element 2830 located in recess 2804. Light absorbing element 2830 preferably absorbs light reflected from regions of cartridge 110 outside liquid detection chamber 2822, thus preventing such reflected light from reaching light sensor 2812 and interfering with the light signal detected thereby. Light absorbing element 2830 is preferably a black element formed of silicon rubber. Light absorbing element 2830 is preferably small enough so as not to intersect with the path of light reflected from liquid detection chamber 2822, and thus only to absorb unwanted light reflected from regions other than liquid detection chamber 2822.

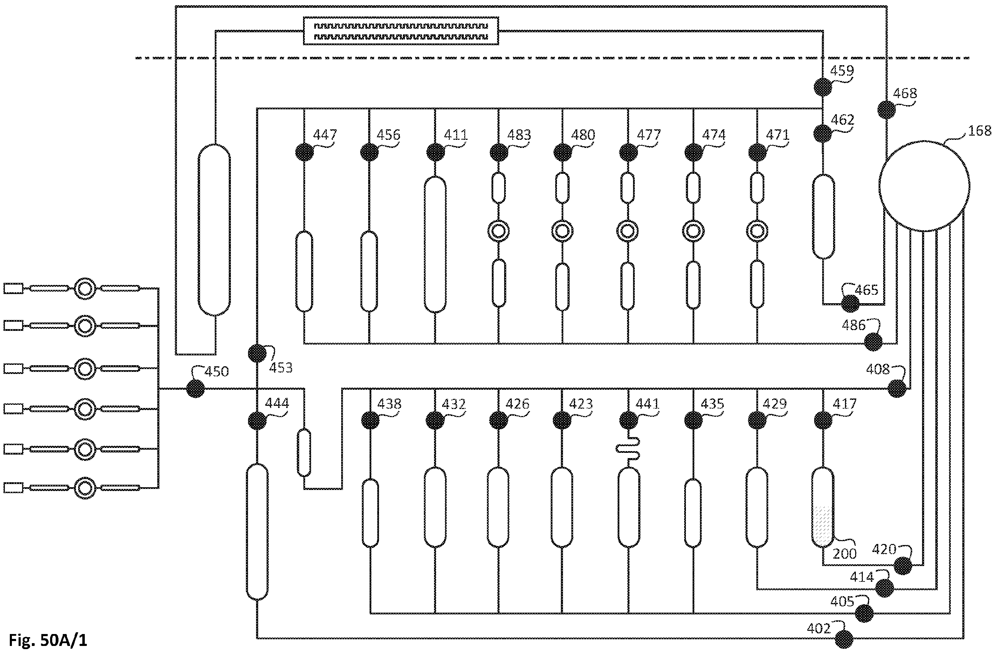

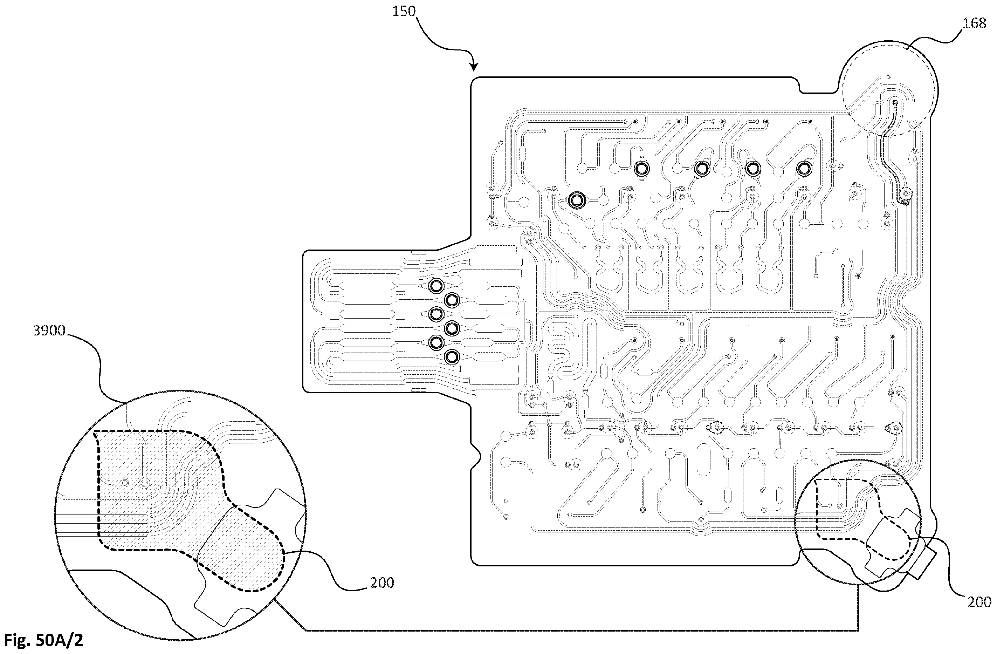

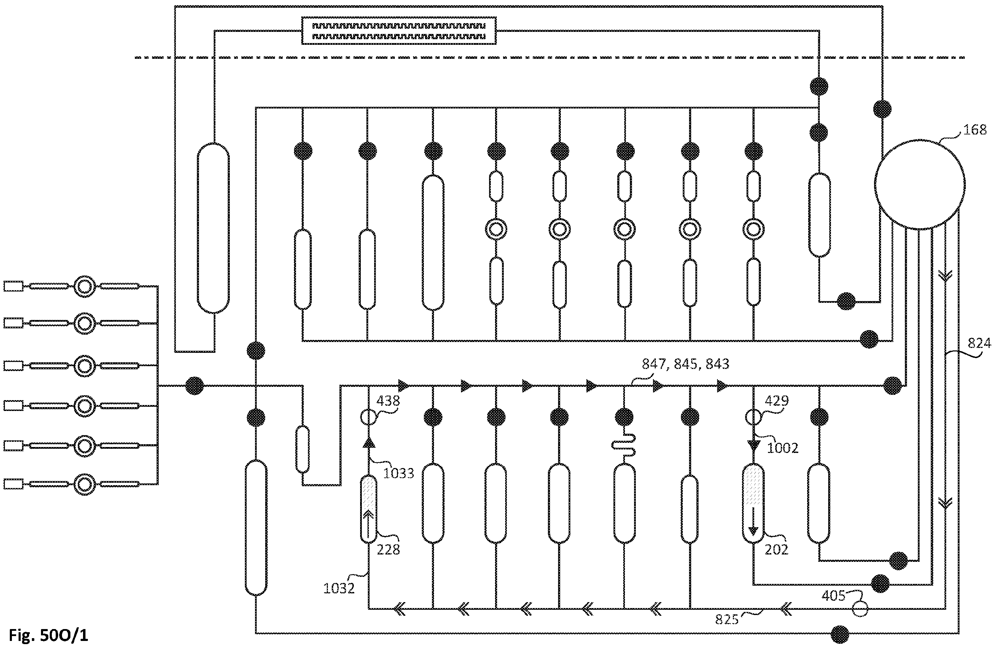

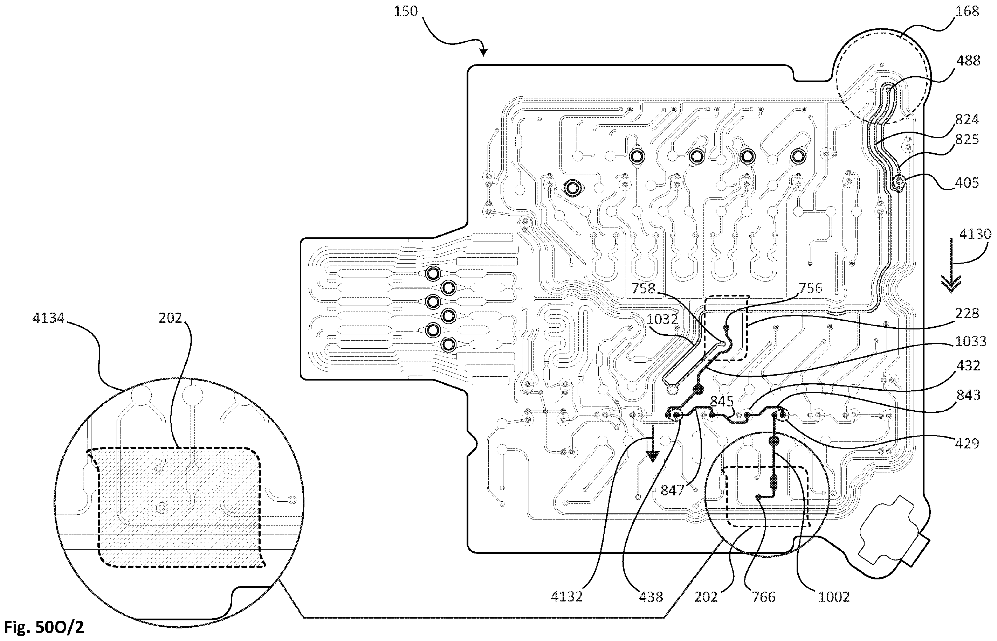

Stages in the operation of cartridge 110 following the functionalization thereof within instrument 100 are now described with reference to Figs. 50A/1 - 50AK/2. In Figs. 50A/1 - 50AK/2, each stage in the operation of cartridge 110 is illustrated by both a schematic illustration and a flow illustration, which schematic and flow illustrations correspond to two alternative representations of the state of cartridge 110 at a given stage in operation thereof.

During operation of cartridge 110, pump 168 (Figs. 2A/1 - 2J) of cartridge 110 is operative to pump air through channels within cartridge 110 and thereby drive liquid between enclosures thereof, for the processing of sample 1402. Pump 168 is actuated by pump actuator 2112, as described hereinabove particularly with reference to Figs. 40, 45 and 46.

Furthermore, during operation of cartridge 110 various ones of valves 402, 405, 408, 411, 414, 417, 420, 423, 426, 429, 432, 435, 438, 441, 444, 447, 450, 453, 456, 459, 462, 465, 468, 471, 474, 477, 480, 483 and 486 described hereinabove with reference to Fig. 3D/1 are selectively opened and closed in order to direct the passage of fluid within cartridge 110. The selectable opening and closing of valves 402, 405, 408, 411, 414, 417, 420, 423, 426, 429, 432, 435, 438, 441, 444, 447, 450, 453, 456, 459, 462, 465, 468, 471, 474, 477, 480, 483 and 486 is controlled by controller 2640, which controls valve pin linear driving motors 2166 to linearly displace valve pin tips 2160, as described hereinabove, particularly with reference to Figs. 41 and 43.

It is appreciated that various valves 402, 405, 408, 411, 414, 417, 420, 423, 426, 429, 432, 435, 438, 441, 444, 447, 450, 453, 456, 459, 462, 465, 468, 471, 474, 477, 480, 483 and 486 (Fig. 3D/1) are selectively opened and closed during the operation of cartridge 110 in order to vent air from liquid enclosures therein. It is appreciated that the open or closed states of the respective valves and corresponding passage of air shown in Figs. 50A/1 - 50AK/2, are only shown with respect to the flow of air driving liquid flow and are not shown with respect to the flow of air for venting. It is appreciated that closed valves are represented by filled in circles and open valves are represented by empty circles in schematic drawings 50A/1 - 50AK/1.

Reference is now made to Figs. S0A/1 and 50A/2, which are simplified respective schematic and flow illustrations of a first stage in the operation of the cartridge 110 of Figs. 2A/1 - 10D by the instrument 100 of Figs. 11A - 49.

As seen in Figs. S0A/1 and 50A/2, all of valves 402, 405, 408, 411, 414, 417, 420, 423, 426, 429, 432, 435, 438, 441, 444, 447, 450, 453, 456, 459, 462, 465, 468, 471, 474, 477, 480, 483 and 486 are closed, thereby preventing the flow of fluid within channels of cartridge 110.

Sample 1402 is located in sample receiving enclosure 200 of cartridge 110, as seen particularly at an enlargement 3900 in Fig. 50A/2, and pump 168 is in a non-operative state.

The stage of operation of cartridge 110 shown in Figs. 50A/1 and 50A/2 corresponds to the state of cartridge 110 immediately following the final clamping thereof by instrument 100, as shown in Figs. 36A and 36B, in which state all valves are closed by valve pins 2144 of instrument 100, as shown in Fig. 41.

Reference is now made to Figs. 50B/1 and 50B/2, which are simplified respective schematic and flow illustrations of a second stage in the operation of the cartridge 110 of Figs. 2A/1 - 10D by the instrument 100 of Figs. 11A - 49, which second stage follows the first stage in operation of the cartridge shown in Figs. S0A/1 and 50A/2.

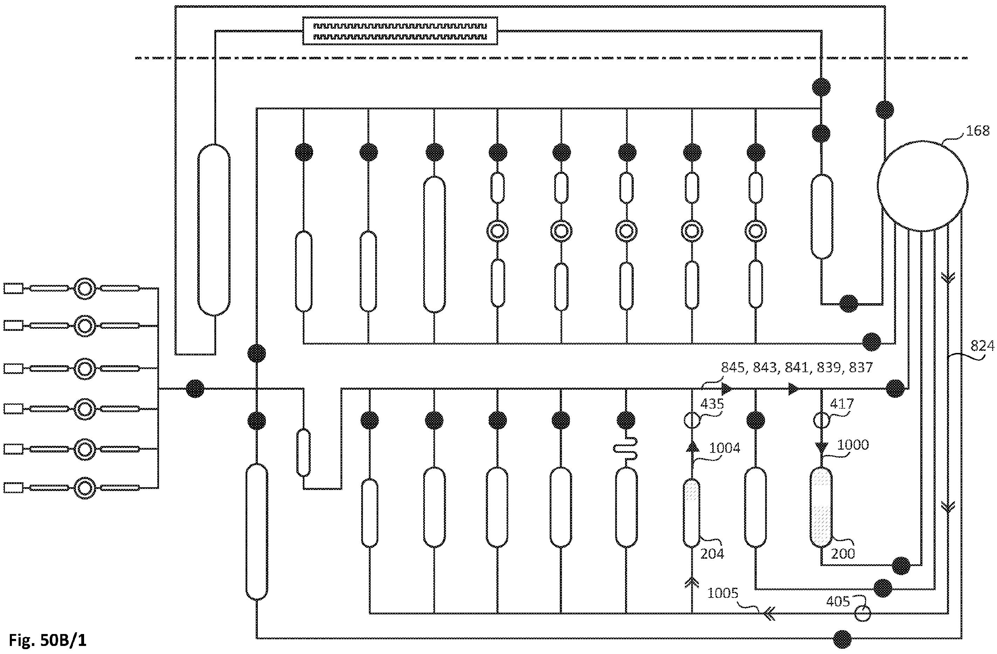

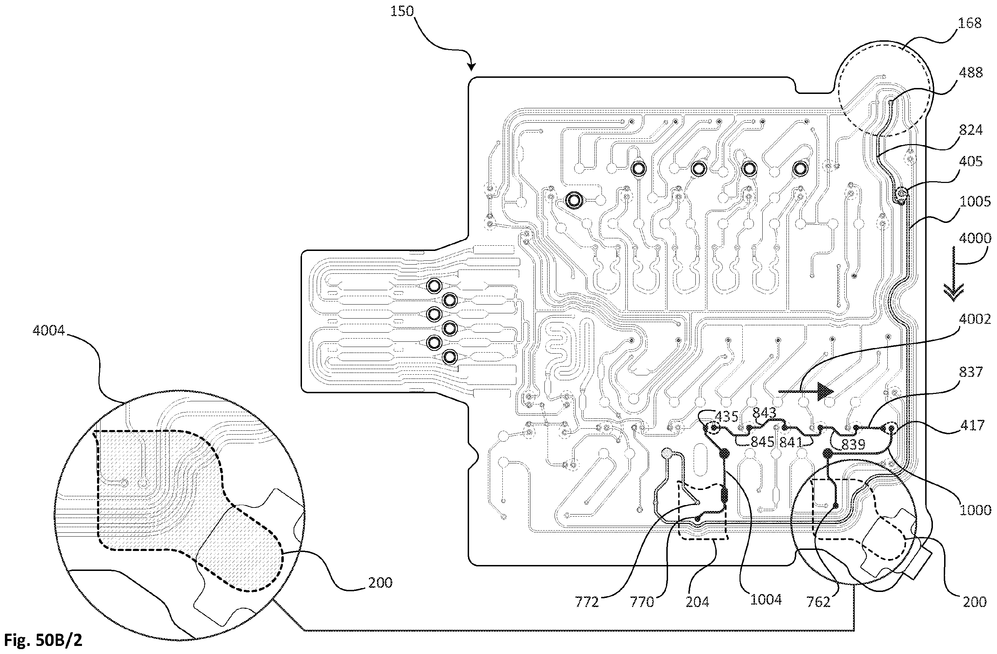

As seen in Figs. 50B/1 and 50B/2, valve 405 is opened, thereby allowing the pumping of air from pump 168 to Protinease K (PK) containing chamber 204 and hence the application of pressure to chamber 204. Air is pumped from pump 168 into chamber 204 along an air flow path comprising channel 824, thereafter open valve 405 and thereafter pathway 1005. Air enters PK containing chamber 204 via through hole 772. The direction of passage of air along the air flow path is indicated by an air flow arrow 4000 in Fig. 50B/2.

As a result of the pressure created by the pumping of air thereinto, the PK liquid held in chamber 204 is driven out of chamber 204 via through hole 770. The PK liquid travels along a liquid flow path comprising pathway 1004, thereafter open valve 435, thereafter channel 845, thereafter channel 843, thereafter channel 841, thereafter channel 839, thereafter channel 837, thereafter open valve 417 and thereafter pathway 1000 leading into sample receiving enclosure 200. The liquid enters sample receiving enclosure 200 via through hole 762. The direction of passage of PK liquid along the liquid flow path is indicated by a liquid flow arrow 4002 in Fig. 50B/2.

The content of sample receiving enclosure 200, following the pumping of air and liquid as described hereinabove, is shown at an enlargement 4004 in Fig. 50B/2. It is appreciated that the representation of the content of sample receiving enclosure 200 in enlargement 4004 is highly schematic and does not distinguish between the various liquids, namely PK and sample 1402, comprising the content.

Reference is now made to Figs. 50C/1 and 50C/2, which are simplified respective schematic and flow illustrations of a third stage in the operation of the cartridge 110 of Figs. 2A/1 - 10D by the instrument 100 of Figs. 11A - 49, which third stage follows the second stage in operation of the cartridge shown in Figs. 50B/1 and 50B/2.

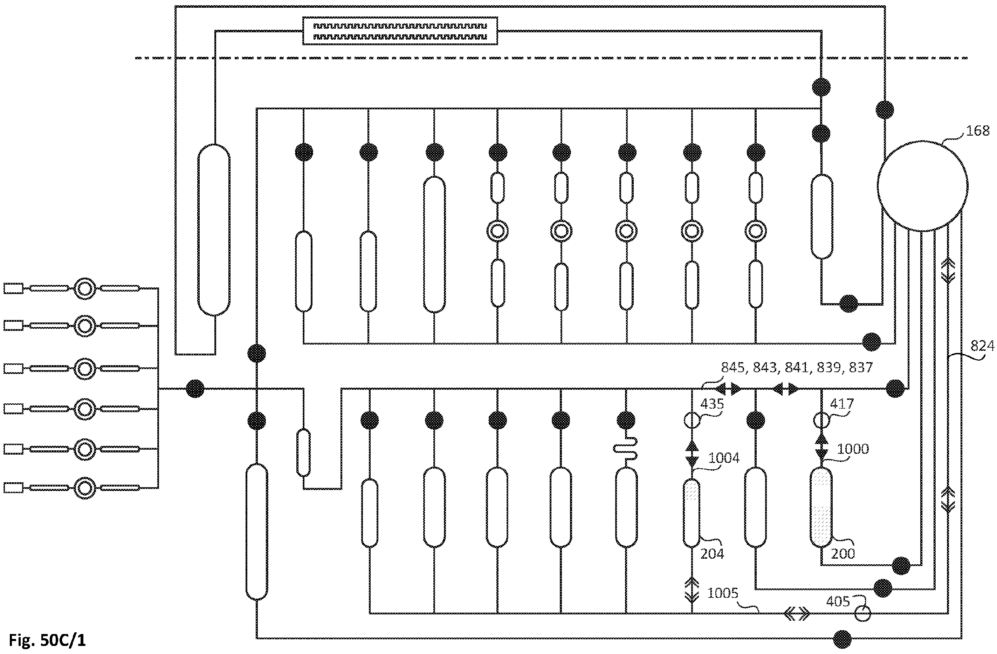

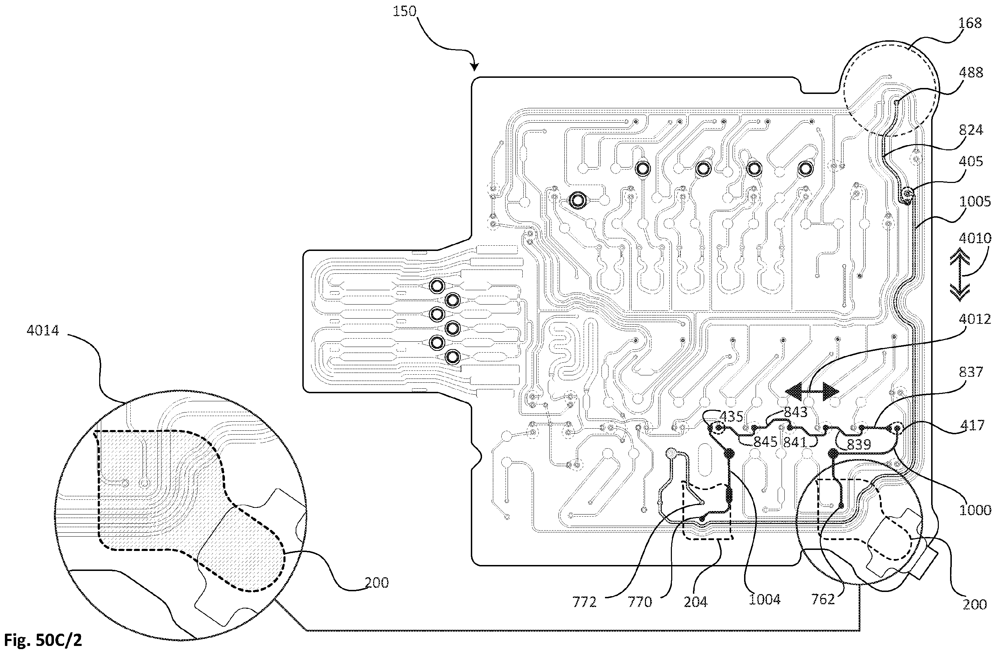

As seen in Figs. 50C/1 and 50C/2, valve 405 remains open and air is repeatedly pumped back and forth between pump 168 and chamber 204. Air travels between pump 168 and chamber 204 along an air flow path comprising channel 824, open valve 405, pathway 1005 and through hole 772. The bi-directional flow of air between pump 168 and chamber 204 is indicated by a double-headed air flow arrow 4010 in Fig. 50C/2.

As a result of the repeated back and forward pressure created by the back and forth passage of air, the liquid contents of sample receiving enclosure 200 are repeatedly driven back and forth between sample receiving enclosure 200 and chamber 204, thereby mixing the PK and sample 1402. The liquid contents of sample receiving enclosure 200 are driven between sample receiving enclosure 200 and chamber 204 along a liquid flow path comprising through hole 770, pathway 1004, open valve 435, channel 845, channel 843, channel 841, channel 839, channel 837, open valve 417, pathway 1000 and through hole 762. The bi-directional passage of liquid between enclosure 200 and chamber 204 is indicated by a double-headed liquid flow arrow 4012 in Fig. 50C/2.

At the completion of mixing, following the passage of air and liquid described hereinabove, the mixed content is returned to sample receiving enclosure 200, as shown schematically at an enlargement 4014 in Fig. 50C/2.

It is appreciated that the contents of sample receiving enclosure 200 may be heated, by activating heater 2108, following the mixing thereof, depending on the processing requirements of sample 1402.

Reference is now made to Figs. 50D/1 and 50D/2, which are simplified respective schematic and flow illustrations of a fourth stage in the operation of the cartridge 110 of Figs. 2A/1 - 10D by the instrument 100 of Figs. 11A - 49, which fourth stage follows the third stage in operation of the cartridge shown in Figs. 50C/1 and 50C/2.

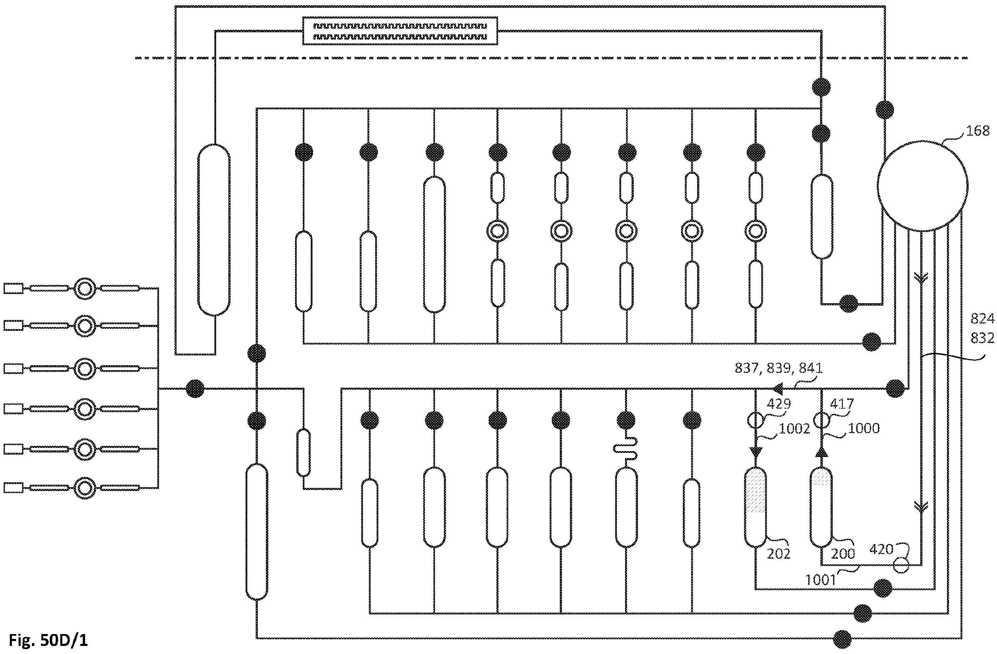

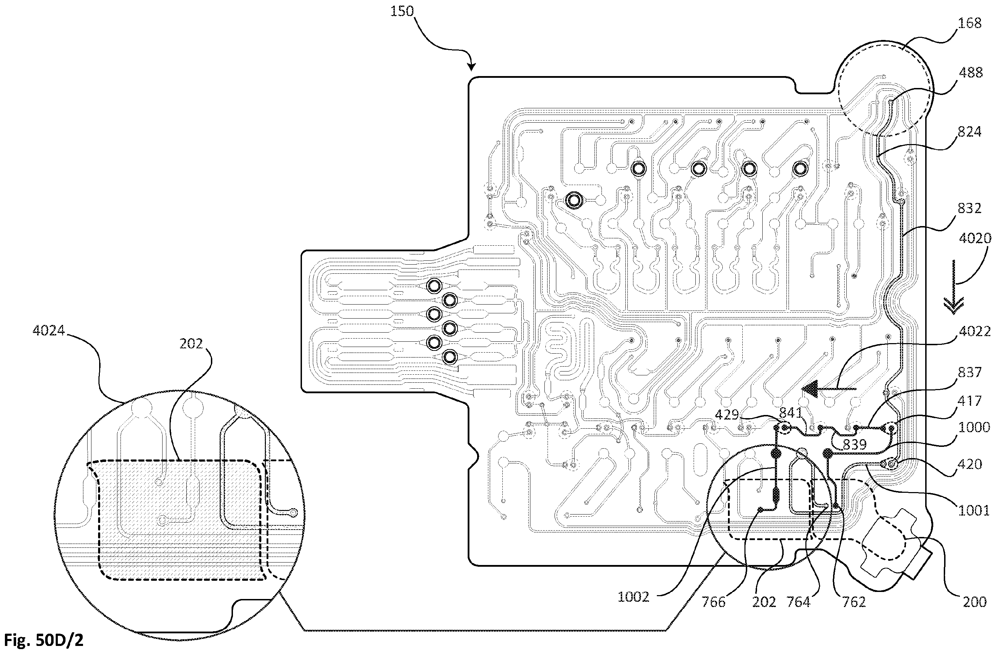

As seen in Figs. 50D/1 and 50D/2, valve 420 is opened, thereby allowing the pumping of air from pump 168 to sample receiving enclosure 200 and hence the application of pressure thereto. Air is pumped from pump 168 into sample receiving enclosure 200 along an air flow path comprising channel 824, thereafter channel 832, thereafter open valve 420 and thereafter pathway 1001. Air enters sample receiving enclosure 200 via through hole 764. The direction of passage of air along the air flow path is indicated by an air flow arrow 4020 in Fig. 50D/2.

As a result of the pressure created by the pumping of air thereinto, the mixed liquid held in sample receiving enclosure 200 is driven out of sample receiving enclosure 200 via through hole 762. The liquid travels along a liquid flow path comprising pathway 1000, thereafter open valve 417, thereafter channel 837, thereafter channel 839, thereafter channel 841, thereafter open valve 429 and thereafter pathway 1002 leading into lysis bead containing chamber 202 via through hole 766. The direction of passage of liquid along the liquid flow path is indicated by a liquid flow arrow 4022 Fig. 50D/2.

The content of chamber 202, following the pumping of air and liquid as described hereinabove, is shown at an enlargement 4024 in Fig. 50D/2. It is appreciated that the representation of the content of chamber 202 in enlargement 4024 is highly schematic and does not distinguish between the various components, namely sample 1402, PK and lysis beads, comprising the content.

Reference is now made to Figs. 50E/1 and 50E/2, which are simplified respective schematic and flow illustrations of a fifth stage in the operation of the cartridge 110 of Figs. 2A/1 - 10D by the instrument 100 of Figs. 11A - 49, which fifth stage follows the fourth stage in operation of the cartridge shown in Figs. 50D/1 and 50D/2.

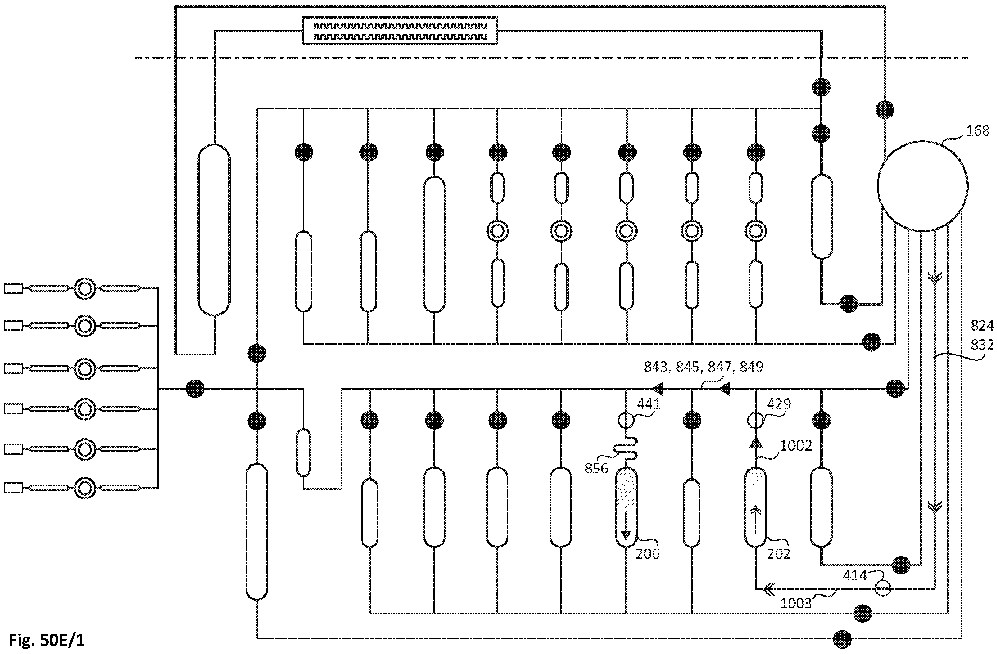

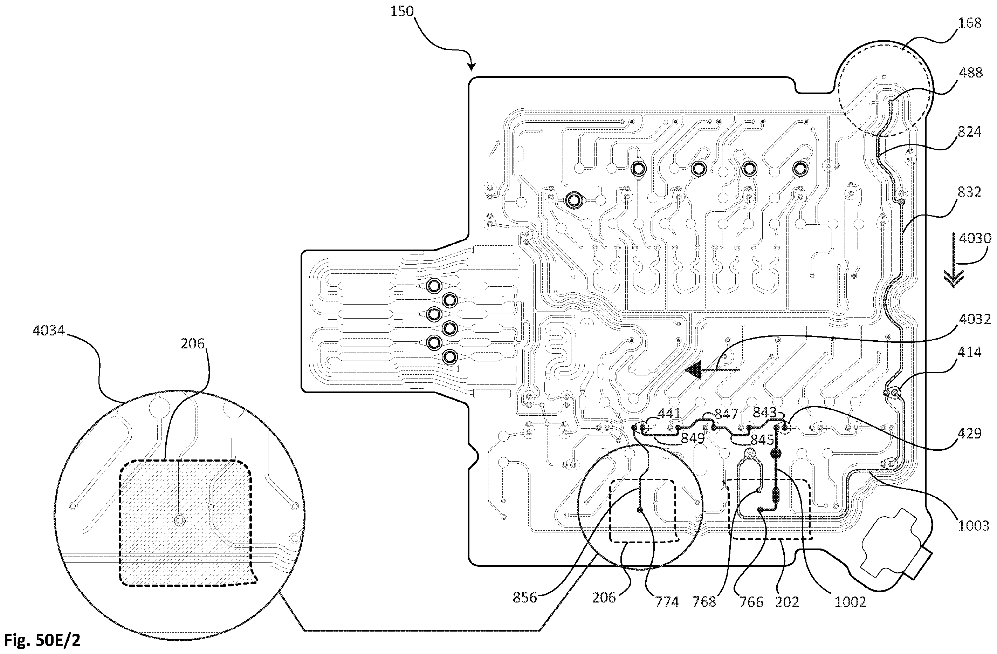

As seen in Figs. 50E/1 and 50E/2, valve 414 is opened, thereby allowing the pumping of air from pump 168 to lysis bead containing chamber 202 and hence the application of pressure to chamber 202. Air is pumped from pump 168 into chamber 202 along an air flow path comprising channel 824, thereafter channel 832, thereafter open valve 414 and thereafter pathway 1003. Air enters lysis bead containing chamber 202 via through hole 768. The direction of passage of air along the air flow path is indicated by an air flow arrow 4030 in Fig. 50E/2.

As a result of the pressure created by the pumping of air thereinto, the liquids held in chamber 202, together with the lysis beads, are driven out of chamber 202 via through hole 766. The liquids and beads travel along a liquid flow path comprising pathway 1002, thereafter open valve 429, thereafter channel 843, thereafter channel 845, thereafter channel 847, thereafter channel 849, thereafter open valve 441 and thereafter channel 856 leading into lysis mixing chamber 206. The liquids and beads enters lysis mixing chamber 206 via through hole 774. The direction of passage of liquids and beads along the liquid flow path is indicated by a liquid flow arrow 4032 in Fig. 50E/2.

The content of lysis mixing chamber 206 following the pumping of air and liquid as described hereinabove, is shown at an enlargement 4034 in Fig. 50E/2. It is appreciated that the representation of the content of lysis mixing chamber 206 in enlargement 4034 is highly schematic and does not distinguish between the various liquids, namely PK and sample 1402, and the lysis beads, comprising the content.

Reference is now made to Figs. 50F/1 and 50F/2, which are simplified respective schematic and flow illustrations of a sixth stage in the operation of the cartridge 110 of Figs. 2A/1 - 10D by the instrument 100 of Figs. 11A - 49, which sixth stage follows the fifth stage in operation of the cartridge shown in Figs. S0E/1 and 50E/2.

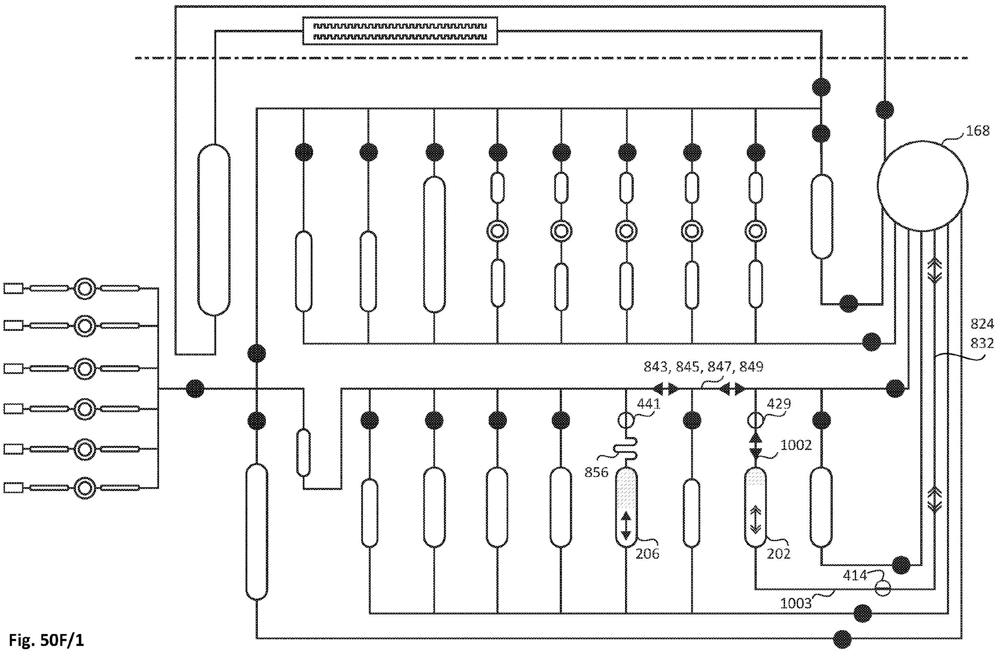

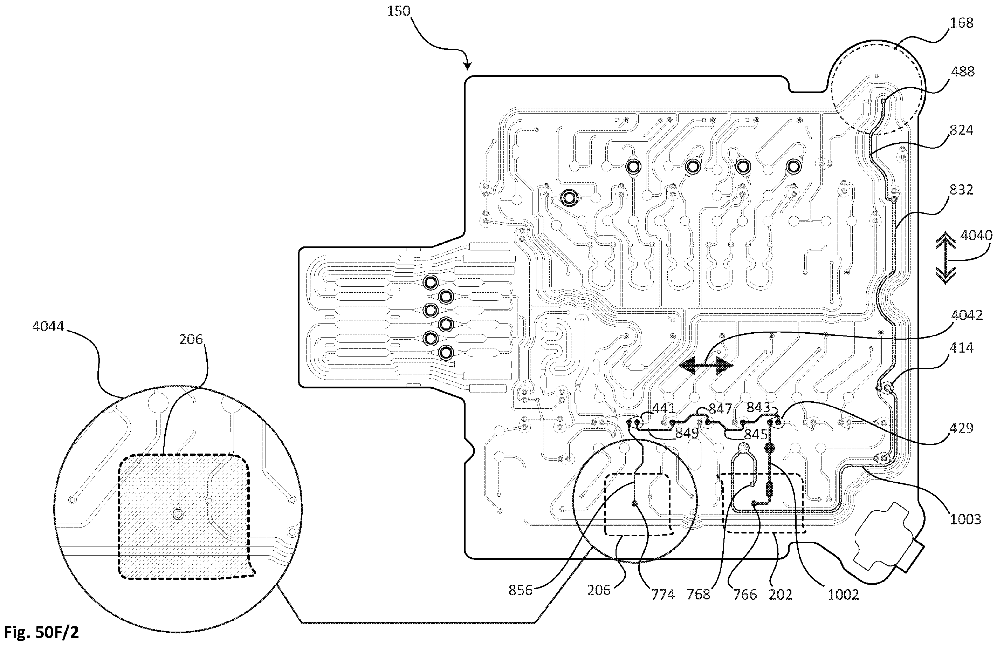

As seen in Figs. 50F/1 and 50F/2, valve 414 remains open and air is repeatedly pumped back and forth between pump 168 and chamber 202. Air travels between pump 168 and chamber 202 along an air flow path comprising channel 824, channel 832, open valve 414, pathway 1003 and through hole 768. The bi-directional flow of air between pump 168 and chamber 202 is indicated by a double-headed air flow arrow 4040 in Fig. 50F/2.

As a result of the repeated back and forward pressure created by the back and forth passage of air, the liquid contents, together with the lysis beads, of lysis mixing chamber 206 are repeatedly driven back and forth between lysis mixing chamber 206 and chamber 202, thereby mixing the PK, sample 1402 and lysis beads. The liquid contents of lysis mixing chamber 206 and the lysis beads are driven between lysis mixing chamber 206 and chamber 202 along a liquid flow path comprising through hole 766, pathway 1002, open valve 429, channel 843, channel 845, channel 847, channel 849, open valve 441, channel 856 and through hole 774. The bi-directional passage of liquid between lysis mixing chamber 206 and chamber 202 is indicated by a double-headed liquid flow arrow 4042 in Fig. 50F/2.

At the completion of mixing, following the passage of air and liquid described hereinabove, the mixed content is returned to lysis mixing chamber 206, as shown schematically at an enlargement 4044 in Fig. 50F/2.

Reference is now made to Figs. 50G/1 and 50G/2, which are simplified respective schematic and flow illustrations of a seventh stage in the operation of the cartridge 110 of Figs. 2A/1 - 10D by the instrument 100 of Figs. 11A - 49, which seventh stage follows the sixth stage in operation of the cartridge shown in Figs. 50F/1 and 50F/2.

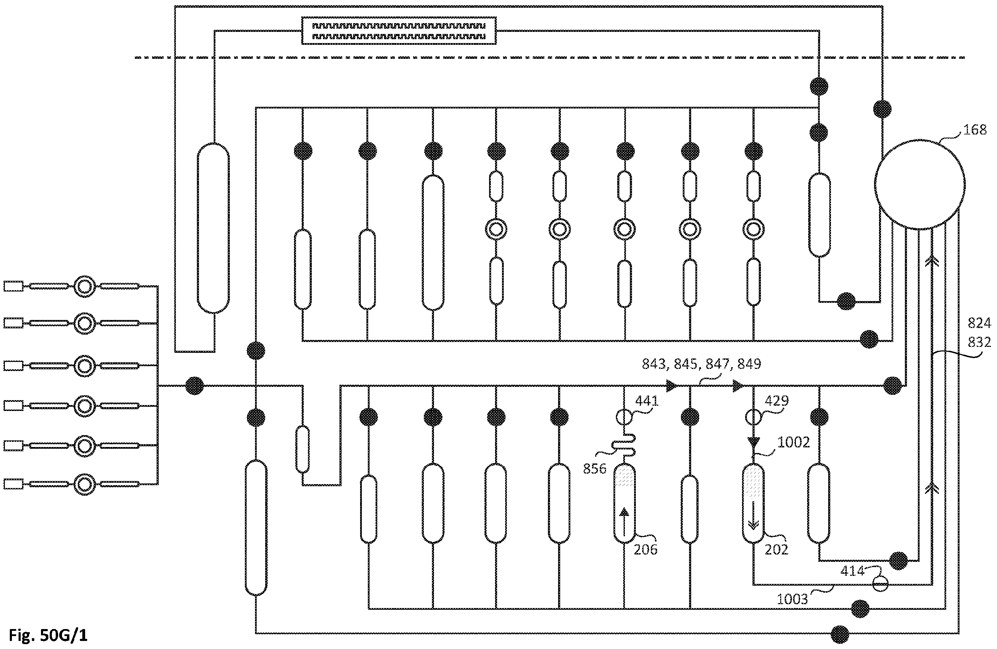

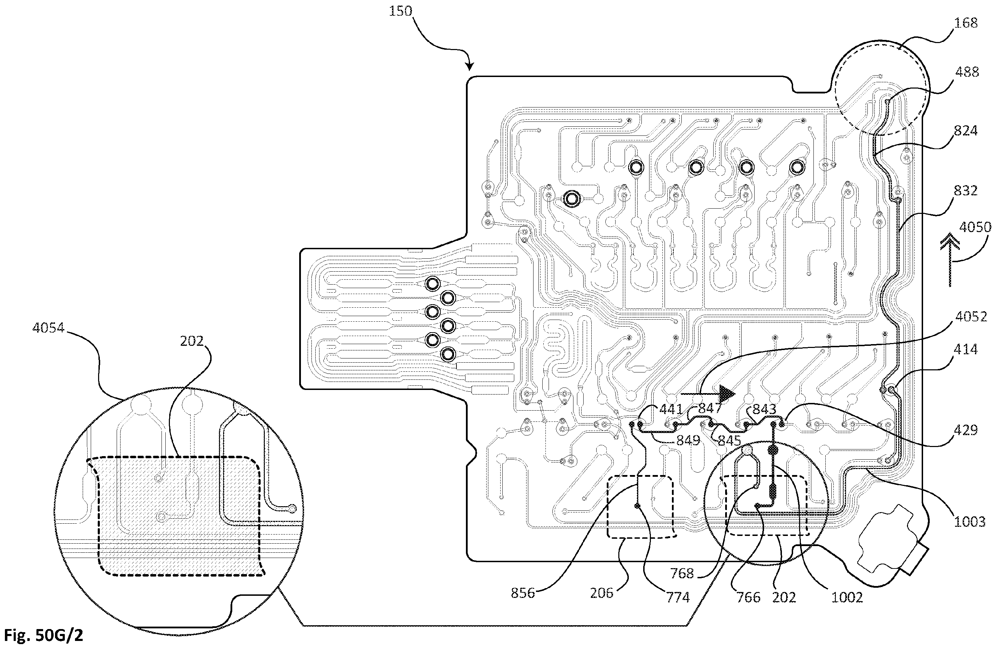

As seen in Figs. 50G/1 and 50G/2, valve 414 remains open, thereby allowing the suctioning of air therethrough by pump 168 and hence the application of back pressure to chamber 202. Air is suctioned from chamber 202 towards pump 168 via through hole 768 along an air flow path comprising pathway 1003, thereafter open valve 414, thereafter channel 832 and thereafter channel 824. The direction of passage of air along the air flow path is indicated by an air flow arrow 4050 in Fig. 50G/2.

As a result of the back-pressure created by the suctioning of air, the liquids held in chamber 206, together with the lysis beads, are driven out of chamber 206 via through hole 774. The liquids, together with the lysis beads, travel along a liquid flow path comprising channel 856, thereafter open valve 441, thereafter channel 849, thereafter channel 847, thereafter channel 845, thereafter channel 843, thereafter open valve 429 and thereafter pathway 1002 leading into chamber 202. The liquids, together with the lysis beads, enter chamber 202 via through hole 766. The direction of passage of liquid along the liquid flow path is indicated by a liquid flow arrow 4052 in Fig. 50G/2.

The content of chamber 202, following the pumping of air and liquid as described hereinabove, is shown at an enlargement 4054 in Fig. 50G/2. It is appreciated that the representation of the content of chamber 202 in enlargement 4054 is highly schematic and does not distinguish between the various liquids, namely PK and sample 1402, and lysis beads, comprising the content.

Reference is now made to Figs. 50H/1 and 50H/2, which are simplified respective schematic and flow illustrations of an eighth stage in the operation of the cartridge 110 of Figs. 2A/1 - 10D by the instrument 100 of Figs. 11A - 49, which eighth stage follows the seventh stage in operation of the cartridge shown in Figs. 50G/1 and 50G/2.

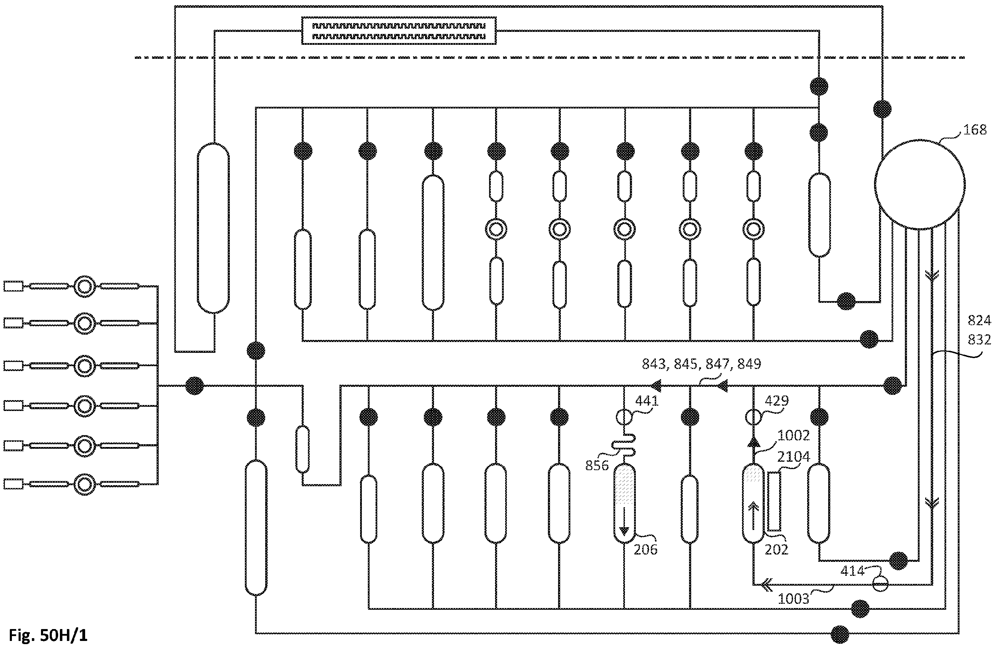

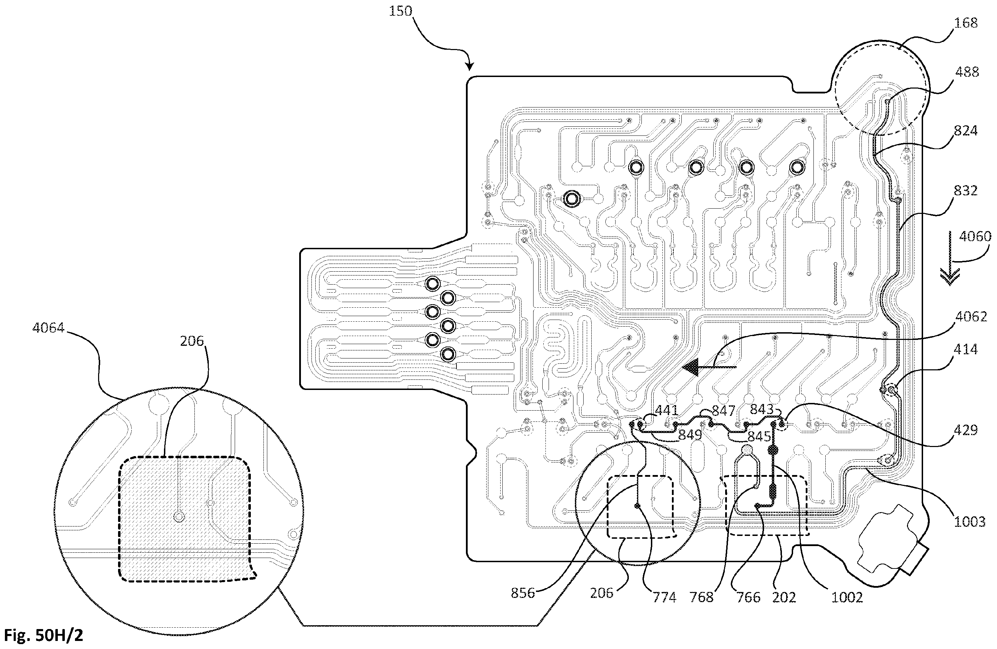

As seen in Fig. 50H/1, magnet 2104 is brought into propinquity with chamber 202. Magnet 2104 is preferably operated by instrument 100 as described hereinabove with reference to Fig. 44.

As further seen in Figs. 50H/1 and 50H/2, valve 414 remains open, thereby allowing the pumping of air from pump 168 to chamber 202 and hence the application of pressure to chamber 202. Air is pumped from pump 168 into chamber 202 along an air flow path comprising channel 824, thereafter channel 832, thereafter open valve 414 and thereafter pathway 1003. Air enters chamber 202 via through hole 768. The direction of passage of air along the air flow path is indicated by an air flow arrow 4060 in Fig. 50H/2.

As a result of the pressure created by the pumping of air thereinto, the liquids held in chamber 202 are driven out of chamber 202, and the magnetic lysis beads, having nucleic acids of sample 1402 attached thereto, are retained in chamber 202 by magnet 2104. The liquids exit chamber 202 via through hole 766 and subsequently travel along a liquid flow path comprising pathway 1002, thereafter open valve 429, thereafter channel 843, thereafter channel 845, thereafter channel 847, thereafter channel 849, thereafter open valve 441 and thereafter channel 856 leading into lysis mixing chamber 206. The expelled liquid enters lysis mixing chamber 206 via through hole 774. The direction of passage of liquids along the liquid flow path is indicated by a liquid flow arrow 4062 in Fig. 50H/2.

The content of chamber 206, following the pumping of air and liquid as described hereinabove, is shown at an enlargement 4064 in Fig. 50H/2. It is appreciated that the representation of the content of chamber 206 in enlargement 4064 is highly schematic and does not distinguish between the various liquids comprising the content. It is further appreciated that, at this stage of operation of cartridge 110, chamber 206 acts as a waste chamber and that the liquids expelled thereinto do not participate further in the processing of cartridge 110.

Reference is now made to Figs. 50I/1 and 50I/2, which are simplified respective schematic and flow illustrations of a ninth stage in the operation of the cartridge 110 of Figs. 2A/1 - 10D by the instrument 100 of Figs. 11A - 49, which ninth stage follows the eighth stage in operation of the cartridge shown in Figs. 50H/1 and 50H/2.

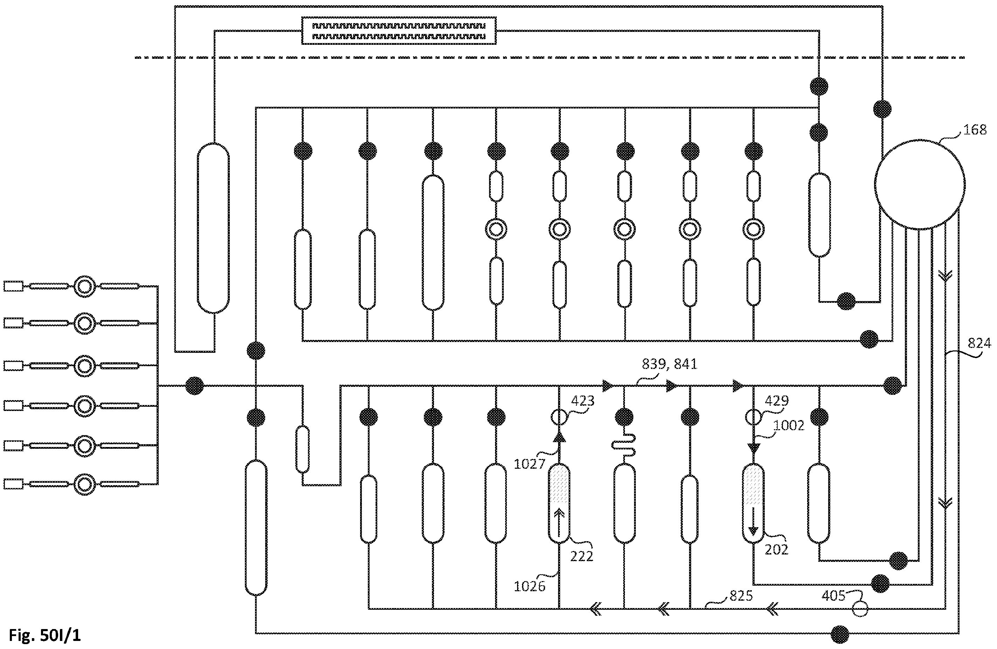

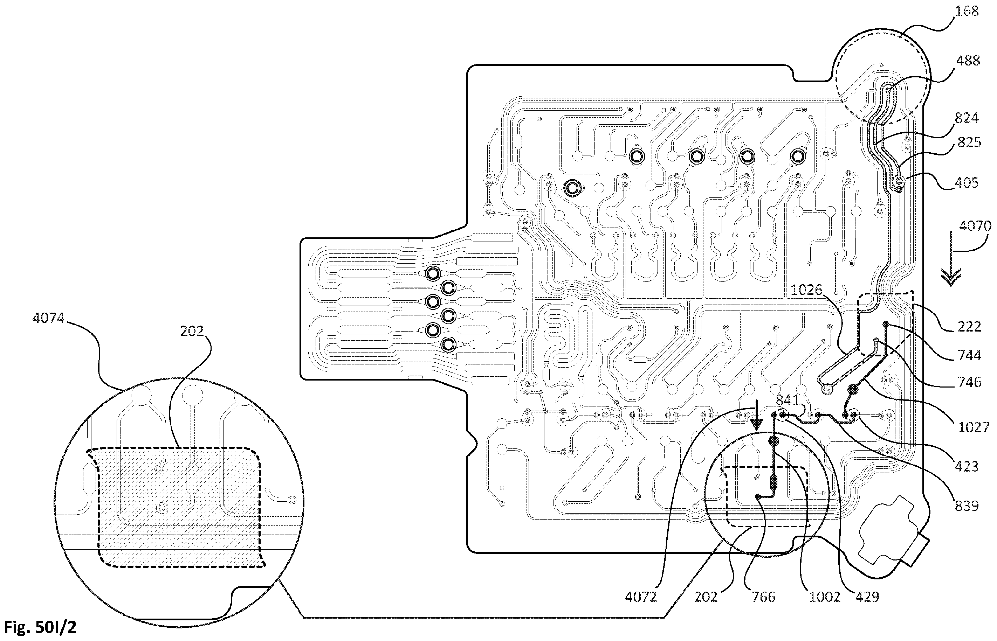

As seen in Figs. 50I/1 and 50I/2, valve 405 is opened, thereby allowing the pumping of air from pump 168 to Wash Buffer I containing chamber 222 and hence the application of pressure to chamber 222. Air is pumped from pump 168 into chamber 222 along an air flow path comprising channel 824, thereafter open valve 405, thereafter channel 825 and thereafter pathway 1026. Air enters chamber 222 via through hole 746. The direction of passage of air along the air flow path is indicated by an air flow arrow 4070 in Fig. 50I/2.

As a result of the pressure created by the pumping of air thereinto, the liquid Wash Buffer I held in chamber 222 is driven out of chamber 222 via through hole 744. Wash Buffer I travels along a liquid flow path comprising pathway 1027, thereafter open valve 423, thereafter channel 839, thereafter channel 841, thereafter open valve 429 and thereafter pathway 1002 leading into chamber 202, which chamber 202 already holds magnetic lysis beads with nucleic acids attached thereto. Wash Buffer I enters chamber 202 via through hole 766. The direction of passage of Wash Buffer I along the liquid flow path is indicated by a liquid flow arrow 4072 in Fig. 50I/2.

The content of chamber 202, following the pumping of air and liquid as described hereinabove, is shown at an enlargement 4074 in Fig. 50I/2. It is appreciated that the representation of the content of chamber 202 in enlargement 4074 is highly schematic and does not distinguish between the various liquids, namely primarily Wash Buffer I, and lysis beads, having nucleic acids attached thereto, comprising the content. It is appreciated that lysis beads having nucleic acids attached thereto are preferably washed by Wash Buffer I at this stage in the operation of cartridge 110.

Reference is now made to Figs. 50J/1 and 50J/2, which are simplified respective schematic and flow illustrations of a tenth stage in the operation of the cartridge 110 of Figs. 2A/1 - 10D by the instrument 100 of Figs. 11A - 49, which tenth stage follows the ninth stage in operation of the cartridge shown in Figs. 50I/1 and 50I/2.

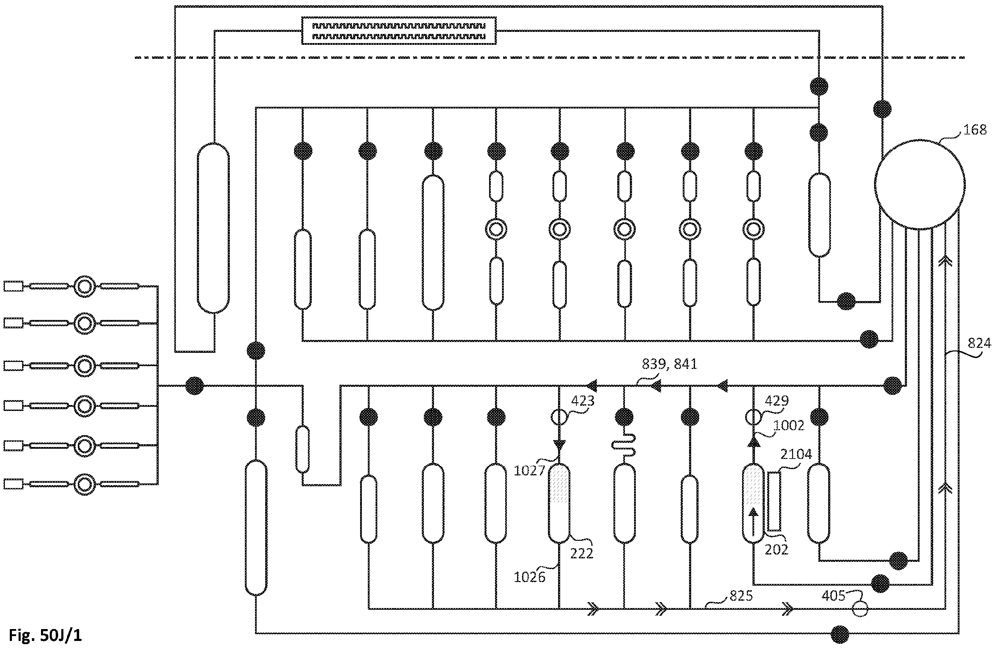

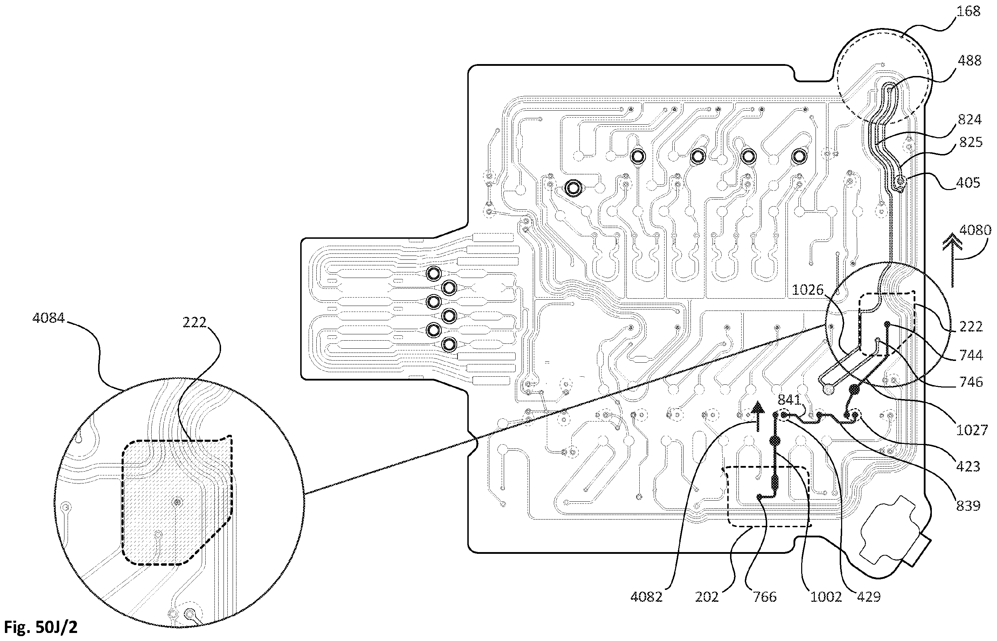

As seen in Fig. 50J/1, magnet 2104 is brought into propinquity with chamber 202. Magnet 2104 is preferably operated by instrument 100 as described hereinabove with reference to Fig. 44.

As further seen in Figs. 50J/1 and 50J/2, valve 405 remains open, thereby allowing the suctioning of air therethrough by pump 168 and hence the application of back pressure to chamber 222. Air is suctioned from chamber 222 towards pump 168 via through hole 746 along an air flow path comprising pathway 1026, thereafter channel 825, thereafter open valve 405 and thereafter channel 824. The direction of passage of air along the air flow path is indicated by an air flow arrow 4080 in Fig. 50J/2.

As a result of the back-pressure created by the suctioning of air, the liquids held in chamber 202 are driven out from chamber 202, and the magnetic lysis beads, having nucleic acids of sample 1402 attached thereto and having now been washed by Wash Buffer I, are retained in chamber 202 by magnet 2104. The liquids, primarily comprising Wash Buffer I, exit chamber 202 via through hole 766 and subsequently travel along a liquid flow path comprising pathway 1002, thereafter open valve 429, thereafter channel 841, thereafter channel 839, thereafter open valve 423 and thereafter pathway 1027 leading into chamber 222. The expelled Wash Buffer I enters chamber 222 via through hole 744. The direction of passage of liquids along the liquid flow path is indicated by a liquid flow arrow 4082 in Fig. 50J/2.

The content of chamber 222, following the pumping of air and liquid as described hereinabove, is shown at an enlargement 4084 in Fig. 50J/2. It is appreciated that the representation of the content of chamber 222 in enlargement 4084 is highly schematic and does not distinguish between the various liquids comprising the content. It is further appreciated that, in this stage of operation of cartridge 110, chamber 222 acts as a waste chamber and that the liquids expelled thereinto do not participate further in the processing of cartridge 110.

Reference is now made to Figs. 50K/1 and 50K/2, which are simplified respective schematic and flow illustrations of an eleventh stage in the operation of the cartridge 110 of Figs. 2A/1 - 10D by the instrument 100 of Figs. 11A - 49, which eleventh stage follows the tenth stage in operation of the cartridge shown in Figs. 50J/1 and 50J/2.

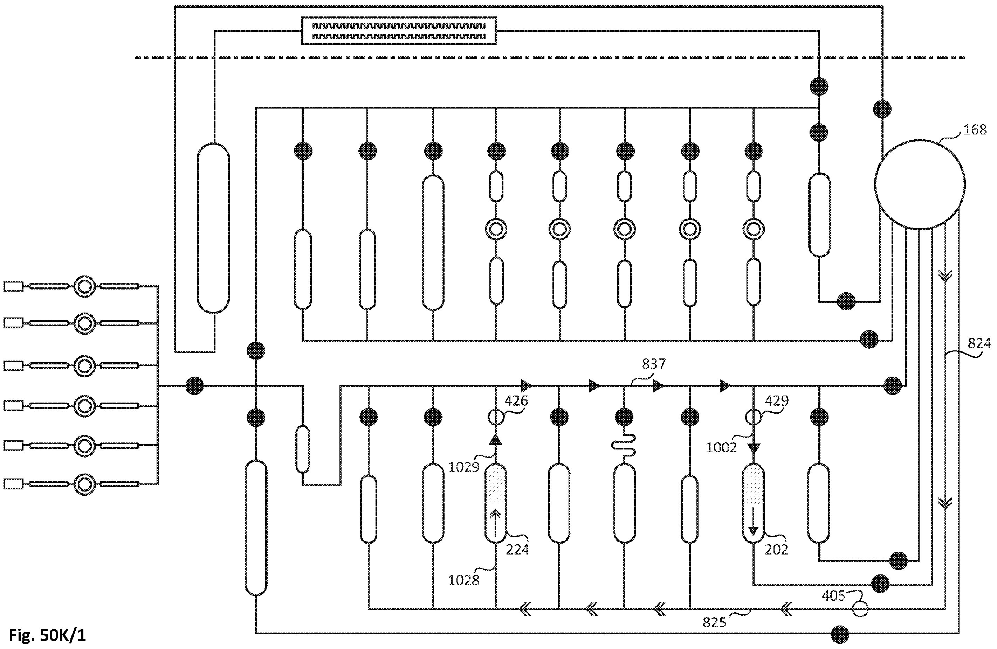

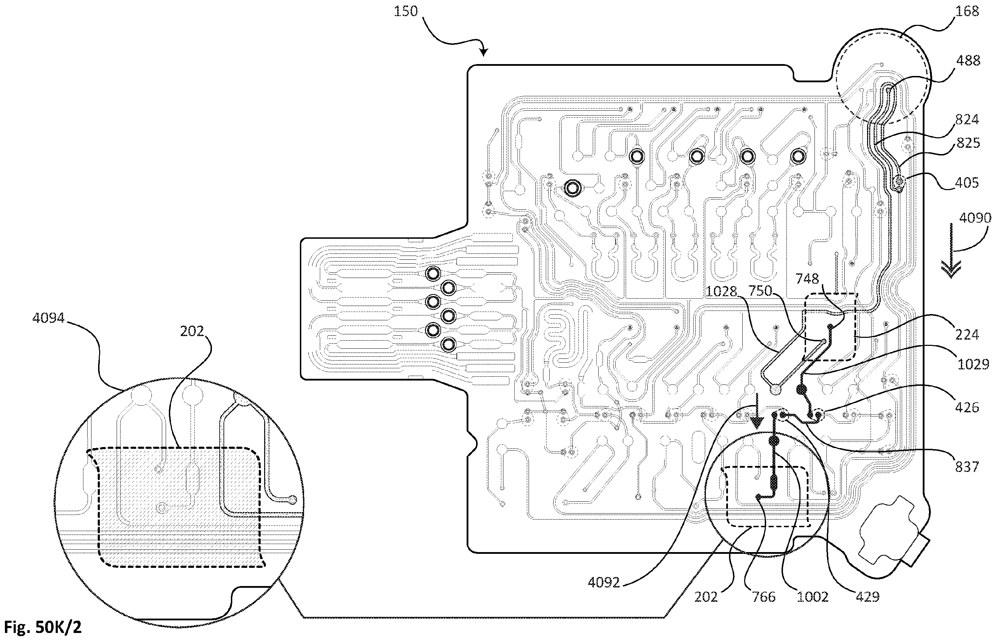

As seen in Figs. 50K/1 and 50K/2, valve 405 is opened, thereby allowing the pumping of air from pump 168 to Wash Buffer II containing chamber 224 and hence the application of pressure to chamber 224. Air is pumped from pump 168 into chamber 224 along an air flow path comprising channel 824, thereafter open valve 405, thereafter channel 825 and thereafter pathway 1028. Air enters chamber 224 via through hole 750. The direction of passage of air along the air flow path is indicated by an air flow arrow 4090 in Fig. 50K/2.

As a result of the pressure created by the pumping of air thereinto, the liquid Wash Buffer II held in chamber 224 is driven out of chamber 224 via through hole 748. Wash Buffer II travels along a liquid flow path comprising pathway 1029, thereafter open valve 426, thereafter channel 837, thereafter open valve 429 and thereafter pathway 1002 leading into chamber 202, which contains magnetic lysis beads with nucleic acids attached thereto. Wash Buffer II enters chamber 202 via through hole 766. The direction of passage of Wash Buffer II along the liquid flow path is indicated by a liquid flow arrow 4092 in Fig. 50K/2.

The content of chamber 202, following the pumping of air and liquid as described hereinabove, is shown at an enlargement 4094 in Fig. 50K/2. It is appreciated that the representation of the content of chamber 202 in enlargement 4094 is highly schematic and does not distinguish between the various liquids, namely primarily Wash Buffer II, and lysis beads, having nucleic acids attached thereto, comprising the content. It is appreciated that lysis beads having nucleic acids attached thereto are preferably washed by Wash Buffer II at this stage in the operation of cartridge 110.

Reference is now made to Figs. 50L/1 and 50L/2, which are simplified respective schematic and flow illustrations of a twelfth stage in the operation of the cartridge 110 of Figs. 2A/1 - 10D by the instrument 100 of Figs. 11A - 49, which twelfth stage follows the eleventh stage in operation of the cartridge shown in Figs. 50K/1 and 50K/2.

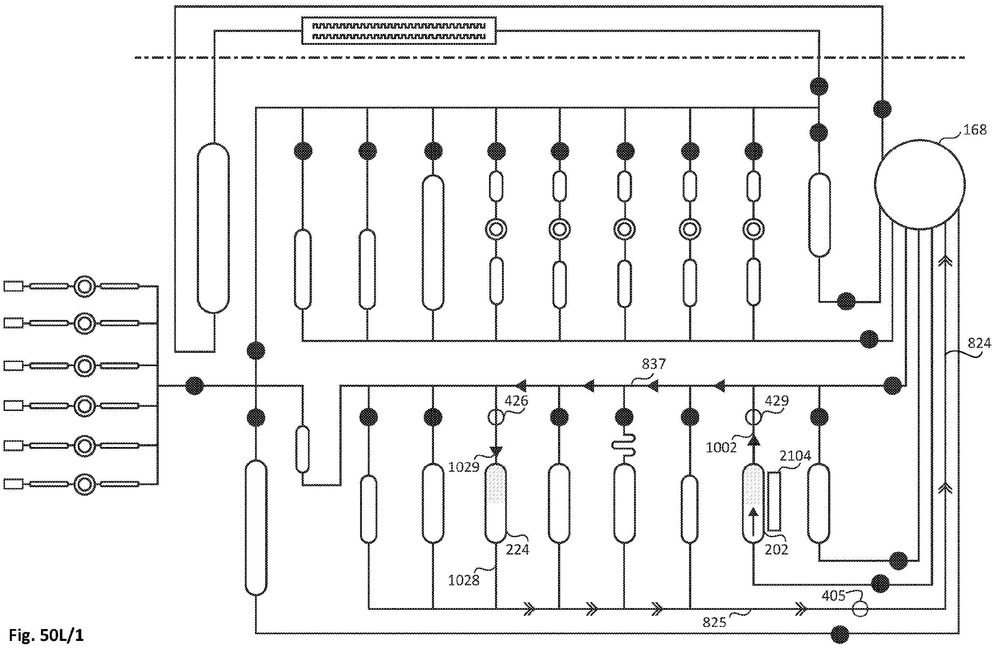

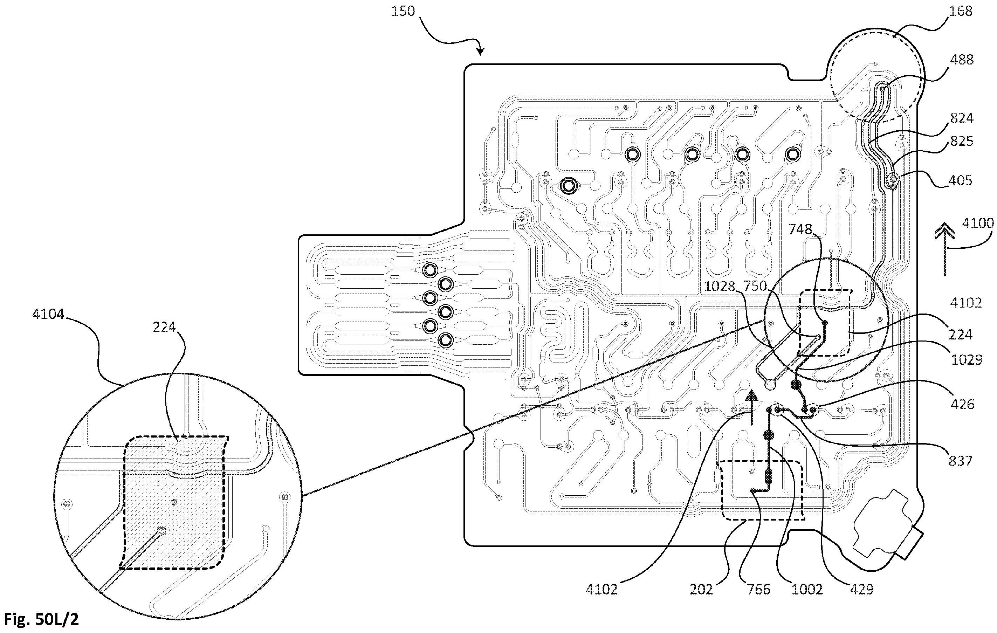

As seen in Fig. 50L/1, magnet 2104 is brought into propinquity with chamber 202. Magnet 2104 is preferably operated by instrument 100 as described hereinabove with reference to Fig. 44.

As further seen in Figs. 50L/1 and 50L/2, valve 405 remains open, thereby allowing the suctioning of air therethrough by pump 168 and hence the application of back pressure to chamber 224. Air is suctioned from chamber 224 towards pump 168 via through hole 750 along an air flow path comprising pathway 1028, thereafter channel 825, thereafter open valve 405 and thereafter channel 824. The direction of passage of air along the air flow path is indicated by an air flow arrow 4100 in Fig. 50L/2.

As a result of the back-pressure created by the suctioning of air, the liquids held in chamber 202 are driven out from chamber 202, and the magnetic lysis beads, having nucleic acids of sample 1402 attached thereto and having now been washed by Wash Buffer II, are retained in chamber 202 by magnet 2104. The liquids, primarily comprising Wash Buffer II, exit chamber 202 via through hole 766 and subsequently travel along a liquid flow path comprising pathway 1002, thereafter open valve 429, thereafter channel 837, thereafter open valve 426 and thereafter pathway 1029 leading into chamber 224. The expelled Wash Buffer II enters chamber 224 via through hole 748. The direction of passage of liquids along the liquid flow path is indicated by a liquid flow arrow 4102 in Fig. 50L/2.

The content of chamber 224, following the pumping of air and liquid as described hereinabove, is shown at an enlargement 4104 in Fig. S0L/2. It is appreciated that the representation of the content of chamber 224 in enlargement 4104 is highly schematic and does not distinguish between the various liquids comprising the content. It is further appreciated that, at this stage of operation of cartridge 110, chamber 224 acts as a waste chamber and that the liquids expelled thereinto do not participate further in the processing of cartridge 110.

Reference is now made to Figs. 50M/1 and 50M/2, which are simplified respective schematic and flow illustrations of a thirteenth stage in the operation of the cartridge 110 of Figs. 2A/1 - 10D by the instrument 100 of Figs. 11A - 49, which thirteenth stage follows the twelfth stage in operation of the cartridge shown in Figs. 50L/1 and 50L/2.

As seen in Figs. 50M/1 and 50M/2, valve 405 is opened, thereby allowing the pumping of air from pump 168 to Wash Buffer III containing chamber 226 and hence the application of pressure to chamber 226. Air is pumped from pump 168 into chamber 226 along an air flow path comprising channel 824, thereafter open valve 405, thereafter channel 825 and thereafter pathway 1030. Air enters chamber 226 via through hole 754. The direction of passage of air along the air flow path is indicated by an air flow arrow 4110 in Fig. S0L/2.

As a result of the pressure created by the pumping of air thereinto, the liquid Wash Buffer III held in chamber 226 is driven out of chamber 226 via through hole 752. Wash Buffer III travels along a liquid flow path comprising pathway 1031, thereafter open valve 432, thereafter channel 843, thereafter open valve 429 and thereafter pathway 1002 leading into chamber 202, which contains magnetic lysis beads with nucleic acids attached thereto. Wash Buffer III enters chamber 202 via through hole 766. The direction of passage of Wash Buffer III along the liquid flow path is indicated by a liquid flow arrow 4112 in Fig. 50L/2.

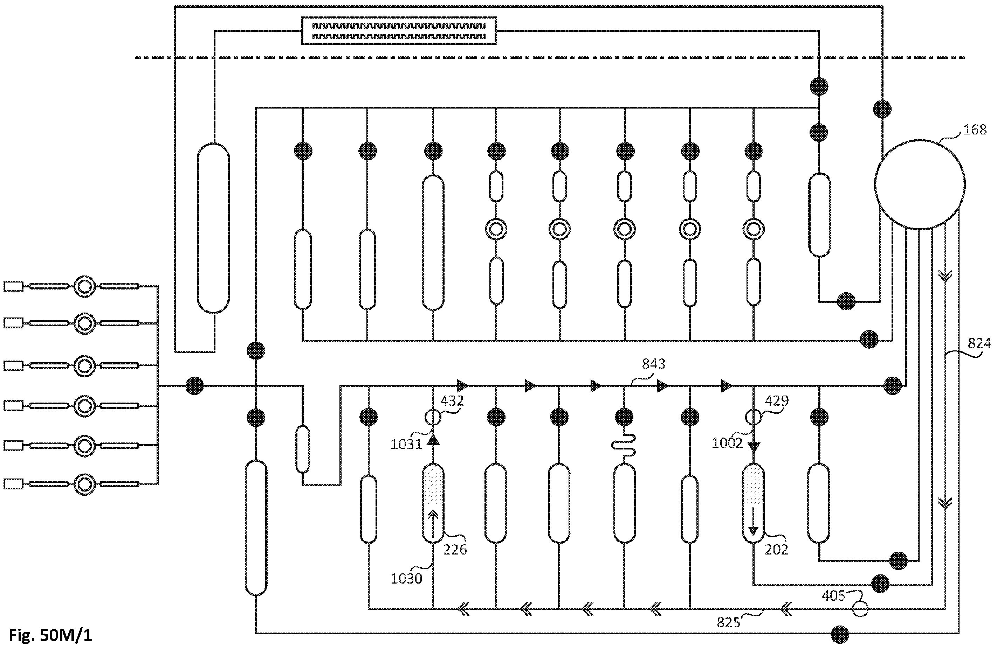

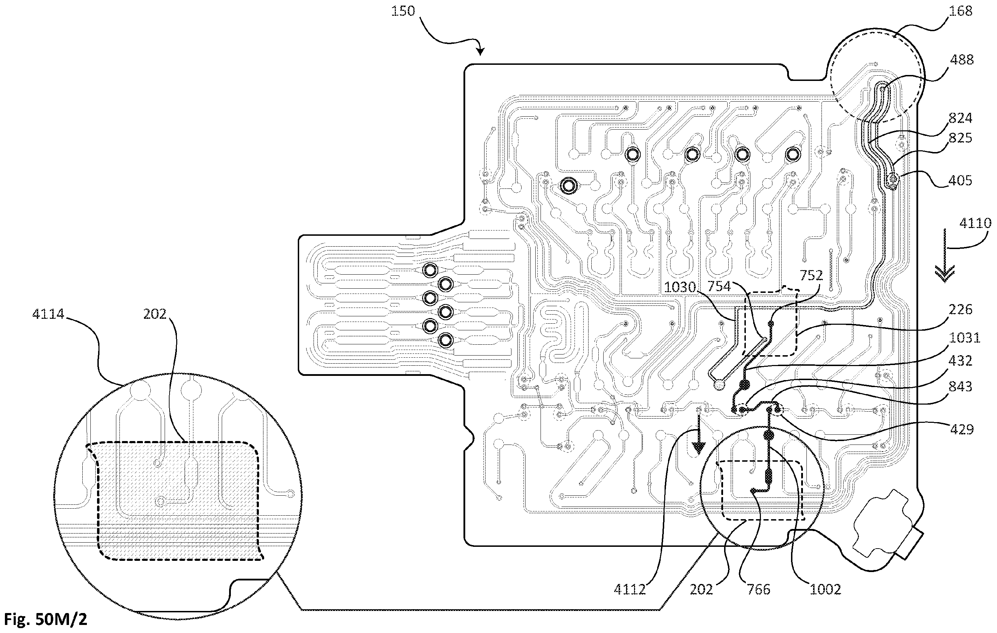

The content of chamber 202, following the pumping of air and liquid as described hereinabove, is shown at an enlargement 4114 in Fig. 50M/2. It is appreciated that the representation of the content of chamber 202 in enlargement 4114 is highly schematic and does not distinguish between the various liquids, namely primarily Wash Buffer III, and lysis beads, having nucleic acids attached thereto, comprising the content. It is appreciated that lysis beads having nucleic acids attached thereto are preferably washed by Wash Buffer III at this stage in the operation of cartridge 110.

Reference is now made to Figs. 50N/1 and 50N/2, which are simplified respective schematic and flow illustrations of a fourteenth stage in the operation of the cartridge 110 of Figs. 2A/1 - 10D by the instrument 100 of Figs. 11A - 49, which fourteenth stage follows the thirteenth stage in operation of the cartridge shown in Figs. 50M/1 and 50M/2.

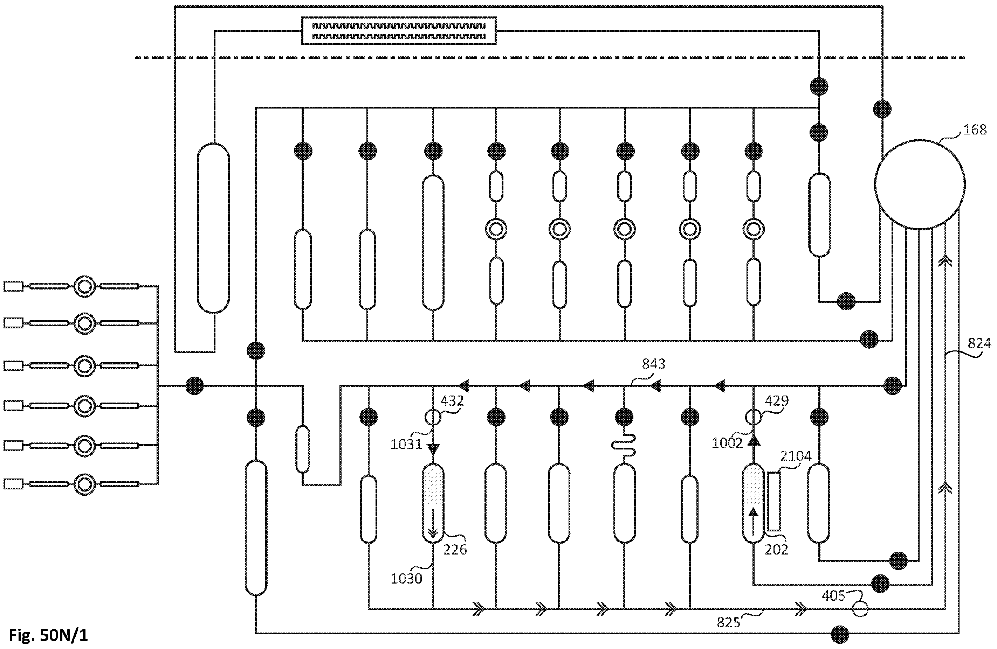

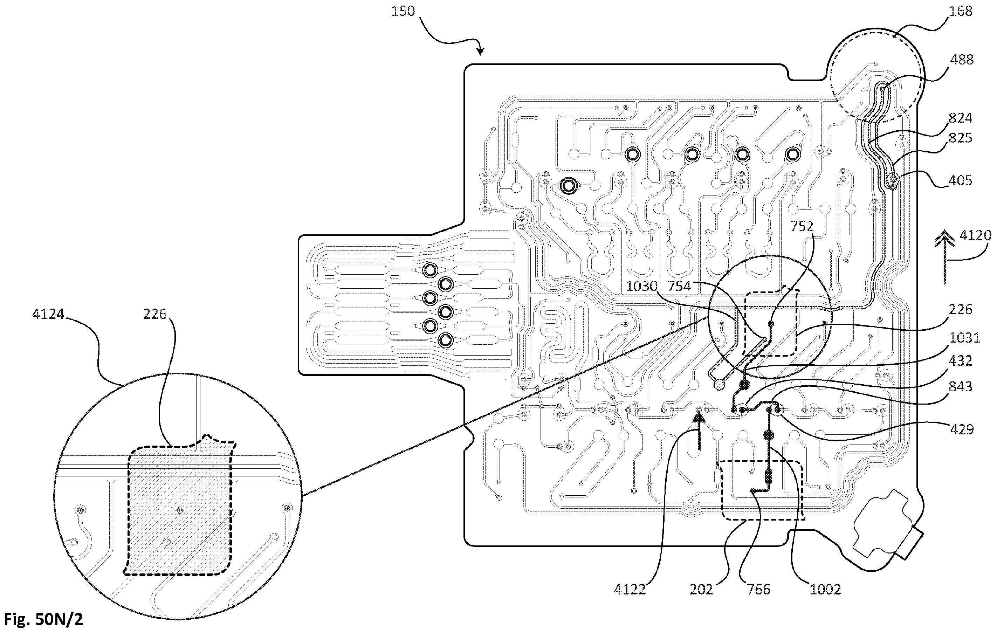

As seen in Fig. 50N/1, magnet 2104 is brought into propinquity with chamber 202. Magnet 2104 is preferably operated by instrument 100 as described hereinabove with reference to Fig. 44.

As further seen in Figs. 50N/1 and 50N/2, valve 405 remains open, thereby allowing the suctioning of air therethrough by pump 168 and hence the application of back pressure to chamber 226. Air is suctioned from chamber 226 towards pump 168 via through hole 754 along an air flow path comprising pathway 1030, thereafter channel 825, thereafter open valve 405 and thereafter channel 824. The direction of passage of air along the air flow path is indicated by an air flow arrow 4120 in 50N/2.

As a result of the back-pressure created by the suctioning of air, the liquids held in chamber 202 are driven out from chamber 202, and the magnetic lysis beads, having nucleic acids of sample 1402 attached thereto and having now been washed by Wash Buffer III, are retained in chamber 202 by magnet 2104. The liquids, primarily comprising Wash Buffer III, exit chamber 202 via through hole 766 and subsequently travel along a liquid flow path comprising pathway 1002, thereafter open valve 429, thereafter channel 843, thereafter open valve 432 and thereafter pathway 1031 leading into chamber 226. The expelled Wash Buffer III enters chamber 226 via through hole 752. The direction of passage of liquids along the liquid flow path is indicated by a liquid flow arrow 4122 in 50N/2.

The content of chamber 226, following the pumping of air and liquid as described hereinabove, is shown at an enlargement 4124 in 50N/2. It is appreciated that the representation of the content of chamber 226 in enlargement 4124 is highly schematic and does not distinguish between the various liquids comprising the content. It is further appreciated that, at this stage of operation of cartridge 110, chamber 226 acts as a waste chamber and that the liquids expelled thereinto do not participate further in the processing of cartridge 110.

Reference is now made to Figs. 50O/1 and 50O/2, which are simplified respective schematic and flow illustrations of a fifteenth stage in the operation of the cartridge 110 of Figs. 2A/1 - 10D by the instrument 100 of Figs. 11A - 49, which fifteenth stage follows the fourteenth stage in operation of the cartridge shown in Figs. 50N/1 and 50N/2.

As seen in Figs. 50O/1 and 50O/2, valve 405 is opened, thereby allowing the pumping of air from pump 168 to elution buffer containing chamber 228 and hence the application of pressure to chamber 228. Air is pumped from pump 168 into chamber 228 along an air flow path comprising channel 824, thereafter open valve 405, thereafter channel 825 and thereafter pathway 1032. Air enters chamber 228 via through hole 758. The direction of passage of air along the air flow path is indicated by an air flow arrow 4130 in 50O/2.

As a result of the pressure created by the pumping of air thereinto, the elution buffer held in chamber 228 is driven out of chamber 228 via through hole 756. The elution buffer travels along a liquid flow path comprising pathway 1033, thereafter open valve 438, thereafter channel 847, thereafter channel 845, thereafter channel 843, thereafter open valve 429 and thereafter pathway 1002 leading into chamber 202, which contains magnetic lysis beads with nucleic acids attached thereto. The elution buffer enters chamber 202 via through hole 766. The direction of passage of the elution buffer along the liquid flow path is indicated by a liquid flow arrow 4132 in 50O/2.

The content of chamber 202 following the pumping of air and liquid as described hereinabove, is shown at an enlargement 4134 in 50O/2. It is appreciated that the representation of the content of sample receiving enclosure 202 in enlargement 4134 is highly schematic and does not distinguish between the various liquids, namely primarily the elution buffer, and magnetic lysis beads, having nucleic acids attached thereto, comprising the content. It is further appreciated that this stage in the operation of cartridge 110 is preferably followed by a mixing step, not shown here.

Reference is now made to Figs. 50P/1 and 50P/2, which are simplified respective schematic and flow illustrations of a sixteenth stage in the operation of the cartridge 110 of Figs. 2A/1 - 10D by the instrument 100 of Figs. 11A - 49, which sixteenth stage follows the fifteenth stage in operation of the cartridge shown in Figs. 50O/1 and 50O/2.

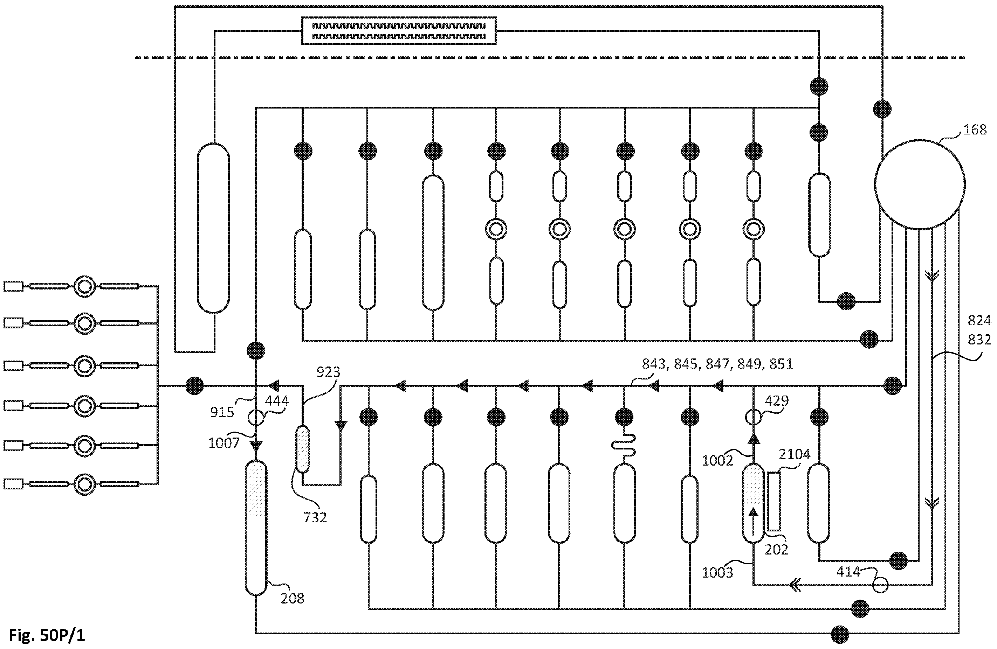

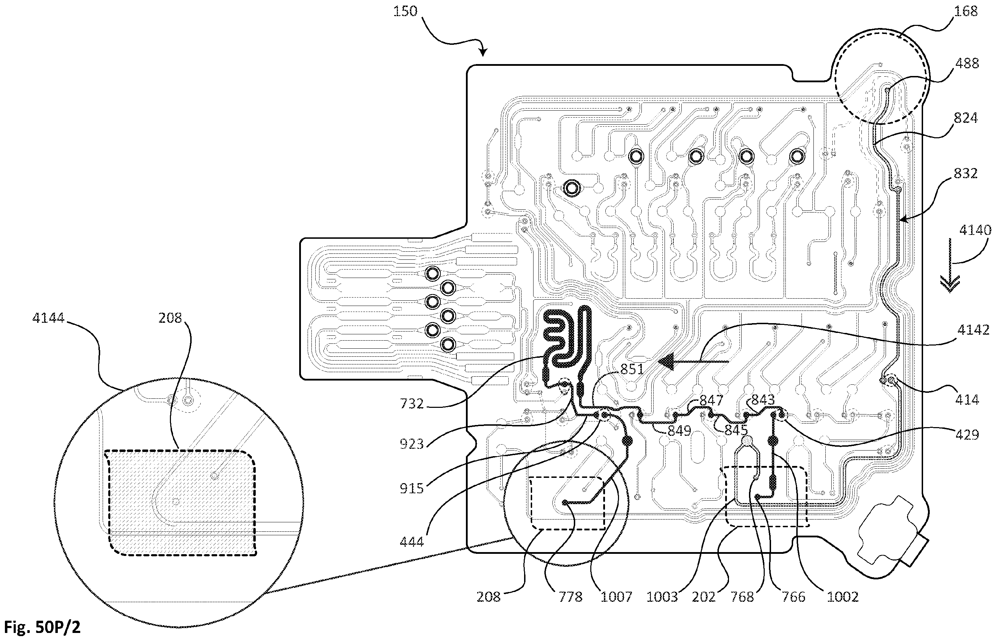

As seen in Fig. 50P/1, magnet 2104 is brought into propinquity with chamber 202. Magnet 2104 is preferably operated by instrument 100 as described hereinabove with reference to Fig. 44.

As further seen in Figs. 50P/1 and 50P/2, valve 414 is opened, thereby allowing the pumping of air from pump 168 to chamber 202 and hence the application of pressure to chamber 202. Air is pumped from pump 168 into chamber 202 along an air flow path comprising channel 824, thereafter channel 832, thereafter open valve 414 and thereafter pathway 1003. Air enters chamber 202 via through hole 768. The direction of passage of air along the air flow path is indicated by an air flow arrow 4140 in 50P/2.

As a result of the pressure created by the pumping of air thereinto, the liquids held in chamber 202 are driven out of chamber 202, and the magnetic lysis beads, now having the nucleic acids of sample 1402 removed therefrom by the elution buffer, are retained in chamber 202 by magnet 2104. The liquids, primarily comprising the elution buffer and nucleic acids of sample 1402, exit chamber 202 via through hole 766 and subsequently travel along a liquid flow path comprising pathway 1002, thereafter open valve 429, thereafter channel 843, thereafter channel 845, thereafter channel 847, thereafter channel 849, thereafter channel 851, thereafter metering chamber 732, thereafter channel 923, thereafter channel 915, thereafter open valve 444 and thereafter pathway 1007 leading into dilution chamber 208. It is appreciated that metering chamber 732 here forms a part of the liquid flow path between chamber 202 and dilution chamber 208. The liquids enter dilution chamber 208 via through hole 778. The direction of passage of the liquids along the liquid flow path is indicated by a liquid flow arrow 4142 in 50P/2.

The content of chamber 208, following the pumping of air and liquid as described hereinabove, is shown at an enlargement 4144 in 50P/2. It is appreciated that the representation of the content of chamber 208 in enlargement 4144 is highly schematic and does not distinguish between the various components, namely primarily nucleic acids, elution buffer and dilution buffer, comprising the content.

Reference is now made to Figs. 50Q/1 and 50Q/2, which are simplified respective schematic and flow illustrations of a seventeenth stage in the operation of the cartridge 110 of Figs. 2A/1 - 10D by the instrument 100 of Figs. 11A - 49, which seventeenth stage follows the sixteenth stage in operation of the cartridge shown in Figs. 50P/1 and 50P/2.

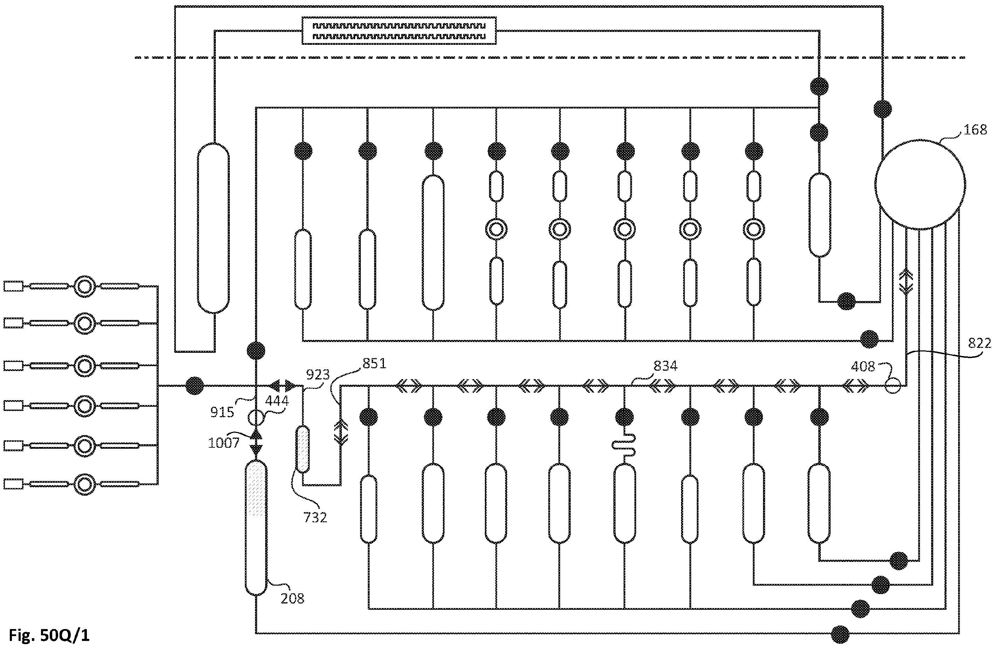

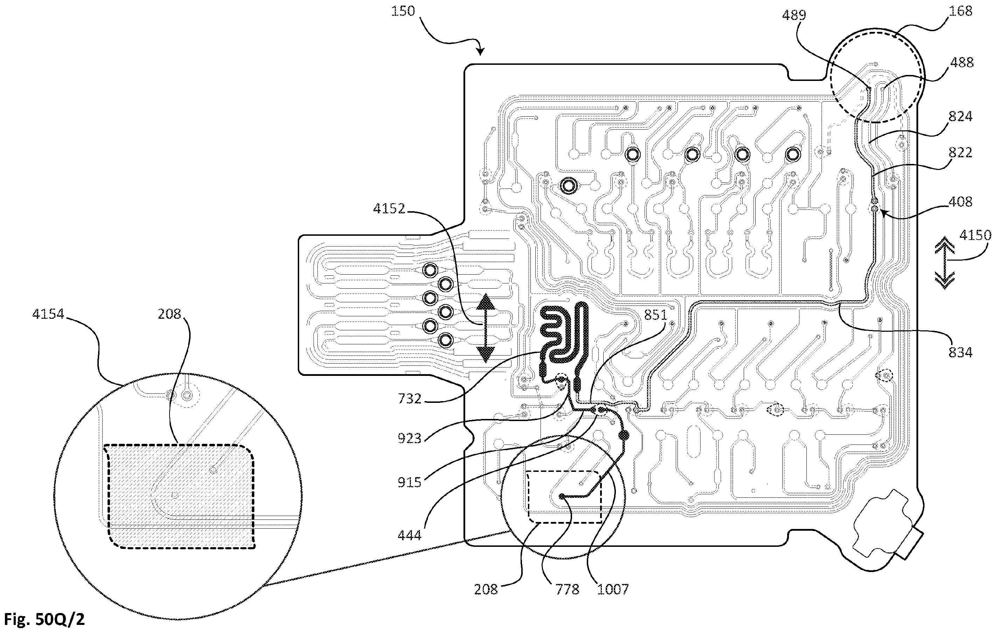

As seen in Figs. 50Q/1 and 50Q/2, valve 408 is opened, thereby allowing the repeated pumping of air back and forth between pump 168 and metering chamber 732. Air travels between pump 168 and metering chamber 732 along an air flow path comprising channel 822, open valve 408, channel 834 and channel 851. The bi-directional flow of air between pump 168 and metering chamber 732 is indicated by a double-headed air flow arrow 4150 in Fig. 50Q/2.

As a result of the repeated back and forward pressure created by the back and forth passage of air, the liquid contents of dilution chamber 208 are repeatedly driven back and forth between dilution chamber 208 and metering chamber 732, thereby mixing the elution buffer and nucleic acids. The liquid contents of chamber 208 are repeatedly driven back and forth between metering chamber 732 and dilution chamber 208 along a liquid flow path comprising channel 923, channel 915, open valve 444 and pathway 1007 and through hole 778. The bi-directional passage of liquid between chamber 208 and metering chamber 732 is indicated by a double-headed liquid flow arrow 4152 in Fig. 50Q/2.

The mixed content of chamber 208, following the passage of air and liquid described hereinabove, is shown schematically at an enlargement 4154 in Fig. 50Q/2.

Reference is now made to Figs. 50R/1 and 50R/2, which are simplified respective schematic and flow illustrations of an eighteenth stage in the operation of the cartridge 110 of Figs. 2A/1 - 10D by the instrument 100 of Figs. 11A - 49, which eighteenth stage follows the seventeenth stage in operation of the cartridge shown in Figs. 50Q/1 and 50Q/2.

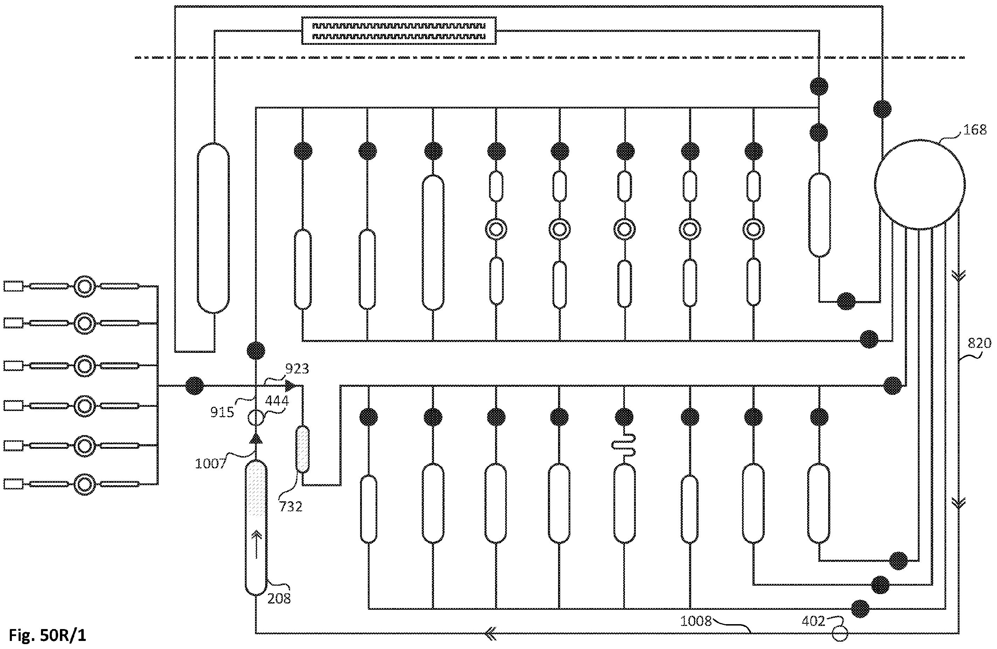

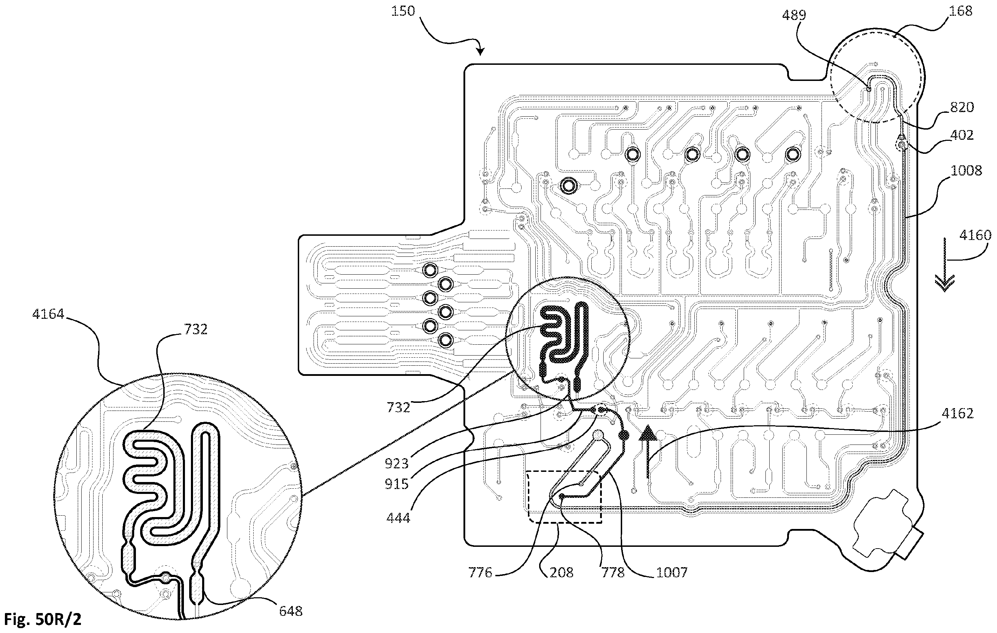

As seen in Figs. S0R/1 and 50R/2, valve 402 is opened, thereby allowing the pumping of air from pump 168 to dilution chamber 208 and hence the application of pressure to dilution chamber 208. Air is pumped from pump 168 into chamber 208 along an air flow path comprising channel 820, thereafter open valve 402 and thereafter pathway 1008. Air enters dilution chamber 208 via through hole 776. The direction of passage of air along the air flow path is indicated by an air flow arrow 4160 in Fig. 50R/2.

As a result of the pressure created by the pumping of air thereinto, part of the liquid held in dilution chamber 208 is driven out of chamber 208 via through hole 778. The liquid travels along a liquid flow path comprising pathway 1007, thereafter open valve 444, thereafter channel 915 and thereafter channel 923 leading into metering chamber 732, which metering chamber 732 terminates at liquid detection chamber 648. The entry of liquid to liquid detection chamber 648 is indicative that metering chamber 732 is full and hence that no further liquid flow is required. Preferably, metering chamber 732 is capable of holding approximately 120 microlitres of liquid. The direction of passage of liquid along the liquid flow path is indicated by a liquid flow arrow 4162 in Fig. 50R/2.

It is appreciated that the entry of liquid to liquid detection chamber 648 is sensed by a corresponding one of optical sensors 1682, as described hereinabove with reference to Figs. 47 - 49.

The content of metering chamber 732, following the pumping of air and liquid as described hereinabove, is shown at an enlargement 4164 in Fig. 50R/2. It is appreciated that the representation of the content of metering chamber 732 in enlargement 4164 is highly schematic and does not distinguish between the various components, namely primarily elution buffer, dilution buffer and nucleic acids, comprising the content.

Reference is now made to Figs. 50S/1 and 50S/2, which are simplified respective schematic and flow illustrations of a nineteenth stage in the operation of the cartridge 110 of Figs. 2A/1 - 10D by the instrument 100 of Figs. 11A - 49, which nineteenth stage follows the eighteenth stage in operation of the cartridge shown in Figs. 50R/1 and 50R/2.

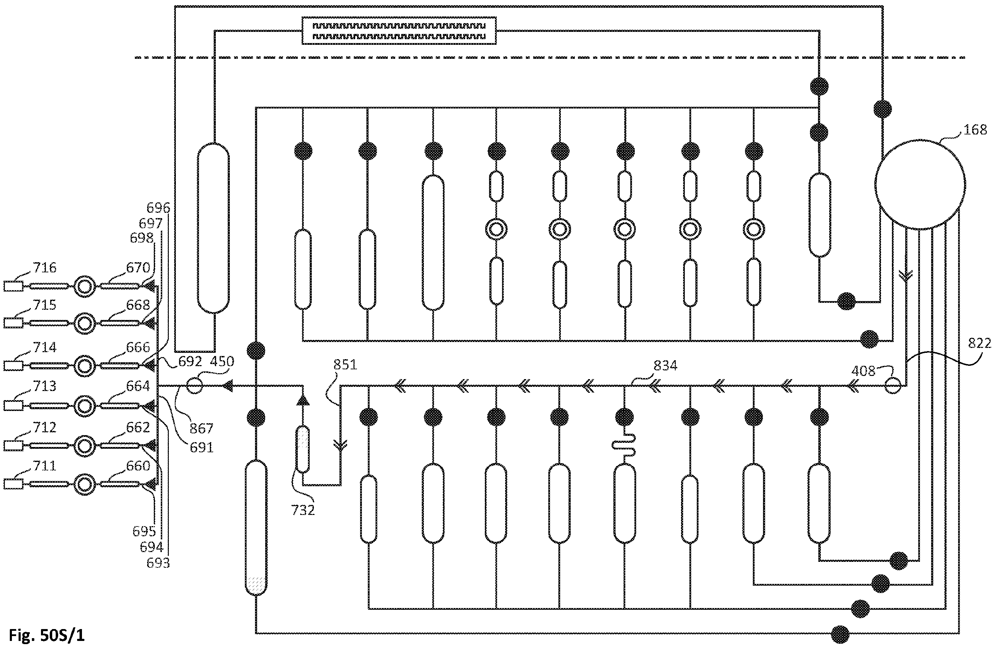

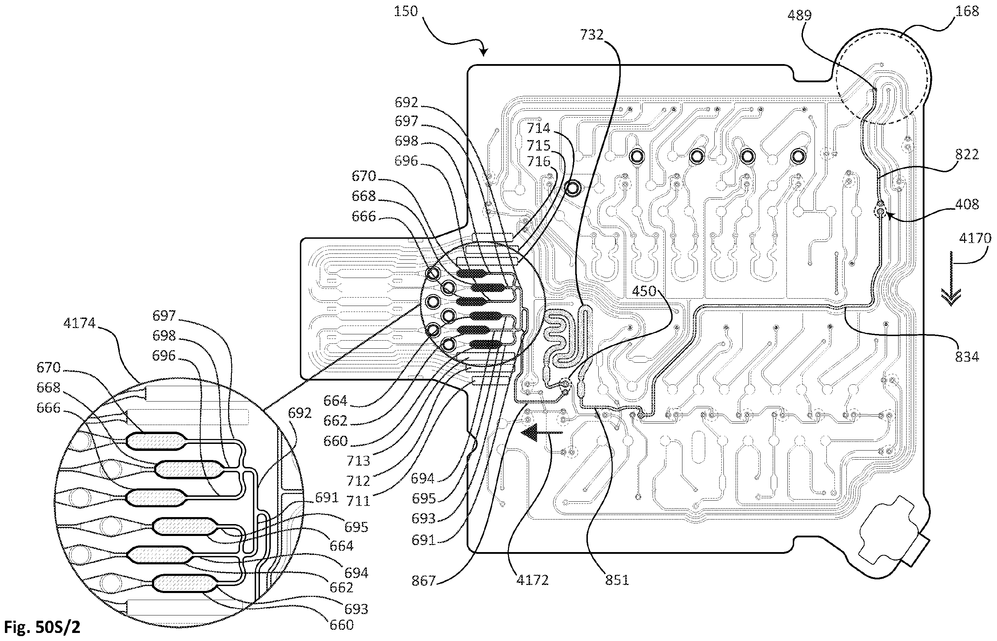

As seen in Figs. 50S/1 and 50S/2, valve 408 is opened, thereby allowing the pumping of air from pump 168 to metering chamber 732 and hence the application of pressure to metering chamber 732. Air is pumped from pump 168 into metering chamber 732 along an air flow path comprising channel 822, thereafter open valve 408, thereafter channel 834 and thereafter channel 851. The direction of passage of air along the air flow path is indicated by an air flow arrow 4170 in Fig. 50S/2.

As a result of the pressure created by the pumping of air thereinto, the liquids held in metering chamber 732 are driven out therefrom. The liquids travel along a liquid flow path comprising open valve 450 and thereafter channel 867. The liquid flow path additionally thereafter comprises channels 691 and 692, through which the liquids flow in parallel, and channels 693, 694, 695, 696, 697 and 698 through which liquids further flow in parallel into respective PCR aliquot chambers 660, 662, 664, 666, 668 and 670. The direction of the passage of liquid along the liquid flow path is indicated by a liquid flow arrow 4172 in Fig. 50S/2. It is appreciated that in Fig. 50S/2 only the final location of liquid in PCR aliquot chambers 660, 662, 664, 666, 668 and 670 is indicated, rather than the entirety of the liquid flow path thereinto, for the sake of clarity.

It is appreciated that the flow of liquid into PCR aliquot chambers 660, 662, 664, 666, 668 and 670 pressurizes gas springs 711, 712, 713, 714, 715 and 716.

The content of PCR aliquot chambers 660, 662, 664, 666, 668 and 670 following the pumping of air and liquid as described hereinabove, is shown at an enlargement 4174 in Fig. 50S/2. It is appreciated that the representation of the content of PCR aliquot chambers 660, 662, 664, 666, 668 and 670 in enlargement 4174 is highly schematic and does not distinguish between the various liquids, namely primarily elution buffer, dilution buffer and nucleic acids, comprising the content.

Reference is now made to Figs. 50T/1 and 50T/2, which are simplified respective schematic and flow illustrations of a twentieth stage in the operation of the cartridge 110 of Figs. 2A/1 - 10D by the instrument 100 of Figs. 11A - 49, which twentieth stage follows the nineteenth stage in operation of the cartridge shown in Figs. 50S/1 and 50S/2.

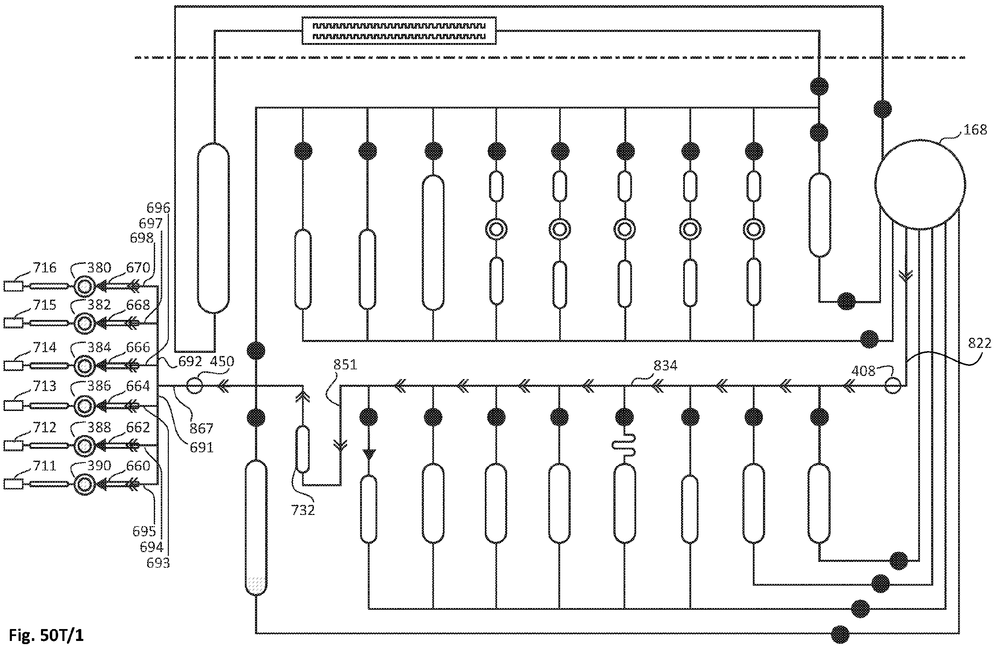

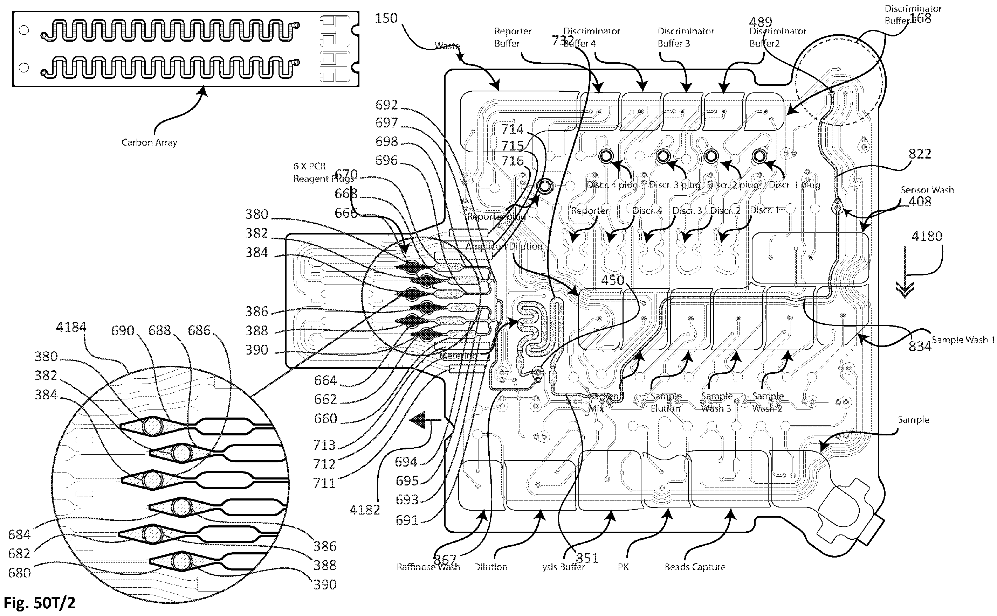

As seen in Figs. 50T/1 and 50T/2, valve 408 remains open thereby allowing the further pumping of air from pump 168 towards metering chamber 732 and PCR aliquot chambers 660, 662, 664, 666, 668 and 670, and hence the application of pressure to PCR aliquot chambers 660, 662, 664, 666, 668 and 670. Air is pumped from pump 168 into PCR aliquot chambers 660, 662, 664, 666, 668 and 670 along an air flow path comprising channel 822, thereafter open valve 408, thereafter channel 834, thereafter channel 851, thereafter metering chamber 732, thereafter open valve 450 and thereafter channel 867. The air flow path further comprises thereafter channels 691 and 692 and channels 693, 694, 695, 696, 697 and 698 leading into respective PCR aliquot chambers 660, 662, 664, 666, 668 and 670. The direction of passage of air along the air flow path is indicated by an air flow arrow 4180 in Fig. 50T/2.

As a result of the pressure created by the pumping of air thereinto, the liquids held in PCR aliquot chambers 660, 662, 664, 666, 668 and 670 are driven out therefrom in parallel towards respective reagent plugs 380, 382, 384, 386, 388 and 390 communicating with corresponding reagent plug hydration chambers 680, 682, 684, 686, 688 and 690. The direction of the passage of liquid along the liquid flow path is indicated by a liquid flow arrow 4182 in Fig. 50T/2. It is appreciated that in Fig. 50T/2 only the final location of liquid in reagent plugs 380, 382, 384, 386, 388 and 390 and corresponding reagent plug hydration chambers 680, 682, 684, 686, 688 and 690 is indicated, rather than the entirety of the liquid flow path thereinto, for the sake of clarity.

It is appreciated that the flow of liquid from PCR aliquot chambers 660, 662, 664, 666, 668 and 670 over reagent plugs 380, 382, 384, 386, 388 and 390 further pressurizes gas springs 711, 712, 713, 714, 715 and 716.

Reference is now made to Figs. 50U/1 and 50U/2, which are simplified respective schematic and flow illustrations of a twenty first stage in the operation of the cartridge 110 of Figs. 2A/1 - 10D by the instrument 100 of Figs. 11A - 49, which twenty first stage follows the twentieth stage in operation of the cartridge shown in Figs. 50T/1 and 50T/2.

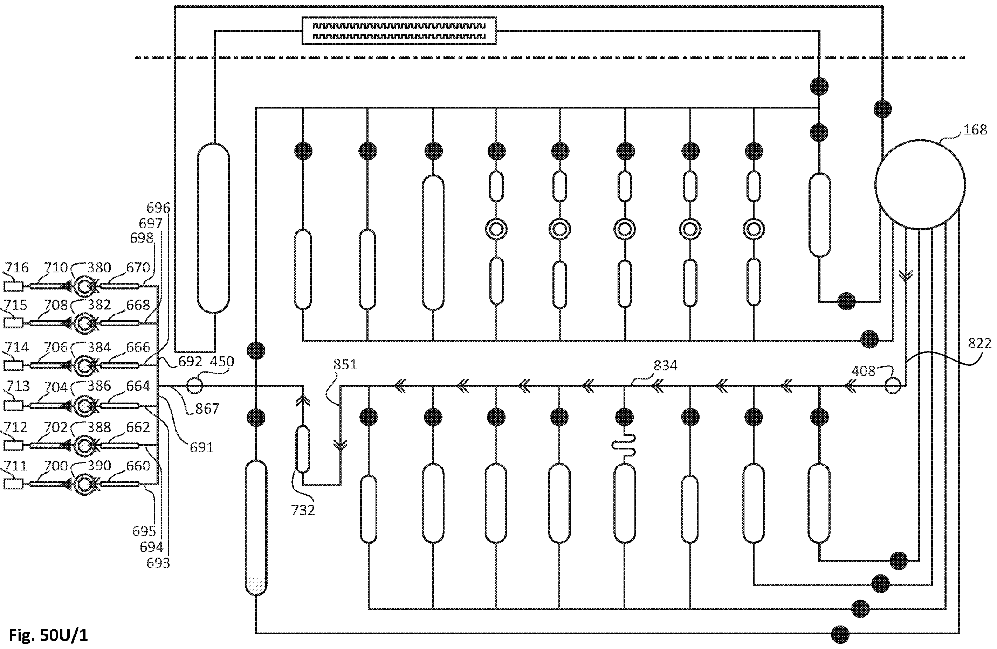

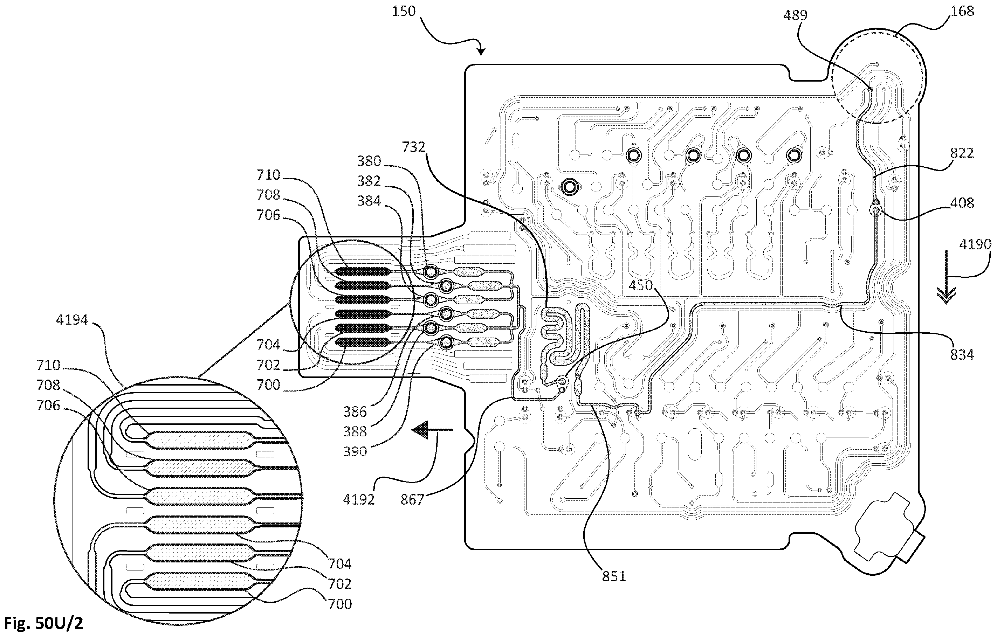

As seen in Figs. 50U/1 and 50U/2, valve 408 remains open, thereby allowing the further pumping of air from pump 168 towards metering chamber 732 and reagent plug hydration chambers 680, 682, 684, 686, 688 and 690, and hence the application of pressure to reagent plug hydration chambers 680, 682, 684, 686, 688 and 690. Air is pumped from pump 168 into reagent plug hydration chambers 680, 682, 684, 686, 688 and 690 along an air flow path comprising channel 822, thereafter open valve 408, thereafter channel 834, thereafter channel 851, thereafter metering chamber 732, thereafter open valve 450 and thereafter channel 867. The air flow path further comprises channels 691 and 692 and parallel channels 693, 694, 695, 696, 697 and 698 leading into respective PCR aliquot chambers 660, 662, 664, 666, 668 and 670 connected to reagent plug hydration chambers 680, 682, 684, 686, 688 and 690. The direction of passage of air along the air flow path is indicated by an air flow arrow 4190 in Fig. 50U/2.

As a result of the pressure created by the pumping of air thereinto, the liquids held in reagent plug hydration chambers 680, 682, 684, 686, 688 and 690 are driven out therefrom in parallel towards respective PCR chambers 700, 702, 704, 706, 708 and 710. The direction of the passage of liquid is indicated by a liquid flow arrow 4192 in Fig. 50U/2. It is appreciated that in Fig. 50U/2 only the final location of liquid in PCR chambers 700, 702, 704, 706, 708 and 710 is indicated, rather than the entirety of the liquid flow path thereinto, for the sake of clarity.

It is appreciated that the flow of liquid from reagent plug hydration chambers 680, 682, 684, 686, 688 and 690 and reagent plugs 380, 382, 384, 386, 388 and 390 towards PCR chambers 700, 702, 704, 706, 708 and 710 further pressurizes gas springs 711, 712, 713, 714, 715 and 716.

It is understood that PCR reactions take place in PCR chambers 700, 702, 704, 706, 708 and 710, seen particularly at an enlargement 4194 in Fig. 50U/2. PCR chambers 700, 702, 704, 706, 708 and 710 are preferably heated by at least one of, and preferably both of, upper thermocycling assembly 1560 and lower thermocycling assembly 1570 of instrument 100 (Fig. 11B) as described hereinabove with reference to Figs. 38 and 39.

Reference is now made to Figs. 50V/1 and 50V/2, which are simplified respective schematic and flow illustrations of a twenty second stage in the operation of the cartridge 110 of Figs. 2A/1 - 10D by the instrument 100 of Figs. 11A - 49, which twenty second stage follows the twenty first stage in operation of the cartridge shown in Figs. 50U/1 and 50U/2.

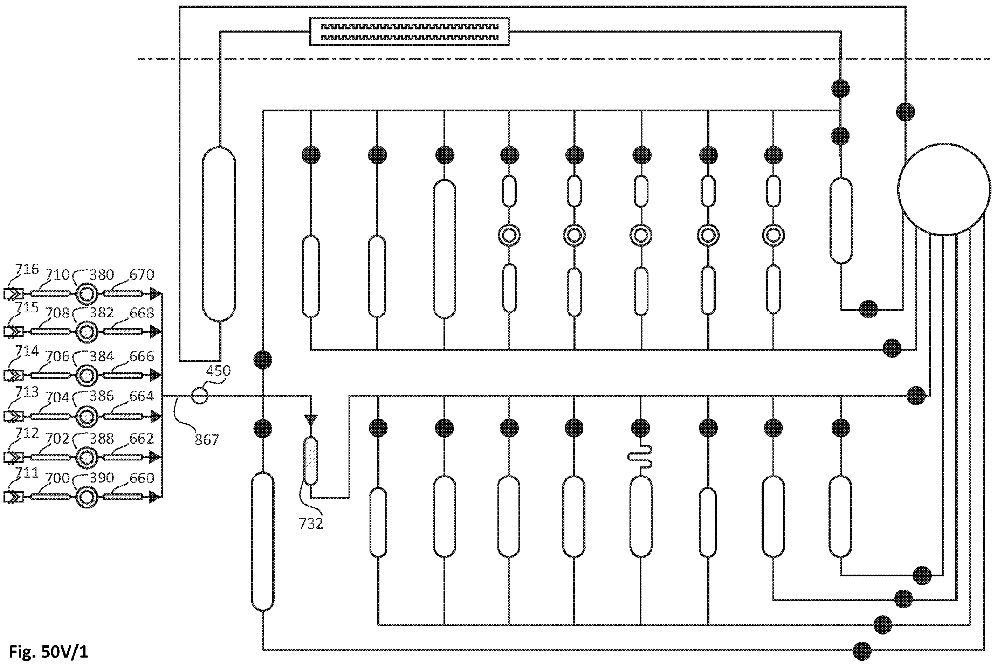

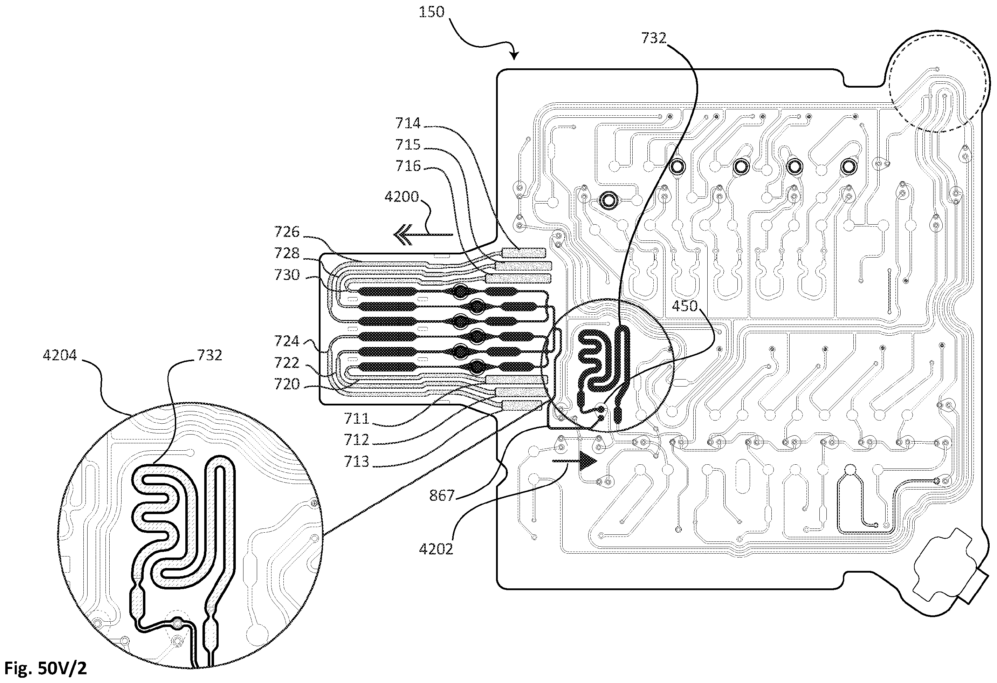

As seen in Figs. 50V/1 and 50V/2, valve 450 remains open and all other valves are closed, thereby allowing the release of pressure from gas springs 711, 712, 713, 714, 715 and 716. Air is driven from gas springs 711, 712, 713, 714, 715 and 716 through respective channels 720, 722, 724, 726, 728 and 730 towards corresponding PCR chambers 700, 702, 704, 706, 708 and 710. The direction of passage of air is indicated by an air flow arrow 4200 in Fig. 50V/2.

Liquid containing amplicons produced by the PCR reactions in PCR chambers 700, 702, 704, 706, 708 and 710 is forced by positive pressure from gas springs 711, 712, 713, 714, 715 and 716 out of PCR chambers 700, 702, 704, 706, 708 and 710 towards metering chamber 732. Liquid containing amplicons is driven along a liquid flow path comprising reagent plug hydration chambers 680, 682, 684, 686, 688 and 690, thereafter PCR aliquot chambers 660, 662, 664, 666, 668 and 670, thereafter parallel channels 693, 694, 695, 696, 697 and 698, thereafter channels 691 and 692, thereafter channel 867 and thereafter open valve 450 leading to metering chamber 732. The direction of passage of air along the liquid flow path is indicated by a liquid flow arrow 4202 in Fig. 50V/2.

The content of metering chamber 732 following the pumping of air and liquid as described hereinabove, is shown at an enlargement 4204 in Fig. 50V/2. It is appreciated that the representation of the content of metering chamber 732 in enlargement 4184 is highly schematic and does not distinguish between the various liquids, namely primarily liquid containing amplicons, comprising the content.

Reference is now made to Figs. 50W/1 and 50W/2, which are simplified respective schematic and flow illustrations of a twenty third stage in the operation of the cartridge 110 of Figs. 2A/1 - 10D by the instrument 100 of Figs. 11A - 49, which twenty third stage follows the twenty second stage in operation of the cartridge shown in Figs. 50V/1 and 50V/2.

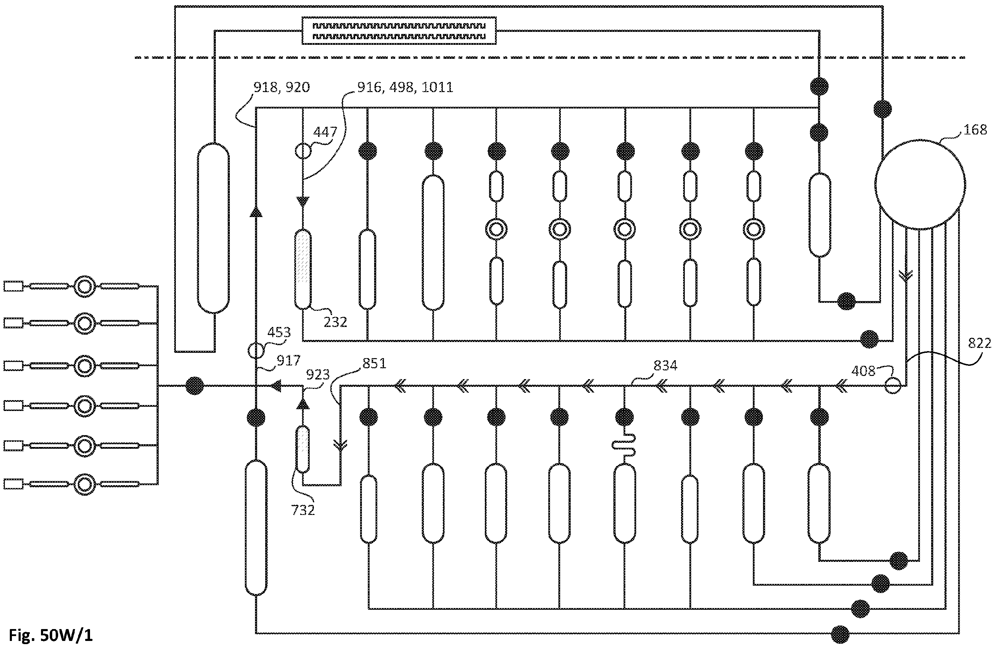

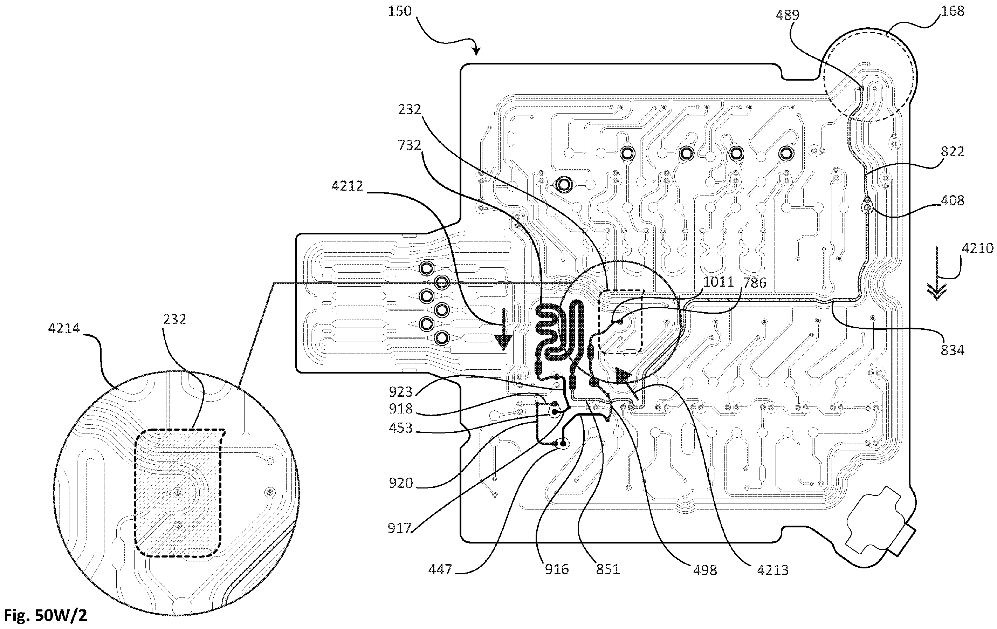

As seen in Figs. 50W/1 and 50W/2, valve 408 is opened, thereby allowing the pumping of air from pump 168 to metering chamber 732 and hence the application of pressure to metering chamber 732. Air is pumped from pump 168 into metering chamber 732 along an air flow path comprising channel 822, thereafter open valve 408, thereafter channel 834 and thereafter channel 851. The direction of passage of air along the air flow path is indicated by an air flow arrow 4210 in Fig. 50W/2.

As a result of the pressure created by the pumping of air thereinto, the amplicon containing liquid held in metering chamber 732 is driven out of metering chamber 732. The liquid travels along a liquid flow path comprising channel 923, thereafter channel 917, thereafter open valve 453, thereafter channel 918, thereafter channel 920, thereafter open valve 447, thereafter channel 916, thereafter channel 498 and thereafter pathway 1011 into amplicon dilution buffer dilution chamber 232. The liquid enters chamber 232 via through hole 786. The direction of passage of liquid along the liquid flow path is indicated sequentially by first and second liquid flow arrows 4212 and 4213 in Fig. 50W/2.

The content of chamber 232 following the pumping of air and liquid as described hereinabove, is shown at an enlargement 4214 in Fig. 50W/2. It is appreciated that the representation of the content of chamber 232 in enlargement 4214 is highly schematic and does not distinguish between the various liquids, namely primarily amplicons and amplicon dilution buffer, comprising the content.

Reference is now made to Figs. 50X/1 and 50X/2, which are simplified respective schematic and flow illustrations of a twenty fourth stage in the operation of the cartridge 110 of Figs. 2A/1 - 10D by the instrument 100 of Figs. 11A - 49, which twenty fourth stage follows the twenty third stage in operation of the cartridge shown in Figs. 50W/1 and 50W/2.

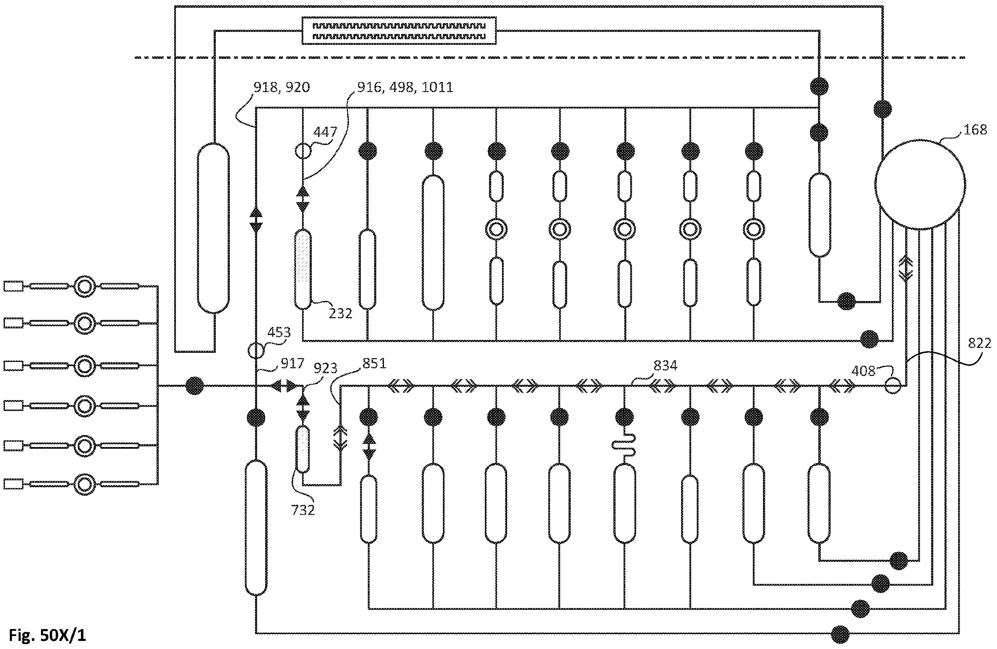

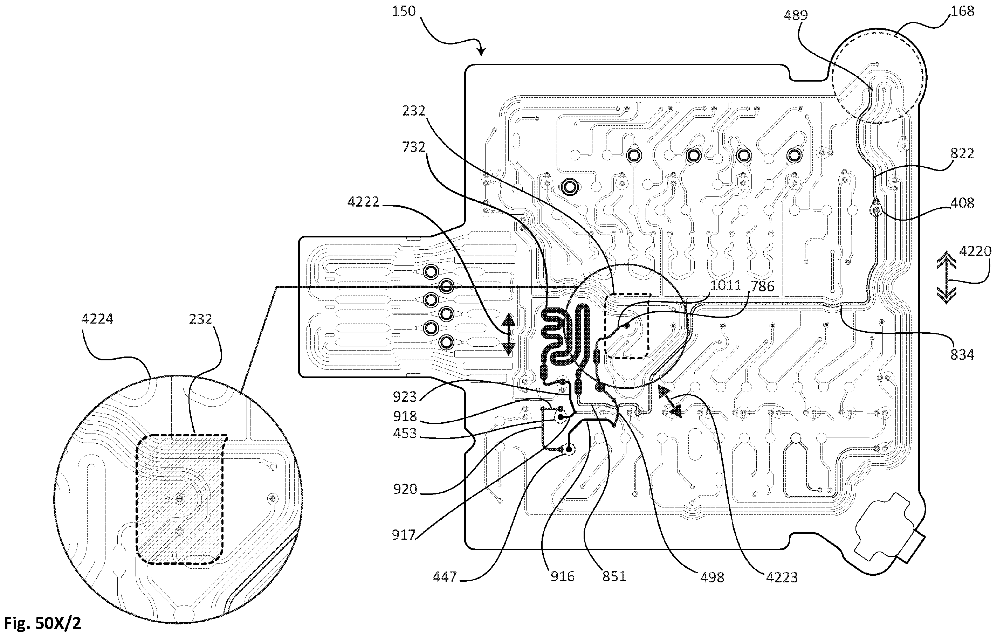

As seen in Figs. 50X/1 and 50X/2, valve 408 remains open and air is repeatedly pumped back and forth between pump 168 and metering chamber 732. Air travels between pump 168 and metering chamber 732 along an air flow path comprising channel 822, open valve 408, channel 834 and channel 851. The bi-directional flow of air between pump 168 and metering chamber 732 is indicated by a double-headed air flow arrow 4220 in Fig. 50X/2.

As a result of the repeated back and forward pressure created by the back and forth passage of air, the liquid contents of chamber 232 are repeatedly driven back and forth between chamber 232 and metering chamber 732, thereby mixing the amplicons and the amplicon dilution buffer. The liquid contents of chamber 232 are driven between chamber 232 and metering chamber 732 along a liquid flow path comprising channel 923, channel 917, open valve 453, channel 918, channel 920, open valve 447, channel 916, channel 498, pathway 1011 and through hole 786. The bi-directional passage of liquid between chamber 232 and metering chamber 732 is indicated by first and second double-headed liquid flow arrows 4222 and 4223 in Fig. 50X/2.

The mixed content of chamber 232 following the passage of air and liquid described hereinabove, is shown schematically at an enlargement 4224 in Fig. 50X/2. It is appreciated that the representation of the content of chamber 232 in enlargement 4224 is highly schematic and does not distinguish between the various liquids, namely primarily amplicons and amplicon dilution buffer, comprising the content.

Reference is now made to Figs. 50Y/1 and 50Y/2, which are simplified respective schematic and flow illustrations of a twenty fifth stage in the operation of the cartridge 110 of Figs. 2A/1 - 10D by the instrument 100 of Figs. 11A - 49, which twenty fifth stage follows the twenty fourth stage in operation of the cartridge shown in Figs. 50X/1 and 50X/2.

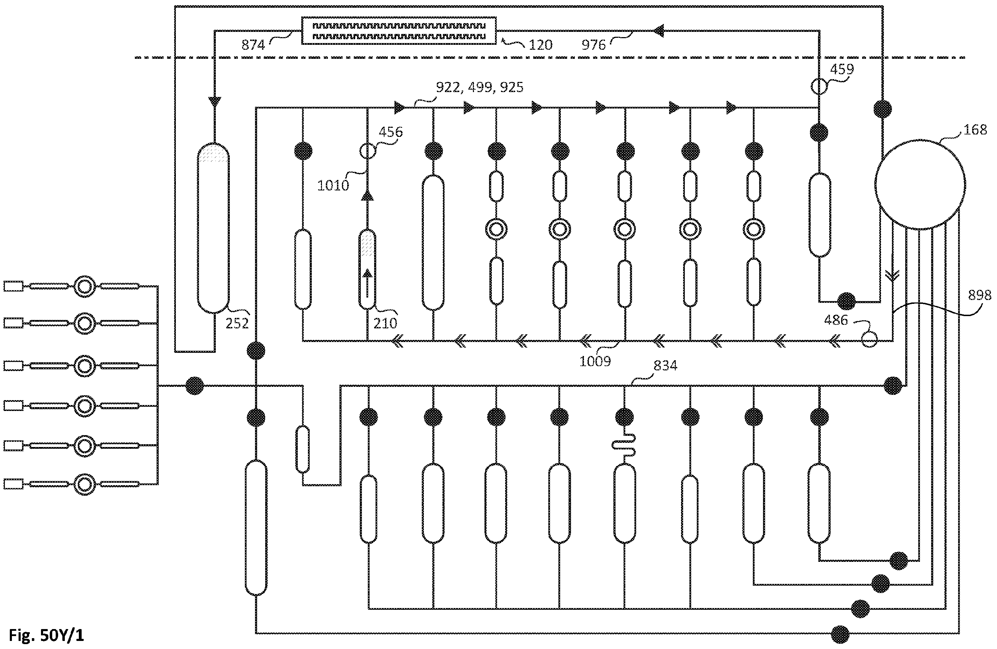

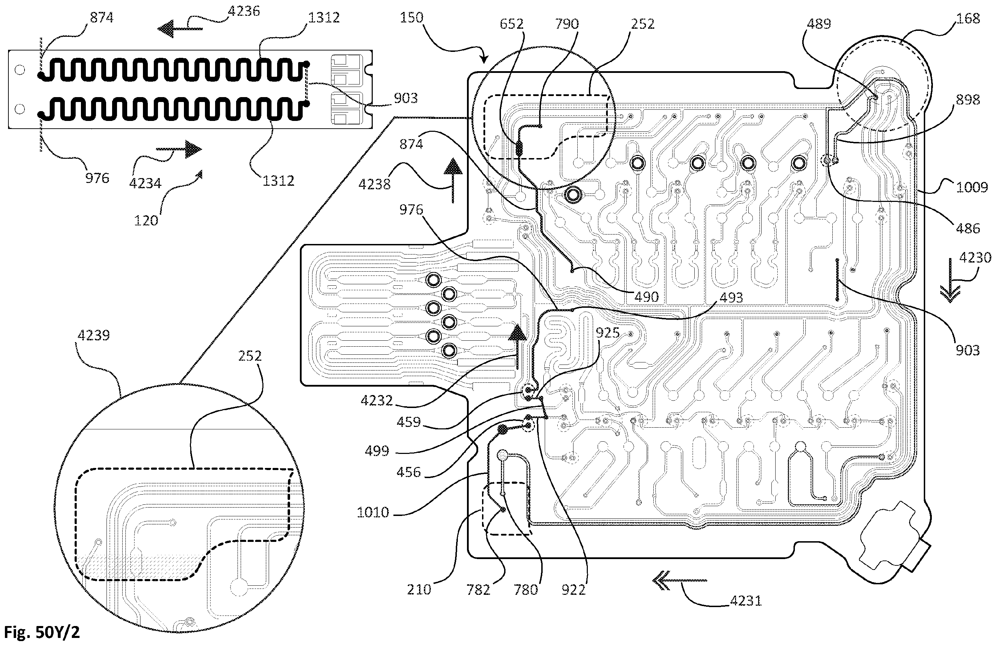

As seen in Figs. 50Y/1 and 50Y/2, valve 486 is opened, thereby allowing the pumping of air from pump 168 to Raffinose washing liquid containing chamber 210 and hence the application of pressure to Raffinose washing liquid containing chamber 210. Air is pumped from pump 168 into Raffinose washing liquid containing chamber 210 along an air flow path comprising channel 898, open valve 486 and pathway 1009. Air enters chamber 210 via through hole 780. The direction of passage of air along the air flow path is indicated sequentially by first and second air flow arrows 4230 and 4231 in Fig. 50Y/2.

As a result of the pressure created by the pumping of air thereinto, the Raffinose washing liquid held in chamber 210 is driven out of chamber 210. The liquid travels along a liquid flow path comprising pathway 1010, thereafter open valve 456, thereafter channel 922, thereafter channel 499, thereafter channel 925, thereafter open valve 459 and thereafter channel 976 into carbon array assembly 120. The direction of liquid flow along the liquid flow path into carbon array assembly 120 is indicated by a first liquid flow arrow 4232. The Raffinose washing liquid enters carbon array assembly 120 via aperture 493 and travels along first snake-shaped cut out 1312 in a direction indicated by a second liquid flow arrow 4234, thereafter through channel 903 and thereafter along second snake-shaped cut out 1312 in a direction indicated by a third liquid flow arrow 4236. The Raffinose washing liquid leaves carbon array assembly 120 via aperture 490 leading to channel 874 and enters waste chamber 252 via through hole 790, in a direction indicated by a fourth liquid flow arrow 4238.

A liquid sensor 1682 (Fig. 13C) may be used in conjunction with liquid detection chamber 652, into which liquid flows from carbon array assembly 120, in order to detect a presence or absence of liquid therein.

It is appreciated that the Raffinose washing liquid washes away raffinose in the carbon array assembly 120, thus readying carbon array assembly 120 for use. The raffinose and Raffinose washing liquid drained from carbon array assembly 120 and held in waste chamber 252 is shown schematically at an enlargement 4239 in Fig. 50Y/2.

Reference is now made to Figs. 50Z/1 and 50Z/2, which are simplified respective schematic and flow illustrations of a twenty sixth stage in the operation of the cartridge 110 of Figs. 2A/1 - 10D by the instrument 100 of Figs. 11A - 49, which twenty sixth stage follows the twenty fifth stage in operation of the cartridge shown in Figs. 50Y/1 and 50Y/2.

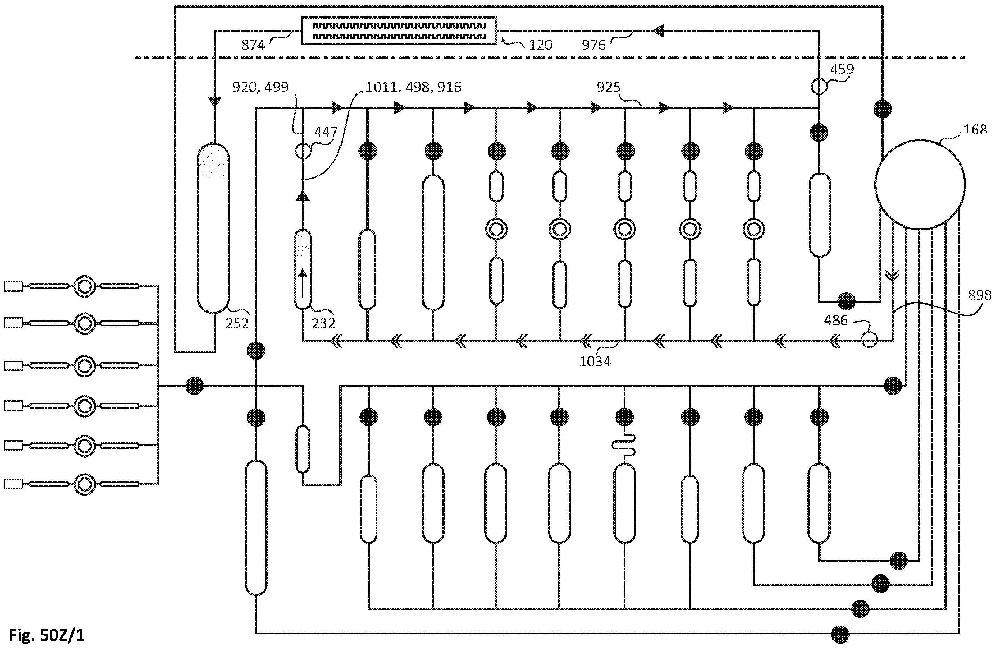

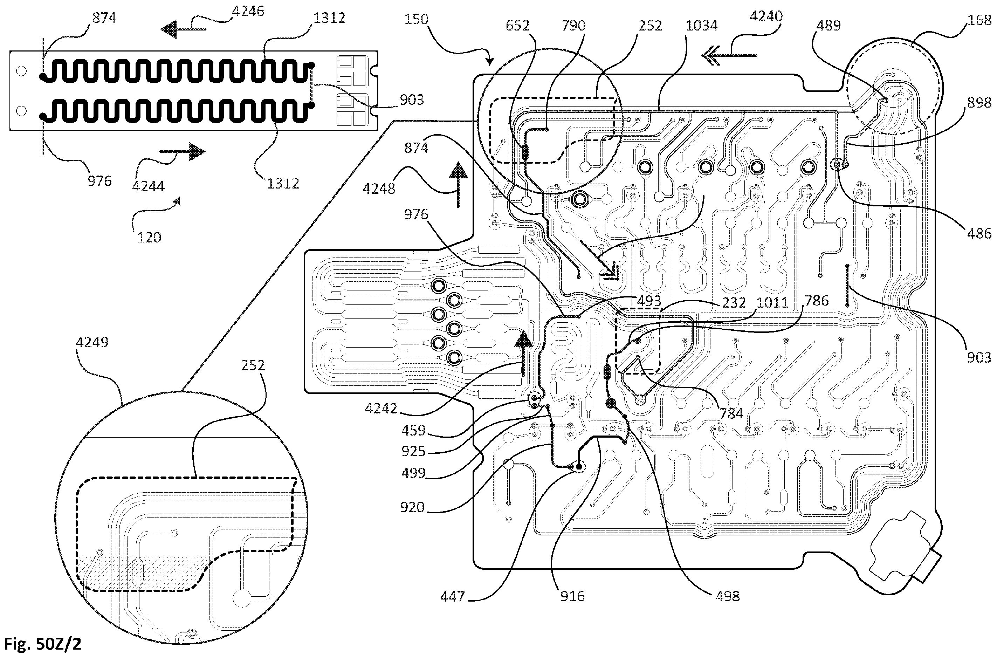

As seen in Figs. 50Z/1 and 50Z/2, valve 486 is opened, thereby allowing the pumping of air from pump 168 to chamber 232 and hence the application of pressure to chamber 232, which chamber 232 contains diluted amplicons. Air is pumped from pump 168 into chamber 232 along an air flow path comprising channel 898, thereafter open valve 486 and thereafter pathway 1034. Air enters chamber 232 via through hole 784. The direction of passage of air along the air flow path is indicated by an air flow arrow 4240 in Fig. 50Z/2.

As a result of the pressure created by the pumping of air thereinto, the liquid containing diluted amplicons held in chamber 232 is driven out of chamber 232. The liquid travels along a liquid flow path comprising through hole 786, pathway 1011, thereafter channel 498, thereafter channel 916, thereafter open valve 447, thereafter channel 920, thereafter channel 499, thereafter channel 925, thereafter open valve 459 and thereafter channel 976 into carbon array assembly 120. The direction of liquid flow along the liquid flow path into carbon array assembly 120 is indicated by a first liquid flow arrow 4242 in Fig. 50Z/2. The liquid enters carbon array assembly 120 via aperture 493 and travels along first snake-shaped cut out 1312 in a direction indicated by a second liquid flow arrow 4244 in Fig. 50Z/2, thereafter through channel 903 and thereafter along second snake-shaped cut out 1312 in a direction indicated by a third liquid flow arrow 4246 in Fig. 50Z/2. Excess diluted amplicons not having bound to carbon array assembly 120 leave carbon array assembly 120 via aperture 490 leading to channel 874 and enter waste chamber 252 via through hole 790, in a direction indicated by a fourth liquid flow arrow 4248 in Fig. 50Z/2.

A liquid sensor 1682 may be used in conjunction with liquid detection chamber 652, into which liquid flows from carbon array assembly 120, in order to detect in order to detect a presence or absence of liquid therein.

The content of waste chamber 252, following the pumping of air and liquid as described hereinabove, is shown at an enlargement 4249 in Fig. 50Z/2. It is appreciated that the representation of the content of chamber 252 in enlargement 4249 is highly schematic and does not distinguish between the various waste liquids, namely Raffinose washing liquid and diluted amplicons, comprising the content.

Reference is now made to Figs. 50AA/1 and 50AA/2, which are simplified respective schematic and flow illustrations of a twenty seventh stage in the operation of the cartridge 110 of Figs. 2A/1 - 10D by the instrument 100 of Figs. 11A - 49, which twenty seventh stage follows the twenty sixth stage in operation of the cartridge shown in Figs. 50Z/1 and 50Z/2.

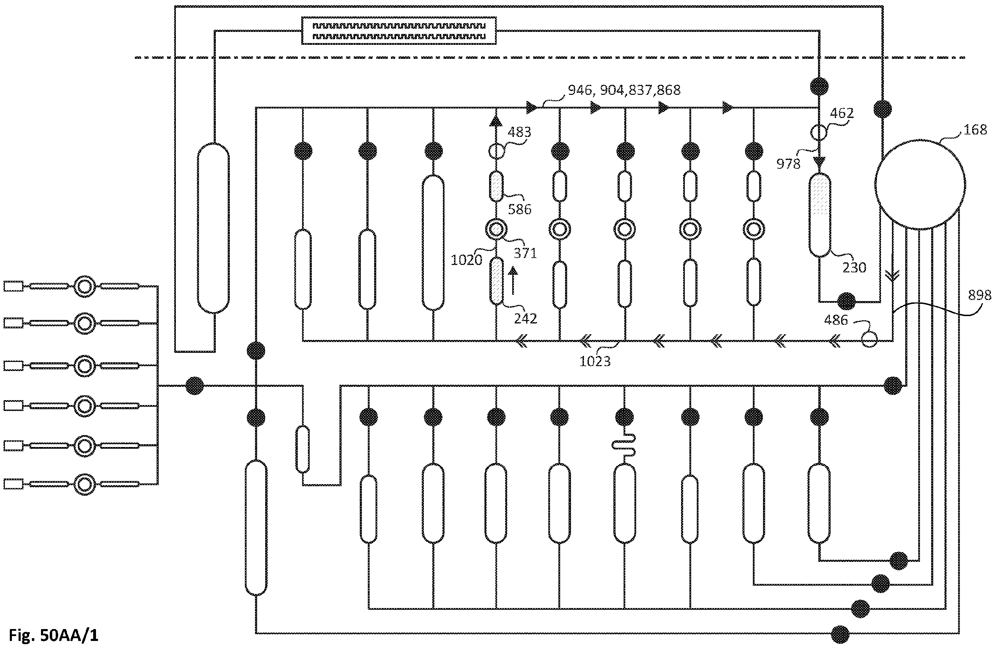

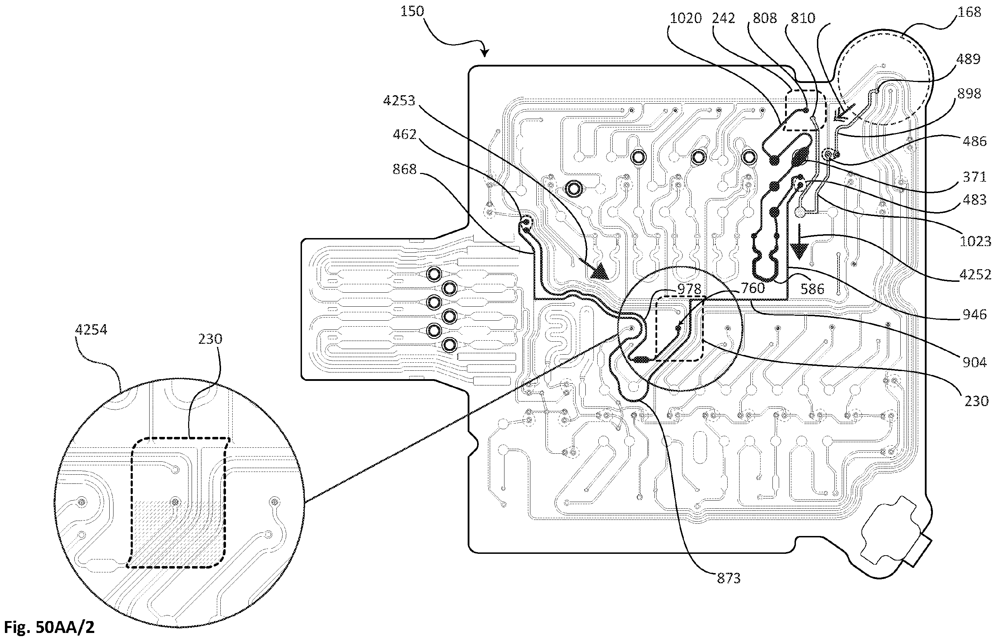

As seen in Figs. 50AA/1 and 50AA/2, valve 486 is opened thereby allowing the pumping of air from pump 168 to Discriminator I Buffer containing chamber 242 and hence the application of pressure to chamber 242. Air is pumped from pump 168 into chamber 242 along an air flow path comprising channel 898, thereafter open valve 486 and thereafter pathway 1023. Air enters chamber 242 via through hole 810. The direction of passage of air along the air flow path is indicated by an air flow arrow 4250 in Fig. 50AA/2.

As a result of the pressure created by the pumping of air thereinto, the Discriminator I Buffer liquid held in chamber 242 is driven out of chamber 242. Discriminator I Buffer travels along a liquid flow path comprising pathway 1020, thereafter Discriminator I reagent plug 371, thereafter reservoir 586, thereafter open valve 483, thereafter channel 946, thereafter channel 904, thereafter channel 837, thereafter channel 868, thereafter open valve 462 and thereafter channel 978 into backend mixing chamber 230. Discriminator I Buffer enters backend mixing chamber 230 via through hole 760. The direction of passage of liquid along the liquid flow path is indicated by sequential first and second liquid flow arrows 4252 and 4253 in Fig. 50AA/2.

The content of backend mixing chamber 230 following the pumping of air and liquid as described hereinabove, is shown schematically at an enlargement 4254 in Fig. 50AA/2. It is appreciated that the representation of the content of chamber 230 in enlargement 4234 is highly schematic and does not distinguish between the various components, namely dried discriminator I and Discriminator I buffer, comprising the content. It is further appreciated that the twenty seventh stage of operation of cartridge 110 illustrated in Figs. 50AA/1 and 50AA/2 may be followed by a mixing step, in order to reconstitute the dried Discriminator I with the Discriminator I buffer.

Reference is now made to Figs. 50AB/1 and 50AB/2, which are simplified respective schematic and flow illustrations of a twenty eighth stage in the operation of the cartridge 110 of Figs. 2A/1 - 10D by the instrument 100 of Figs. 11A - 49, which twenty eighth stage follows the twenty seventh stage in operation of the cartridge shown in Figs. 50AA/1 and 50AA/2.

As seen in Figs. 50AB/1 and 50AB/2, valve 465 is opened, thereby allowing the pumping of air from pump 168 to backend mixing chamber 230 and hence the application of pressure to chamber 230, which chamber 230 contains reconstituted Discriminator I. Air is pumped from pump 168 into chamber 230 along an air flow path comprising channel 894, thereafter channel 927, thereafter open valve 465 and thereafter channel 870. Air enters chamber 230 via through hole 759. The direction of passage of air along the air flow path is indicated by an air flow arrow 4260.

As a result of the pressure created by the pumping of air thereinto, the liquid reconstituted Discriminator I held in chamber 230 is driven out of chamber 230. The liquid travels along a liquid flow path comprising channel 978, thereafter open valve 462, thereafter channel 868, thereafter channel 865, thereafter open valve 459 and thereafter channel 976 into carbon array assembly 120. The direction of liquid flow along the liquid flow path into carbon array assembly 120 is indicated by sequential first, second and third liquid flow arrows 4261, 4262 and 4263. The liquid enters carbon array assembly 120 via aperture 493 and travels along first snake-shaped cut out 1312 in a direction indicated by a fourth liquid flow arrow 4264, thereafter through channel 903 and thereafter along second snake-shaped cut out 1312 in a direction indicated by a fifth liquid flow arrow 4265. Excess Discriminator I not having bound to carbon array assembly 120 leaves carbon array assembly 120 via aperture 490 leading to channel 874 and enters waste chamber 252 via through hole 790, in a direction indicated by a sixth liquid flow arrow 4266.

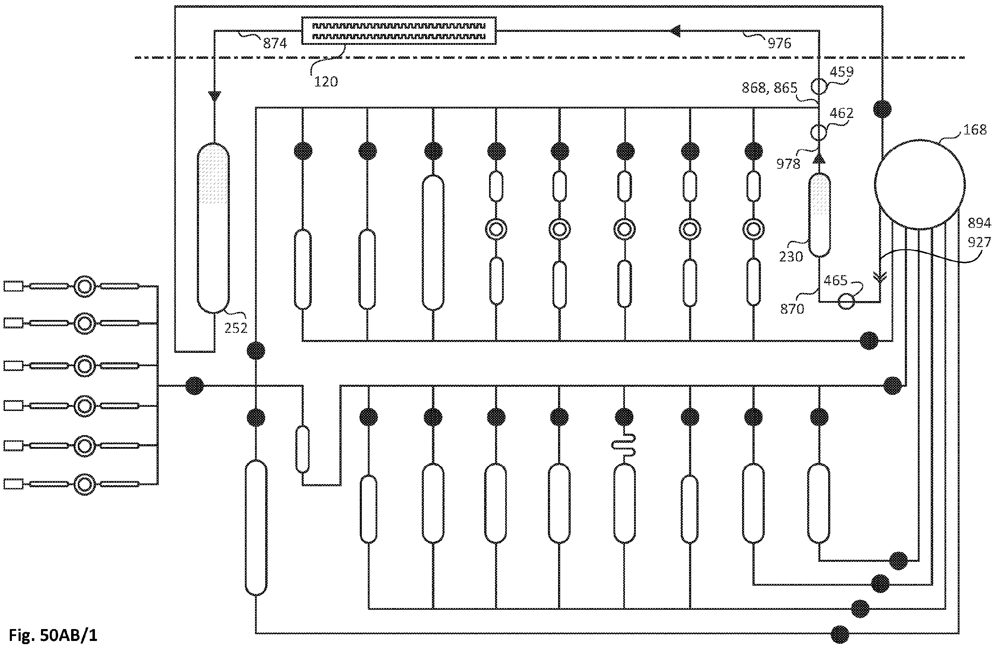

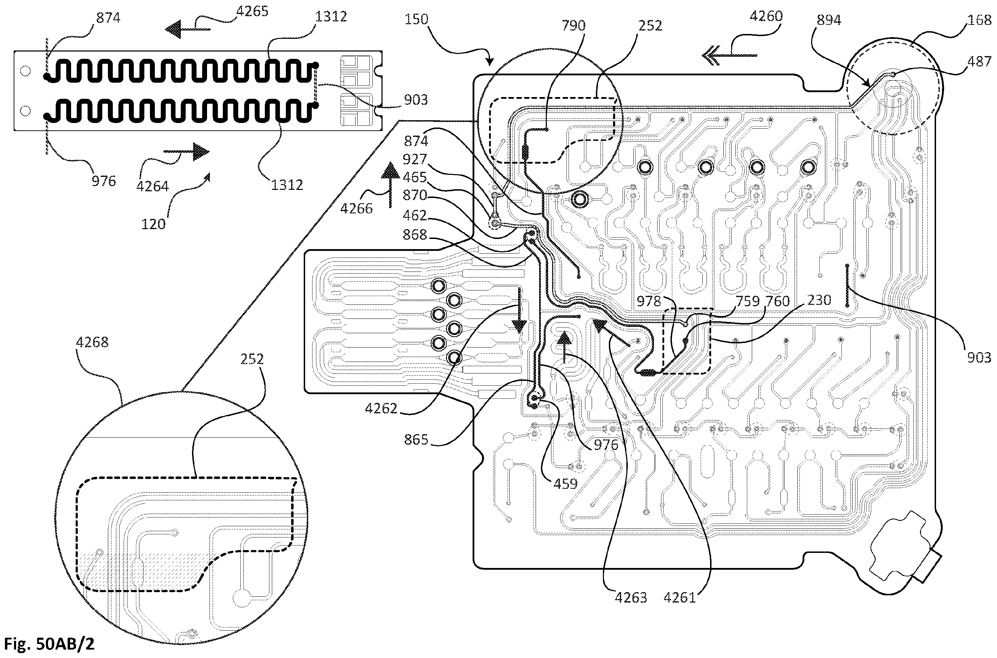

The content of waste chamber 252 following the pumping of air and liquid as described hereinabove, is shown at an enlargement 4268 in Fig. 50AB/2. It is appreciated that the representation of the content of chamber 252 in enlargement 4249 is highly schematic and does not distinguish between the various waste liquids, namely Raffinose washing liquid, diluted amplicons and reconstituted Discriminator I, comprising the content.

Reference is now made to Figs. 50AC/1 and 50AC/2, which are simplified respective schematic and flow illustrations of a twenty ninth stage in the operation of the cartridge 110 of Figs. 2A/1 - 10D by the instrument 100 of Figs. 11A - 49, which twenty ninth stage follows the twenty eighth stage in operation of the cartridge shown in Figs. 50AB/1 and 50AB/2.

As seen in Figs. 50AC/1 and 50AC/2, valve 486 is opened thereby allowing the pumping of air from pump 168 to Discriminator II Buffer containing chamber 244 and hence the application of pressure to chamber 244. Air is pumped from pump 168 into chamber 244 along an air flow path comprising channel 898, open valve 486 and pathway 1018. Air enters chamber 244 via through hole 804. The direction of passage of air along the air flow path is indicated by an air flow arrow 4270.

As a result of the pressure created by the pumping of air thereinto, the Discriminator II Buffer liquid held in chamber 244 is driven out of chamber 244. Discriminator II Buffer travels along a liquid flow path comprising pathway 1019, Discriminator II reagent plug 372, reservoir 588, open valve 480, channel 948, channel 904, channel 873, channel 868, open valve 462 and channel 978 into backend mixing chamber 230. Discriminator II Buffer enters backend mixing chamber 230 via through hole 760. The direction of passage of liquid along the liquid flow path is indicated by sequential first and second liquid flow arrows 4272 and 4273.

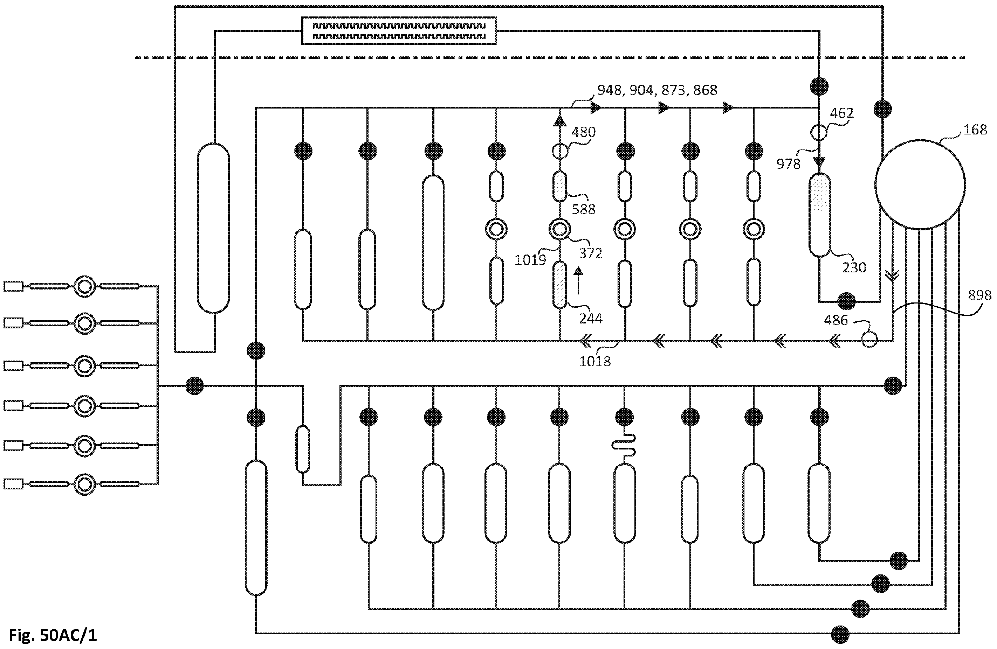

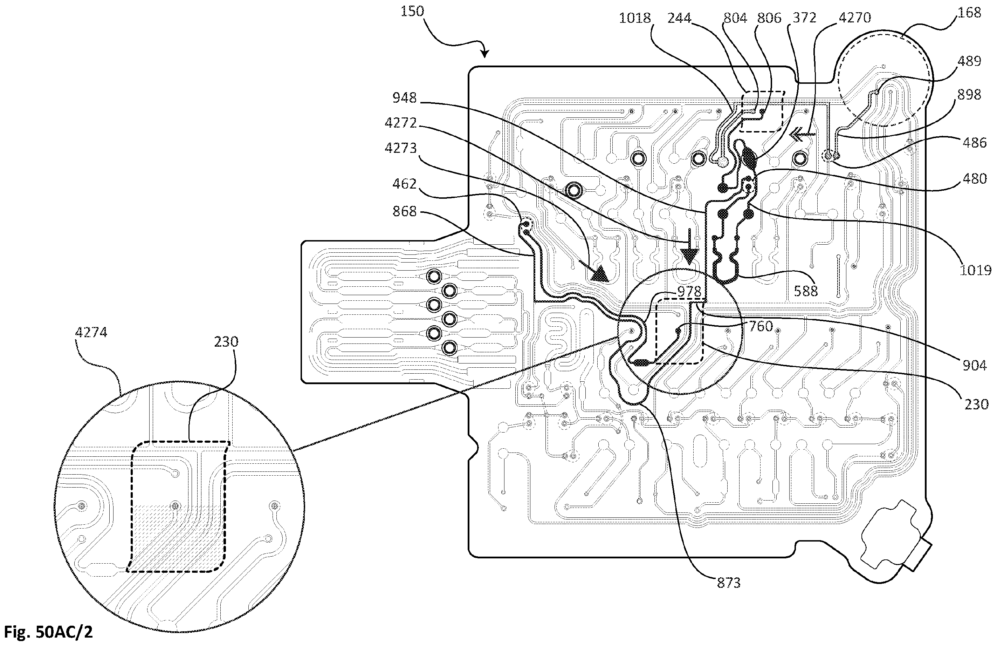

The content of backend mixing chamber 230 following the pumping of air and liquid as described hereinabove, is shown schematically at an enlargement 4274 in Fig. 50AC/2. It is appreciated that the representation of the content of chamber 230 in enlargement 4254 is highly schematic and does not distinguish between the various components, namely dried discriminator II and Discriminator II buffer, comprising the content. It is further appreciated that the twenty ninth stage of operation of cartridge 110 illustrated in Figs. 50AC/1 and 50AC/2 may be followed by a mixing step, in order to reconstitute the dried Discriminator II with the Discriminator II buffer.

Reference is now made to Figs. 50AD/1 and 50AD/2, which are simplified respective schematic and flow illustrations of a thirtieth stage in the operation of the cartridge 110 of Figs. 2A/1 - 10D by the instrument 100 of Figs. 11A - 49, which thirtieth stage follows the twenty ninth stage in operation of the cartridge shown in Figs. 50AC/1 and 50AC/2.

As seen in Figs. 50AD/1 and 50AD/2, valve 465 is opened thereby allowing the pumping of air from pump 168 to backend mixing chamber 230 and hence the application of pressure to chamber 230, which chamber 230 contains reconstituted Discriminator II. Air is pumped from pump 168 into chamber 230 along an air flow path comprising channel 894, channel 927, open valve 465 and channel 870. Air enters chamber 230 via through hole 759. The direction of passage of air along the air flow path is indicated by an air flow arrow 4280.

As a result of the pressure created by the pumping of air thereinto, the liquid reconstituted Discriminator II held in chamber 230 is driven out of chamber 230. The liquid travels along a liquid flow path comprising channel 978, open valve 462, channel 868, channel 865, open valve 459 and channel 976 into carbon array assembly 120. The direction of liquid flow along the liquid flow path into carbon array assembly 120 is indicated by first, second and third liquid flow arrows 4281, 4282 and 4283. The liquid enters carbon array assembly 120 via aperture 493 and travels along first snake-shaped cut out 1312 in a direction indicated by a fourth liquid flow arrow 4284, thereafter through channel 903 and thereafter along second snake-shaped cut out 1312 in a direction indicated by a fifth liquid flow arrow 4285. Excess Discriminator II not having bound to carbon array assembly 120 leaves carbon array assembly 120 via aperture 490 leading to channel 874 and enter waste chamber 252 via through hole 790, in a direction indicated by a sixth liquid flow arrow 4286.

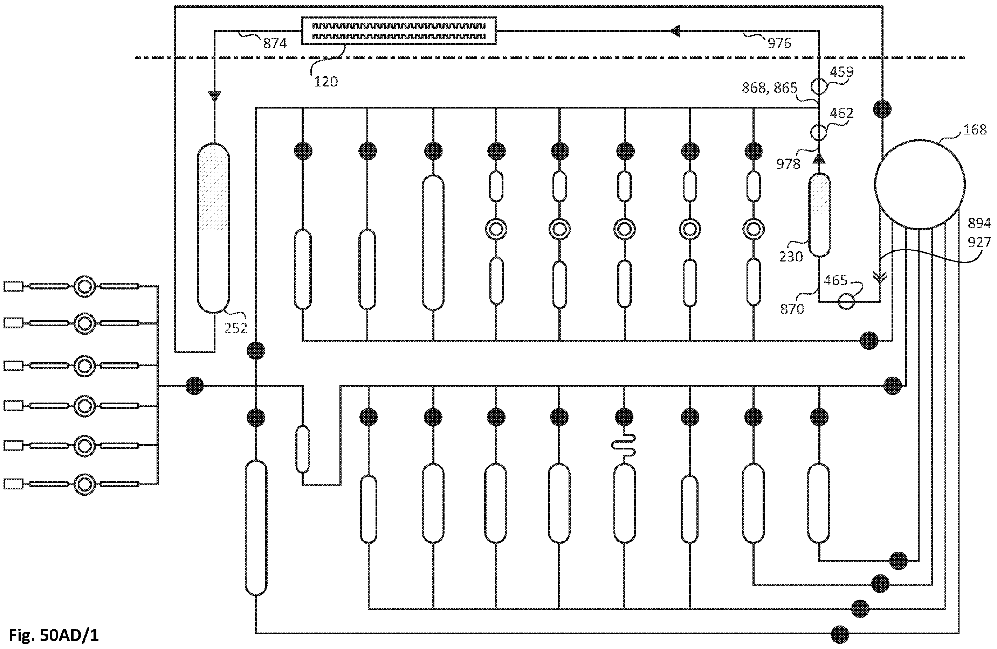

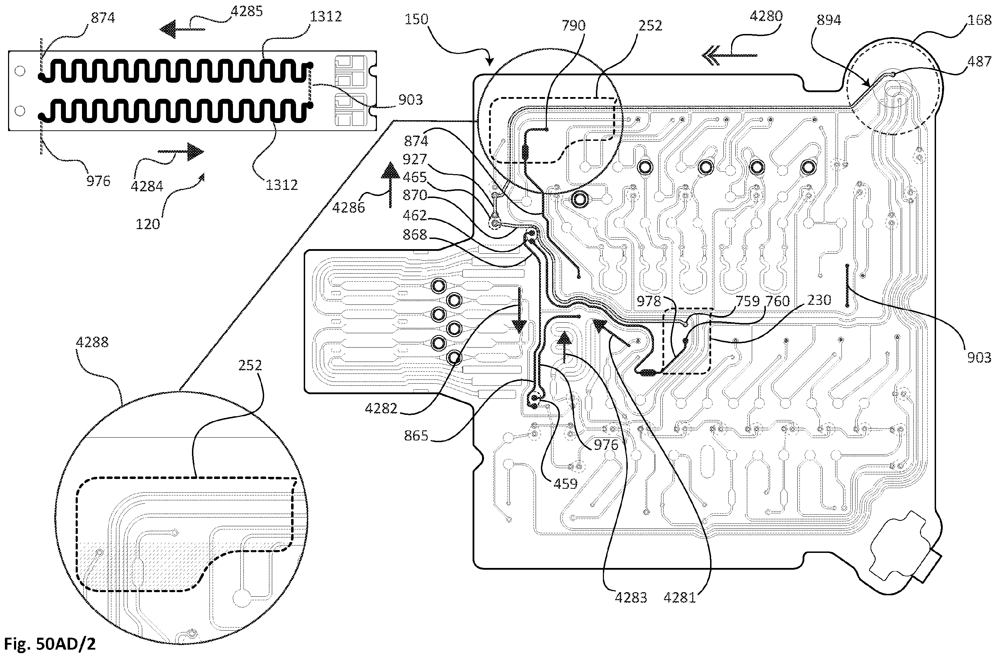

The content of waste chamber 252 following the pumping of air and liquid as described hereinabove, is shown at an enlargement 4288 in Fig. 50AD/2. It is appreciated that the representation of the content of chamber 252 in enlargement 4269 is highly schematic and does not distinguish between the various waste liquids, namely Raffinose washing liquid, diluted amplicons and reconstituted Discriminators I and II, comprising the content.

Reference is now made to Figs. 50AE/1 and 50AE/2, which are simplified respective schematic and flow illustrations of a thirty first stage in the operation of the cartridge 110 of Figs. 2A/1 - 10D by the instrument 100 of Figs. 11A - 49, which thirty first stage follows the thirtieth stage in operation of the cartridge shown in Figs. 50AD/1 and 50AD/2.

As seen in Figs. 50AE/1 and 50AE/2, valve 486 is opened thereby allowing the pumping of air from pump 168 to Discriminator III Buffer containing chamber 246 and hence the application of pressure to chamber 246. Air is pumped from pump 168 into chamber 246 along an air flow path comprising channel 898, open valve 486 and pathway 1016. Air enters chamber 246 via through hole 800. The direction of passage of air along the air flow path is indicated by an air flow arrow 4290.

As a result of the pressure created by the pumping of air thereinto, the Discriminator III Buffer liquid held in chamber 246 is driven out of chamber 246. Discriminator III Buffer travels along a liquid flow path comprising pathway 1017, Discriminator III reagent plug 374, reservoir 590, open valve 477, channel 950, channel 904, channel 873, channel 868, open valve 462 and channel 978 into backend mixing chamber 230. Discriminator III Buffer enters backend mixing chamber 230 via through hole 760. The direction of passage of liquid along the liquid flow path is indicated by sequential first and second liquid flow arrows 4292 and 4293.

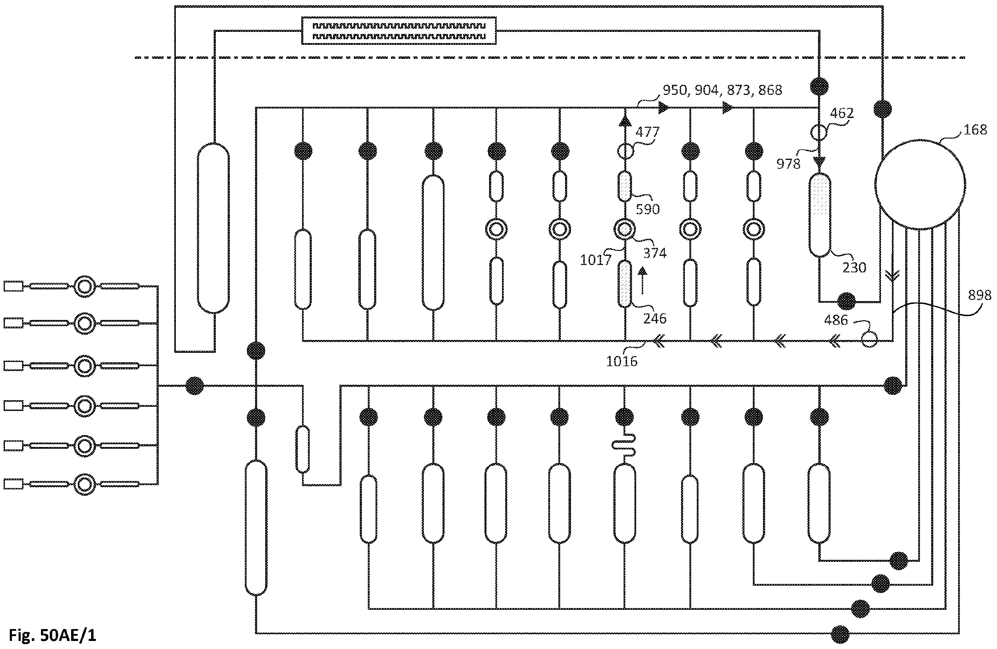

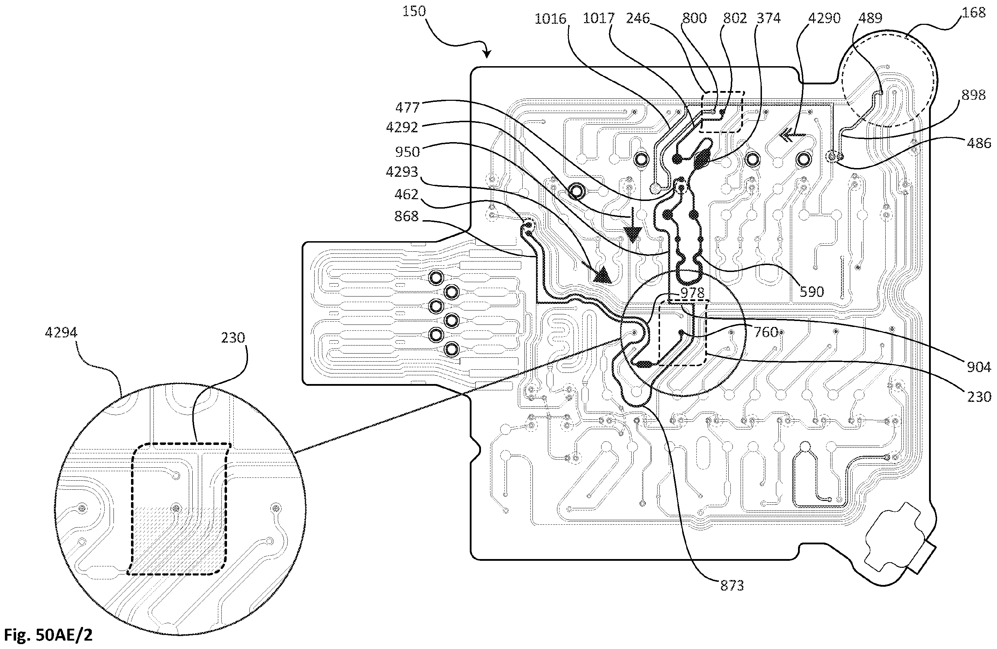

The content of backend mixing chamber 230 following the pumping of air and liquid as described hereinabove, is shown schematically at an enlargement 4294 in Fig. 50AE/2. It is appreciated that the representation of the content of chamber 230 in enlargement 4274 is highly schematic and does not distinguish between the various components, namely dried discriminator **III** and Discriminator **III** buffer, comprising the content. It is further appreciated that the thirty first stage of operation of cartridge 110 illustrated in Figs. 50AE/1 and 50AE/2 may be followed by a mixing step, in order to reconstitute the dried Discriminator III with the Discriminator III buffer.

Reference is now made to Figs. 50AF/1 and 50AF/2, which are simplified respective schematic and flow illustrations of a thirty second stage in the operation of the cartridge 110 of Figs. 2A/1 - 10D by the instrument 100 of Figs. 11A - 49, which thirty second stage follows the thirty first stage in operation of the cartridge shown in Figs. 50AE/1 and 50AE/2.

As seen in Figs. 50AF/1 and 50AF/2, valve 465 is opened thereby allowing the pumping of air from pump 168 to backend mixing chamber 230 and hence the application of pressure to chamber 230, which chamber 230 contains reconstituted Discriminator III. Air is pumped from pump 168 into chamber 230 along an air flow path comprising channel 894, channel 927, open valve 465 and channel 870. Air enters chamber 230 via through hole 759. The direction of passage of air along the air flow path is indicated by an air flow arrow 4300.

As a result of the pressure created by the pumping of air thereinto, the liquid reconstituted Discriminator III held in chamber 230 is driven out of chamber 230. The liquid travels along a liquid flow path comprising channel 978, open valve 462, channel 868, channel 865, open valve 459 and channel 976 into carbon array assembly 120. The direction of liquid flow along the liquid flow path into carbon array assembly 120 is indicated by first, second and third liquid flow arrows 4301, 4302 and 4303. The liquid enters carbon array assembly 120 via aperture 493 and travels along first snake-shaped cut out 1312 in a direction indicated by a fourth liquid flow arrow 4304, thereafter through channel 903 and thereafter along second snake-shaped cut out 1312 in a direction indicated by a fifth liquid flow arrow 4305. Excess Discriminator III not having bound to carbon array assembly 120 leaves carbon array assembly 120 via aperture 490 leading to channel 874 and enters waste chamber 252 via through hole 790, in a direction indicated by a sixth liquid flow arrow 4306.

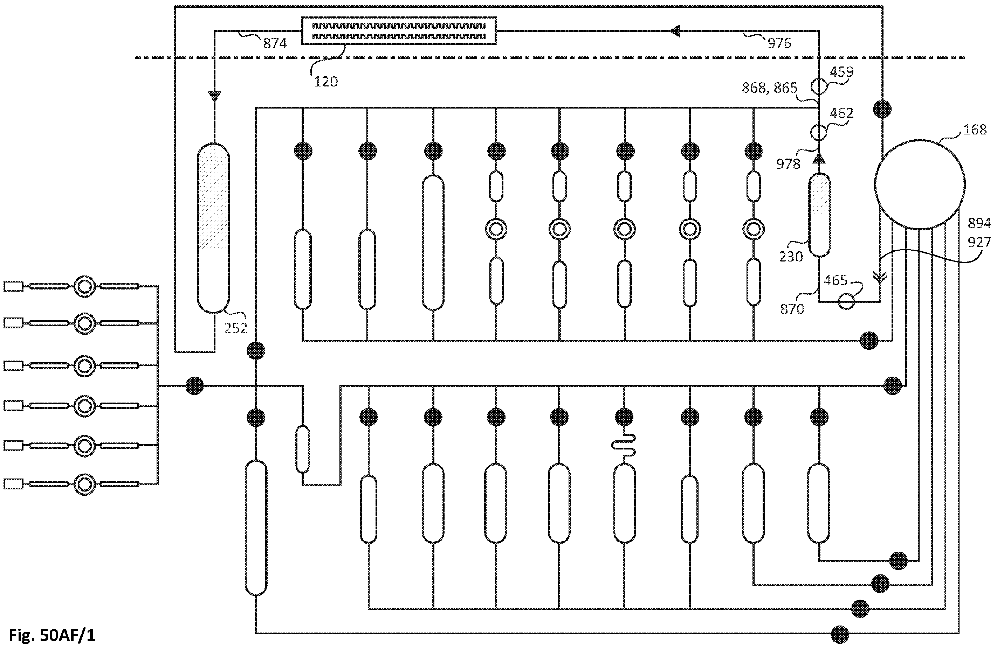

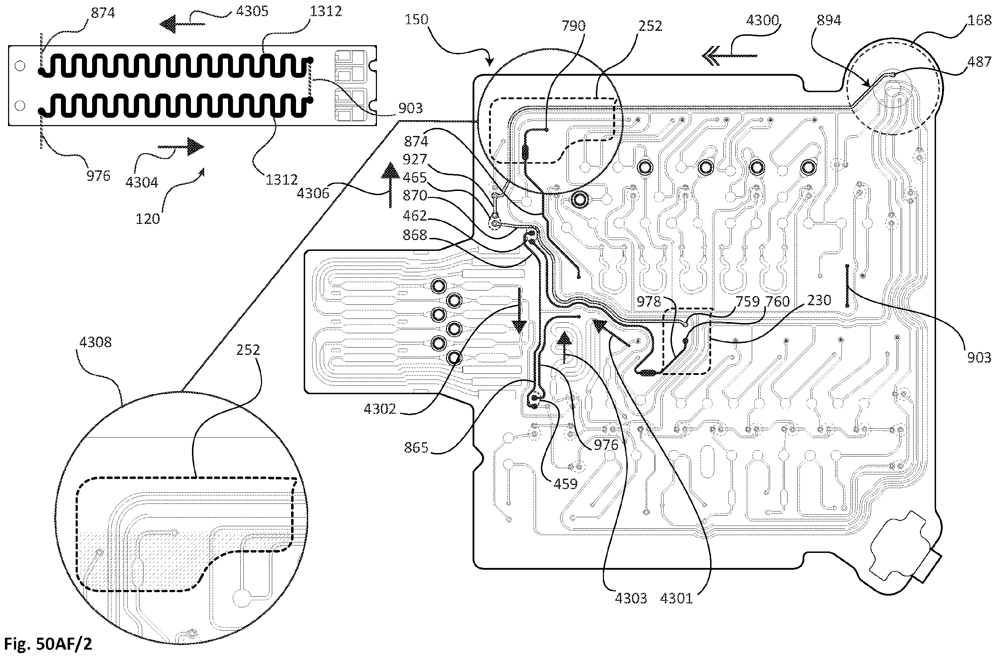

The content of waste chamber 252 following the pumping of air and liquid as described hereinabove, is shown at an enlargement 4308 in Fig. 50AF/2. It is appreciated that the representation of the content of chamber 252 in enlargement 4289 is highly schematic and does not distinguish between the various waste liquids, namely Raffinose washing liquid, diluted amplicons and reconstituted Discriminators I, II and III, comprising the content.

Reference is now made to Figs. 50AG/1 and 50AG/2, which are simplified respective schematic and flow illustrations of a thirty third stage in the operation of the cartridge 110 of Figs. 2A/1 - 10D by the instrument 100 of Figs. 11A - 49, which thirty third stage follows the thirty second stage in operation of the cartridge shown in Figs. 50AD/1 and 50AD/2.

As seen in Figs. 50AG/1 and 50AG/2, valve 486 is opened thereby allowing the pumping of air from pump 168 to Discriminator IV Buffer containing chamber 248 and hence the application of pressure to chamber 248. Air is pumped from pump 168 into chamber 248 along an air flow path comprising channel 898, open valve 486 and pathway 1014. Air enters chamber 248 via through hole 796. The direction of passage of air along the air flow path is indicated by an air flow arrow 4310.

As a result of the pressure created by the pumping of air thereinto, the Discriminator IV Buffer liquid held in chamber 248 is driven out of chamber 248. Discriminator IV Buffer travels along a liquid flow path comprising pathway 1015, Discriminator IV reagent plug 376, reservoir 592, open valve 474, channel 950, channel 904, channel 873, channel 868, open valve 462 and channel 978 into backend mixing chamber 230. Discriminator IV Buffer enters backend mixing chamber 230 via through hole 760. The direction of passage of liquid along the liquid flow path is indicated by sequential first and second liquid flow arrows 4312 and 4313.

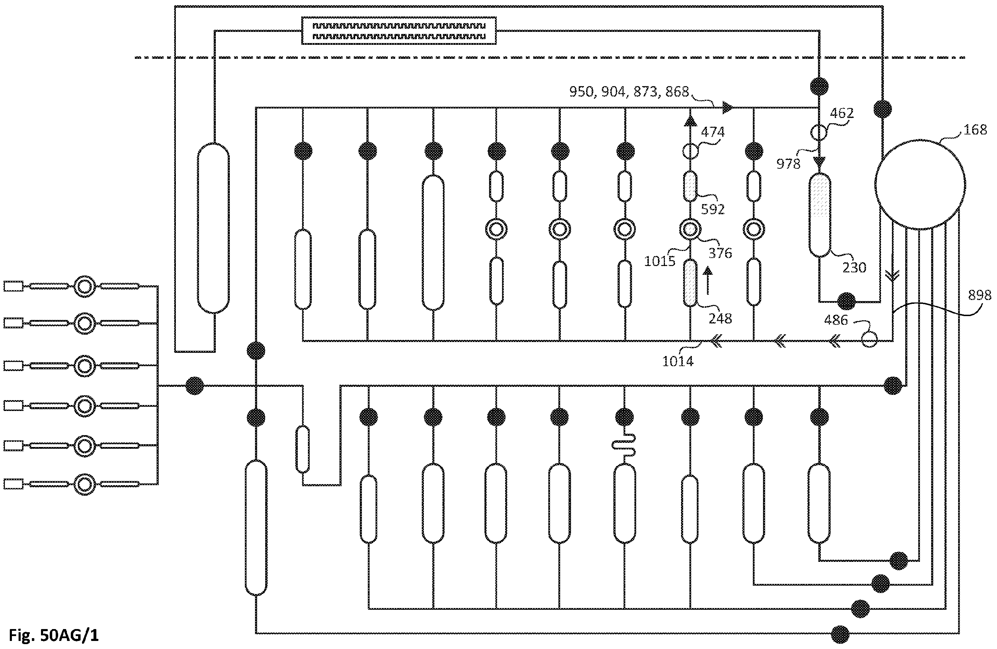

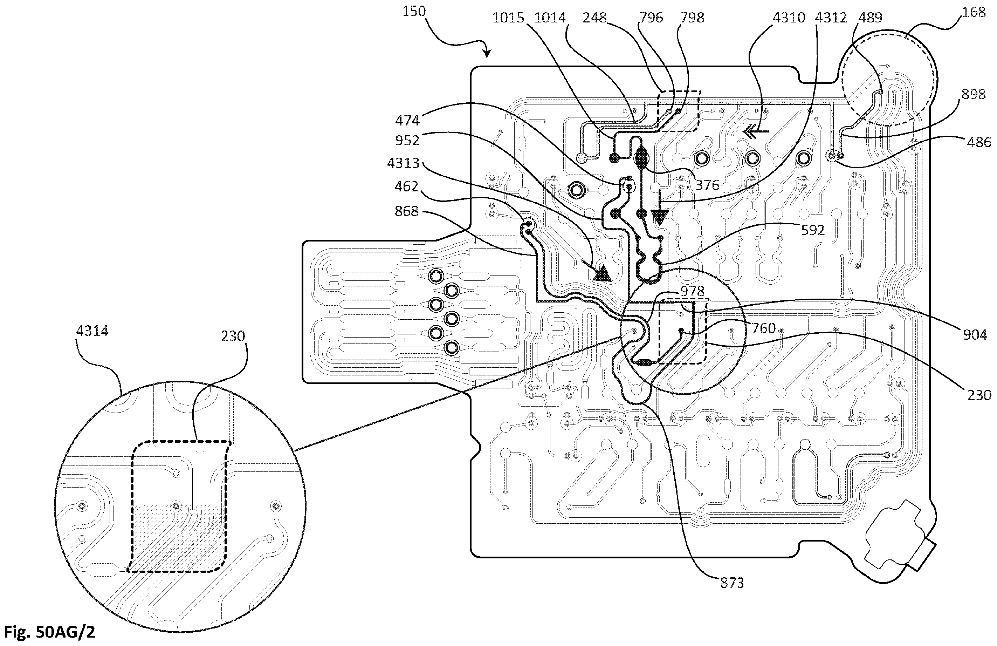

The content of backend mixing chamber 230 following the pumping of air and liquid as described hereinabove, is shown schematically at an enlargement 4314 in Fig. 50AG/2. It is appreciated that the representation of the content of chamber 230 in enlargement 4314 is highly schematic and does not distinguish between the various components, namely dried discriminator IV and Discriminator IV buffer, comprising the content. It is further appreciated that the thirty third stage of operation of cartridge 110 illustrated in Figs. 50AG/1 and 50AG/2 may be followed by a mixing step, in order to reconstitute the dried Discriminator IV with the Discriminator IV buffer.

Reference is now made to Figs. 50AH/1 and 50AH/2, which are simplified respective schematic and flow illustrations of a thirty fourth stage in the operation of the cartridge 110 of Figs. 2A/1 - 10D by the instrument 100 of Figs. 11A - 49, which thirty fourth stage follows the thirty third stage in operation of the cartridge shown in Figs. 50AG/1 and 50AG/2.

As seen in Figs. 50AH/1 and 50AH/2, valve 465 is opened thereby allowing the pumping of air from pump 168 to backend mixing chamber 230 and hence the application of pressure to chamber 230, which chamber 230 contains reconstituted Discriminator IV. Air is pumped from pump 168 into chamber 230 along an air flow path comprising channel 894, channel 927, open valve 465 and channel 870. Air enters chamber 230 via through hole 759. The direction of passage of air along the air flow path is indicated by an air flow arrow 4320.

As a result of the pressure created by the pumping of air thereinto, the liquid reconstituted Discriminator IV held in chamber 230 is driven out of chamber 230. The liquid travels along a liquid flow path comprising channel 978, open valve 462, channel 868, channel 865, open valve 459 and channel 976 into carbon array assembly 120. The direction of liquid flow along the liquid flow path into carbon array assembly 120 is indicated by first, second and third liquid flow arrow 4321, 4322, 4323. The liquid enters carbon array assembly 120 via aperture 493 and travels along first snake-shaped cut out 1312 in a direction indicated by a fourth liquid flow arrow 4324, thereafter through channel 903 and thereafter along second snake-shaped cut out 1312 in a direction indicated by a fifth liquid flow arrow 4325. Excess Discriminator III not having bound to carbon array assembly 120 leaves carbon array assembly 120 via aperture 490 leading to channel 874 and enters waste chamber 252 via through hole 790, in a direction indicated by a sixth liquid flow arrow 4326.

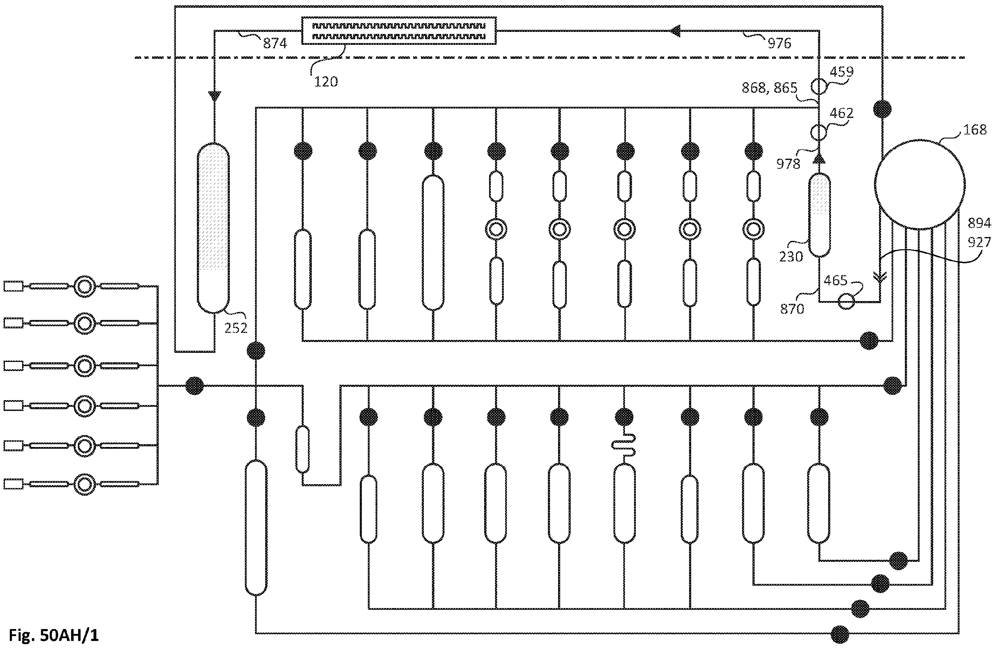

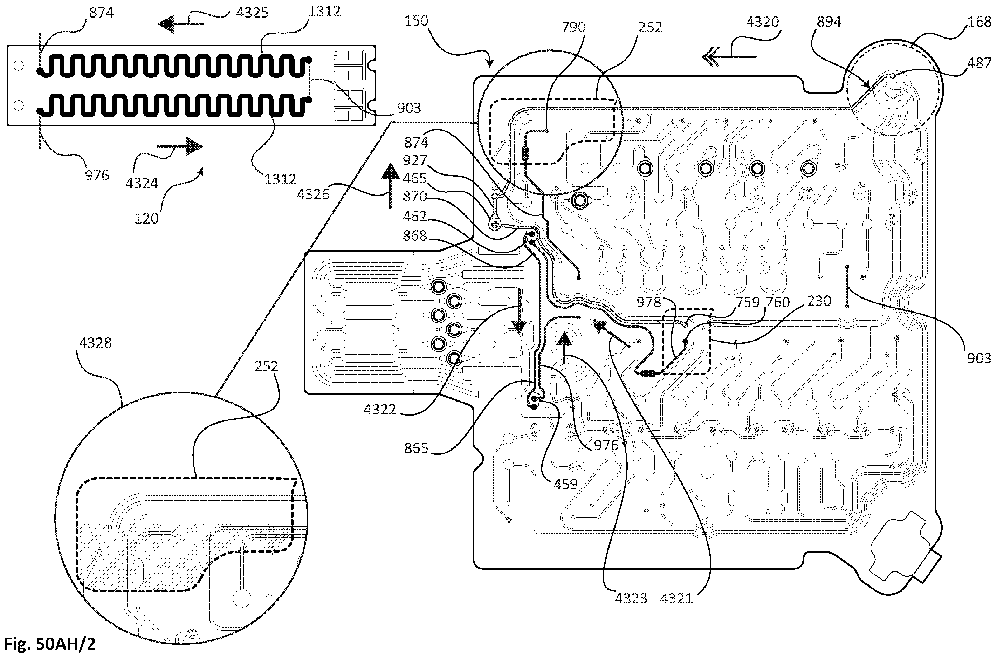

The content of waste chamber 252 following the pumping of air and liquid as described hereinabove, is shown at an enlargement 4328 in Fig. 50AH/2. It is appreciated that the representation of the content of chamber 252 in enlargement 4328 is highly schematic and does not distinguish between the various waste liquids, namely Raffinose washing liquid, diluted amplicons and reconstituted Discriminators I, II, III and IV, comprising the content.

Reference is now made to Figs. 50AI/1 and 50AI/2, which are simplified respective schematic and flow illustrations of a thirty fifth stage in the operation of the cartridge 110 of Figs. 2A/1 - 10D by the instrument 100 of Figs. 11A - 49, which thirty fifth stage follows the thirty fourth stage in operation of the cartridge shown in Figs. 50AH/1 and 50AH/2.

As seen in Figs. 50AI/1 and 50AI/2, valve 486 is opened thereby allowing the pumping of air from pump 168 to reporter buffer containing chamber 250 and hence the application of pressure to chamber 250. Air is pumped from pump 168 into chamber 250 along an air flow path comprising channel 898, open valve 486 and pathway 1012. Air enters chamber 250 via through hole 792. The direction of passage of air along the air flow path is indicated by an air flow arrow 4330.

As a result of the pressure created by the pumping of air thereinto, the reporter buffer liquid held in chamber 250 is driven out of chamber 250. The reporter buffer travels along a liquid flow path comprising pathway 1013, reporter reagent plug 378, reservoir 594, open valve 471, channel 954, channel 904, channel 873, channel 868, open valve 462 and channel 978 into backend mixing chamber 230. The reporter buffer enters backend mixing chamber 230 via through hole 760. The direction of passage of liquid along the liquid flow path is indicated by sequential first and second liquid flow arrow 4332 and 4333.

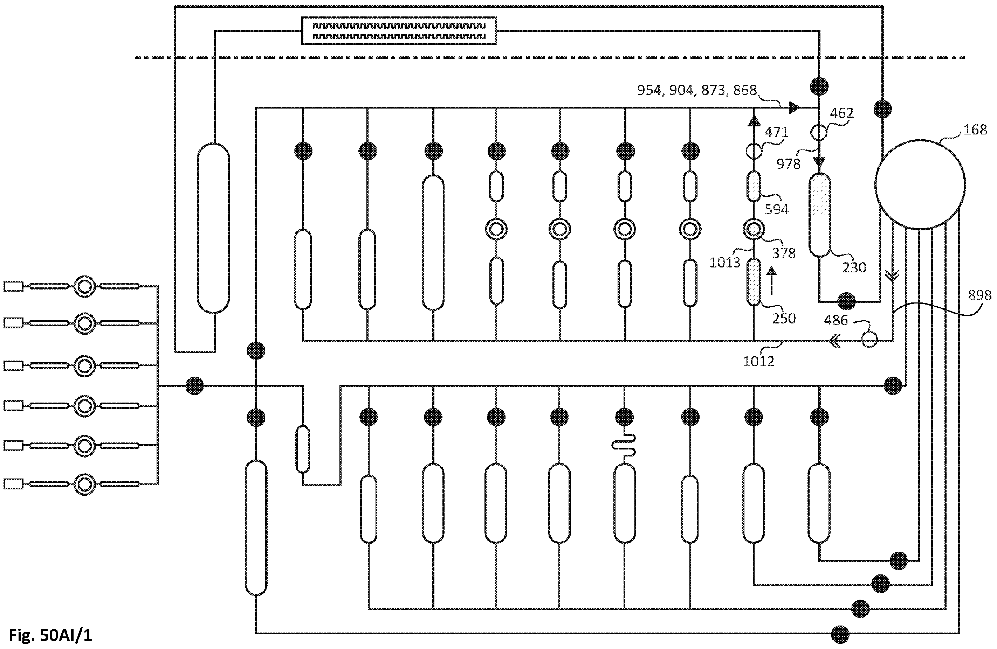

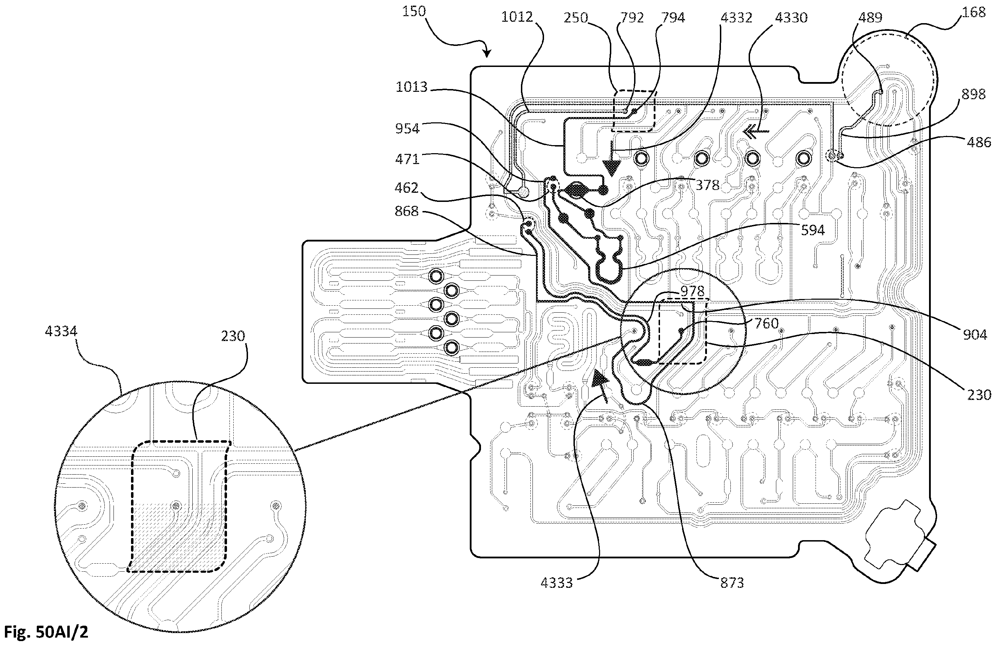

The content of backend mixing chamber 230 following the pumping of air and liquid as described hereinabove, is shown schematically at an enlargement 4334 in Fig. 50AI/2. It is appreciated that the representation of the content of chamber 230 in enlargement 4334 is highly schematic and does not distinguish between the various components, namely dried reporter and reporter buffer, comprising the content. It is further appreciated that the thirty fifth stage of operation of cartridge 110 illustrated in Figs. 50AI/1 and 50AI/2 may be followed by a mixing step, in order to reconstitute the dried reporter with the reporter buffer.

Reference is now made to Figs. 50AJ/1 and 50AJ/2, which are simplified respective schematic and flow illustrations of a thirty sixth stage in the operation of the cartridge 110 of Figs. 2A/1 - 10D by the instrument 100 of Figs. 11A - 49, which thirty sixth stage follows the thirty fifth stage in operation of the cartridge shown in Figs. 50AI/1 and 50AI/2.

As seen in Figs. 50AJ/1 and 50AJ/2, valve 465 is opened thereby allowing the pumping of air from pump 168 to backend mixing chamber 230 and hence the application of pressure to chamber 230, which chamber 230 contains reconstituted reporter. Air is pumped from pump 168 into chamber 230 along an air flow path comprising channel 894, channel 927, open valve 465 and channel 870. Air enters chamber 230 via through hole 759. The direction of passage of air along the air flow path is indicated by an air flow arrow 4340.

As a result of the pressure created by the pumping of air thereinto, the liquid reconstituted reporter held in chamber 230 is driven out of chamber 230. The liquid travels along a liquid flow path comprising channel 978, open valve 462, channel 868, channel 865, open valve 459 and channel 976 into carbon array assembly 120. The direction of liquid flow along the liquid flow path into carbon array assembly 120 is indicated by first, second and third liquid flow arrow 4341, 4342, 4343. The liquid enters carbon array assembly 120 via aperture 493 and travels along first snake-shaped cut out 1312 in a direction indicated by a fourth liquid flow arrow 4344, thereafter through channel 903 and thereafter along second snake-shaped cut out 1312 in a direction indicated by a fifth liquid flow arrow 4345. Excess reporter not having bound to carbon array assembly 120 leaves carbon array assembly 120 via aperture 490 leading to channel 874 and enters waste chamber 252 via through hole 790, in a direction indicated by a sixth liquid flow arrow 4346.

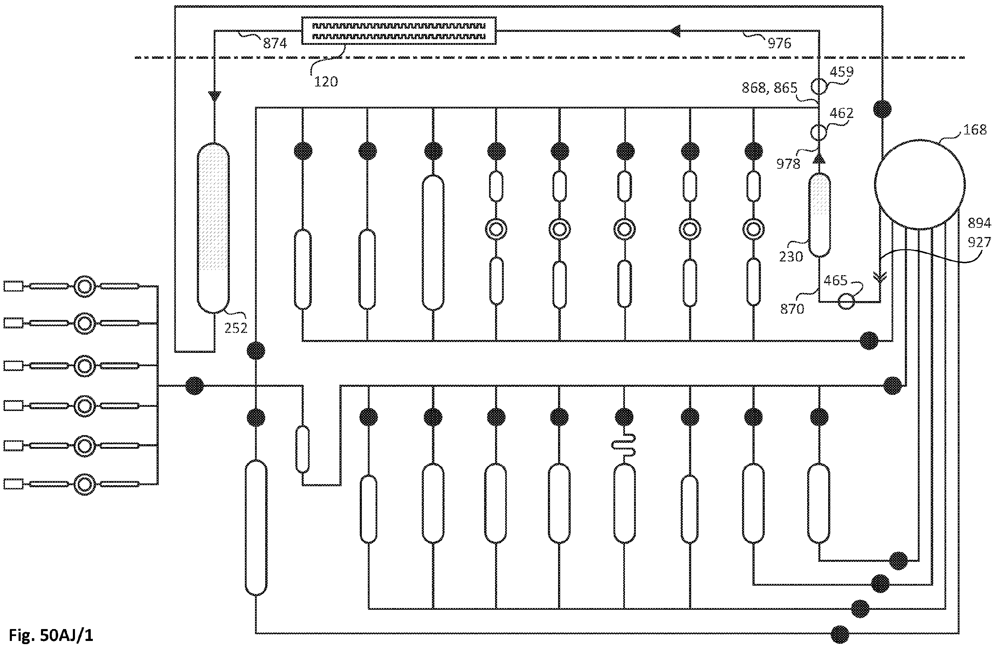

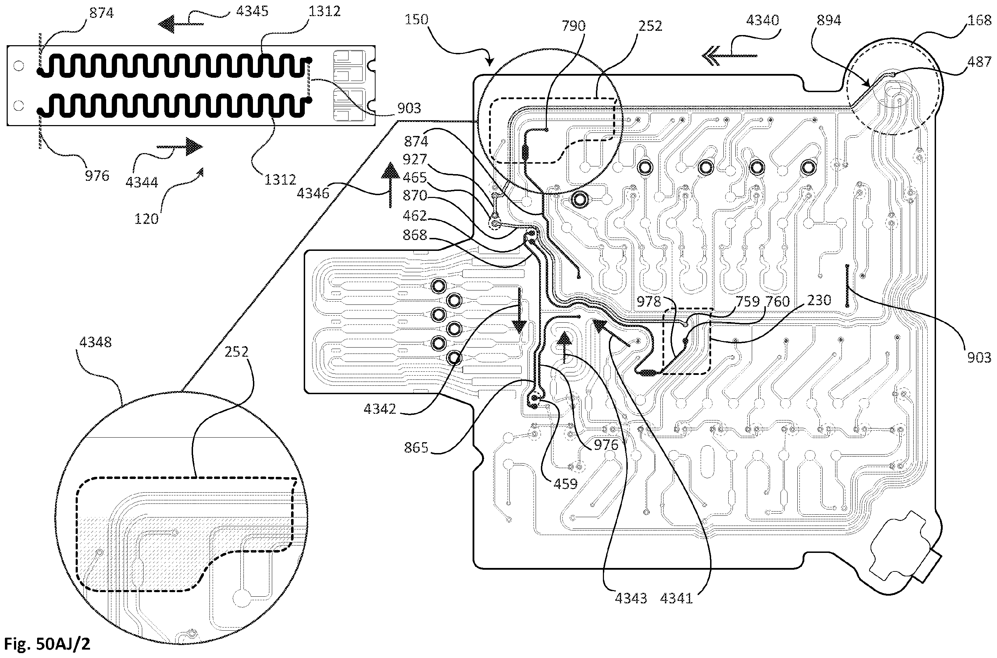

The content of waste chamber 252 following the pumping of air and liquid as described hereinabove, is shown at an enlargement 4348 in Fig. 50AJ/2. It is appreciated that the representation of the content of chamber 252 in enlargement 4348 is highly schematic and does not distinguish between the various waste liquids, namely Raffinose washing liquid, diluted amplicons, reconstituted Discriminators I, II, III and IV and reconstituted reporter, comprising the content.

Reference is now made to Figs. S0AK/1 and 50AK/2, which are simplified respective schematic and flow illustrations of a thirty seventh stage in the operation of the cartridge 110 of Figs. 2A/1 - 10D by the instrument 100 of Figs. 11A - 49, which thirty seventh stage follows the thirty sixth stage in operation of the cartridge shown in Figs. 50AJ/1 and 50AJ/2.

As seen in Figs. 50AK/1 and 50AK/2, valve 486 is opened thereby allowing the pumping of air from pump 168 to sensor wash containing chamber 234 and hence the application of pressure to chamber 234. Air is pumped from pump 168 into chamber 234 along an air flow path comprising channel 898, open valve 486 and pathway 1024. Air enters chamber 234 via through hole 740. The direction of passage of air along the air flow path is indicated by an air flow arrow 4350.

As a result of the pressure created by the pumping of air thereinto, the sensor wash liquid held in chamber 234 is driven out of chamber 234. The sensor wash travels along a liquid flow path comprising pathway 1025, open valve 411, channel 904, channel 873, channel 865, open valve 459 and channel 976 into carbon array assembly 120. The direction of liquid flow along the liquid flow path into carbon array assembly 120 is indicated by first and second liquid flow arrows 4352 and 4353. The liquid enters carbon array assembly 120 via aperture 493 and travels along first snake-shaped cut out 1312 in a direction indicated by a third liquid flow arrow 4354, thereafter through channel 903 and thereafter along second snake-shaped cut out 1312 in a direction indicated by a fourth liquid flow arrow 4356. The sensor wash leaves carbon array assembly 120 via aperture 490 leading to channel 874 and enters waste chamber 252 via through hole 790, in a direction indicated by a fourth liquid flow arrow 4357.

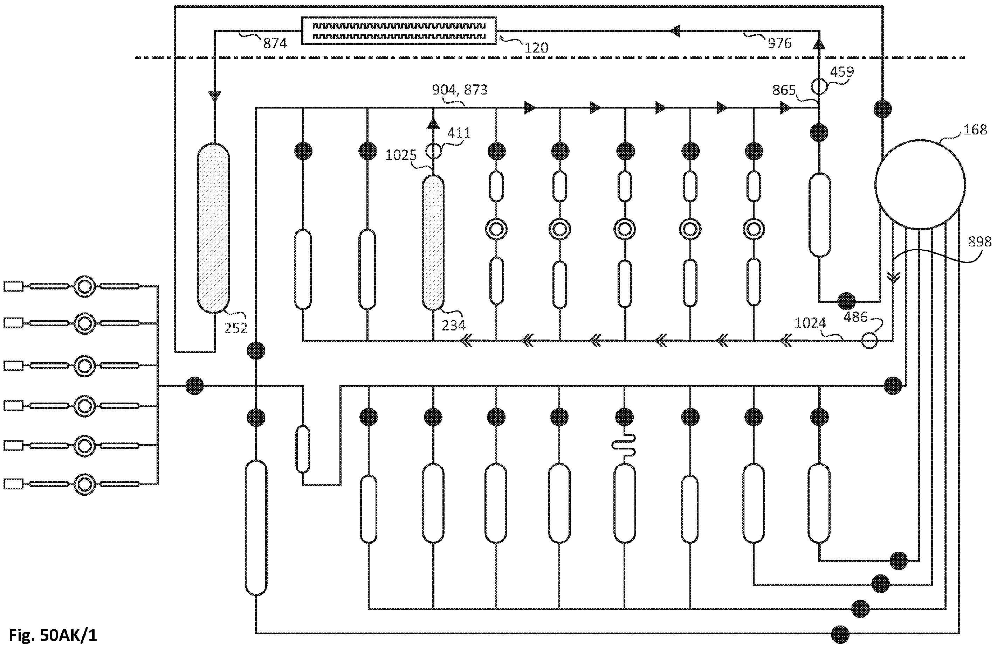

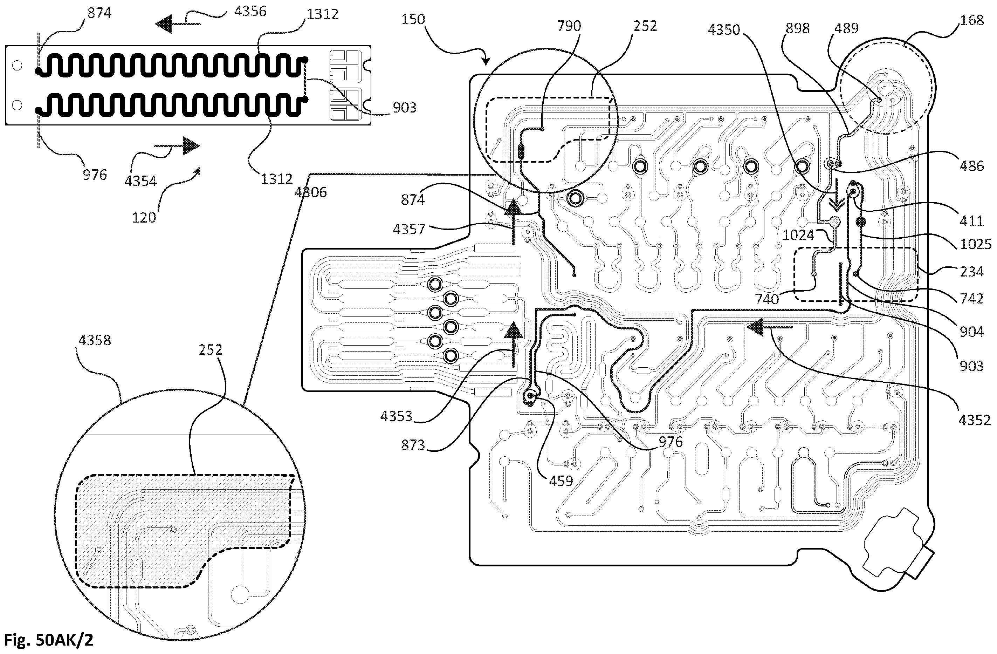

The content of waste chamber 252 following the pumping of air and liquid as described hereinabove, is shown schematically at an enlargement 4358 in Fig. 50AK/2. It is appreciated that the representation of the content of chamber 252 in enlargement 4358 is highly schematic and does not distinguish between the various components, namely Raffinose washing liquid, diluted amplicons, reconstituted Discriminators I, II, III and IV, reconstituted reporter and sensor wash, comprising the content.

It is appreciated that following the thirty seventh stage in the operation of cartridge 110, carbon array assembly 120 is preferably imaged by imaging assembly 1580 (Fig. 11B) of instrument 100.

Although most of the foregoing description references PCR amplification, it is appreciated that other types of amplification, including rolling-circle amplification (RCA), may be advantageously employed using the cartridge and the instrument described hereinabove with relatively minor modifications, which are within the ability of a person skilled in the art. The following description relates to apparatus and techniques for RCA application, which may be employed in various arrangements of the present disclosure.

Reference is now made to Figs. 51A and 51B, which are simplified pictorial assembled and exploded view illustrations of an electrophoretic array assembly 5100 constructed and operative in accordance with a preferred arrangement of the disclosure, which defines a multiplicity of immobilized, mutually spaced and mutually electrically separated target molecule-specific microgel regions during rolling-circle amplification (RCA) operation, and to Figs. 52A and 52B, which are simplified pictorial assembled and exploded view illustrations of the electrophoretic array assembly of Figs. 51A and 51B in which the multiplicity of immobilized, mutually spaced and mutually electrically separated microgel regions are shown in a dehydrated storage operative orientation. It is appreciated that electrophoretic array assembly 5100 is particularly suitable for use in a method described hereinbelow with reference to Figs. 57A - 57J.

As seen in Figs. 51A and 51B, the electrophoretic array assembly 5100 comprises an enclosure defined by a substrate 5110, a peripheral wall structure 5120 and a window 5130. Access to the interior of the enclosure is preferably provided by a solution ingress aperture 5140 and a solution egress aperture 5150 formed in substrate 5110.

An electrophoretic array 5160 is formed onto substrate 5110, as will be described hereinbelow in greater detail with reference to Figs. 53A - 53I. A multiplicity of target molecule-specific microgel deposits 5170, preferably having bound thereto different RCA circular probes, forward primers and reverse primers, here indicated generally by reference numeral 5175 in a symbolic manner, are immobilized onto discrete working electrode locations 5180, defined by the electrophoretic array 5160. In Figs. 51A and 51B, the target molecule-specific microgel deposits 5170 are shown in an operative orientation suitable for rolling circle amplification.

In Figs. 52A and 52B, the target molecule-specific microgel deposits are shown in a dehydrated state suitable for storage and are designated by reference numerals 5190. It is appreciated that the target molecule-specific microgel deposits 5190 of Figs. 52A and 52B, when hydrated, by supply of a suitable solution, preferably a nucleic acid target molecule containing solution, to the interior of the electrophoretic array assembly 5100, preferably assume the operative orientation shown in Figs. 51A & 51B, where they are designated by reference numerals 5170.

Preferred dimensions of the electrophoretic array assembly 5100 and various components thereof, assuming, for ease of calculation, that each microgel deposit 5170 exposed to solution assumes a generally hemispherical shape, are as follows:
Solution volume: approximately 100 mm³
Interior height between substrate 5110 and window 5130: 0.8 mm - 2.0 mm
Height of target molecule-specific microgel deposits 5170 above substrate 5110 in the operative orientation of Figs. 51A & 51B: 0.5 mm - 1.25 mm
Height of target molecule-specific microgel deposits 5190 above substrate 5110 in the operative orientation of Figs. 52A & 52B: 0.1 mm - 0.3 mm
. Surface area of each target molecule-specific microgel deposit 5170 above substrate 5110 in the operative orientation of Figs. 51A and 51B: .0016-.009 mm²
Ratio of surface area of each target molecule-specific microgel deposit 5170 exposed to solution to solution volume: 0.000016 - 0.00009 mm² of exposed surface area of microgel deposit per mm³ of solution.

It is appreciated that the actual surface area and the actual ratio of surface area to solution volume are greater than or equal to the surface area and ratio calculated using the simplifying assumption of a hemispherical shape.

The structure and construction of the electrophoretic array assembly 5100 will now be described with additional reference to Figs. 53A - 53I.

Turning initially to Fig. 53A, it is seen that substrate 5110 is typically a polyester sheet, such as polyethylene terephthalate, preferably of thickness 0.1 mm. Substrate 5110 may be provided with solution ingress aperture 5140 and solution egress aperture 5150 at this stage, preferably by laser cutting. Alternatively, solution ingress aperture 5140 and solution egress aperture 5150 may be formed in substrate 5110 at a later stage.

Turning now to Fig. 53B, it is seen that substrate 5110 is formed with an initial patterned layer 5200 of a highly conductive material, preferably by screen printing of Henkel 479SS ink. The thickness of layer 5200 is preferably 0.03 mm. Layer 5200 provides uniform electrical current conduction to each of the microgel deposits in the electrophoretic array assembly 5100.

Fig. 53C shows subsequent forming of a carbon layer 5210 in registration with the initial patterned layer 5200, preferably by screen printing of DuPont 7102 and BQ 221. The thickness of layer 5210 is preferably 0.03 mm. Layer 5210 serves as the working electrode material, which is exposed to the solution as will be described hereinbelow. Layer 5210 also controls the voltage applied between an inner working electrode 5230 and an outer counter electrode 5240.

Mutually registered layers 5200 and 5210 together define outer counter electrode 5240 and inner working electrode 5230, which are connected to respective electrical contacts 5250 and 5260.

Referring now additionally to Fig. 53D, it is seen that a patterned dielectric layer 5270 is formed over layers 5200 and 5210 and over substrate 5100, preferably by screen printing of CMI-101-80 79SS ink, commercially available from Creative Materials, Inc., Ayer, MA. Dielectric layer 5270 preferably has a thickness of 0.04mm.

As seen in Fig. 53D, dielectric layer 5270 is preferably formed with apertures 5272 and 5274, which preferably correspond in size and location to solution ingress aperture 5140 and solution egress aperture 5150. Dielectric layer 5270 is also preferably provided with elongate apertures 5276 and 5278, which overlie parts of counter electrode 5240. The length of each of elongate apertures 5276 and 5278 are preferably 100 mm. Additionally, dielectric layer 5270 is formed with a multiplicity of apertures 5280, here shown as an array of eight apertures 5280, which overlie working electrode 5230 and define therewith discrete working electrode locations 5180 (Figs. 51B and 52B). Preferably, apertures 5280 are all identical circular apertures having a diameter of 0.2 mm - 0.5mm and a minimum spacing therebetween of 1 mm.

Turning now to Fig. 53E, it is seen that an array of microgel deposits 5300 is formed, as by robotic spotting over working electrode locations 5180. Microgel deposits 5300 preferably have a generally hemispherical shape when hydrated and a diameter in the plane of the dielectric layer 5270 of 0.70 mm so that they are mutually physically separated from each other. The microgel deposits 5300 preferably have a height of between 0.5 mm and 1.2 mm and an exposed surface area of 0.0016 -0.009 square millimeters. The microgel deposits 5300 are preferably formed of (bis)acrylamide hydrogel containing streptavidin.

Turning now to Fig. 53F, it is seen that the microgel deposits 5300 are preferably illuminated by UV illumination to polymerize components of the microgel deposits 5300.Histidine is added to the microgel deposits 5300 and the microgel deposits 5300 are then washed to remove excess histidine.

Following polymerization, the microgel deposits 5300 are dried as by air drying, producing dried microgel deposits 5310, as seen in Fig 53G,.

Turning to Fig. 53H, it is seen that different nucleic acid target molecule specific RCA circular probes 5320, and, preferably, also forward primers 5322 and reverse primers 5324 are bound to corresponding different ones of the dried microgel deposits 5310, as by robotic spotting, thereby producing different target molecule-specific microgel deposits 5190 (Figs. 52A & 52B). It is appreciated that throughout the drawings, nucleic acid target molecule specific RCA circular probes 5320, forward primers 5322 and reverse primers 5324 are shown symbolically and not to scale.

It is appreciated that although, in the arrangement shown in Figs. 53H-53I and in Figs. 51A-52B, nucleic acid target molecule specific RCA circular probes 5320, forward primers 5322 and reverse primers 5324 are all shown as being bound to microgel deposits 5310, in alternative arrangements one or more of nucleic acid target molecule specific RCA circular probes 5320, forward primers 5322 and reverse primers 5324 may not be bound to microgel deposits 5310 and may be provided in a solution together with the nucleic acid target molecules. In the arrangement shown in Figs. 53H - 53I and in the alternative arrangements, the nucleic acid target molecule specific RCA circular probes 5320, forward primers 5322 and reverse primers 5324 are located in the immobilized, mutually spaced and mutually electrically separated target molecule-specific microgel regions, as described hereinbelow with reference to Figs. 55A - 57J.

As seen in Fig. 53I, following suitable washing of the target molecule-specific microgel dried microgel deposits 5190 and the addition of raffinose as a preservative the peripheral wall structure 5120 and the window 5130 are assembled onto the substrate 5110, thereby completing manufacture of the electrophoretic array assembly 5100 in a shelf storable state, as described above with reference to Figs. 52A & 52B. The electrophoretic array assembly 5100 is ready for use in accordance with a preferred arrangement of the disclosure, which provides a method for rapid detection of the presence of at least one nucleic acid target molecule, from among a multiplicity of pre-selected nucleic acid target molecules, in a solution, including the steps of:
introducing the solution to at least a multiplicity of immobilized, mutually spaced and mutually electrically separated microgel regions on an electrophoretic array, each of the multiplicity of immobilized, mutually spaced and mutually electrically separated microgel regions containing a microgel deposit containing materials suitable for binding of a different one of the multiplicity of pre-selected nucleic acid target molecules and performing rolling circle amplification;
performing rolling circle amplification at least generally simultaneously at each of the immobilized, mutually spaced and mutually electrically separated microgel regions, while applying electric fields thereto during various stages of the rolling circle amplification; and
detecting the presence of at least one of the multiplicity of pre-selected nucleic acid target molecules at at least one corresponding one of the immobilized, mutually spaced and mutually electrically separated microgel regions,
wherein the detecting occurs within a short time period of the introducing, the short time period preferably being less than 30 minutes, more preferably less than 25 minutes and even more preferably less than 20 minutes.

In the description which follows, four variations of carrying out the above method are described in detail with reference to Figs. 54A - 54J, Figs. 55A - 55J, Figs. 56A - 56J and Figs. 57A - 57J, respectively. The electrophoretic array assembly 5100 described hereinabove is particularly suitable for use in carrying out the method of Figs. 57A - 57J.

All of these methods employ rolling circle amplification. Rolling circle amplification is a known technique and is described, inter alia, in the following publications:
U.S. Patent No. 5,854,033; Lizardi et al., Nature Genetics 19(3):225-232 (1998),
Michael G. Mohsen and Eric T. Kool,The Discovery of Rolling Circle Amplification and Rolling Circle Transcription, Acc Chem Res. 2016, 49(11): 2540-2550
Peiying Feng, et al., Identification and Typing of Isolates of Cyphellophora and Relatives by Use of Amplified Fragment Length Polymorphism and Rolling Circle Amplification, Journal of Clinical Microbiology, 2013 Volume 51 Number 3, p. 931-937.
Signal Amplification by Rolling Circle Amplification on DNA Microarrays, G. Nallur et al., Nucleic Acids Research, 2001, Vol. 29,. Vol. 29, No. 123, e118
M. Monsur Ali et al., Rolling circle amplification: a versatile tool for chemical biology, materials science and medicine, Chemical Society Review, Chem. Soc. Rev., 2014, 43, 3324-3341.
Ali MM, Li F, Zhang Z, Zhang K, Kang D, Ankrum JA, Le XC, Zhao W. Rolling circle amplification: a versatile tool for chemical biology, materials science and medicine. Chem Soc Rev. 2014; 43:3324.
Kool, ET. Rolling circle synthesis of oligonucleotides and amplification of select randomized circular oligonucleotides. U.S. Patent No. 5,714,320, Feb 3. 1998.
Fire A, Xu S. Rolling replication of short DNA circles. Proc Natl Acad Sci U S A. 1995; 92:4641- 4645.
Nilsson M, Malmgren H, Samiotaki M, Kwiatkowski M, Chowdhary BP, Landegren U. Padlock Probes: Circularizing Oligonucleotides for Localized DNA Detection. Science. 1994; 265:2085- 2088.

The various methods which are described hereinbelow include features which are novel and unobvious in view of the prior art rolling circle amplification techniques.

Reference is now made to Figs. 54A - 54J, which illustrate principal stages in rapid detection of the presence of at least one nucleic acid target molecule, from among a multiplicity of pre-selected nucleic acid target molecules in accordance with one preferred arrangement of the present disclosure.

The method of Figs. 54A - 54J is preferably carried out using electrophoretic array assembly 5100 in a shelf storable state, as described above with reference to Figs. 52A & 52B, wherein only capture probes are bound to the immobilized, mutually spaced and mutually electrically separated microgel deposits 5190. It is appreciated that for simplicity, the substrate 5110, the peripheral wall structure 5120 and the window 5130 as well as the various layers constituting the electrophoretic array 5160, as described hereinabove with reference to Figs. 51A - 52B, are not specifically shown in Figs. 54A - 54J. Each of Figs. 54A - 54J is a simplified side view sectional illustration taken generally along lines 54 - 54 in Fig. 52A.

Fig. 54A shows the electrophoretic array assembly 5100 in its shelf storable state as shown in Fig. 52A, with symbolic, not to scale, indications of various different oligonucleotide nucleic acid specific capture probes 5400, such as those specific but not limited to identification of meningitis infectious disease pathogens, or other diseases, which are bound to corresponding different ones of the dried microgel deposits 5190. Fig. 54A indicates, in millimeter units, the interior dimensions of a preferred arrangement of the electrophoretic array assembly 5100, as well as the general dimensions of the immobilized dried target molecule-specific microgel deposits 5190, which are centered on but extend somewhat beyond working electrode locations 5180 (Figs. 51A -53I).

Fig. 54B illustrates the introduction, symbolized by an arrow 5401, of a solution 5402 containing one or more different types of nucleic acid target molecules 5403, so as to fill the interior of the electrophoretic array assembly 5100. Solution 5402 preferably includes, in addition to nucleic acid target molecules 5403, forward primers 5322 and nucleic acid target molecule specific RCA circular probes 5320.

Preparation of solution 5402 is not part of the present claimed invention and is carried out in accordance with conventional techniques, such as those described in "Nasir Ali, Rita de Cássia Pontello Rampazzo, Alexandre Dias Tavares Costa, and Marco Aurelio Krieger, Current Nucleic Acid Extraction Methods and Their Implications to Point-of-Care Diagnostics, BioMed Research International Volume 2017, Article ID 9306564, 13 pages". Solution 5402 preferably includes a low conductivity eluent liquid, typically introduced during preparation of the solution 5402, that promotes electronic addressing of nucleic acids and promotes activity of restriction enzymes in solution 5402. A preferred eluent liquid includes histidine and a restriction enzyme buffer. Fig. 54B shows the dried target molecule-specific microgel deposits 5190 in their dehydrated state. The introduction of solution 5402 into electrophoretic array assembly 5100, so that solution 5402 contacts dried target molecule-specific microgel deposits 5190, defines a time T0 and causes dried target molecule-specific microgel deposits 5190 to assume their hydrated state, designated by reference numeral 5170 (Figs. 51A & 51B).

Fig. 54C illustrates immobilized target molecule-specific microgel deposits 5170 in a hydrated state, as the result of the introduction of solution 5402, containing nucleic acid target molecules 5403, which fills the interior of the electrophoretic array assembly 5100. Fig. 54C illustrates that the hydrated target molecule-specific electrically separated microgel deposits 5170 have a typical maximum height of 1.25 mm. The hydration of the target molecule-specific microgel deposits 5170 to their state shown in Fig. 54C preferably takes about 10 seconds and is preferably completed at time T = T0 + 10 seconds.

Fig. 54D illustrates electrophoretic addressing of nucleic acids to the hydrated immobilized, mutually electrically separated microgel deposits 5170 in the presence of a DC electric field preferably of 10 - 300 Volts per centimeter applied between working electrode locations 5180 on working electrode 5230 and counter electrode 5240 (Figs. 51A & 51B). Portions of the electric field lines are indicated by reference numerals 5410 and the direction of the electric field is indicated by arrows 5412. It may be appreciated that the electric field lines define three-dimensional, immobilized, mutually spaced and mutually electrically separated target molecule-specific microgel regions 5420, each centered about a different target molecule-specific microgel deposit 5170.

It is appreciated that addressing, as well as the various steps described hereinbelow with reference to Figs. 54E - 54J, occur not only on the surface of microgel deposits 5170, but also within the volume of microgel deposits 5170.

As seen in Fig. 54D, the application of the DC electric field to the three-dimensional, immobilized, mutually spaced and mutually electrically separated target molecule-specific microgel regions 5420 causes rapid transport of nucleic acid target molecules 5403, nucleic acid target molecule specific RCA circular probes 5320 and forward primers 5322 to the target molecule-specific microgel deposits 5170, which in turn facilitates specific hybridization between the nucleic acid target molecules 5403 and the nucleic acid target molecule specific RCA circular probes 5320, specific hybridization between the forward primers 5322 and the nucleic acid target molecule specific RCA circular probes 5320 and capture of the nucleic acid target molecule specific RCA circular probes 5320 by target molecule-specific capture probes 5400, which capture probes 5400 are bound to hydrated target molecule-specific electrically separated microgel deposits 5170.

The duration of the stage illustrated in Fig. 54D is between 30 seconds and 120 seconds. The stage illustrated in Fig. 54D is preferably completed at a time T = T0 + [40 to 130] seconds.

Reference is now made to Fig. 54E, which illustrates a ligation stage that normally follows the addressing stage shown in Fig. 54D and preferably takes place in the presence of a DC electric field, preferably of 10 - 300 Volts per centimeter in the same direction as the electric field in the step of Fig. 54D. The ligation stage preferably occurs in the presence of a ligation enzyme 5428, for example, T-4 ligase, which is introduced into electrophoretic array assembly 5100 through a solution. The duration of the ligation stage is typically 120 to 240 seconds. The ligation stage is preferably completed at T=TO + [160 to 370] seconds.

Reference is now made to Fig. 54F, which illustrates an RCA polymerization stage that normally follows the ligation stage shown in Fig. 54E and preferably occurs in the presence of a DC electric field, preferably of 10 - 300 Volts per centimeter in the same direction as the electric field in the step of Fig. 54E.

The RCA polymerization stage preferably occurs in the presence of a *Bst* polymerase enzyme 5429, dNTPs (not shown) and a reverse primer 5324, which are introduced into electrophoretic array assembly 5100 through a solution, and forward primer 5322, which is bound to RCA circular probe 5320, which in turn bound to capture probe 5400, which in turn is bound to target-molecule specific microgel deposit 5170, preferably at a temperature of 65 degrees Celsius. A result of the RCA polymerization stage is generation of long RCA amplicons 5440. As seen in Fig. 54F, following binding of polymerase enzyme 5429 to nucleic acid target molecule specific RCA circular probes 5320, nucleic acid target molecules 5403 are displaced from nucleic acid target molecule specific RCA circular probes 5320, as indicated by arrows 5442. The duration of the RCA polymerization stage is typically 300 to 720 seconds. The RCA polymerization stage is preferably completed at T=TO + [460 to 1090] seconds.

Reference is now made to Fig. 54G, which illustrates an amplicon stretching stage that normally occurs during the RCA polymerization stage of Fig. 54F and preferably occurs in the presence of a DC electric field, preferably of 10 - 300 Volts per centimeter in a direction opposite to that of the electric field in the step of Fig. 54F as indicated by arrows 5444. In the illustrated arrangement, stretching of amplicon 5440 occurs in a direction indicated by an arrow 5448. The amplicon stretching stage preferably occurs in the presence of a *Bst* polymerase enzyme 5429, dNTPs (not shown) and reverse primer 5324 at a temperature of 65 degrees Celsius. The duration of the amplicon stretching stage is typically 5 to 15 seconds. The RCA polymerization stage is preferably completed at T=TO + [465 to 1105] seconds.

It is appreciated that the stages shown in Figs. 54F and 54G may be repeated intermittently multiple times, with multiple stages shown in Fig. 54F each being of a shorter duration than that indicated above and being separated by a stage shown in Fig. 54G.

Reference is now made to Fig. 54H, which illustrates an exponential RCA amplification stage that normally occurs during the RCA polymerization stage of Fig. 54F and the amplicon stretching stage of Fig. 54G and preferably occurs in the presence of a DC electric field, preferably of 10 - 300 Volts per centimeter, which is normally in an opposite direction from that of the electric field in the step of Fig. 54G, as indicated by arrow 5412, but preferably includes short periods in which the polarity of the electric field is reversed. In this stage, additional amplicons 5450 are generated using reverse primers 5324.

The exponential RCA amplification stage preferably occurs in the presence of a *Bst* polymerase enzyme 5429, dNTPs (not shown) and reverse primer 5324 at a temperature of 65 degrees Celsius. The duration of the exponential RCA amplification stage, which occurs during the RCA polymerization stage of Fig. 54F and the amplicon stretching stage of Fig. 54G, is typically 5 - 15 seconds. The RCA polymerization stage of Fig. 54F, the amplicon stretching stage of Fig. 54G and the exponential RCA amplification stage of Fig. 54H, preferably are completed at T=TO + [465 to 1105] seconds.

Reference is now made to Fig. 54I, which illustrates a post-RCA polymerization addressing stage that normally occurs following the completion of RCA polymerization stage of Fig. 54F, the amplicon stretching stage of Fig. 54G and the exponential RCA amplification stage of Fig. 54H, and preferably occurs in the presence of a DC electric field, preferably of 10 - 300 Volts per centimeter, which is normally in the same direction as that of the electric field in the step of Fig. 54H. The post-RCA polymerization addressing stage is particularly useful for concentrating amplicons 5440 that are in solution 5402 at a distance from target molecule-specific microgel deposits 5170 and recapturing them at the target molecule-specific microgel deposits 5170, as indicated by arrows 5460, and preferably gathering the amplicons 5440 at one location within each microgel region 5420. The duration of the post-RCA polymerization addressing stage is typically 10 - 30 seconds. The post-RCA polymerization addressing stage is preferably completed at T=TO + [475 to 1135] seconds.

Reference is now made to Fig. 54J, which illustrates a reporting stage that normally follows the post-RCA polymerization addressing stage. The reporting stage preferably occurs in the presence of fluorescence reporters 5470 complementary to amplicons 5440 and 5450, which is introduced to electrophoretic array assembly 5100 through a solution. The duration of the reporting stage is typically 10 - 30 seconds. The reporting stage is preferably completed at T=TO + [485 to 1165] seconds.

Upon completion of the reporting stage and a subsequent washing stage, not shown, the detection of the presence of at least one nucleic acid target molecule, from among a multiplicity of pre-selected nucleic acid target molecules, may be carried out by conventional fluorescence detection techniques. It is thus appreciated that detection of at least one nucleic acid target molecule is preferably completed within between 8 minutes and 20 minutes of the initial supply of solution 5402 to the interior of the electrophoretic array assembly 5100.

It is appreciated that if preparation of solution 5402 is completed within 4 - 5 minutes of acquisition of a sample, as by taking a blood sample from a patient, detection at least one nucleic acid target molecule may be completed within 12 - 25 minutes from sample acquisition.

Reference is now made to Figs. 55A - 55J, which illustrate principal stages in rapid detection of the presence of at least one nucleic acid target molecule, from among a multiplicity of pre-selected nucleic acid target molecules in accordance with another preferred arrangement of the present disclosure.

The method of Figs. 55A - 55J is preferably carried out using an electrophoretic array assembly 5500 in a shelf storable state, as described above with reference to Figs. 52A & 52B, wherein forward primers 5322 are bound to the immobilized and mutually spaced microgel deposits 5190. It is appreciated that for simplicity, the substrate 5110, the peripheral wall structure 5120 and the window 5130 as well as the various layers constituting the electrophoretic array 5160 are not specifically shown. Each of Figs. 55A - 55J is a simplified side view sectional illustration taken generally along lines 54 - 54 in Fig. 52A.

Fig. 55A shows an electrophoretic array assembly 5500 in its dried, dehydrated, operative orientation, similar to that shown in Fig. 52A, with symbolic, not to scale, indications of various different oligonucleotide forward primers 5322, which are bound to corresponding different ones of the dried microgel deposits 5190.

Fig. 55A indicates, in millimeter units, the interior dimensions of a preferred arrangement of the electrophoretic array assembly 5500, as well as the general dimensions of the immobilized dried target molecule-specific microgel deposits 5190.

It is appreciated that the method of Figs. 55A - 55J differs from the method of Figs. 54A - 54J in that instead of capture probes 5400 being bound to the immobilized dried target molecule specific microgel deposits 5190 in the method of Figs. 54A - 54J, forward primers 5322, which also serve as capture probes, are bound to the immobilized dried target molecule specific microgel deposits 5190 in the method of Figs. 55A - 55J.

Fig. 55B illustrates the introduction, symbolized by an arrow 5501, of a solution 5502 containing nucleic acid target molecules 5503, so as to fill the interior of the electrophoretic array assembly 5500. This solution preferably includes nucleic acid target molecule specific RCA circular probes 5320 in addition to nucleic acid target molecules 5503.

Preparation of solution 5502 is not part of the present claimed invention and is carried out in accordance with conventional techniques, such as those described in "Nasir Ali, Rita de Cássia Pontello Rampazzo, Alexandre Dias Tavares Costa, and Marco Aurelio Krieger, Current Nucleic Acid Extraction Methods and Their Implications to Point-of-Care Diagnostics, BioMed Research International Volume 2017, Article ID 9306564, 13 pages". Solution 5502 preferably includes a low conductivity eluent liquid, typically introduced during preparation of the solution, that promotes electronic addressing of nucleic acids and promotes activity of restriction enzymes in solution 5502. A preferred eluent liquid includes histidine and a restriction enzyme buffer. Fig. 55B shows the dried target molecule-specific microgel deposits 5190 in their dehydrated state. The introduction of solution 5502 into electrophoretic array assembly 5500, so that solution 5502 contacts dried target molecule-specific microgel deposits 5190, defines a time T0 and causes dried target molecule-specific microgel deposits 5190 (Figs. 52A & 52B) to be hydrated to their hydrated form, designated by reference numeral 5170 (Figs. 51A & 51B).

Fig. 55C illustrates immobilized target molecule-specific microgel deposits 5170 in a hydrated state, as the result of the introduction of solution 5502, containing nucleic acid target molecules 5503, which fills the interior of the electrophoretic array assembly 5500. Fig. 55C illustrates that the hydrated target molecule-specific electrically separated microgel deposits 5170 have a typical maximum height of 1.25 mm. The hydration of the target molecule-specific microgel deposits 5170 to their state shown in Fig. 55C preferably takes about 10 seconds and is preferably completed at time T = T0 + 10 seconds.

Fig. 55D illustrates electrophoretic addressing of nucleic acids to the hydrated immobilized, mutually electrically separated target molecule-specific microgel deposits 5170 in the presence of a DC electric field preferably of 10 - 300 Volts per centimeter. Portions of the electric field lines are indicated by reference numerals 5510 and the direction of the electric field is indicated by arrows 5512. It may be appreciated that the electric field lines define three-dimensional, immobilized, mutually spaced and mutually electrically separated microgel regions 5520, each centered about a different target molecule-specific microgel deposit 5170.

It is appreciated that addressing as well as the various steps described hereinbelow with reference to Figs. 55E - 55J occur not only on the surface of microgel deposits 5170 but also within the volume of the microgel deposits 5170.

As seen in Fig. 55D, the application of the DC electric field to the three-dimensional, immobilized, mutually spaced and mutually electrically separated target molecule-specific microgel regions 5520 causes rapid transport of nucleic acid target molecules 5503 and nucleic acid target molecule specific RCA circular probes 5320 to the target molecule-specific microgel deposits 5170, which in turn facilitates specific hybridization between the nucleic acid target molecules 5503 and the nucleic acid target molecule specific RCA circular probes 5320. Rapid transport of nucleic acid target molecule specific RCA circular probes 5320 to the target molecule-specific microgel deposits 5170 also facilitates specific hybridization between the forward primers 5322, which are bound to the target molecule-specific microgel deposits 5170, and the nucleic acid target molecule specific RCA circular probes 5320.

The duration of the stage illustrated in Fig. 55D is between 30 seconds and 120 seconds. The stage illustrated in Fig. 55D is preferably completed at a time T = T0 + [40 to 130] seconds.

Reference is now made to Fig. 55E, which illustrates a ligation stage that normally follows the addressing stage shown in Fig. 55D and preferably takes place in the presence of a DC electric field, preferably of 10 - 300 Volts per centimeter in the same direction as the electric field in the step of Fig. 55D. The ligation stage preferably occurs in the presence of a ligation enzyme 5528, for example, T-4 ligase, which is introduced into electrophoretic array assembly 5500 through a solution. The duration of the ligation stage is typically 120 to 240 seconds. The ligation stage is preferably completed at T=TO + [160 to 370] seconds.

Reference is now made to Fig. 55F, which illustrates an RCA polymerization stage that normally follows the ligation stage shown in Fig. 55E and preferably occurs in the presence of a DC electric field, preferably of 10 - 300 Volts per centimeter in the same direction as the electric field in the step of Fig. 55E. The RCA polymerization stage preferably occurs in the presence of a *Bst* polymerase enzyme 5529, dNTPs (not shown) and a reverse primer 5324, which are introduced into electrophoretic array assembly 5500 through solution, and forward primer 5322, which is bound to target-molecule specific microgel deposit 5170, preferably at a temperature of 65 degrees Celsius. A result of the RCA polymerization stage is generation of long RCA amplicons 5540 (Fig. 55G). As seen in Fig. 55F, following binding of polymerase enzyme 5529 to nucleic acid target molecule specific RCA circular probes 5320, nucleic acid target molecules 5503 are displaced from nucleic acid target molecule specific RCA circular probes 5320, as indicated by arrows 5542. The duration of the RCA polymerization stage is typically 300 to 720 seconds. The RCA polymerization stage is preferably completed at T = T0 + [560 to 1090] seconds.

Reference is now made to Fig. 55G, which illustrates an amplicon stretching stage that normally occurs during the RCA polymerization stage of Fig. 55F and preferably occurs in the presence of a DC electric field, preferably of 10 - 300 Volts per centimeter in a direction opposite to that of the electric field in the step of Fig. 55F as indicated by an arrow 5544. Stretching of amplicon 5540 occurs in a direction indicated by an arrow 5548. The amplicon stretching stage preferably occurs in the presence of a *Bst* polymerase enzyme 5529, dNTPs (not shown) and reverse primer 5324 at a temperature of 65 degrees Celsius. The duration of the amplicon stretching stage is typically 5 to 15 seconds. The RCA polymerization stage is preferably completed at T=TO + [565 to 1105] seconds.

It is appreciated that the stages shown in Figs. 55F and 55G may be repeated intermittently multiple times, with multiple stages shown in Fig. 55F each being of a shorter duration than that indicated above and being separated by a stage shown in Fig. 55G.

Reference is now made to Fig. 55H, which illustrates an exponential RCA amplification stage that normally occurs during the RCA polymerization stage of Fig. 55F and the amplicon stretching stage of Fig. 55G and preferably occurs in the presence of a DC electric field, preferably of 10 - 300 Volts per centimeter, which is normally in an opposite direction, as indicated by arrows 5512, from that of the electric field in the step of Fig. 55G but preferably includes short periods in which the polarity of the electric field is reversed. In this stage, additional amplicons 5550 are generated using reverse primers 5324.

The exponential RCA amplification stage preferably occurs in the presence of a *Bst* polymerase enzyme 5529, dNTPs (not shown) and reverse primer 5324 at a temperature of 65 degrees Celsius. The duration of the exponential RCA amplification stage, which occurs during the RCA polymerization stage of Fig. 55F and the amplicon stretching stage of Fig. 55G, is typically 5 - 15 seconds. The RCA polymerization stage of Fig. 55F, the amplicon stretching stage of Fig. 55G and the exponential RCA amplification stage of Fig. 55H, preferably are completed at T=TO + [465 to 1105] seconds.

Reference is now made to Fig. 55I, which illustrates a post-RCA polymerization addressing stage that normally occurs following the completion of RCA polymerization stage of Fig. 55F, the amplicon stretching stage of Fig. 55G and the exponential RCA amplification stage of Fig. 55H, and preferably occurs in the presence of a DC electric field, preferably of 10 - 300 Volts per centimeter, which is normally in the same direction as that of the electric field in the step of Fig. 55H. The post-RCA polymerization addressing stage is particularly useful for gathering amplicons 5540 that are in solution 5502 at a distance from target molecule-specific microgel deposits 5170 and recapturing them at the target molecule-specific microgel deposits 5170, as indicated by arrows5560, and preferably concentrating the amplicons 5540 at one location within each microgel region 5520. The duration of the post-RCA polymerization addressing stage is typically 10 - 30 seconds. The post-RCA polymerization addressing stage is preferably completed at T=TO + [475 to 1135] seconds.

Reference is now made to Fig. 55J, which illustrates a reporting stage that normally follows the post-RCA polymerization addressing stage. The reporting stage preferably occurs in the presence of a fluorescence reporter 5570 complementary to amplicons 5540 and 5550, which is introduced to electrophoretic array assembly 5500 through a solution. The duration of the reporting stage is typically 10 - 30 seconds. The reporting stage is preferably completed at T = T0 + [585 to 1165] seconds.

Upon completion of the reporting stage and a subsequent washing stage, not shown, the detection of the presence of at least one nucleic acid target molecule, from among a multiplicity of pre-selected nucleic acid target molecules, may be carried out by conventional fluorescence detection techniques. It is thus appreciated that detection of at least one nucleic acid target molecule is preferably completed within between 8 minutes and 20 minutes of the initial supply of solution 5502 to the interior of the electrophoretic array assembly 5100.

It is appreciated that if preparation of solution 5502 is completed within 4 - 5 minutes of acquisition of a sample as by taking a blood sample from a patient, detection at least one nucleic acid target molecule may be completed within 12 - 25 minutes from sample acquisition.

Reference is now made to Figs. 56A - 56J, which illustrate principal stages in rapid detection of the presence of at least one nucleic acid target molecule, from among a multiplicity of pre-selected nucleic acid target molecules in accordance with yet another preferred arrangement of the present disclosure.

The method of Figs. 56A - 56J is preferably carried out using an electrophoretic array assembly 5600 in a shelf storable state, as described above with reference to Figs. 52A & 52B, wherein forward primers 5322 and nucleic acid target molecule specific RCA circular probes 5320 are bound to the immobilized and mutually spaced microgel deposits 5190. It is appreciated that for simplicity, the substrate 5110, the peripheral wall structure 5120 and the window 5130 as well as the various layers constituting the electrophoretic array 5160 are not specifically shown. Each of Figs. 56A - 56J is a simplified side view sectional illustration taken generally along lines 54 - 54 in Fig. 52A.

Fig. 56A shows an electrophoretic array assembly 5600 in its dried, dehydrated, operative orientation, similar to that shown in Fig. 52A, with symbolic, not to scale, indications of various different oligonucleotide forward primers 5322 and nucleic acid target molecule specific RCA circular probes 5320, which are bound to corresponding different ones of the dried microgel deposits 5190.

Fig. 56A indicates, in millimeter units, the interior dimensions of a preferred arrangement of the electrophoretic array assembly 5600, as well as the general dimensions of the immobilized dried target molecule-specific microgel deposits 5190.

It is appreciated that the method of Figs. 56A - 56J differs from the method of Figs. 54A - 54J in that instead of capture probes 5400 being bound to the immobilized dried target molecule-specific microgel deposits 5190 in the method of Figs. 54A - 54J, nucleic acid target molecule specific RCA circular probes 5320 are specifically hybridized to forward primers 5322, which are bound to the immobilized dried target molecule-specific microgel deposits 5190 in the method of Figs. 56A - 56J.

Fig. 56B illustrates the introduction, symbolized by an arrow 5601, of a solution 5602 containing nucleic acid target molecules 5603, so as to fill the interior of the electrophoretic array assembly 5600.

Preparation of solution 5602 is not part of the present claimed invention and is carried out in accordance with conventional techniques, such as those described in"Nasir Ali, Rita de Cássia Pontello Rampazzo, Alexandre Dias Tavares Costa, and Marco Aurelio Krieger, Current Nucleic Acid Extraction Methods and Their Implications to Point-of-Care Diagnostics, BioMed Research International Volume 2017, Article ID 93065. Solution 5602 preferably includes a low conductivity eluent liquid, typically introduced during preparation of the solution, that promotes electronic addressing of nucleic acids and promotes activity of restriction enzymes in solution 5602. A preferred eluent liquid includes histidine and a restriction enzyme buffer. Fig. 56B shows the dried target molecule-specific microgel deposits 5190 in their dehydrated state. The introduction of solution 602 into electrophoretic array assembly 5100, so that solution 602 contacts dried target molecule-specific microgel deposits 5190, defines a time T0 and causes dried target molecule-specific microgel deposits 5190 to assume their hydrated state, designated by reference numeral 5170 (Figs. 51A & 51B).

Fig. 56C illustrates immobilized target molecule-specific microgel deposits 5170 in a hydrated state, as the result of the introduction of solution 5602, containing nucleic acid target molecules 5603, which fills the interior of the electrophoretic array assembly 5600. Fig. 56C illustrates that the hydrated target molecule-specific electrically separated microgel deposits 5170 have a typical maximum height of 1.25 mm. The hydration of the target molecule-specific microgel deposits 5190 to their state shown in Fig. 56C preferably takes about 10 seconds and is preferably completed at time T = T0 + 10 seconds.

Fig. 56D illustrates electrophoretic addressing of nucleic acids to the hydrated immobilized, mutually electrically separated target molecule-specific microgel deposits 5170 in the presence of a DC electric field preferably of 10 - 300 Volts per centimeter. Portions of the electric field lines are indicated by reference numerals 5610 and the direction of the electric field is indicated by arrows 5612. It may be appreciated that the electric field lines define three-dimensional, immobilized, mutually spaced and mutually electrically separated microgel regions 5620, each centered about a different target molecule-specific microgel deposit 5170.

It is appreciated that addressing as well as the various steps described hereinbelow with reference to Figs. 56E - 56J occur not only on the surface of microgel deposits 5170 but also within the volume of the microgel deposits 5170.

As seen in Fig. 56D, the application of the DC electric field to the three-dimensional, immobilized, mutually spaced and mutually electrically separated target molecule-specific microgel regions 5620 causes rapid transport of nucleic acid target molecules 5603 to the target molecule-specific microgel deposits 5170, which in turn facilitates specific hybridization between the nucleic acid target molecules 5603 and the nucleic acid target molecule specific RCA circular probes 5320, which RCA circular probes 5320 are bound to forward primers 5322, which forward primers 5322 are bound to hydrated target molecule specific electrically separated microgel deposits 5170.

The duration of the stage illustrated in Fig. 56D is between 30 seconds and 120 seconds. The stage illustrated in Fig. 56D is preferably completed at a time T = T0 +[40 to 130] seconds.

Reference is now made to Fig. 56E, which illustrates a ligation stage that normally follows the addressing stage shown in Fig. 56D and preferably takes place in the presence of a DC electric field, preferably of 10 - 300 Volts per centimeter in the same direction as the electric field in the step of Fig. 56D. The ligation stage preferably occurs in the presence of a ligation enzyme 5628, for example, T-4 ligase, which is introduced into electrophoretic array assembly 5600 through a solution. The duration of the ligation stage is typically 120 to 240 seconds. The ligation stage is preferably completed at T = T0 + [160 to 370] seconds.

Reference is now made to Fig. 56F, which illustrates an RCA polymerization stage that normally follows the ligation stage shown in Fig. 56E and preferably occurs in the presence of a DC electric field, preferably of 10 - 300 Volts per centimeter in the same direction as the electric field in the step of Fig. 56E. The RCA polymerization stage preferably occurs in the presence of a *Bst* polymerase enzyme 5629, dNTPs (not shown) and a reverse primer 5324, which are introduced into electrophoretic array assembly 5600 through solution, and forward primer 5322, which is bound to target-molecule specific microgel deposit 5170, preferably at a temperature of 65 degrees Celsius. A result of the RCA polymerization stage is generation of long RCA amplicons 5640 (Fig. 56G). As seen in Fig. 56F, following binding of polymerase enzyme 5629 to nucleic acid target molecule specific RCA circular probes 5320, nucleic acid target molecules 5603 are displaced from nucleic acid target molecule specific RCA circular probes 5320, as indicated by arrows 5642. The duration of the RCA polymerization stage is typically 300 to 720 seconds. The RCA polymerization stage is preferably completed at T = T0 + [460 to 1090] seconds.

Reference is now made to Fig. 56G, which illustrates an amplicon stretching stage that normally occurs during the RCA polymerization stage of Fig. 56F and preferably occurs in the presence of a DC electric field, preferably of 10 - 300 Volts per centimeter in a direction opposite to that of the electric field in the step of Fig. 56F as indicated by an arrow 5644. Stretching of amplicon 5640 occurs in a direction indicated by an arrow 5648. The amplicon stretching stage preferably occurs in the presence of a *Bst* polymerase enzyme 5629, dNTPs (not shown) and reverse primer 5324 at a temperature of 65 degrees Celsius. The duration of the amplicon stretching stage is typically 5 to 15 seconds. The RCA polymerization stage is preferably completed at T= T0 + [465 to 1105] seconds.

It is appreciated that the stages shown in Figs.56F and 56G may be repeated intermittently multiple times, with multiple stages shown in Fig. 56F each being of a shorter duration than that indicated above and being separated by a stage shown in Fig. 56G.

Reference is now made to Fig. 56H, which illustrates an exponential RCA amplification stage that normally occurs during the RCA polymerization stage of Fig. 56F and the amplicon stretching stage of Fig. 56G and preferably occurs in the presence of a DC electric field, preferably of 10 - 300 Volts per centimeter, which is normally in an opposite direction from that of the electric field in the step of Fig. 56G but preferably includes short periods in which the polarity of the electric field is reversed. In this stage, additional amplicons 5650 are generated using reverse primers 5324.

The exponential RCA amplification stage preferably occurs in the presence of a *Bst* polymerase enzyme 5629, dNTPs (not shown) and reverse primer 5324 at a temperature of 65 degrees Celsius. The duration of the exponential RCA amplification stage, which occurs during the RCA polymerization stage of Fig. 56F and the amplicon stretching stage of Fig. 56G, is typically 5 - 15 seconds. The RCA polymerization stage of Fig. 56F, the amplicon stretching stage of Fig. 56G and the exponential RCA amplification stage of Fig. 56H, preferably are completed at T=TO + [465 to 1105] seconds.

Reference is now made to Fig. 56I, which illustrates a post-RCA polymerization addressing stage that normally occurs following the completion of RCA polymerization stage of Fig. 56F, the amplicon stretching stage of Fig. 56G and the exponential RCA amplification stage of Fig. 56H, and preferably occurs in the presence of a DC electric field, preferably of 10 - 300 Volts per centimeter, which is normally in the same direction as that of the electric field in the step of Fig. 56H. The post-RCA polymerization addressing stage is particularly useful for gathering amplicons 5640 that are in solution 5602 at a distance from target molecule-specific microgel deposits 5170 and recapturing them at the target molecule-specific microgel deposits 5170, as indicated by arrows 5660, and preferably concentrating the amplicons 5640 at one location within each microgel region 5620. The duration of the post-RCA polymerization addressing stage is typically 10 - 30 seconds. The post-RCA polymerization addressing stage is preferably completed at T=TO + [475 to 1135] seconds.

Reference is now made to Fig. 56J, which illustrates a reporting stage that normally follows the post-RCA polymerization addressing stage. The reporting stage preferably occurs in the presence of a fluorescence reporter 5670 complementary to amplicons 5640 and 5650, which is introduced to electrophoretic array assembly 5600 through a solution. The duration of the reporting stage is typically 10 - 30 seconds. The reporting stage is preferably completed at T = T0 + [485 to 1165] seconds.

Upon completion of the reporting stage and a subsequent washing stage, not shown, the detection of the presence of at least one nucleic acid target molecule, from among a multiplicity of pre-selected nucleic acid target molecules, may be carried out by conventional fluorescence detection techniques. It is thus appreciated that detection of at least one nucleic acid target molecule is preferably completed within between 8 minutes and 20 minutes of the initial supply of solution 5602 to the interior of the electrophoretic array assembly 5600.

It is appreciated that if preparation of solution 5602 is completed within 4 - 5 minutes of acquisition of a sample as by taking a blood sample from a patient, detection at least one nucleic acid target molecule may be completed within 12 - 25 minutes from sample acquisition.

Reference is now made to Figs. 57A - 57J, which illustrate principal stages in rapid detection of the presence of at least one nucleic acid target molecule, from among a multiplicity of pre-selected nucleic acid target molecules in accordance with still another preferred arrangement of the present disclosure. It is appreciated that for simplicity, the substrate 5110, the peripheral wall structure 5120 and the window 5130 as well as the various layers constituting the electrophoretic array 5160 are not specifically shown. Each of Figs. 57A - 57J is a simplified side view sectional illustration taken generally along lines 54 - 54 in Fig. 52A.

Fig. 57A shows an electrophoretic array assembly 5700 in its dried, dehydrated, operative orientation, similar to that shown in Fig. 52A, with symbolic indications of various different oligonucleotide forward primers 5322, nucleic acid target molecule specific RCA circular probes 5320 and reverse primers 5324 which are bound to corresponding different ones of the dried microgel deposits 5190. Fig. 57A indicates, in millimeter units, the interior dimensions of a preferred arrangement of the electrophoretic array assembly 5700, as well as the general dimensions of the immobilized dried target molecule-specific microgel deposits 5190.

It is appreciated that the method of Figs. 57A - 57J differs from the method of Figs. 54A - 54J in that instead of capture probes 5400 being bound to the immobilized dried target molecule specific microgel deposits 5190 in the method of Figs. 54A - 54J, nucleic acid target molecule specific RCA circular probes 5320 are specifically hybridized to forward primers 5322, which are bound to immobilized dried target molecule-specific microgel deposits 5190, and reverse primers 5324 are also bound to immmobilized dried target molecule-specific microgel deposits 5190 in the method of Figs. 57A - 57J.

Fig. 57B illustrates the introduction, symbolized by an arrow 5701, of a solution 5702 containing nucleic acid target molecules 5703, so as to fill the interior of the electrophoretic array assembly 5700.

Preparation of solution 5702 is not part of the present claimed invention and is carried out in accordance with conventional techniques, such as those described in "Nasir Ali, Rita de Cássia Pontello Rampazzo, Alexandre Dias Tavares Costa, and Marco Aurelio Krieger, Current Nucleic Acid Extraction Methods and Their Implications to Point-of-Care Diagnostics, BioMed Research International Volume 2017, Article ID 93065. Solution 5702 preferably includes a low conductivity eluent liquid, typically introduced during preparation of the solution, that promotes electronic addressing of nucleic acids and promotes activity of restriction enzymes in solution 5702. A preferred eluent liquid includes histidine and a restriction enzyme buffer. Fig. 57B shows the dried target molecule-specific microgel deposits 5190 in their dehydrated state. The introduction of solution 5702 into electrophoretic array assembly 5700, so that solution 5702 contacts dried target molecule-specific microgel deposits 5190, defines a time T0 and causes dried target molecule-specific microgel deposits 5190 to assume their hydrated state, designated by reference numeral 5170 (Figs. 51A & 51B).

Fig. 57C illustrates the immobilized target molecule-specific microgel deposits in a hydrated state, as indicated by reference numerals 5170 (Figs. 51A & 51B) as the result of the introduction of solution 5702, containing nucleic acid target molecules 5703, which fills the interior of the electrophoretic array assembly 5700. Fig. 57C illustrates that the hydrated target molecule-specific electrically separated microgel deposits 5170 have a typical maximum height of 1.25 mm. The hydration of the target molecule-specific microgel deposits 5170 to their hydrated state shown in Fig. 57C preferably takes about 10 seconds and is preferably completed at time T = T0 + 10 seconds.

Fig. 57D illustrates electrophoretic addressing of nucleic acid target molecules 5703 to the hydrated immobilized, mutually electrically separated microgel deposits 5170 in the presence of a DC electric field preferably of 10 - 300 Volts per centimeter. Portions of the electric field lines are indicated by reference numerals 5710 and the direction of the electric field is indicated by arrows 5712. It may be appreciated that the electric field lines define three-dimensional, immobilized, mutually spaced and mutually electrically separated microgel regions 5720, each centered about a different target molecule-specific microgel deposit 5170.

It is appreciated that addressing as well as the various steps described hereinbelow with reference to Figs. 57E - 57J occur not only on the surface of microgel deposits 5170 but also within the volume of the microgel deposits 5170.

As seen in Fig. 57D, the application of the DC electric field to the three-dimensional, immobilized, mutually spaced and mutually electrically separated target molecule-specific microgel regions 5720 causes rapid transport of nucleic acid target molecules 5703 to the target molecule-specific microgel deposits 5170, , which in turn facilitates specific hybridization between the nucleic acid target molecules 5703 and the nucleic acid target molecule specific RCA circular probes 5320, which RCA circular probes 5320 are bound to forward primers 5322, which forward primers 5322 are bound to the target molecule-specific microgel deposits 5170.

The duration of the stage illustrated in Fig. 57D is between 30 seconds and 120 seconds. The stage illustrated in Fig. 57D is preferably completed at a time T = T0 + [40 to 130] seconds.

It is appreciated that an optional removing stage (not shown) may be added following the addressing stage shown in Fig. 57D, prior to the stages described below in reference to 57E - 57J, and preferably takes place in the presence of a DC electric field, preferably of 10 - 300 Volts per centimeter in the opposite direction as the electric field in the step of Fig. 57D. The removing stage preferably is operative to remove non-specific target molecules which had hybridized to RCA circular probes 5320.

Reference is now made to Fig. 57E, which illustrates a ligation stage that normally follows the addressing stage shown in Fig. 57D and preferably takes place in the presence of a DC electric field, preferably of 10 - 300 Volts per centimeter in the same direction as the electric field in the step of Fig. 57D. The ligation stage preferably occurs in the presence of a ligation enzyme 5728, for example, T-4 ligase, which is introduced into electrophoretic array assembly 5700 through a solution. The duration of the ligation stage is typically 120 to 240 seconds. The ligation stage is preferably completed at T=TO + [160 to 370] seconds.

Reference is now made to Fig. 57F, which illustrates an RCA polymerization stage that normally follows the ligation stage shown in Fig. 57E and preferably occurs in the presence of a DC electric field, preferably of 10 - 300 Volts per centimeter in the same direction as the electric field in the step of Fig. 57E. The RCA polymerization stage preferably occurs in the presence of a *Bst* polymerase enzyme 5729 and dNTPs (not shown), which are introduced into electrophoretic array assembly 5700 through a solution, and forward primer 5322 and reverse primer 5324, which are bound to the target molecule-specific microgel deposit 5170, preferably at a temperature of 65 degrees Celsius. A result of the RCA polymerization stage is generation of long RCA amplicons 5740 (Fig. 57G). As seen in Fig. 57F, following binding of polymerase enzyme 5729 to nucleic acid target molecule specific RCA circular probes 5320, nucleic acid target molecules 5703 are displaced from nucleic acid target molecule specific RCA circular probes 5320, as indicated by arrows 5742. The duration of the RCA polymerization stage is typically 300 to 5720 seconds. The RCA polymerization stage is preferably completed at T = T0 + [460 to 1090] seconds.

Reference is now made to Fig. 57G, which illustrates an amplicon stretching and compressing stage that normally occurs during the RCA polymerization stage of Fig. 57F and preferably occurs in the presence of a DC electric field, preferably of 10 - 300 Volts per centimeter in directions both the same and opposite to that of the electric field in the step of Fig. 57F as indicated by an arrow 5744. Stretching of amplicon 5740 occurs in a direction indicated by an arrow 5748. Compressing typically occurs in a direction opposite to that shown by arrow 5748. Amplicon stretching and compressing enhances hybridization of reverse primers 5324, which are bound to the target molecule-specific microgel deposits 5170, to amplicon 5740. The amplicon stretching and compressing stage preferably occurs in the presence of a *Bst* polymerase enzyme 5729, dNTPs (not shown) at a temperature of 65 degrees Celsius. The duration of the amplicon stretching stage is typically 5 to 15 seconds. The RCA polymerization stage is preferably completed at T=TO + [465 to 1105] seconds.

It is appreciated that the stages shown in Figs. 57F and 57G may be repeated intermittently multiple times, with the stages shown in Fig. 57F being each of a shorter duration than that indicated above and being separated by a stage shown in Fig. 57G.

Reference is now made to Fig. 57H, which illustrates an exponential RCA amplification stage that normally occurs during the RCA polymerization stage of Fig. 57F and the amplicon compressing stretching stage of Fig. 57G and preferably occurs in the presence of a DC electric field, preferably of 10 - 300 Volts per centimeter, which is normally in an opposite direction from that of the electric field in the step of Fig. 57G, but preferably includes short periods in which the polarity of the electric field is reversed. In this stage, additional amplicons 5750 are generated using reverse primers 5324.

The exponential RCA amplification stage preferably occurs in the presence of a *Bst* polymerase enzyme 5729, dNTPs (not shown) and reverse primer 5324 at a temperature of 65 degrees Celsius. The duration of the exponential RCA amplification stage, which occurs during the RCA polymerization stage of Fig. 57F and the amplicon stretching stage of Fig. 57G, is typically 5 - 15 seconds. The RCA polymerization stage of Fig. 57F, the amplicon stretching stage of Fig. 57G and the exponential RCA amplification stage of Fig. 57H, preferably are completed at T=TO + [465 to 1105] seconds.

Reference is now made to Fig. 57I, which illustrates a post-RCA amplicon concentrating stage that normally occurs following the completion of RCA polymerization stage of Fig. 57F, the amplicon stretching and compressing stage of Fig. 57G and the exponential RCA amplification stage of Fig. 57H, and preferably occurs in the presence of a DC electric field, preferably of 10 - 300 Volts per centimeter, which is normally in the same direction as that of the electric field in the step of Fig. 57H. The amplicon concentrating stage is particularly useful for concentrating amplicons 5740 and 5750, as indicated by arrows 5760, at one location within each microgel region 5720. The duration of the amplicon concentrating stage is typically 10 - 30 seconds. The post-RCA polymerization addressing stage is preferably completed at T=TO + [475 to 1135] seconds.

Reference is now made to Fig. 57J, which illustrates a reporting stage that normally follows the post-RCA polymerization addressing. The reporting stage preferably occurs in the presence of a fluorescence reporter 5770 complementary to amplicons 5740 and 5750, which is introduced to electrophoretic array assembly 5700 through a solution. The duration of the reporting stage is typically 10 - 30 seconds. The reporting stage is preferably completed at T = T0 + [485 to 1165] seconds.

Upon completion of the reporting stage and a subsequent washing stage, not shown, the detection of the presence of at least one nucleic acid target molecule, from among a multiplicity of pre-selected nucleic acid target molecules, may be carried out by conventional fluorescence detection techniques. It is thus appreciated that detection of at least one nucleic acid target molecule is preferably completed within between approximately 8 minutes and 20 minutes of the initial supply of solution 5702 to the interior of the electrophoretic array assembly 5100.

It is appreciated that if preparation of solution 5701 is completed within 4 - 5 minutes of acquisition of a sample as by taking a blood sample from a patient, detection at least one nucleic acid target molecule may be completed within 12 - 25 minutes from sample acquisition.

### EXAMPLES

### Example 1.

### DETECTION OF MENINGITIS PATHOGENS EMPLOYING THE METHOD OF FIGS. 57A - 57J AND USING A SYNTHETIC DNA TARGET MOLECULE REPRESENTING Neisseria meningitidis

An electrophoretic array assembly similar to electrophoretic array assembly 5700 (Fig. 57A) containing 100 immobilized dried target molecule-specific microgel deposits 5190 having a base diameter of 0.45 mm and a height of approximately 0.2 mm - 0.3 mm was provided. 48 immobilized dried target molecule-specific microgel deposits 5190 were spotted with RCA circular probes 5320 pre-hybridized to forward primers 5322 and with reverse primers 5324 specific to nine different pathogen DNA targets each relevant to detection of meningitis, including inter alia *Neisseria meningitidis.* Accordingly, the 48 spotted immobilized dried target molecule-specific microgel deposits 5190 were target molecule-specific as follows:
Deposit 1 -Specific to *Neisseria meningitidis*
Deposit 2 -Specific to *Neisseria meningitidis*
Deposit 3 -Specific to *Neisseria meningitidis*
Deposit 4.-Specific to *Escherichia coli*
Deposit 5.-Specific to *Escherichia coli*
Deposit 6.-Specific to *Escherichia coli*
Deposit 7 -Specific to *Neisseria meningitidis*
Deposit 8 -Specific to *Neisseria meningitidis*
Deposit 9 -Specific to *Neisseria meningitidis*
Deposit 10. Specific to *Enterovirus*
Deposit 11. Specific to *Enterovirus*
Deposit 12 -Specific to *Neisseria meningitidis*
Deposit 13 -Specific to *Neisseria meningitidis*
Deposit 14 -Specific to *Neisseria meningitidis*
Deposit 15. Group B *Streptococcus*
Deposit 16. Group B *Streptococcus*
Deposit 17. Group B *Streptococcus*
Deposit 18 -Specific to *Neisseria meningitidis*
Deposit 19 -Specific to *Neisseria meningitidis*
Deposit 20 -Specific to *Neisseria meningitidis*
Deposit 21 -Specific to *Haemophilus influenzae*
Deposit 22 -Specific to *Haemophilus influenzae*
Deposit 23 -Specific to *Haemophilus influenzae*
Deposit 24 -Specific to *Neisseria meningitidis*
Deposit 25 -Specific to *Neisseria meningitidis*
Deposit 26 -Specific to *Neisseria meningitidis*
Deposit 27 -Specific to *Human herpes virus*
Deposit 28 -Specific to *Human herpes virus*
Deposit 29 -Specific to *Human herpes virus*
Deposit 30 -Specific to *Human herpes virus*
Deposit 31 -Specific to *Neisseria meningitidis*
Deposit 32 -Specific to *Neisseria meningitidis*
Deposit 33 -Specific to *Neisseria meningitidis*
Deposit 34 -Specific to *Human parechovirus*
Deposit 35 -Specific to *Human parechovirus*
Deposit 36 -Specific to *Human parechovirus*
Deposit 37 -Specific to *Neisseria meningitidis*
Deposit 38 -Specific to *Neisseria meningitidis*
Deposit 39 -Specific to *Neisseria meningitidis*
Deposit 40 -Specific to *Lysteria monocytogenes*
Deposit 41 -Specific to *Lysteria monocytogenes*
Deposit 42 -Specific to *Lysteria monocytogenes*
Deposit 43 -Specific to *Neisseria meningitidis*
Deposit 44 -Specific to *Neisseria meningitidis*
Deposit 45 -Specific to *Neisseria meningitidis*
Deposit 46. Specific to *Varicella zoster*
Deposit 47. Specific to *Varicella zoster*
Deposit 48. Specific to *Varicella zoster*

A solution 5702 containing nucleic acid target molecules 5703 (100 nM concentration) representing *Neisseria meningitidis* was supplied to the interior volume of the electrophoretic array, at a time defined as T0. The solution 5702 also included a low conductivity buffer supporting rapid DNA transport and hybridization to the RCA probes deposited on the microgels.

Supplying solution 5702 caused dried target molecule-specific microgel deposits 5190 to assume their hydrated state, designated by reference numeral 5170, after a duration of 10 seconds. (Figs. 57B-57C)

At time T= T0 + 10 seconds, a constant current of 1.6 mA was applied across the working and counter electrode contacts 5260 and 5250 respectively, resulting in voltages of 4.5 V, yielding an electric field applied across the electrophoretic array of 12.5 V per cm and producing electrophoretic addressing (Fig. 57D). The duration of the electrophoretic addressing was 40 seconds.

At time T= T0 + 50 seconds, a ligation reaction solution including ligation reaction enzyme T4 ligase (Blunt T/A, from New England Biolabs) was supplied to the interior volume of the electrophoretic array, replacing solution 5702, for a duration of approximately 180 seconds. (Fig. 57E)

At time T = T0 + 230 seconds, a polymerase solution containing *Bst* polymerase enzyme 5729 and dNTPs (from New England Biolabs) was supplied to the interior volume of the electrophoretic array, replacing the ligation reaction solution, for a duration of approximately 720 seconds. (Fig. 57F).

At time T = T0 + 950 seconds, a constant current of 1.6 mA was applied across the working and counter electrode contacts 5260 and 5250 respectively, resulting in voltages of 4.5 V, yielding an electric field applied across the electrophoretic array of 12.5 V per cm and providing recapture of RCA amplicons from the polymerase solution. The duration of this step was approximately 20 seconds. (Fig. 57I)

At time T = T0 + 970 seconds, a red reporter solution containing fluorescently labeled oligonucleotides (Alexa 647 from Integrated Device Technology, Inc., San Jose, CA) was supplied to the interior volume of the electrophoretic array, replacing the polymerase solution for a duration of approximately 30 seconds. (Fig. 57J). Following washing out of the red reporter solution, a fluorescence image of the electrophoretic array assembly 5700 was taken via window 5130 and the presence of nucleic acid target molecules 5703 representing *Neisseria meningitidis* was detected at the following ones of immobilized dried target molecule-specific microgel deposits 5170: 1,2,3,7,8,9,13,14,15,19,20,21,25,26,27,31,32,33,37,38,39,43,44, and 45. The presence of nucleic acid target molecules 5703 representing *Neisseria meningitides* was not detected at the following ones of immobilized dried target molecule-specific microgel deposits 5170: 4,5,6,10,11,12,16,17,18,22,23,24,28,29,30,34,35,36,40,41,42,46,47 and 48.

The detection results are summarized in Fig. 8. It is noted that the average ratio of intensities of the fluorescence signal obtained from deposits 1,2,3,7,8,9,13,14,15,19,20,21,25,26,27,31,32,33,37,38,39,43,44, and 45 to the fluorescence signal obtained from deposits 4,5,6,10,11,12,16,17,18,22,23,24,28,29,30,34,35,36,40,41,42,46,47 and 48 was approximately 8.5.

### Example 2.

### DETECTION OF MENINGITIS PATHOGENS EMPLOYING THE METHOD OF FIGS. 57A - 57J AND USING A GENOMIC DNA TARGET MOLECULE EXTRACTED FROM Neisseria meningitides PATHOGEN SPIKED INTO CEREBROSPINAL FLUID CLINICAL SAMPLE

An electrophoretic array assembly similar to electrophoretic array assembly 5700 (Fig. 57A) containing 100 immobilized dried target molecule-specific microgel deposits 5190 having a base diameter of 0.45 mm and a height of approximately 0.2 mm - 0.3 mm was provided. 21 immobilized dried target molecule-specific microgel deposits 5190 were spotted with RCA circular probes 5320 pre-hybridized to forward primers 5322 and with reverse primers 5324 specific to nine different pathogen DNA targets each relevant to detection of meningitis, including inter alia *Neisseria meningitidis.* Accordingly, the 21 spotted immobilized dried target molecule-specific microgel deposits 5190 were target molecule-specific as follows:
Deposit 1. Specific to *Escherichia coli*
Deposit 2. Specific to *Escherichia coli*
Deposit 3 -Specific to *Neisseria meningitidis*
Deposit 4 -Specific to *Neisseria meningitidis*
Deposit 5 -Specific to *Neisseria meningitidis*
Deposit 6. Specific to *Enterovirus*
Deposit 7. Specific to *Enterovirus*
Deposit 8 -Specific to *Neisseria meningitidis*
Deposit 9 -Specific to *Neisseria meningitidis*
Deposit 10 -Specific to *Neisseria meningitidis*
Deposit 11. Group B *Streptococcus*
Deposit 12. Group B *Streptococcus*
Deposit 13 -Specific to *Haemophilus influenzae*
Deposit 14 -Specific to *Haemophilus influenzae*
Deposit 15 -Specific to *Neisseria meningitidis*
Deposit 16 -Specific to *Neisseria meningitidis*
Deposit 17 -Specific to *Neisseria meningitidis*
Deposit 18 -Specific to *Lysteria monocytogenes*
Deposit 19 -Specific to *Lysteria monocytogenes*
Deposit 20. Specific to *Varicella zoster*
Deposit 21. Specific to *Varicella zoster*

A clinical sample of cerebrospinal fluid (CSF) was spiked with *Neisseria meningitides* pathogen, and genomic DNA extraction performed using a common magnetic bead-based DNA extraction method. The input concentration of DNA target in cerebrospinal fluid was determined by a reference real-time PCR method that yielded *Neisseria meningitides* pathogen concentration in clinical sample of cerebrospinal fluid of 720 copies of DNA per microliter of CSF.

A solution 5702, prepared from the spiked clinical sample, was supplied to the interior volume of the electrophoretic array, at a time defined as T0. The solution 5702 also included a low conductivity buffer supporting rapid DNA transport and hybridization to the RCA probes deposited on the microgels.

Supplying solution 5702 caused dried target molecule-specific microgel deposits 5190 to assume their hydrated state, designated by reference numeral 5170, after a duration of 10 seconds. (Figs. 57B-57C)

At time T= T0 + 10 seconds, a constant current of 1.6 mA was applied across the working and counter electrode contacts 5260 and 5250 respectively, resulting in voltages of 4.5 V, yielding an electric field applied across the electrophoretic array of 12.5 V per cm and producing electrophoretic addressing (Fig. 57D). The duration of the electrophoretic addressing was 40 seconds.

At time T= T0 + 50 seconds, a reverse polarity electric field was applied by applying a constant current of negative 1.6 mA across the working and counter electrode contacts 5260 and 5250 respectively, resulting in voltages of 4.5 V, yielding an electric field applied across the electrophoretic array of 12.5 V per cm and enhancing removal of nonspecifically bound DNA targets. The duration of the electrophoretic addressing was 10 seconds. (Fig. 57G)

At time T= T0 + 60 seconds, a ligation reaction solution including ligation reaction enzyme T4 ligase (Blunt T/A, from New England Biolabs) was supplied to the interior volume of the electrophoretic array, replacing solution 5702, for a duration of approximately 180 seconds. (Fig. 57E)

At time T = T0 + 240 seconds, a polymerase solution containing *Bst* polymerase enzyme 5729 and dNTPs (from New England Biolabs) was supplied to the interior volume of the electrophoretic array, replacing the ligation reaction solution, for a duration of approximately 720 seconds. (Fig. 57F).

At time T = T0 + 960 seconds, a constant current of 1.6 mA was applied across the working and counter electrode contacts 5260 and 5250 respectively, resulting in voltages of 4.5 V, yielding an electric field applied across the electrophoretic array of 12.5 V per cm and providing recapture of RCA amplicons from the polymerase solution. The duration of this step was approximately 20 seconds. (Fig. 57I)

At time T = T0 + 980 seconds, a red reporter solution containing fluorescently labeled oligonucleotides (Alexa 647 from Integrated Device Technology, Inc., San Jose, CA) was supplied to the interior volume of the electrophoretic array, replacing the polymerase solution for a duration of approximately 30 seconds. (Fig. 57J). Following washing out of the red reporter solution, a fluorescence image of the electrophoretic array assembly 5700 was taken via window 5130 and the presence of nucleic acid target molecules 5703 representing *Neisseria meningitidis* was detected at the following ones of immobilized dried target molecule-specific microgel deposits 5170: 3,4,5,8,9,10, 15,16, and 17. The presence of nucleic acid target molecules 5703 representing *Neisseria meningitidis* was not detected at the following ones of immobilized dried target molecule-specific microgel deposits 5170: 1,2,6,7,11,12,13,14,18,19,20, and 21.

The detection results are summarized in Fig. 9. It is noted that the average ratio of intensities of the fluorescence signal obtained from deposits 3,4,5,8,9,10, 15,16, and 17 to the the fluorescence signal obtained from deposits 1,2,6,7,11,12,13,14,18,19,20, and 21 was approximately 4.3.

It will be appreciated by persons skilled in the art that the present invention is defined by the appended claims, and may not be limited to what has been specifically described and shown herein but may also include combinations and sub-combinations of features described herein and modifications thereof which are not in the prior art.

## Claims

1. A computerized automated diagnostic system comprising:
a cartridge (122) including reagents for carrying out a diagnostic biological process and configured to receive a biological sample to be tested using said reagents, said cartridge including a carbon array assembly (120) mounted thereon; and
an instrument (100) operative to interact with said cartridge (122) to carry out multiple steps in said diagnostic biological process,
wherein:
said cartridge has multiple operative states including:
a non-functionalized state in which said cartridge does not include said reagents and does not include said biological sample;
a partially functionalized state in which said cartridge does include said reagents and does include said biological sample and in which a carbon array assembly is mounted onto said cartridge and forms part of said cartridge; and
a fully functionalized state upon insertion of said cartridge, onto which said carbon array assembly is mounted, into clamped engagement with said instrument, and
said carbon array assembly (120) comprises:
a carbon array subassembly (1200) comprising a double-sided adhesive layer (1220) formed with registration apertures (1222) and registration cut outs (1224) as well as liquid inlet apertures (1226) and liquid outlet apertures (1228); and
a cover assembly (1210)
with said carbon array subassembly (1200) further comprising:
a black background layer (1240);
a substrate layer formed (1250) over said black background layer (1240) and formed with registration apertures (1252) and registration cut outs (1254) as well as liquid inlet apertures (1256) and liquid outlet apertures (1258);
an array of carbon resistors (1260) formed onto said substrate layer (1250);
an electrode array (1280) formed over said array of carbon resistors (1260) onto said substrate layer (1250) and defining a plurality of electrode arrays (1282), each of which includes a peripheral electrode (1284) and a counter electrode (1286) as well as two rows of working electrodes (1288); and
a carbon array (1290) printed over said electrode array (1280) onto said substrate layer (1250) and defining a pair of carbon arrays (1292), each of which includes a central carbon electrode (1294) as well as two rows of working carbon electrodes (1298).

2. A computerized automated diagnostic system according to claim 1 and wherein said carbon array assembly includes a flow cell including multiple simultaneous detection regions.

3. A computerized automated diagnostic system according to claim 1 or claim 2 and wherein said cartridge (122) comprises a Polymerase Chain Reaction (PCR) amplification assembly, said PCR amplification assembly including:
a plurality of aliquot chambers (660, 662, 664, 666, 668, 670) configured to receive purified sample nucleic acid material;
a plurality of different dry reagent plugs (390, 388, 386, 384, 382, 380), each in liquid communication with one of said plurality of aliquot chambers (660, 662, 664, 666, 668, 670);
a plurality of PCR chambers (700, 702, 704, 706, 708, 710), each in liquid communication with one of said plurality of different dry reagent plugs (390, 388, 386, 384, 382, 380); and
a plurality of gas springs (711, 712, 713, 714, 715, 716), each in liquid communication with one of said plurality of PCR chambers (700, 702, 704, 706, 708, 710),
wherein said plurality of PCR chambers (700, 702, 704, 706, 708, 710) are located adjacent an edge of said cartridge (122).

4. A computerized automated diagnostic system according to any of the preceding claims and wherein said cartridge (122) also comprises:
a fluid sealing layer (130) having first (132, 134) and second pairs (136, 138) of apertures providing fluid communication with an interior of said carbon array assembly (120);
a main cartridge element (150), having a three-dimensionally patterned fluid conduit defining surface (152) which cooperates with said sealing layer (130) to define a multiplicity of fluid conduits, said main cartridge element (150) having a fluid conduit defining and liquid enclosure engagement surface (154) opposite to said three-dimensionally patterned fluid conduit defining surface (152); and
a plurality of elastomeric sealing layers (162, 164) which are sealingly joined to said fluid conduit defining and liquid enclosure engagement surface (154) of said main cartridge element (150) and define therewith a plurality of valves (402, 405, 408, ..., 486), said main cartridge element (150) also comprising a plurality of through holes (400, 401, 403, 404, 406, 407, ..., 484, 485) which cooperate with said sealing layer (130) and said plurality of elastomeric sealing layers (162, 164) to define a plurality of frangible seals (501, 503, 505, ..., 583), said frangible seals (501, 503, 505, ..., 583) being normally closed and being simultaneously opened when said cartridge (122) is in clamped engagement with said instrument (100).

5. A computerized automated diagnostic system according to claim 1, and wherein said cartridge (122) comprises a room-temperature shelf-storable electrophoretic array (5160) including:
a multiplicity of immobilized, mutually spaced and mutually electrically separated microgel deposits (5170, 5310), each of said multiplicity of immobilized, mutually spaced and mutually electrically separated microgel deposits (5170) containing materials suitable for performing rolling circle amplification and binding of at least one of said multiplicity of pre-selected nucleic acid target molecules, each of said microgel deposits containing at least the following elements pre-anchored therein:
an RCA probe (5320) specific to of at least one of said multiplicity of pre-selected nucleic acid target molecules; and
at least one primer including at least one forward primer (5322) and at least one reverse primer (5324).

6. A computerized automated diagnostic method comprising:
providing a computerized automated diagnostic system according to claim 5, wherein said multiplicity of immobilized, mutually spaced and mutually electrically separated microgel deposits (5170) define a corresponding multiplicity of immobilized, mutually spaced and mutually electrically separated microgel regions (5420, 5520, 5620, 5720);
introducing said solution to each of said multiplicity of immobilized, mutually spaced and mutually electrically separated microgel regions;
performing rolling circle amplification at least generally simultaneously at each of said multiplicity of immobilized, mutually spaced and mutually electrically separated microgel regions, while applying electric fields thereto during various stages of said rolling circle amplification; and
detecting the presence of at least one of said multiplicity of pre-selected nucleic acid target molecules at at least one corresponding one of said immobilized, mutually spaced and mutually electrically separated microgel regions,
wherein said detecting occurs within a short time period of said introducing, said short time period being less than 30 minutes, and
said applying electric fields during said rolling circle amplification comprises at least two of the following:
applying an electric field to said immobilized, mutually spaced and mutually electrically separated microgel regions for driving nucleic acid target molecules in said solution to said microgel deposits;
applying an electric field to said immobilized, mutually spaced and mutually electrically separated microgel regions for driving nucleic acid target molecule-RCA probe hybridization products in said solution to said microgel deposits;
applying an electric field to said immobilized, mutually spaced and mutually electrically separated microgel regions for recapturing RCA amplicons that drift away from said microgel deposits;
applying an electric field to said immobilized, mutually spaced and mutually electrically separated microgel regions for driving RCA probes into said microgel deposits for hybridization with at least one of capture probes and primers already bound to said microgel deposits;
applying an electric field to said immobilized, mutually spaced and mutually electrically separated microgel regions for removing undesired molecules from said microgel regions;
applying an electric field to said immobilized, mutually spaced and mutually electrically separated microgel regions for stretching RCA amplicons that are bound to said microgel deposits;
applying an electric field to said immobilized, mutually spaced and mutually electrically separated microgel regions for compressing RCA amplicons that are bound to said microgel deposits;
applying an electric field to said immobilized, mutually spaced and mutually electrically separated microgel regions for stirring RCA reagents in the vicinity of RCA amplicons that are bound to said microgel deposits;
applying an electric field to said immobilized, mutually spaced and mutually electrically separated microgel regions for enhancing the speed of enzyme activity in RCA; and
applying electric field of sequentially reversing polarity to said immobilized, mutually spaced and mutually electrically separated microgel regions for enhancing stringency of binding of RCA amplicons to said microgel deposits.

## Patentansprüche

1. Rechnergestütztes automatisiertes Diagnosesystem, umfassend:
eine Kartusche (122), die Reagenzien zum Ausführen eines biologischen Diagnoseprozesses beinhaltet und dazu konfiguriert ist, eine biologische Probe aufzunehmen, die unter Verwendung der Reagenzien zu testen ist, wobei die Kartusche eine darauf montierte Kohlenstoffarray-Baugruppe (120) beinhaltet; und
ein Instrument (100), das betriebsfähig ist, um mit der Kartusche (122) zu interagieren, um mehrere Schritte in dem biologischen Diagnoseprozess auszuführen,
wobei:
die Kartusche mehrere Betriebszustände aufweist, beinhaltend:
einen nicht funktionalisierten Zustand, in dem die Kartusche die Reagenzien nicht beinhaltet und die biologische Probe nicht beinhaltet;
einen teilweise funktionalisierten Zustand, in dem die Kartusche die Reagenzien beinhaltet und die biologische Probe beinhaltet und in dem eine Kohlenstoffarray-Baugruppe auf der Kartusche montiert ist und einen Teil der Kartusche bildet; und
einen vollständig funktionalisierten Zustand beim Einsetzen der Kartusche, auf der die Kohlenstoffarray-Baugruppe in Klemmeingriff mit dem Instrument montiert ist, und
die Kohlenstoffarray-Baugruppe (120) Folgendes umfasst:
eine Kohlenstoffarray-Unterbaugruppe (1200), die eine doppelseitige Klebstoffschicht (1220) umfasst, die mit Ausrichtungsöffnungen (1222) und Ausrichtungsausschnitten (1224) sowie Flüssigkeitseinlassöffnungen (1226) und Flüssigkeitsauslassöffnungen (1228) gebildet ist; und
eine Abdeckungsbaugruppe (1210),
wobei die Kohlenstoffarray-Unterbaugruppe (1200) ferner Folgendes umfasst:
eine schwarze Hintergrundschicht (1240);
eine Substratschicht (1250), die über der schwarzen Hintergrundschicht (1240) gebildet ist und mit Ausrichtungsöffnungen (1252) und Ausrichtungsausschnitten (1254) sowie Flüssigkeitseinlassöffnungen (1256) und Flüssigkeitsauslassöffnungen (1258) gebildet ist;
ein Array von Kohlenstoffwiderständen (1260), das auf der Substratschicht (1250) gebildet ist;
ein Elektrodenarray (1280), das über dem Array von Kohlenstoffwiderständen (1260) auf der Substratschicht (1250) gebildet ist und eine Vielzahl von Elektrodenarrays (1282) definiert, von denen jedes eine Umfangselektrode (1284) und eine Gegenelektrode (1286) sowie zwei Reihen von Arbeitselektroden (1288) beinhaltet; und
ein Kohlenstoffarray (1290), das über dem Elektrodenarray (1280) auf die Substratschicht (1250) gedruckt ist und ein Paar von Kohlenstoffarrays (1292) definiert, von denen jedes eine zentrale Kohlenstoffelektrode (1294) sowie zwei Reihen von Arbeitskohlenstoffelektroden (1298) beinhaltet.

2. Rechnergestütztes automatisiertes Diagnosesystem nach Anspruch 1, und wobei die Kohlenstoffarray-Baugruppe eine Durchflusszelle beinhaltet, die mehrere Regionen zur gleichzeitigen Detektion beinhaltet.

3. Rechnergestütztes automatisiertes Diagnosesystem nach Anspruch 1 oder Anspruch 2, und wobei die Kartusche (122) eine Polymerase-Kettenreaktion-(PCR-)Verstärkungsbaugruppe umfasst, wobei die PCR-Verstärkungsbaugruppe Folgendes beinhaltet:
eine Vielzahl von Aliquotkammern (660, 662, 664, 666, 668, 670), die dazu konfiguriert sind, gereinigtes Probennukleinsäurematerial aufzunehmen;
eine Vielzahl von verschiedenen Trockenreagenzstopfen (390, 388, 386, 384, 382, 380), die jeweils in Flüssigkeitskommunikation mit einer der Vielzahl von Aliquotkammern (660, 662, 664, 666, 668, 670) stehen;
eine Vielzahl von PCR-Kammern (700, 702, 704, 706, 708, 710), die jeweils in Flüssigkeitskommunikation mit einem der Vielzahl von verschiedenen Trockenreagenzstopfen (390, 388, 386, 384, 382, 380) stehen; und
eine Vielzahl von Gasfedern (711, 712, 713, 714, 715, 716), die jeweils in Flüssigkeitskommunikation mit einer der Vielzahl von PCR-Kammern (700, 702, 704, 706, 708, 710) stehen,
wobei sich die Vielzahl von PCR-Kammern (700, 702, 704, 706, 708, 710) benachbart zu einer Kante der Kartusche (122) befindet.

4. Rechnergestütztes automatisiertes Diagnosesystem nach einem der vorhergehenden Ansprüche, und wobei die Kartusche (122) außerdem Folgendes umfasst:
eine Fluiddichtungsschicht (130), die ein erstes (132, 134) und ein zweites Paar (136, 138) von Öffnungen aufweist, die eine Fluidkommunikation mit einem Innenraum der Kohlenstoffarray-Baugruppe (120) bereitstellen;
ein Hauptkartuschenelement (150), das eine dreidimensional gemusterte Fluidleitungsdefinitionsfläche (152) aufweist, die mit der Dichtungsschicht (130) zusammenwirkt, um eine Mehrzahl von Fluidleitungen zu definieren, wobei das Hauptkartuschenelement (150) eine Fluidleitungsdefinitions- und Flüssigkeitseinfassungseingriffsfläche (154) gegenüber der dreidimensional gemusterten Fluidleitungsdefinitionsfläche (152) aufweist; und
eine Vielzahl von elastomeren Dichtungsschichten (162, 164), die dichtend mit der Fluidleitungsdefinitions- und Flüssigkeitseinfassungseingriffsfläche (154) des Hauptkartuschenelements (150) verbunden sind und damit eine Vielzahl von Ventilen (402, 405, 408, ..., 486) definieren, wobei das Hauptkartuschenelement (150) außerdem eine Vielzahl von Durchgangslöchern (400, 401, 403, 404, 406, 407, ..., 484, 485) umfasst, die mit der Dichtungsschicht (130) und der Vielzahl von elastomeren Dichtungsschichten (162, 164) zusammenwirken, um eine Vielzahl von zerbrechlichen Dichtungen (501, 503, 505, ..., 583) zu definieren, wobei die zerbrechlichen Dichtungen (501, 503, 505, ..., 583) normalerweise geschlossen sind und gleichzeitig geöffnet werden, wenn sich die Kartusche (122) in Klemmeingriff mit dem Instrument (100) befindet.

5. Rechnergestütztes automatisiertes Diagnosesystem nach Anspruch 1, und wobei die Kartusche (122) ein bei Raumtemperatur in einem Regal lagerbares elektrophoretisches Array (5160) umfasst, das Folgendes beinhaltet:
eine Mehrzahl von immobilisierten, voneinander beabstandeten und elektrisch voneinander getrennten Mikrogelablagerungen (5170, 5310), wobei jede der Mehrzahl von immobilisierten, voneinander beabstandeten und elektrisch voneinander getrennten Mikrogelablagerungen (5170) Materialien enthält, die zum Durchführen einer Rolling-Circle-Verstärkung und -Bindung von mindestens einem der Mehrzahl von vorgewählten Nukleinsäurezielmolekülen geeignet sind, wobei jede der Mikrogelablagerungen mindestens die folgenden darin vorverankerten Elemente enthält:
eine RCA-Sonde (5320), die für mindestens eines der Mehrzahl von vorgewählten Nukleinsäurezielmolekülen spezifisch ist; und
mindestens einen Primer, der mindestens einen Vorwärtsprimer (5322) und mindestens einen Rückwärtsprimer (5324) beinhaltet.

6. Rechnergestütztes automatisiertes Diagnoseverfahren, umfassend:
Bereitstellen eines rechnergestützten automatisierten Diagnosesystems nach Anspruch 5, wobei die Mehrzahl von immobilisierten, voneinander beabstandeten und elektrisch voneinander getrennten Mikrogelablagerungen (5170) eine entsprechende Mehrzahl von immobilisierten, voneinander beabstandeten und elektrisch voneinander getrennten Mikrogelregionen (5420, 5520, 5620, 5720) definiert;
Einführen der Lösung in jede der Mehrzahl von immobilisierten, voneinander beabstandeten und elektrisch voneinander getrennten Mikrogelregionen;
Durchführen einer Rolling-Circle-Verstärkung mindestens im Allgemeinen gleichzeitig an jeder der Mehrzahl von immobilisierten, voneinander beabstandeten und elektrisch voneinander getrennten Mikrogelregionen, während elektrische Felder während verschiedener Phasen der Rolling-Circle-Verstärkung daran angelegt werden; und
Detektieren des Vorhandenseins von mindestens einem der Mehrzahl von vorgewählten Nukleinsäurezielmolekülen an mindestens einem entsprechenden der immobilisierten, voneinander beabstandeten und elektrisch voneinander getrennten Mikrogelregionen,
wobei das Detektieren innerhalb einer kurzen Zeitspanne nach dem Einführen erfolgt, wobei die kurze Zeitspanne weniger als 30 Minuten beträgt, und
wobei das Anlegen elektrischer Felder während der Rolling-Circle-Verstärkung mindestens zwei der Folgenden umfasst:
Anlegen eines elektrischen Feldes an die immobilisierten, voneinander beabstandeten und elektrisch voneinander getrennten Mikrogelregionen, um Nukleinsäurezielmoleküle in der Lösung zu den Mikrogelablagerungen zu treiben;
Anlegen eines elektrischen Feldes an die immobilisierten, voneinander beabstandeten und elektrisch voneinander getrennten Mikrogelregionen, um Nukleinsäurezielmolekül-RCA-Sonden-Hybridisierungsprodukte in der Lösung zu den Mikrogelablagerungen zu treiben;
Anlegen eines elektrischen Feldes an die immobilisierten, voneinander beabstandeten und elektrisch voneinander getrennten Mikrogelregionen, um RCA-Amplikons zurückzugewinnen, die sich von den Mikrogelablagerungen weg bewegen;
Anlegen eines elektrischen Feldes an die immobilisierten, voneinander beabstandeten und elektrisch voneinander getrennten Mikrogelregionen, um RCA-Sonden zur Hybridisierung mit mindestens einem von Erfassungssonden und Primern, die bereits an die Mikrogelablagerungen gebunden sind, in die Mikrogelablagerungen zu treiben;
Anlegen eines elektrischen Feldes an die immobilisierten, voneinander beabstandeten und elektrisch voneinander getrennten Mikrogelregionen, um unerwünschte Moleküle aus den Mikrogelregionen zu entfernen;
Anlegen eines elektrischen Feldes an die immobilisierten, voneinander beabstandeten und elektrisch voneinander getrennten Mikrogelregionen, um RCA-Amplikons zu dehnen, die an die Mikrogelablagerungen gebunden sind;
Anlegen eines elektrischen Feldes an die immobilisierten, voneinander beabstandeten und elektrisch voneinander getrennten Mikrogelregionen, um RCA-Amplikons zu komprimieren, die an die Mikrogelablagerungen gebunden sind;
Anlegen eines elektrischen Feldes an die immobilisierten, voneinander beabstandeten und elektrisch voneinander getrennten Mikrogelregionen, um RCA-Reagenzien in der Nähe von RCA-Amplikons, die an die Mikrogelablagerungen gebunden sind, zu rühren;
Anlegen eines elektrischen Felds an die immobilisierten, voneinander beabstandeten und elektrisch voneinander getrennten Mikrogelregionen, um die Geschwindigkeit von Enzymaktivität in RCA zu verbessern; und
Anlegen eines elektrischen Feldes mit sequentiell umkehrender Polarität an die immobilisierten, voneinander beabstandeten und elektrisch voneinander getrennten Mikrogelregionen, um die Stringenz der Bindung von RCA-Amplikons an die Mikrogelablagerungen zu verbessern.

## Revendications

1. Système de diagnostic automatisé informatisé comprenant :
une cartouche (122) comprenant des réactifs pour mettre en œuvre un processus biologique de diagnostic et conçue pour recevoir un échantillon biologique à tester à l'aide desdits réactifs, ladite cartouche comprenant un ensemble réseau de carbone (120) monté sur celle-ci ; et
un instrument (100) servant à interagir avec ladite cartouche (122) pour mettre en œuvre de multiples étapes dans ledit processus biologique de diagnostic, dans lequel ladite cartouche présente de multiples états opérationnels comprenant :
un état non fonctionnalisé où ladite cartouche ne comprend pas lesdits réactifs et ne comprend pas ledit échantillon biologique ;
un état partiellement fonctionnalisé où ladite cartouche comprend lesdits réactifs et comprend ledit échantillon biologique et où un ensemble réseau de carbone est monté sur ladite cartouche et fait partie de ladite cartouche ; et
un état entièrement fonctionnalisé lors de l'insertion de ladite cartouche, sur laquelle ledit ensemble réseau de carbone est monté, en prise serrée avec ledit instrument, et
ledit ensemble réseau de carbone (120) comprend :
un sous-ensemble réseau de carbone (1200) comprenant une couche adhésive double face (1220) formée avec des ouvertures de repérage (1222) et des découpes de repérage (1224) ainsi que des ouvertures d'entrée de liquide (1226) et des ouvertures de sortie de liquide (1228) ; et
un ensemble couvercle (1210) avec ledit sous-ensemble réseau de carbone (1200) comprenant en outre :
une couche de fond noir (1240) ;
une couche de substrat formée (1250) sur ladite couche de fond noir (1240) et formée avec des ouvertures de repérage (1252) et des découpes de repérage (1254) ainsi que des ouvertures d'entrée de liquide (1256) et des ouvertures de sortie de liquide (1258) ;
un réseau de résistances de carbone (1260) formé sur ladite couche de substrat (1250) ;
un réseau d'électrodes (1280) formé par dessus ledit réseau de résistances de carbone (1260) sur ladite couche de substrat (1250) et définissant une pluralité de réseaux d'électrodes (1282), dont chacun comprend une électrode périphérique (1284) et une contre-électrode (1286) ainsi que deux rangées d'électrodes de travail (1288) ; et
un réseau de carbone (1290) imprimé par dessus ledit réseau d'électrodes (1280) sur ladite couche de substrat (1250) et définissant une paire de réseaux de carbone (1292), dont chacun comprend une électrode de carbone centrale (1294) ainsi que deux rangées d'électrodes de carbone de travail (1298).

2. Système de diagnostic automatisé informatisé selon la revendication 1 et dans lequel ledit ensemble réseau de carbone comprend une cellule d'écoulement comprenant de multiples régions de détection simultanée.

3. Système de diagnostic automatisé informatisé selon la revendication 1 ou la revendication 2 et dans lequel ladite cartouche (122) comprend un ensemble d'amplification par réaction en chaîne par polymérase (PCR), ledit ensemble d'amplification par PCR comprenant :
une pluralité de chambres aliquotes (660, 662, 664, 666, 668, 670) conçues pour recevoir un matériau d'acide nucléique d'échantillon purifié ;
une pluralité de bouchons de réactif sec différents (390, 388, 386, 384, 382, 380), chacun en communication liquide avec l'une de ladite pluralité de chambres aliquotes (660, 662, 664, 666, 668, 670) ;
une pluralité de chambres de PCR (700, 702, 704, 706, 708, 710), chacune en communication liquide avec l'un de ladite pluralité de différents bouchons de réactif sec (390, 388, 386, 384, 382, 380) ; et
une pluralité de ressorts à gaz (711, 712, 713, 714, 715, 716), chacun en communication liquide avec l'une de ladite pluralité de chambres de PCR (700, 702, 704, 706, 708, 710),
dans lequel ladite pluralité de chambres de PCR (700, 702, 704, 706, 708, 710) sont situées adjacentes à un bord de ladite cartouche (122).

4. Système de diagnostic automatisé informatisé selon l'une quelconque des revendications précédentes et dans lequel ladite cartouche (122) comprend également :
une couche d'étanchéité aux fluides (130) présentant des première (132, 134) et seconde (136, 138) paires d'ouvertures assurant une communication fluidique avec un intérieur dudit ensemble réseau de carbone (120) ;
un élément de cartouche principal (150), présentant une surface de définition de conduit de fluide à motif tridimensionnel (152) qui coopère avec ladite couche d'étanchéité (130) pour définir une multiplicité de conduits de fluide, ledit élément de cartouche principal (150) présentant une surface de définition de conduit de fluide et de prise d'enceinte de liquide (154) opposée à ladite surface de définition de conduit de fluide à motif tridimensionnel (152) ; et
une pluralité de couches d'étanchéité élastomères (162, 164) qui sont jointes de manière étanche à ladite surface de définition de conduit de fluide et de prise d'enceinte de liquide (154) dudit élément de cartouche principal (150) et définissent avec celle-ci une pluralité de vannes (402, 405, 408, ..., 486), ledit élément de cartouche principal (150) comprenant également une pluralité de trous traversants (400, 401, 403, 404, 406, 407, ..., 484, 485) qui coopèrent avec ladite couche d'étanchéité (130) et ladite pluralité de couches d'étanchéité élastomères (162, 164) pour définir une pluralité de joints friables (501, 503, 505, ..., 583), lesdits joints friables (501, 503, 505, ..., 583) étant normalement fermés et étant ouverts simultanément lorsque ladite cartouche (122) est en prise serrée avec ledit instrument (100).

5. Système de diagnostic automatisé informatisé selon la revendication 1, et dans lequel ladite cartouche (122) comprend un réseau électrophorétique stockable sur étagère à température ambiante (5160) comprenant :
une multiplicité de dépôts de microgel immobilisés, mutuellement espacés et mutuellement séparés électriquement (5170, 5310), chacun de ladite multiplicité de dépôts de microgel immobilisés, mutuellement espacés et mutuellement séparés électriquement (5170) contenant des matériaux appropriés pour réaliser une amplification en cercle roulant et une liaison d'au moins une de ladite multiplicité de molécules cibles d'acide nucléique présélectionnées, chacun desdits dépôts de microgel contenant au moins les éléments suivants pré-ancrés sur celui-ci :
une sonde de RCA (5320) spécifique d'au moins l'une de ladite multiplicité de molécules cibles d'acide nucléique présélectionnées ; et
au moins une amorce comprenant au moins une amorce avant (5322) et au moins une amorce inverse (5324).

6. Procédé de diagnostic automatisé informatisé comprenant :
la fourniture d'un système de diagnostic automatisé informatisé selon la revendication 5, dans lequel ladite multiplicité de dépôts de microgel immobilisés, mutuellement espacés et mutuellement séparés électriquement (5170) définit une multiplicité correspondante de régions de microgel immobilisées, mutuellement espacées et mutuellement séparées électriquement (5420, 5520, 5620, 5720) ;
l'introduction de ladite solution dans chacune de ladite multiplicité de régions de microgel immobilisées, mutuellement espacées et mutuellement séparées électriquement ;
la réalisation d'une amplification en cercle roulant au moins généralement simultanément au niveau de chacune de ladite multiplicité de régions de microgel immobilisées, mutuellement espacées et mutuellement séparées électriquement, tout en y appliquant des champs électriques pendant diverses étapes de ladite amplification en cercle roulant ; et
la détection de la présence d'au moins l'une de ladite multiplicité de molécules cibles d'acide nucléique présélectionnées au niveau d'au moins l'une correspondante desdites régions de microgel immobilisées, mutuellement espacées et mutuellement séparées électriquement,
dans lequel ladite détection se produit dans un court laps de temps de ladite introduction, ledit court laps de temps étant inférieur à 30 minutes, et
ladite application de champs électriques pendant ladite amplification de cercle roulant comprend au moins deux des étapes suivantes :
l'application d'un champ électrique auxdites régions de microgel immobilisées, mutuellement espacées et mutuellement séparées pour entraîner des molécules cibles d'acide nucléique dans ladite solution vers lesdits dépôts de microgel ;
l'application d'un champ électrique auxdites régions de microgel immobilisées, mutuellement espacées et mutuellement séparées pour entraîner des produits d'hybridation de la molécule cible d'acide nucléique et de la sonde de RCA dans ladite solution vers lesdits dépôts de microgel ;
l'application d'un champ électrique auxdites régions de microgel immobilisées, mutuellement espacées et mutuellement séparées pour recapturer des amplicons de RCA qui s'éloignent desdits dépôts de microgel ;
l'application d'un champ électrique auxdites régions de microgel immobilisées, mutuellement espacées et mutuellement séparées pour entraîner des sondes de RCA dans lesdits dépôts de microgel pour une hybridation avec au moins l'une des sondes de capture et des amorces déjà liées auxdits dépôts de microgel ;
l'application d'un champ électrique auxdites régions de microgel immobilisées, mutuellement espacées et mutuellement séparées pour éliminer des molécules indésirables desdites régions de microgel ;
l'application d'un champ électrique auxdites régions de microgel immobilisées, mutuellement espacées et mutuellement séparées pour étirer des amplicons de RCA qui sont liés auxdits dépôts de microgel ;
l'application d'un champ électrique auxdites régions de microgel immobilisées, mutuellement espacées et mutuellement séparées pour comprimer des amplicons de RCA qui sont liés auxdits dépôts de microgel ;
l'application d'un champ électrique auxdites régions de microgel immobilisées, mutuellement espacées et mutuellement séparées pour agiter des réactifs de RCA à proximité d'amplicons de RCA qui sont liés auxdits dépôts de microgel ;
l'application d'un champ électrique auxdites régions de microgel immobilisées, mutuellement espacées et mutuellement séparées pour améliorer la vitesse de l'activité d'enzymes en RCA ; et
l'application d'un champ électrique de polarité inversée de manière séquentielle auxdites régions de microgel immobilisées, mutuellement espacées et mutuellement séparées pour améliorer une rigueur de liaison des amplicons de RCA auxdits dépôts de microgel.
